# EUROPEAN PATENT SPECIFICATION

(11) **EP 2 274 287 B1**
(45) Date of publication and mention of the grant of the patent: **09.03.2016**
(21) Application number: 09722738.3
(22) Date of filing: 18.03.2009
(51) Int. Cl.: C07D 233/32, C07D 263/22, C07D 263/24, C07D 401/04, C07D 403/06, C07D 409/10, C07D 413/04, C07D 413/06, C07D 413/10, C07D 417/10, A61K 31/445

(54) **INHIBITORS OF 11BETA-HYDROXYSTEROID DEHYDROGENASE TYPE 1**
INHIBITOREN DER 11BETA-HYDROXYSTEROIDDEHYDROGENASE VOM TYP 1
INHIBITEURS DE LA 11-BÊTA-HYDROXYSTÉROÏDE DÉSHYDROGÉNASE DE TYPE 1

(30) Priority: 18.03.2008 US 37646
(43) Date of publication of application: 19.01.2011
(73) Proprietor: Vitae Pharmaceuticals, Inc., Fort Washington, PA 19034 (US)
(72) Inventor: CLAREMON, David, A., Maple Glen PA 19002 (US); ZHUANG, Linghang, Chalfont PA 18914 (US); YE, Yuanjie, Ambler PA 19002 (US); SINGH, Suresh, B., Kendall Park NJ 08824 (US); TICE, Colin, M., Ambler PA 19002 (US); MCGEEHAN, Gerard, Garnet Valley PA 19061 (US)
(74) Representative: Potter Clarkson LLP
(86) International application number: PCT/US2009/001712
(87) International publication number: WO 2009/117109

(56) References cited:
- EP-A- 0 645 387
- EP-A- 1 156 049
- EP-A- 1 852 425
- WO-A-2006/031715
- JP-A- 2007 254 409
- GOUBET F ET AL: "Conversion of a Thiohydantoin to the Corresponding Hydantoin via a Ring-Opening/Ring Closure Mechanism" TETRAHEDRON LETTERS, ELSEVIER, AMSTERDAM, vol. 37, no. 43, 21 October 1996 (1996-10-21), pages 7727-7730, XP004030953 ISSN: 0040-4039
- SCHOELLKOPF U ET AL: "UMSETZUNGEN ALPHAMETALLIERTER ISOCYANIDE MIT EINIGEN 1,3-DIPOLEN//REACTIONS OF ALPHA-METALATED OSICYANIDES WITH SOME 1,3-DIPOLES" LIEBIGS ANNALEN DER CHEMIE, VERLAG CHEMIE GMBH. WEINHEIM, DE, vol. 4, 1 January 1980 (1980-01-01), pages 600-610, XP009064419 ISSN: 0170-2041
- YOSHINAO TAMARU: "Palladium(2+)-Catalyzed Intramolecular Aminocarbonylation of 3-Hydroxy-4-pentenylamines and 4-Hydroxy-5-hexenylamines" J. ORG. CHEM., 1988, pages 5731-5741, XP002534130

## Description

### RELATED APPLICATIONS

The application claims the benefit of U.S. Provisional Application No. 61/037,646, filed March 18, 2008, the entire teachings of which are incorporated herein by reference.

### FIELD OF THE INVENTION

The present invention relates to inhibitors of 11β-hydroxy steroid dehydrogenase type 1 (11β-HSD1), pharmaceutical compositions thereof and methods of using the same.

### BACKGROUND OF THE INVENTION

Glucocorticoids, such as cortisol (hydrocortisone), are steroid hormones that regulate fat metabolism, function and distribution, and play a role in carbohydrate, protein and fat metabolism. Glucocorticoids are also known to have physiological effects on development, neurobiology, inflammation, blood pressure, metabolism and programmed cell death. Cortisol and other corticosteroids bind both the glucocorticoid receptor (GR) and the mineralocorticoid receptor (MR), which are members of the nuclear hormone receptor superfamily and have been shown to mediate cortisol function in vivo. These receptors directly modulate transcription via DNA-binding zinc finger domains and transcriptional activation domains.

Until recently, the major determinants of glucocorticoid action were attributed to three primary factors: (1) circulating levels of glucocorticoid (driven primarily by the hypothalamic-pituitary-adrenal (HP A) axis); (2) protein binding of glucocorticoids in circulation; and (3) intracellular receptor density inside target tissues. Recently, a fourth determinant of glucocorticoid function has been identified: tissue-specific pre-receptor metabolism by glucocorticoid-activating and -inactivating enzymes. These 11β-hydroxysteroid dehydrogenase (11β-HSD) pre-receptor control enzymes modulate activation of GR and MR by regulation of glucocorticoid hormones. To date, two distinct isozymes of 11-beta-HSD have been cloned and characterized: 11β-HSD1 (also known as 11-beta-HSD type 1, 11betaHSD1, HSD11B1, and HSD11L) and 11β-HSD2. 11β-HSD1 is a bi-directional oxidoreductase that regenerates active cortisol from inactive 11-keto forms, whereas 11β-HSD2 is a unidirectional dehydrogenase that inactivates biologically active cortisol by converting it into cortisone.

The two isoforms are expressed in a distinct tissue-specific fashion, consistent with the differences in their physiological roles. 11β-HSD1 is widely distributed in rat and human tissues; expression of the enzyme and corresponding mRNA have been detected in human liver, adipose tissue, lung, testis, bone and ciliary epithelium. In adipose tissue, increased cortisol concentrations stimulate adipocyte differentiation and may play a role in promoting visceral obesity. In the eye, 11β-HSD1 may regulate intraocular pressure and may contribute to glaucoma; some data suggests that inhibition of 11β-HSD1 may cause a drop in intraocular pressure in patients with intraocular hypertension (Kotelevtsev, et al., (1997), Proc. Nat'l Acad. Sci. USA 94(26):14924-9). Although 11β-HSD1 catalyzes both 11-beta-dehydrogenation and the reverse 11-oxoreduction reaction, 11β-HSD1 acts predominantly as a NADPH-dependent oxoreductase in intact cells and tissues, catalyzing the formation of active cortisol from inert cortisone (Low, et al., (1994) J. Mol. Endocrin. 13: 167-174). In contrast, 11β-HSD2 expression is found mainly in mineralocorticoid target tissues such as kidney (cortex and medulla), placenta, sigmoid and rectal colon, salivary gland and colonic epithelial cell lines. 11β-HSD2 acts as an NAD-dependent dehydrogenase catalyzing the inactivation of cortisol to cortisone (Albiston, et al., (1994) Mol. Cell. Endocrin. 105: R11-R17), and has been shown to protect the MR from glucocorticoid excess (e.g., high levels of receptor-active cortisol) (Blum, et al., (2003) Prog. Nucl. Acid Res. Mol. Biol. 75:173-216).

Mutations in either the 11β-HSD1 or the 11β-HSD2 genes result in human pathology. For example, individuals with mutations in 11β-HSD2 are deficient in this cortisol-inactivation activity and, as a result, present with a syndrome of apparent mineralocorticoid excess (also referred to as "SAME") characterized by hypertension, hypokalemia, and sodium retention (Edwards, et al., (1988) Lancet 2: 986-989; Wilson, et al., (1998) Proc. Nat'l Acad. Sci. 95: 10200-10205). Similarly, mutations in 11β-HSD1 and in the gene encoding a co-localized NADPH-generating enzyme, hexose 6-phosphate dehydrogenase (H6PD), can result in cortisone reductase deficiency (CRD); these individuals present with ACTH-mediated androgen excess (hirsutism, menstrual irregularity, hyperandrogenism), a phenotype resembling polycystic ovary syndrome (PCOS) (Draper, et al., (2003) Nat. Genet. 34: 434-439).

Notably, disruption of homeostasis in the HPA axis by either deficient or excess secretion or action results in Cushing's syndrome or Addison's disease, respectively (Miller & Chrousos, Endocrinology and Metabolism (Felig & Frohman eds., McGraw-Hill: New York, 4th Ed. (2001)) 387-524). Patients with Cushing's syndrome or receiving glucocorticoid therapy develop reversible visceral fat obesity. The phenotype of Cushing's syndrome patients closely resembles that of Reaven's metabolic syndrome (also known as Syndrome X or insulin resistance syndrome), the symptoms of which include visceral obesity, glucose intolerance, insulin resistance, hypertension, type 2 diabetes and hyperlipidemia (Reaven, (1993) Ann. Rev. Med. 44, 121-131). Although the role of glucocorticoids in human obesity is not fully characterized, there is mounting evidence that 11β-HSD1 activity plays an important role in obesity and metabolic syndrome (Bujalska, et al., (1997) Lancet 349: 1210-1213); (Livingstone, et al., (2000) Endocrinology 131, 560-563; Rask, et al., (2001) J. Clin. Endocrinol. Metab. 86, 1418-1421; Lindsay, et al., (2003) J. Clin. Endocrinol. Metab. 88: 2738-2744; Wake, et al., (2003) J. Clin. Endocrinol. Metab. 88, 3983-3988).

Data from studies in mouse transgenic models supports the hypothesis that adipocyte 11β-HSD1 activity plays a central role in visceral obesity and metabolic syndrome (Alberts, et al., (2002) Diabetologia. 45(11), 1526-32). Over-expression in adipose tissue of 11 β-HSD1 under the control of the aP2 promoter in transgenic mice produced a phenotype remarkably similar to human metabolic syndrome (Masuzaki, et al., (2001) Science 294, 2166-2170; Masuzaki, et al., (2003) J. Clinical Invest. 112, 83-90). Moreover, the increased activity of 11β-HSD1 in these mice is very similar to that observed in human obesity (Rask, et al., (2001) J. Clin. Endocrinol. Metab. 86, 1418-1421). In addition, data from studies with 11βHSD1-deficient mice produced by homologous recombination demonstrate that the loss of 11β-HSD1 leads to an increase in insulin sensitivity and glucose tolerance due to a tissue-specific deficiency in active glucocorticoid levels (Kotelevstev, et al., (1997) Proc. Nat'l Acad. Sci. 94: 14924-14929; Morton, et al., (2001) J. Biol. Chem. 276, 41293-41300; Morton, et al., (2004) Diabetes 53,931-938).

The published data supports the hypothesis that increased expression of 11β-HSD1 contributes to increased local conversion of cortisone to cortisol in adipose tissue and hence that 11β-HSD1 plays a role in the pathogenesis of central obesity and the appearance of the metabolic syndrome in humans (Engeli, et al., (2004) Obes. Res. 12: 9-17). Therefore, 11β-HSD1 is a promising pharmaceutical target for the treatment of the metabolic syndrome (Masuzaki, et al., (2003) Curr. Drug Targets Immune Endocr. Metabol. Disord. 3: 255-62). Furthermore, inhibition of 11β-HSD1 activity may prove beneficial in treating numerous glucocorticoid-related disorders. For example, 11β-HSD1 inhibitors could be effective in combating obesity and/or other aspects of the metabolic syndrome cluster, including glucose intolerance, insulin resistance, hyperglycemia, hypertension, and/or hyperlipidemia (Kotelevstev, et al., (1997) Proc. Nat'l Acad. Sci. 94, 14924-14929; Morton, et al., (2001) J. Biol. Chem. 276, 41293-41300; Morton, et al., (2004) Diabetes 53, 931-938). In addition, inhibition of 11β-HSD1 activity may have beneficial effects on the pancreas, including the enhancement of glucose-stimulated insulin release (Billaudel & Sutter, (1979) Horm. Metab. Res. 11, 555-560; Ogawa, et al., (1992) J. Clin. Invest. 90, 497-504; Davani, et al., (2000) J. Biol. Chem. 275, 34841-34844). Inter-individual differences in general cognitive function has been linked to variability in the long-term exposure to glucocorticoids (Lupien, et al., (1998) Nat. Neurosci. 1: 69-73) and dysregulation of the HPA axis. Such chronic exposure to glucocorticoid excess in certain brain subregions has been theorized to contribute to the decline of cognitive function (McEwen & Sapolsky (1995) Curr. Opin. Neurobiol. 5, 205-216). Therefore, inhibition of 11β-HSD1 may reduce exposure to glucocorticoids in the brain and thereby protect against deleterious glucocorticoid effects on neuronal function, including cognitive impairment, dementia, and/or depression.

There is also evidence that glucocorticoids and 11β-HSD1 play a role in regulation of in intra-ocular pressure (IOP) (Stokes, et al., (2000) Invest. Ophthalmol. Vis. Sci. 41: 1629-1683; Rauz, et al., (2001) Invest. Ophthalmol. Vis. Sci. 42: 2037-2042). If left untreated, elevated IOP can lead to partial visual field loss and eventually blindness. Thus, inhibition of 11β-HSD1 in the eye could reduce local glucocorticoid concentrations and IOP, and hence could be used to treat or prevent glaucoma and other visual disorders.

Transgenic aP2-11β-HSD1 mice exhibit high arterial blood pressure and have increased sensitivity to dietary salt. Additionally, plasma angiotensinogen levels are elevated in the transgenic mice, as are angiotensin II and aldosterone. Treatment of the mice with an angiotensin II antagonist alleviates the hypertension (Masuzaki, et al., (2003) J. Clinical Invest. 112, 83-90). This suggests that hypertension may be caused or exacerbated by 11β-HSD1 activity. Thus, 11β-HSD1 inhibitors may be useful for treatment of hypertension and hypertension-related cardiovascular disorders.

Glucocorticoids can have adverse effects on skeletal tissues, and prolonged exposure to even moderate glucocorticoid doses can result in osteoporosis (Cannalis, (1996) J. Clin. Endocrinol. Metab. 81, 3441-3447). In addition, 11β-HSD1 has been shown to be present in cultures of human primary osteoblasts as well as cells from adult bone (Cooper, et al., (2000) Bone 27: 375-381), and the 11β-HSD1 inhibitor carbenoxolone has been shown to attenuate the negative effects of glucocorticoids on bone nodule formation (Bellows, et al., (1998) Bone 23: 119-125). Thus, inhibition of 11β-HSD1 is predicted to decrease the local glucocorticoid concentration within osteoblasts and osteoclasts, thereby producing beneficial effects in various forms of bone disease, including osteoporosis.

WO 2006/031715 describes specified imidazolidin-2-one derivatives as selective androgen receptor modulators.

EP-A-1156049 discloses compounds which function as antagonists of the receptors of the neurokinines, and are used to treat physiological disorders associated with an excess of tachykinines.

Goubet F et al (Tetrahedron Letters, vol. 37, no. 43, 21 October 1996) describes methodology for the conversion of N,N' disubstituted arylthiohydantoins to the corresponding hydantoins.

EP-A-0645387 discloses specified thiazolidine compounds for lowering blood sugar and lipid levels.

JP 2007 254409 describes specified imidazolidine derivatives having 11β-HSD1 inhibiting action.

As evidenced herein, there is a continuing need for new and improved drugs that inhibit 11β-HSD1. The novel compounds of the present invention are effective inhibitors of 11β-HSD1.

### SUMMARY OF THE INVENTION

The present invention provides compounds of Formula I: 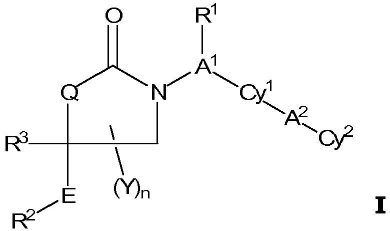 wherein:
R¹ is (a) absent or (b) selected from (C₁-C₆)alkyl, (C₂-C₆)alkenyl, (C₂-C₆)alkynyl and (C₁-C₃)alkoxy(C₁-C₃)alkyl, wherein each is optionally substituted with up to four groups independently selected from fluorine, cyano, oxo, R⁴, R⁴O-, (R⁴)₂N-, R⁴O₂C-, R⁴S, R⁴S(=O)-, R⁴S(=O)₂-, R⁴C(=O)NR⁴-, (R⁴)₂NC(=O)-, (R⁴)₂NC(=O)O-, (R⁴)₂NC(=O)NR⁴-, R⁴OC(=O)NR⁴-, (R⁴)₂NC(=NCN)NR⁴-, (R⁴O)₂P(=O)O-, (R⁴O)₂P(=O)NR⁴-, R⁴OS(=O)₂NR⁴-, (R⁴)₂NS(=O)₂O-, (R⁴)₂NS(=O)₂NR⁴-, R⁴S(=O)₂NR⁴-, R⁴S(=O)₂NHC(=O)-, R⁴S(=O)₂NHC(=O)O-, R⁴S(=O)₂NHC(=O)NR⁴-, R⁴OS(=O)₂NHC(=O)-, R⁴OS(=O)₂NHC(=O)O-, R⁴OS(=O)₂NHC(=O)NR⁴-, (R⁴)₂NS(=O)₂NHC(=O)-, (R⁴)₂NS(=O)₂NHC(=O)O-, (R⁴)₂NS(=O)₂NHC(=O)NR⁴-, R⁴C(=O)NHS(=O)₂-, R⁴C(=O)NHS(=O)₂O-, R⁴C(=O)NHS(=O)₂NR⁴-, R⁴OC(=O)NHS(=O)₂-, R⁴OC(=O)NHS(=O)₂O-, R⁴OC(=O)NHS(=O)₂NR⁴-, (R⁴)₂NC(=O)NHS(=O)₂-, (R⁴)₂NC(=O)NHS(=O)₂O-, (R⁴)₂NC(=O)NHS(=O)₂NR⁴-, aryl, cycloalkyl, heterocyclyl, heteroaryl, arylamino and heteroarylamino;
A¹ is (a) a bond, or (b) (C₁-C₃)alkylene, CH₂CH₂O, wherein the oxygen is attached to Cy¹, or CH₂C(=O), wherein the carbonyl carbon is attached to Cy¹;
Cy¹ is aryl, heteroaryl, monocyclic cycloalkyl or heterocyclyl, wherein each is optionally substituted with 1 to 4 groups independently selected from fluorine, chlorine, bromine, iodine, cyano, nitro, amino, hydroxy, carboxy, (C₁-C₆)alkyl, hydroxy(C₁-C₆)alkyl, (C₃-C₆)cycloalkyl, hydroxy(C₃-C₆)cycloalkyl, (C₄-C₇)cycloalkylalkyl, (C₂-C₆)alkenyl, halo(C₂-C₆)alkenyl, hydroxy(C₂-C₆)alkenyl, (C₂-C₆)alkynyl, (C₃-C₆)cycloalkyl(C₂-C₄)alkynyl, halo(C₁-C₆)alkyl, halo(C₃-C₆)cycloalkyl, halo(C₄-C₇)cycloalkylalkyl, (C₁-C₆)alkoxy, (C₃-C₆)cycloalkoxy, (C₄-C₇)cycloalkylalkoxy, halo(C₁-C₆)alkoxy, halo(C₃-C₆)cycloalkoxy, halo(C₄-C₇)cycloalkylalkoxy, (C₁-C₆)alkylthio, (C₃-C₆)cycloalkythio, (C₄-C₇)cycloalkylalkylthio, halo(C₁-C₆)alkylthio, halo(C₃-C₆)cycloalkythio, halo(C₄-C₇)cycloalkylalkylthio, (C₁-C₆)alkanesulfinyl, (C₃-C₆)cycloalkanesulfinyl, (C₄-C₇)cycloalkylalkanesulfinyl, halo(C₁-C₆)alkanesulfinyl, halo(C₃-C₆)cycloalkanesulfinyl, halo(C₄-C₇)cycloalkylalkanesulfinyl, (C₁-C₆)alkanesulfonyl, (C₃-C₆)cycloalkanesulfonyl, (C₄-C₇)cycloalkylalkanesulfonyl, halo(C₁-C₆)alkanesulfonyl, halo(C₃-C₆)cycloalkanesulfonyl, halo(C₄-C₇)cycloalkylalkanesulfonyl, (C₁-C₆)alkylamino, di(C₁-C₆)alkylamino, (C₁-C₆)alkoxy(C₁-C₆)alkoxy, halo(C₁-C₆)alkoxy(C₁-C₆)alkoxy, (C₁-C₆)alkoxycarbonyl, H₂NCO, H₂NSO₂, (C₁-C₆)alkylaminocarbonyl, di(C₁-C₆)alkylaminocarbonyl, (C₁-C₃)alkoxy(C₁-C₃)alkylaminocarbonyl, heterocyclylcarbonyl, (C₁-C₆)alkylaminosulfonyl, di(C₁-C₆)alkylaminosulfonyl, heterocyclosulfonyl, (C₁-C₆)alkylearbonylamino, (C₁-C₆)alkylcarbonylamino(C₁-C₆)alkyl, (C₁-C₆)alkylsulfonylamino, (C₁-C₆)alkylsulfonylamino(C₁-C₆)alkyl, (C₁-C₆)alkoxycarbonyl(C₁-C₆)alkoxy, (C₁-C₆)alkoxy(C₁-C₆)alkyl, halo(C₁-C₆)alkoxy(C₁-C₆)alkyl, hydroxy(C₁-C₆)alkoxy, heteroaryl oxo, amino(C₁-C₆)alkyl, (C₁-C₆)alkylamino(C₁-C₆)alkyl, di(C₁-C₆)alkylamino(C₁-C₆)alkyl amino(C₂-C₆)alkoxy, (C₁-C₆)alkylamino(C₂-C₆)alkoxy, di(C₁-C₆)alkylamino(C₂-C₆)alkoxy, (C₁-C₆)alkylcarbonyl, (C₃-C₆)cycloalkylcarbonyl, (C₃-C₆)cycloalkylaminocarbonyl, {(C₃-C₆)cycloalkyl} {(C₁-C₆)alkyl}aminocarbonyl, di(C₃-C₆)cycloalkylaminocarbonyl, (C₃-C₆)cycloalkylaminosulfonyl, {(C₃-C₆)cycloalkyl} {(C₁-C₆)alkyl}aminosulfonyl, di(C₃-C₆)cycloalkylaminosulfonyl, cyano(C₁-C₆)alkyl, aminocarbonyl(C₁-C₆)alkyl, (C₁-C₆)alkylaminocarbonyl(C₁-C₆)alkyl, di(C₁-C₆)alkylaminocarbonyl(C₁-C₆)alkyl, (C₃-C₆)cycloalkylaminocarbonyl(C₁-C₆)alkyl, {(C₃-C₆)cycloalkyl} {(C₁-C₆)alkyl}aminocarbonyl(C₁-C₆)alkyl and di(C₃-C₆)cycloalkylaminocarbonyl(C₁-C₆)alky;
A² is (a) a bond, O, S or NR⁴; or (b) (C₁-C₃)alkylene or (C₁-C₂)alkyleneoxy, each of which is optionally substituted with 1 to 4 groups independently selected from methyl, ethyl, trifluoromethyl or oxo;
Cy² is aryl, heteroaryl, cycloalkyl or heterocyclyl, wherein each is optionally substituted with 1 to 4 groups independently selected from fluorine, chlorine, bromine, iodine, cyano, nitro, amino, hydroxy, carboxy, (C₁-C₆)alkyl, hydroxy(C₁-C₆)alkyl, (C₃-C₆)cycloalkyl, hydroxy(C₃-C₆)cycloalkyl, (C₄-C₇)cycloalkylalkyl, (C₂-C₆)alkenyl, halo(C₂-C₆)alkenyl, hydroxy(C₂-C₆)alkenyl, (C₂-C₆)alkynyl, (C₃-C₆)cycloalkyl(C₂-C₄)alkynyl, halo(C₁-C₆)alkyl, halo(C₃-C₆)cycloalkyl, halo(C₄-C₇)cycloalkylalkyl, (C₁-C₆)alkoxy, (C₃-C₆)cycloalkoxy, (C₄-C₇)cycloalkylalkoxy, halo(C₁-C₆)alkoxy, halo(C₃-C₆)cycloalkoxy, halo(C₄-C₇)cycloalkylalkoxy, (C₁-C₆)alkylthio, (C₃-C₆)cycloalkythio, (C₄-C₇)cycloalkylalkylthio, halo(C₁-C₆)alkylthio, halo(C₃-C₆)cycloalkythio, halo(C₄-C₇)cycloalkylalkylthio, (C₁-C₆)alkanesulfinyl, (C₃-C₆)cycloalkanesulfinyl, (C₄-C₇)cycloalkylalkanesulfinyl, halo(C₁-C₆)alkanesulfinyl, halo(C₃-C₆)cycloalkanesulfinyl, halo(C₄-C₇)cycloalkylalkanesulfinyl, (C₁-C₆)alkanesulfonyl, (C₃-C₆)cycloalkanesulfbnyl, (C₄-C₇)cycloalkylalkanesulfbnyl, halo(C₁-C₆)alkanesulfonyl, halo(C₃-C₆)cycloalkanesulfonyl, halo(C₄-C₇)cycloalkylalkanesulfonyl, (C₁-C₆)alkylamino, di(C₁-C₆)alkylamino, (C₁-C₆)alkoxy(C₁-C₆)alkoxy, halo(C₁-C₆)alkoxy(C₁-C₆)alkoxy, (C₁-C₆)alkoxycarbonyl, H₂NCO, H₂NSO₂, (C₁-C₆)alkylaminocarbonyl, di(C₁-C₆)alkylaminocarbonyl, (C₁-C₃)alkoxy(C₁-C₃)alkylaminocarbonyl, heterocyclylcarbonyl, (C₁-C₆)alkylaminosulfonyl, di(C₁-C₆)alkylaminosulfonyl, heterocyclosulfonyl, (C₁-C₆)alkylcarbonylamino, (C₁-C₆)alkylcarbonylamino(C₁-C₆)alkyl, (C₁-C₆)alkylsulfonylamino, (C₁-C₆)alkylsulfonylamino(C₁-C₆)alkyl, (C₁-C₆)alkoxycarbonyl(C₁-C₆)alkoxy, (C₁-C₆)alkoxy(C₁-C₆)alkyl, halo(C₁-C₆)alkoxy(C₁-C₆)alkyl, hydroxy(C₁-C₆)alkoxy, heteroaryl, oxo, amino(C₁-C₆)alkyl, (C₁-C₆)alkylamino(C₁-C₆)alkyl, di(C₁-C₆)alkylamino(C₁-C₆)alkyl amino(C₂-C₆)alkoxy, (C₁-C₆)alkylamino(C₂-C₆)alkoxy, di(C₁-C₆)alkylamino(C₂-C₆)alkoxy, (C₁-C₆)alkylcarbonyl, (C₃-C₆)cycloalkylcarbonyl, (C₃-C₆)cycloalkylaminocarbonyl, {(C₃-C₆)cycloalkyl} {(C₁-C₆)alkyl}aminocarbonyl, di(C₃-C₆)cycloalkylaminocarbonyl, (C₃-C₆)cycloalkylaminosulfonyl, {(C₃-C₆)cycloalkyl} {(C₁-C₆)alkyl}aminosulfonyl, di(C₃-C₆)cycloalkylaminosulfonyl, cyano(C₁-C₆)alkyl, aminocarbonyl(C₁-C₆)alkyl, (C₁-C₆)alkylaminocarbonyl(C₁-C₆)alkyl, di(C₁-C₆)alkylaminocarbonyl(C₁-C₆)alkyl, (C₃-C₆)cycloalkylaminocarbonyl(C₁-C₆)alkyl, {(C₃-C₆)cycloalkyl} {(C₁-C₆)alkyl}aminocarbonyl(C₁-C₆)alkyl and di(C₃-C₆)cycloalkylaminocarbonyl(C₁-C₆)alkyl;
Y is (C₁-C₆)alkyl or halo(C₁-C₆)alkyl;
n is 0, 1 or 2;
E is (a) a bond or (b) (C₁-C₃)alkylene or (C₁-C₂)alkylenyloxy, wherein the O is attached to R², each of which is optionally substituted with 1 to 4 groups independently selected from methyl, ethyl, trifluoromethyl or oxo;
R² is aryl, heteroaryl, cycloalkyl or heterocyclyl, wherein each is optionally substituted with up to 4 groups independently selected from fluorine, chlorine, bromine, iodine, cyano, nitro, amino, hydroxy, carboxy, (C₁-C₆)alkyl, hydroxy(C₁-C₆)alkyl, (C₃-C₆)cycloalkyl, hydroxy(C₃-C₆)cycloalkyl, (C₄-C₇)cycloalkylalkyl, (C₂-C₆)alkenyl, halo(C₂-C₆)alkenyl, hydroxy(C₂-C₆)alkenyl, (C₂-C₆)alkynyl, (C₃-C₆)cycloalkyl(C₂-C₄)alkynyl, halo(C₁-C₆)alkyl, halo(C₃-C₆)cycloalkyl, halo(C₄-C₇)cycloalkylalkyl, (C₁-C₆)alkoxy, (C₃-C₆)cycloalkoxy, (C₄-C₇)cycloalkylalkoxy, halo(C₁-C₆)alkoxy, halo(C₃-C₆)cycloalkoxy, halo(C₄-C₇)cycloalkylalkoxy, (C₁-C₆)alkylthio, (C₃-C₆)cycloalkylthio, (C₄-C₇)cycloalkylalkylthio, halo(C₁-C₆)alkylthio, halo(C₃-C₆)cycloalkylthio, halo(C₄-C₇)cycloalkylalkylthio, (C₁-C₆)alkanesulfinyl, (C₃-C₆)cycloalkanesulfinyl, (C₄-C₇)cycloalkylalkanesulfinyl, halo(C₁-C₆)alkanesulfinyl, halo(C₃-C₆)cycloalkanesulfinyl, halo(C₄-C₇)cycloalkylalkanesulfinyl, (C₁-C₆)alkanesulfonyl, (C₃-C₆)cycloalkanesulfbnyl, (C₄-C₇)cycloalkylalkanesulfbnyl, halo(C₁-C₆)alkanesulfonyl, halo(C₃-C₆)cycloalkanesulfbnyl, halo(C₄-C₇)cycloalkylalkanesulfonyl, (C₁-C₆)alkylamino, di(C₁-C₆)alkylamino, (C₁-C₆)alkoxy(C₁-C₆)alkoxy, halo(C₁-C₆)alkoxy(C₁-C₆)alkoxy, (C₁-C₆)alkoxycarbonyl, H₂NCO, H₂NSO₂, (C₁-C₆)alkylaminocarbonyl, di(C₁-C₆)alkylaminocarbonyl, (C₁-C₃)alkoxy(C₁-C₃)alkylaminocarbonyl, heterocyclylcarbonyl, (C₁-C₆)alkylaminosulfonyl, di(C₁-C₆)alkylaminosulfonyl, heterocyclosulfonyl, (C₁-C₆)alkylcarbonylamino, (C₁-C₆)alkylcarbonylamino(C₁-C₆)alkyl, (C₁-C₆)alkylsulfonylamino, (C₁-C₆)alkylsulfonylamino(C₁-C₆)alkyl, (C₁-C₆)alkoxycarbonyl(C₁-C₆)alkoxy, (C₁-C₆)alkoxy(C₁-C₆)alkyl, halo(C₁-C₆)alkoxy(C₁-C₆)alkyl, hydroxy(C₁-C₆)alkoxy, heteroaryl, oxo, amino(C₁-C₆)alkyl, (C₁-C₆)alkylamino(C₁-C₆)alkyl, di(C₁-C₆)alkylamino(C₁-C₆)alkyl amino(C₂-C₆)alkoxy, (C₁-C₆)alkylamino(C₂-C₆)alkoxy, di(C₁-C₆)alkylamino(C₂-C₆)alkoxy, (C₁-C₆)alkylcarbonyl, (C₃-C₆)cycloalkylcarbonyl, (C₃-C₆)cycloalkylaminocarbonyl, {(C₃-C₆)cycloalkyl} {(C₁-C₆)alkyl}aminocarbonyl, di(C₃-C₆)cycloalkylaminocarbonyl, (C₃-C₆)cycloalkylaminosulfonyl, {(C₃-C₆)cycloalkyl} {(C₁-C₆)alkyl}aminosulfonyl, di(C₃-C₆)cycloalkylaminosulfonyl, cyano(C₁-C₆)alkyl, aminocarbonyl(C₁-C₆)alkyl, (C₁-C₆)alkylaminocarbonyl(C₁-C₆)alkyl, di(C₁-C₆)alkylaminocarbonyl(C₁-C₆)alkyl, (C₃-C₆)cycloalkylaminocarbonyl(C₁-C₆)alkyl, {(C₃-C₆)cycloalkyl} {(C₁-C₆)alkyl}aminocarbonyl(C₁-C₆)alkyl and di(C₃-C₆)cycloalkylaminocarbonyl(C₁-C₆)alkyl;
R³ is selected from (C₂-C₆)alkyl, (C₂-C₆)alkenyl, (C₂-C₆)alkynyl and (C₁-C₃)alkoxy(C₁-C₃)alkyl, wherein the (C₂-C₆)alkyl is substituted with, and each of the (C₂-C₆)alkenyl, (C₂-C₆)alkynyl and (C₁-C₃)alkoxy(C₁-C₃)alkyl is optionally substituted with, up to four groups independently selected from fluorine, cyano, oxo, halo(C₁-C₆)alkyl, amino(C₁-C₆)alkyl, (C₁-C₆)alkylamino(C-C₆)alkyl, di(C₁-C₆)alkylamino(C₁-C₆)alkyl, hydroxy(C₁-C₆)alkyl, (C₁-C₆)alkoxy(C₁-C₆)alkyl R⁴O-, (R⁴)₂N-, R⁴O₂C-, R⁴S, R⁴S(=O)-, R⁴S(=O)₂-, R⁴C(=O)NR⁴-, (R⁴)₂NC(=O)-, (R⁴)₂NC(=O)O-, (R⁴)₂NC(=O)NR⁴-, R⁴OC(=O)NR⁴-, (R⁴) ₂NC(=NCN)NR⁴-, (R⁴O)₂P(=O)O-, (R⁴O)₂P(=O)NR⁴-, R⁴OS(=O)₂NR⁴-, (R⁴)₂NS(=O)₂O-, (R⁴)₂NS(=O)₂NR⁴=, R⁴S(=O)₂NR⁴-, R⁴S(=O)₂NHC(=O)-, R⁴S(=O)₂NHC(=O)O-, R⁴S(=O)₂NHC(=O)NR⁴-, R⁴OS(=O)₂NHC(=O)-, R⁴OS(=O)₂NHC(=O)O-, R⁴OS(=O)₂NHC(=O)NR⁴-, (R⁴)₂NS(=O)₂NHC(=O)-, (R⁴)₂NS(=O)₂NHC(=O)O-, (R⁴)₂NS(=O)₂NHC(=O)NR⁴-, R⁴C(=O)NHS(=O)₂-, R⁴C(=O)NHS(=O)₂O-, R⁴C(=O)NHS(=O)₂NR⁴-, R⁴OC(=O)NHS(=O)₂-, R⁴OC(=O)NHS(=O)₂O-, R⁴OC(=O)NHS(=O)₂NR⁴-, (R⁴)₂NC(=O)NHS(=O)₂-, (R⁴)₂NC(=O)NHS(=O)₂O-, (R⁴)₂NC(=O)NHS(=O)₂NR⁴-, heterocyclyl (which in turn may be optionally substituted with alkyl, haloalkyl or oxo), heteroaryl (which in turn may be optionally substituted with alkyl, haloalkyl, alkoxy, alkylthio, alkylsulfonyl, halogen, trifluoromethyl, dialkylamino, nitro, cyano, CO₂H, CONH₂, N-monoalkyl-substituted amido, N,N-dialkyl-substituted amido, or oxo), arylamino (which in turn may be optionally substituted with alkyl, alkoxy, alkylthio, alkylsulfonyl, halogen, trifluoromethyl, dialkylamino, nitro, cyano, CO₂H, CONH₂, N-monoalkyl-substituted amido and N,N-dialkyl-substituted amido) and heteroarylamino (which in turn may be optionally substituted with alkyl, haloalkyl, alkoxy, alkylthio, alkylsulfonyl, halogen, trifluoromethyl, dialkylamino, nitro, cyano, CO₂H, CONH₂, N-monoalkyl-substituted amido, N,N-dialkyl-substituted amido, or oxo);
R⁴ is independently selected from H, (C₁-C₆)alkyl, halo(C₁-C₆)alkyl, amino(C₁-C₆)alkyl, (C₁-C₆)alkylamino(C₁-C₆)alkyl, di(C₁-C₆)alkylamino(C₁-C₆)alkyl, hydroxy(C₁-C₆)alkyl and (C₁-C₆)alkoxy(C₁-C₆)alkyl;
Q = O, NR⁵; and
R⁵ is H, (C₁-C₆)alkyl, halo(C₁-C₆)alkyl or hydroxy(C₁-C₆)alkyl;
or a pharmaceutically acceptable salt, enantiomer or diastereomer thereof.

Another embodiment of the invention is a compound of Formula I wherein Cy¹ is aryl, heteroaryl, monocyclic cycloalkyl or heterocyclyl, wherein each is optionally substituted with 1 to 4 groups independently selected from fluorine, chlorine, bromine, iodine, cyano, nitro, amino, hydroxy, carboxy, (C₁-C₆)alkyl, hydroxy(C₁-C₆)alkyl, (C₃-C₆)cycloalkyl, hydroxy(C₃-C₆)cycloalkyl, (C₄-C₇)cycloalkylalkyl, (C₂-C₆)alkenyl, halo(C₂-C₆)alkenyl, hydroxy(C₂-C₆)alkenyl, (C₂-C₆)alkynyl, (C₃-C₆)cycloalkyl(C₂-C₄)alkynyl, halo(C₁-C₆)alkyl, halo(C₃-C₆)cycloalkyl, halo(C₄-C₇)cycloalkylalkyl, (C₁-C₆)alkoxy, (C₃-C₆)cycloalkoxy, (C₄-C₇)cycloalkylalkoxy, halo(C₁-C₆)alkoxy, halo(C₃-C₆)cycloalkoxy, halo(C₄-C₇)cycloalkylalkoxy, (C₁-C₆)alkylthio, (C₃-C₆)cycloalkythio, (C₄-C₇)cycloalkylalkylthio, halo(C₁-C₆)alkylthio, halo(C₃-C₆)cycloalkythio, halo(C₄-C₇)cycloalkylalkylthio, (C₁-C₆)alkanesulfinyl, (C₃-C₆)cycloalkanesulfinyl, (C₄-C₇)cycloalkylalkanesulfinyl, halo(C₁-C₆)alkanesulfinyl, halo(C₃-C₆)cycloalkanesulfinyl, halo(C₄-C₇)cycloalkylalkanesulfinyl, (C₁-C₆)alkanesulfonyl, (C₃-C₆)cycloalkanesulfbnyl, (C₄-C₇)cycloalkylalkanesulfbnyl, halo(C₁-C₆)alkanesulfonyl, halo(C₃-C₆)cycloalkanesulfbnyl, halo(C₄-C₇)cyclo-alkylalkanesulfonyl, (C₁-C₆)alkylamino, di(C₁-C₆)alkylamino, (C₁-C₆)alkoxy(C₁-C₆)alkoxy, halo(C₁-C₆)alkoxy(C₁-C₆)alkoxy, (C₁-C₆)alkoxycarbonyl, H₂NCO, H₂NSO₂, (C₁-C₆)alkylaminocarbonyl, di(C₁-C₆)alkylaminocarbonyl, (C₁-C₃)alkoxy(C₁-C₃)alkylaminocarbonyl, heterocyclylcarbonyl, (C₁-C₆)alkylaminosulfonyl, di(C₁-C₆)alkylaminosulfonyl, heterocyclosulfonyl, (C₁-C₆)alkylcarbonylamino, (C₁-C₆)alkylcarbonylamino(C₁-C₆)alkyl, (C₁-C₆)alkylsulfonylamino, (C₁-C₆)alkylsulfonylamino(C₁-C₆)alkyl, (C₁-C₆)alkoxycarbonyl(C₁-C₆)alkoxy, (C₁-C₆)alkoxy(C₁-C₆)alkyl, halo(C₁-C₆)alkoxy(C₁-C₆)alkyl, hydroxy(C₁-C₆)alkoxy,heteroaryl oxo, amino(C₁-C₆)alkyl, (C₁-C₆)alkylamino(C₁-C₆)alkyl, di(C₁-C₆)alkylamino(C₁-C₆)alkyl amino(C₂-C₆)alkoxy, (C₁-C₆)alkylamino(C₂-C₆)alkoxy, di(C₁-C₆)alkylamino(C₂-C₆)alkoxy and (C₁-C₆)alkylcarbonyl;
Cy² is aryl, heteroaryl, cycloalkyl or heterocyclyl, wherein each is optionally substituted with 1 to 4 groups independently selected from fluorine, chlorine, bromine, iodine, cyano, nitro, amino, hydroxy, carboxy, (C₁-C₆)alkyl, hydroxy(C₁-C₆)alkyl, (C₃-C₆)cycloalkyl, hydroxy(C₃-C₆)cycloalkyl, (C₄-C₇)cycloalkylalkyl, (C₂-C₆)alkenyl, halo(C₂-C₆)alkenyl, hydroxy(C₂-C₆)alkenyl, (C₂-C₆)alkynyl, (C₃-C₆)cycloalkyl(C₂-C₄)alkynyl, halo(C₁-C₆)alkyl, halo(C₃-C₆)cycloalkyl, halo(C₄-C₇)cycloalkylalkyl, (C₁-C₆)alkoxy, (C₃-C₆)cycloalkoxy, (C₄-C₇)cycloalkylalkoxy, halo(C₁-C₆)alkoxy, halo(C₃-C₆)cycloalkoxy, halo(C₄-C₇)cycloalkylalkoxy, (C₁-C₆)alkylthio, (C₃-C₆)cycloalkythio, (C₄-C₇)cycloalkylalkylthio, halo(C₁-C₆)alkylthio, halo(C₃-C₆)cycloalkythio, halo(C₄-C₇)cycloalkylalkylthio, (C₁-C₆)alkanesulfinyl, (C₃-C₆)cycloalkanesulfinyl, (C₄-C₇)cycloalkylalkanesulfinyl, halo(C₁-C₆)alkanesulfinyl, halo(C₃-C₆)cycloalkanesulfinyl, halo(C₄-C₇)cycloalkylalkanesulfinyl, (C₁-C₆)alkanesulfonyl, (C₃-C₆)cycloalkanesulfbnyl, (C₄-C₇)cycloalkylalkanesulfbnyl, halo(C₁-C₆)alkanesulfonyl, halo(C₃-C₆)cycloalkanesulfbnyl, halo(C₄-C₇)cycloalkylalkanesulfonyl, (C₁-C₆)alkylamino, di(C₁-C₆)alkylamino, (C₁-C₆)alkoxy(C₁-C₆)alkoxy, halo(C₁-C₆)alkoxy(C₁-C₆)alkoxy, (C₁-C₆)alkoxycarbonyl, H₂NCO, H₂NSO₂, (C₁-C₆)alkylaminocarbonyl, di(C₁-C₆)alkylaminocarbonyl, (C₁-C₃)alkoxy(C₁-C₃)alkylaminocarbonyl, heterocyclylcarbonyl, (C₁-C₆)alkylaminosulfonyl, di(C₁-C₆)alkylaminosulfonyl, heterocyclosulfonyl, (C₁-C₆)alkylcarbonylamino, (C₁-C₆)alkylcarbonylamino(C₁-C₆)alkyl, (C₁-C₆)alkylsulfonylamino, (C₁-C₆)alkylsulfonylamino(C₁-C₆)alkyl, (C₁-C₆)alkoxycarbonyl(C₁-C₆)alkoxy, (C₁-C₆)alkoxy(C₁-C₆)alkyl, halo(C₁-C₆)alkoxy(C₁-C₆)alkyl, hydroxy(C₁-C₆)alkoxy, heteroaryl, oxo, amino(C₁-C₆)alkyl, (C₁-C₆)alkylamino(C₁-C₆)alkyl, di(C₁-C₆)alkylamino(C₁-C₆)alkyl amino(C₂-C₆)alkoxy, (C₁-C₆)alkylamino(C₂-C₆)alkoxy, di(C₁-C₆)alkylamino(C₂-C₆)alkoxy and (C₁-C₆)alkylcarbonyl;
R² is aryl, heteroaryl, cycloalkyl or heterocyclyl, wherein each is optionally substituted with up to 4 groups independently selected from fluorine, chlorine, bromine, iodine, cyano, nitro, amino, hydroxy, carboxy, (C₁-C₆)alkyl, hydroxy(C₁-C₆)alkyl, (C₃-C₆)cycloalkyl, hydroxy(C₃-C₆)cycloalkyl, (C₄-C₇)cycloalkylalkyl, (C₂-C₆)alkenyl, halo(C₂-C₆)alkenyl, hydroxy(C₂-C₆)alkenyl, (C₂-C₆)alkynyl, (C₃-C₆)cycloalkyl(C₂-C₄)alkynyl, halo(C₁-C₆)alkyl, halo(C₃-C₆)cycloalkyl, halo(C₄-C₇)cycloalkylalkyl, (C₁-C₆)alkoxy, (C₃-C₆)cycloalkoxy, (C₄-C₇)cycloalkylalkoxy, halo(C₁-C₆)alkoxy, halo(C₃-C₆)cycloalkoxy, halo(C₄-C₇)cycloalkylalkoxy, (C₁-C₆)alkylthio, (C₃-C₆)cycloalkylthio, (C₄-C₇)cycloalkylalkylthio, halo(C₁-C₆)alkylthio, halo(C₃-C₆)cycloalkylthio, halo(C₄-C₇)cycloalkylalkylthio, (C₁-C₆)alkanesulfinyl, (C₃-C₆)cycloalkanesulfinyl, (C₄-C₇)cycloalkylalkanesulfinyl, halo(C₁-C₆)alkanesulfinyl, halo(C₃-C₆)cycloalkanesulfinyl, halo(C₄-C₇)cycloalkylalkanesulfinyl, (C₁-C₆)alkanesulfonyl, (C₃-C₆)cycloalkanesulfbnyl, (C₄-C₇)cycloalkylalkanesulfbnyl, halo(C₁-C₆)alkanesulfonyl, halo(C₃-C₆)cycloalkanesulfbnyl, halo(C₄-C₇)cyclo-alkylalkanesulfonyl, (C₁-C₆)alkylamino, di(C₁-C₆)alkylamino, (C₁-C₆)alkoxy(C₁-C₆)alkoxy, halo(C₁-C₆)alkoxy(C₁-C₆)alkoxy, (C₁-C₆)alkoxycarbonyl, H₂NCO, H₂NSO₂, (C₁-C₆)alkylaminocarbonyl, di(C₁-C₆)alkylaminocarbonyl, (C₁-C₃)alkoxy(C₁-C₃)alkylaminocarbonyl, heterocyclylcarbonyl, (C₁-C₆)alkylaminosulfonyl, di(C₁-C₆)alkylaminosulfonyl, heterocyclosulfonyl, (C₁-C₆)alkylcarbonylamino, (C₁-C₆)alkylcarbonylamino(C₁-C₆)alkyl, (C₁-C₆)alkylsulfonylamino, (C₁-C₆)alkylsulfonylamino(C₁-C₆)alkyl, (C₁-C₆)alkoxycarbonyl(C₁-C₆)alkoxy, (C₁-C₆)alkoxy(C₁-C₆)alkyl, halo(C₁-C₆)alkoxy(C₁-C₆)alkyl, hydroxy(C₁-C₆)alkoxy, heteroaryl, oxo, amino(C₁-C₆)alkyl, (C₁-C₆)alkylamino(C₁-C₆)alkyl, di(C₁-C₆)alkylamino(C₁-C₆)alkyl amino(C₂-C₆)alkoxy, (C₁-C₆)alkylamino(C₂-C₆)alkoxy, di(C₁-C₆)alkylamino(C₂-C₆)alkoxy and (C₁-C₆)alkylcarbonyl and the reminder of the variables are as defined above.

The present invention also provides a pharmaceutical composition comprising a disclosed 11β-HSD1 inhibitor, including a compound of Formula I, or a pharmaceutically acceptable salt, enantiomer or diastereomer thereof, and a pharmaceutically acceptable carrier or diluent, wherein the values for the variables are as described above for the compounds of Formula I.

Also included in the present invention is the use of a disclosed 11β- HSD1 inhibitor, including a compound of Formula I, or a pharmaceutically acceptable salt thereof, for the manufacture of a medicament for inhibiting 11β-HSD1 activity in a mammal in need of such treatment.

Also included in the present invention is the use of a disclosed 11β-HSD1 inhibitor, including a compound of Formula I, or a pharmaceutically acceptable salt thereof, for the manufacture of a medicament for treating a disease or disorder related to the activity or expression of 11β-HSD1, inhibiting the conversion of cortisone to cortisol in a cell, inhibiting production of cortisol in a cell, increasing insulin sensitivity in a mammal in need thereof, modulating 11β-HSD1 activity in a mammal in need thereof, and/or inhibiting 11β-HSD1 in a mammal in need thereof.

Also included in the present invention is a disclosed 11β-HSD1 inhibitor, including a compound of Formula I, or a pharmaceutically acceptable salt thereof, for use in inhibiting 11β-HSD1 1 activity in a mammal in need of such treatment.

Also included in the present invention is a disclosed 11β-HSD1 inhibitor, including a compound of Formula I, or a pharmaceutically acceptable salt thereof, for use in therapy, e.g., treating a disease or disorder associated with activity or expression of 11β-HSD1 in a subject.

### DETAILED DESCRIPTION OF THE INVENTION

In one embodiment, the 11β-HSD1 inhibitors of the invention are represented by Structural Formula I. Pharmaceutically acceptable salts of the 11β-HSD1 inhibitors disclosed herein (including those represented by Structural Formula I) are also included in the invention. Values and alternative values for the variables in Structural Formula I are provided in the following paragraphs:
R¹ is (a) absent or (b) selected from (C₁-C₆)alkyl, (C₂-C₆)alkenyl, (C₂-C₆)alkynyl and (C₁-C₃)alkoxy(C₁-C₃)alkyl, wherein each is optionally substituted with up to four groups independently selected from fluorine, cyano, oxo, R⁴, R⁴O-, (R⁴)₂N-, R⁴O₂C-, R⁴S, R⁴S(=O)-, R⁴S(=O)₂-, R⁴C(=O)NR⁴-, (R⁴)₂NC(=O)-, (R⁴)₂NC(=O)O-, (R⁴)₂NC(=O)NR⁴-, R⁴OC(=O)NR⁴-, (R⁴)₂NC(=NCN)NR⁴-, (R⁴O)₂P(=O)O-, (R⁴O)₂P(=O)NR⁴-, R⁴OS(=O)₂NR⁴-, (R⁴)₂NS(=O)₂O-, (R⁴)₂NS(=O)₂NR⁴-, R⁴S(=O)₂NR⁴-, R⁴S(=O)₂NHC(=O)-, R⁴S(=O)₂NHC(=O)O-, R⁴S(=O)₂NHC(=O)NR⁴-, R⁴OS(=O)₂NHC(=O)-, R⁴OS(=O)₂NHC(=O)O-, R⁴OS(=O)₂NHC(=O)NR⁴-, (R⁴)₂NS(=O)₂NHC(=O)-, (R⁴)₂NS(=O)₂NHC(=O)O-, (R⁴)₂NS(=O)₂NHC(=O)NR⁴-, R⁴C(=O)NHS(=O)₂-, R⁴C(=O)NHS(=O)₂O-, R⁴C(=O)NHS(=O)₂NR⁴-, R⁴OC(=O)NHS(=O)₂-, R⁴OC(=O)NHS(=O)₂O-, R⁴OC(=O)NHS(=O)₂NR⁴-, (R⁴)₂NC(=O)NHS(=O)₂-, (R⁴)₂NC(=O)NHS(=O)₂O-, (R⁴)₂NC(=O)NHS(=O)₂NR⁴-, aryl, cycloalkyl, heterocyclyl, heteroaryl, arylamino and heteroarylamino. Alternatively, R¹ is absent. Alternatively, R¹ is unsubstitued or substituted (C₁-C₆)alkyl, wherein the substituents are as described above. Alternatively, R¹ is unsubstituted or substitued methyl or ethyl, wherein the substituents are as described above. Alternatively, R¹ is unsubstituted methyl or ethyl.
A¹ is (a) a bond, or (b) (C₁-C₃)alkylene, CH₂CH₂O, wherein the oxygen is attached to Cy¹, or CH₂C(=O), wherein the carbonyl carbon is attached to Cy¹. Alternatively, A¹ is a bond. Alternatively, A¹ is (C₁-C₃)alkylene. Alternatively, A¹ is methylene.
Cy¹ is aryl, heteroaryl, monocyclic cycloalkyl or heterocyclyl, wherein each is optionally substituted with 1 to 4 groups independently selected from fluorine, chlorine, bromine, iodine, cyano, nitro, amino, hydroxy, carboxy, (C₁-C₆)alkyl, hydroxy(C₁-C₆)alkyl, (C₃-C₆)cycloalkyl, hydroxy(C₃-C₆)cycloalkyl, (C₄-C₇)cycloalkylalkyl, (C₂-C₆)alkenyl, halo(C₂-C₆)alkenyl, hydroxy(C₂-C₆)alkenyl, (C₂-C₆)alkynyl, (C₃-C₆)cycloalkyl(C₂-C₄)alkynyl, halo(C₁-C₆)alkyl, halo(C₃-C₆)cycloalkyl, halo(C₄-C₇)cycloalkylalkyl, (C₁-C₆)alkoxy, (C₃-C₆)cycloalkoxy, (C₄-C₇)cycloalkylalkoxy, halo(C₁-C₆)alkoxy, halo(C₃-C₆)cycloalkoxy, halo(C₄-C₇)cycloalkylalkoxy, (C₁-C₆)alkylthio, (C₃-C₆)cycloalkythio, (C₄-C₇)cycloalkylalkylthio, halo(C₁-C₆)alkylthio, halo(C₃-C₆)cycloalkythio, halo(C₄-C₇)cycloalkylalkylthio, (C₁-C₆)alkanesulfinyl, (C₃-C₆)cycloalkanesulfinyl, (C₄-C₇)cycloalkylalkanesulfinyl, halo(C₁-C₆)alkanesulfinyl, halo(C₃-C₆)cycloalkanesulfinyl, halo(C₄-C₇)cycloalkylalkanesulfinyl, (C₁-C₆)alkanesulfonyl, (C₃-C₆)cycloalkanesulfonyl, (C₄-C₇)cycloalkylalkanesulfonyl, halo(C₁-C₆)alkanesulfonyl, halo(C₃-C₆)cycloalkanesulfonyl, halo(C₄-C₇)cyclo-alkylalkanesulfonyl, (C₁-C₆)alkylamino, di(C₁-C₆)alkylamino, (C₁-C₆)alkoxy(C₁-C₆)alkoxy, halo(C₁-C₆)alkoxy(C₁-C₆)alkoxy, (C₁-C₆)alkoxycarbonyl, H₂NCO, H₂NSO₂, (C₁-C₆)alkylaminocarbonyl, di(C₁-C₆)alkylaminocarbonyl; (C₁-C₃)alkoxy(C₁-C₃)alkylaminocarbonyl, heterocyclylcarbonyl, (C₁-C₆)alkylaminosulfonyl, di(C₁-C₆)alkylaminosulfonyl, heterocyclosulfonyl, (C₁-C₆)alkylcarbonylamino, (C₁-C₆)alkylcarbonylamino(C₁-C₆)alkyl, (C₁-C₆)alkylsulfonylamino, (C₁-C₆)alkylsulfonylamino(C₁-C₆)alkyl, (C₁-C₆)alkoxycarbonyl(C₁-C₆)alkoxy, (C₁-C₆)alkoxy(C₁-C₆)alkyl, halo(C₁-C₆)alkoxy(C₁-C₆)alkyl, hydroxy(C₁-C₆)alkoxy, heteroaryl oxo, amino(C₁-C₆)alkyl, (C₁-C₆)alkylamino(C₁-C₆)alkyl, di(C₁-C₆)alkylamino(C₁-C₆)alkyl amino(C₂-C₆)alkoxy, (C₁-C₆)alkylamino(C₂-C₆)alkoxy, di(C₁-C₆)alkylamino(C₂-C₆)alkoxy and (C₁-C₆)alkylcarbonyl.

Alternatively, Cy¹ is optionally substituted aryl or optionally substituted heteroaryl, wherein the substituents are as described above. Alternatively, Cy¹ is optionally substituted phenyl or optionally substituted pyridyl, wherein the substituents are as described above. Alternatively, Cy¹ is optionally substituted phenyl, wherein the substituents are as described above. Alternatively, Cy¹ is optionally substituted monocyclic cycloalkyl, wherein the substituents are as described above. Alternatively, Cy¹ is optionally substituted cyclohexyl, wherein the substituents are as described above. Alternatively, Cy¹ is substituted with fluorine, chlorine, bromine, methoxy, difluoromethoxy, methoxycarbonyl, carboxy, methyl, or trifluoromethyl.

A² is (a) a bond, O, S or NR⁴; or (b) (C₁-C₃)alkylene or (C₁-C₂)alkyleneoxy, each of which is optionally substituted with 1 to 4 groups independently selected from methyl, ethyl, trifluoromethyl or oxo. Alternatively, A² is a bond.

Cy² is aryl, heteroaryl, cycloalkyl or heterocyclyl, wherein each is optionally substituted with 1 to 4 groups independently selected from fluorine, chlorine, bromine, iodine, cyano, nitro, amino, hydroxy, carboxy, (C₁-C₆)alkyl, hydroxy(C₁-C₆)alkyl, (C₃-C₆)cycloalkyl, hydroxy(C₃-C₆)cycloalkyl, (C₄-C₇)cycloalkylalkyl, (C₂-C₆)alkenyl, halo(C₂-C₆)alkenyl, hydroxy(C₂-C₆)alkenyl, (C₂-C₆)alkynyl, (C₃-C₆)cycloalkyl(C₂-C₄)alkynyl, halo(C₁-C₆)alkyl, halo(C₃-C₆)cycloalkyl, halo(C₄-C₇)cycloalkylalkyl, (C₁-C₆)alkoxy, (C₃-C₆)cycloalkoxy, (C₄-C₇)cycloalkylalkoxy, halo(C₁-C₆)alkoxy, halo(C₃-C₆)cycloalkoxy, halo(C₄-C₇)cycloalkylalkoxy, (C₁-C₆)alkylthio, (C₃-C₆)cycloalkythio, (C₄-C₇)cycloalkylalkylthio, halo(C₁-C₆)alkylthio, halo(C₃-C₆)cycloalkythio, halo(C₄-C₇)cycloalkylalkylthio, (C₁-C₆)alkanesulfinyl, (C₃-C₆)cycloalkanesulfinyl, (C₄-C₇)cycloalkylalkanesulfinyl, halo(C₁-C₆)alkanesulfinyl, halo(C₃-C₆)cycloalkanesulfinyl, halo(C₄-C₇)cycloalkylalkanesulfinyl, (C₁-C₆)alkanesulfonyl, (C₃-C₆)cycloalkanesulfonyl, (C₄-C₇)cycloalkylalkanesulfonyl, halo(C₁-C₆)alkanesulfonyl, halo(C₃-C₆)cycloalkanesulfonyl, halo(C₄-C₇)cyclo-alkylalkanesulfonyl, (C₁-C₆)alkylamino, di(C₁-C₆)alkylamino, (C₁-C₆)alkoxy(C₁-C₆)alkoxy, halo(C₁-C₆)alkoxy(C₁-C₆)alkoxy, (C₁-C₆)alkoxycarbonyl, H₂NCO, H₂NSO₂, (C₁-C₆)alkylaminocarbonyl, di(C₁-C₆)alkylaminocarbonyl, (C₁-C₃)alkoxy(C₁-C₃)alkylaminocarbonyl, heterocyclylcarbonyl, (C₁-C₆)alkylaminosulfonyl, di(Ci-C₆)alkylaminosulfonyl, heterocyclosulfonyl, (C₁-C₆)alkylcarbonylamino, (C₁-C₆)alkylcarbonylamino(C₁-C₆)alkyl, (C₁-C₆)alkylsulfonylamino, (C₁-C₆)alkylsulfonylamino(C₁-C₆)alkyl, (C₁-C₆)alkoxycarbonyl(C₁-C₆)alkoxy, (C₁-C₆)alkoxy(C₁-C₆)alkyl, halo(C₁-C₆)alkoxy(C₁-C₆)alkyl, hydroxy(C₁-C₆)alkoxy, heteroaryl, oxo, amino(C₁-C₆)alkyl, (C₁-C₆)alkylamino(C₁-C₆)alkyl, di(C₁-C₆)alkylamino(C₁-C₆)alkyl amino(C₂-C₆)alkoxy, (C₁-C₆)alkylamino(C₂-C₆)alkoxy, di(C₁-C₆)alkylamino(C₂-C₆)alkoxy and (C₁-C₆)alkylcarbonyl.

Alternatively, Cy² is optionally substituted aryl or heteroaryl, wherein the substituents are as described above. Alternatively, Cy² is optionally substituted cycloalkyl or heterocyclyl, wherein the substituents are as described above. Alternatively, Cy² is optionally substituted phenyl or pyridyl, wherein the substituents are as described above. Alternatively, Cy² is substituted with 1 to 4 groups independently selected from chlorine or fluorine. Alternatively, Cy² is difluorophenyl or monofluorophenyl. Alternatively, Cy² is 1,2-dihydro-2-oxopyridyl or 1,2-dihydro-1-methyl-2-oxopyridyl. Alternatively, Cy² is cyclopropyl.

Y is (C₁-C₆)alkyl or halo(C₁-C₆)alkyl.

n is 0, 1 or 2.

E is (a) a bond or (b) (C₁-C₃)alkylene or (C₁-C₂)alkylenyloxy, wherein the O is attached to R², each of which is optionally substituted with 1 to 4 groups independently selected from methyl, ethyl, trifluoromethyl or oxo. Alternatively, E is a bond or alkylene. Alternatively, E is a bond.

R² is aryl, heteroaryl, cycloalkyl or heterocyclyl, wherein each is optionally substituted with up to 4 groups independently selected from fluorine, chlorine, bromine, iodine, cyano, nitro, amino, hydroxy, carboxy, (C₁-C₆)alkyl, hydroxy(C₁-C₆)alkyl, (C₃-C₆)cycloalkyl, hydroxy(C₃-C₆)cycloalkyl, (C₄-C₇)cycloalkylalkyl, (C₂-C₆)alkenyl, halo(C₂-C₆)alkenyl, hydroxy(C₂-C₆)alkenyl, (C₂-C₆)alkynyl, (C₃-C₆)cycloalkyl(C₂-C₄)alkynyl, halo(C₁-C₆)alkyl, halo(C₃-C₆)cycloalkyl, halo(C₄-C₇)cycloalkylalkyl, (C₁-C₆)alkoxy, (C₃-C₆)cycloalkoxy, (C₄-C₇)cycloalkylalkoxy, halo(C₁-C₆)alkoxy, halo(C₃-C₆)cycloalkoxy, halo(C₄-C₇)cycloalkylalkoxy, (C₁-C₆)alkylthio, (C₃-C₆)cycloalkylthio, (C₄-C₇)cycloalkylalkylthio, halo(C₁-C₆)alkylthio, halo(C₃-C₆)cycloalkylthio, halo(C₄-C₇)cycloalkylalkylthio, (C₁-C₆)alkanesulfinyl, (C₃-C₆)cycloalkanesulfinyl, (C₄-C₇)cycloalkylalkanesulfinyl, halo(C₁-C₆)alkanesulfinyl, halo(C₃-C₆)cycloalkanesulfinyl, halo(C₄-C₇)cycloalkylalkanesulfinyl, (C₁-C₆)alkanesulfonyl, (C₃-C₆)cycloalkanesulfonyl, (C₄-C₇)cycloalkylalkanesulfonyl, halo(C₁-C₆)alkanesulfonyl, halo(C₃-C₆)cycloalkanesulfonyl, halo(C₄-C₇)cyclo-alkylalkanesulfonyl, (C₁-C₆)alkylamino, di(C₁-C₆)alkylamino, (C₁-C₆)alkoxy(C₁-C₆)alkoxy, halo(C₁-C₆)alkoxy(C₁-C₆)alkoxy, (C₁-C₆)alkoxycarbonyl, H₂NCO, H₂NSO₂, (C₁-C₆)alkylaminocarbonyl, di(C₁-C₆)alkylaminocarbonyl, (C₁-C₃)alkoxy(C₁-C₃)alkylaminocarbonyl, heterocyclylcarbonyl, (C₁-C₆)alkylaminosulfonyl, di(C₁-C₆)alkylaminosulfonyl, heterocyclosulfonyl, (C₁-C₆)alkylcarbonylamino, (C₁-C₆)alkylcarbonylamino(C₁-C₆)alkyl, (C₁-C₆)alkylsulfonylamino, (C₁-C₆)alkylsulfonylamino(C₁-C₆)alkyl, (C₁-C₆)alkoxycarbonyl(C₁-C₆)alkoxy, (C₁-C₆)alkoxy(C₁-C₆)alkyl, halo(C₁-C₆)alkoxy(C₁-C₆)alkyl, hydroxy(C₁-C₆)alkoxy, heteroaryl, oxo, amino(C₁-C₆)alkyl, (C₁-C₆)alkylamino(C₁-C₆)alkyl, di(C₁-C₆)alkylamino(C₁-C₆)alkyl amino(C₂-C₆)alkoxy, (C₁-C₆)alkylamino(C₂-C₆)alkoxy, di(C₁-C₆)alkylamino(C₂-C₆)alkoxy and (C₁-C₆)alkylcarbonyl.

Alternatively, R² is aryl, heteroaryl, cycloalkyl or heterocyclyl substituted with up to 4 groups independently selected from fluorine, bromine, iodine, cyano, amino, hydroxy, (C₁-C₆)alkyl, hydroxy(C₁-C₆)alkyl, (C₃-C₆)cycloalkyl, hydroxy(C₃-C₆)cycloalkyl, (C₄-C₇)cycloalkylalkyl, (C₂-C₆)alkenyl, halo(C₂-C₆)alkenyl, hydroxy(C₂-C₆)alkenyl, (C₂-C₆)alkynyl, (C₃-C₆)cycloalkyl(C₂-C₄)alkynyl, halo(C₁-C₆)alkyl, halo(C₃-C₆)cycloalkyl, halo(C₄-C₇)cycloalkylalkyl, (C₁-C₆)alkoxy, (C₃-C₆)cycloalkoxy, (C₄-C₇)cycloalkylalkoxy, halo(C₁-C₆)alkoxy, halo(C₃-C₆)cycloalkoxy, halo(C₄-C₇)cycloalkylalkoxy, (C₁-C₆)alkylthio, (C₃-C₆)cycloalkylthio, (C₄-C₇)cycloalkylalkylthio, halo(C₁-C₆)alkylthio, halo(C₃-C₆)cycloalkylthio, halo(C₄-C₇)cycloalkylalkylthio, (C₁-C₆)alkanesulfinyl, (C₃-C₆)cycloalkanesulfinyl, (C₄-C₇)cycloalkylalkanesulfinyl, halo(C₁-C₆)alkanesulfinyl, halo(C₃-C₆)cycloalkanesulfinyl, halo(C₄-C₇)cycloalkylalkanesulfinyl, (C₁-C₆)alkanesulfonyl, (C₃-C₆)cycloalkanesulfonyl, (C₄-C₇)cycloalkylalkanesulfonyl, halo(C₁-C₆)alkanesulfonyl, halo(C₃-C₆)cycloalkanesulfonyl, halo(C₄-C₇)cyclo-alkylalkanesulfonyl, (C₁-C₆)alkylamino, di(C₁-C₆)alkylamino, (C₁-C₆)alkoxy(C₁-C₆)alkoxy, halo(C₁-C₆)alkoxy(C₁-C₆)alkoxy, (C₁-C₆)alkoxycarbonyl, H₂NCO, H₂NSO₂, (C₁-C₆)alkylaminocarbonyl, di(C₁-C₆)alkylaminocarbonyl, (C₁-C₃)alkoxy(C₁-C₃)alkylaminocarbonyl, heterocyclylcarbonyl, (C₁-C₆)alkylaminosulfonyl, di(C₁-C₆)alkylaminosulfonyl, heterocyclosulfonyl, (C₁-C₆)alkylcarbonylamino, (C₁-C₆)alkylcarbonylamino(C₁-C₆)alkyl, (C₁-C₆)alkylsulfonylamino, (C₁-C₆)alkylsulfonylamino(C₁-C₆)alkyl, (C₁-C₆)alkoxycarbonyl(C₁-C₆)alkoxy, (C₁-C₆)alkoxy(C₁-C₆)alkyl, halo(C₁-C₆)alkoxy(C₁-C₆)alkyl, hydroxy(C₁-C₆)alkoxy, heteroaryl, oxo, amino(C₁-C₆)alkyl, (C₁-C₆)alkylamino(C₁-C₆)alkyl, di(C₁-C₆)alkylamino(C₁-C₆)alkyl amino(C₂-C₆)alkoxy, (C₁-C₆)alkylamino(C₂-C₆)alkoxy, di(C₁-C₆)alkylamino(C₂-C₆)alkoxy and (C₁-C₆)alkylcarbonyl.

Alternatively, R² is optionally substituted aryl, optionally substituted heteroaryl, optionally substituted cycloalkyl or optionally substituted heterocyclyl, wherein the substituents are as described above.

Alternatively, R² is optionally substituted phenyl, wherein the substituents are as described above.

Alternatively, R² is fluorophenyl.

R³ is selected from (C₂-C₆)alkyl, (C₂-C₆)alkenyl, (C₂-C₆)alkynyl and (C₁-C₃)alkoxy(C₁-C₃)alkyl, wherein the (C₂-C₆)alkyl is substituted with, and each of the (C₂-C₆)alkenyl, (C₂-C₆)alkynyl and (C₁-C₃)alkoxy(C₁-C₃)alkyl is optionally substituted with, up to four groups independently selected from fluorine, cyano, oxo, halo(C₁-C₆)alkyl, amino(C₁-C₆)alkyl, (C₁-C₆)alkylamino(C₁-C₆)alkyl, di(C₁-C₆)alkylamino(C₁-C₆)alkyl, hydroxy(C₁-C₆)alkyl, (C₁-C₆)alkoxy(C₁-C₆)alkyl, R⁴O-, (R⁴)₂N-, R⁴O₂C-, R⁴S, R⁴S(=O)-, R⁴S(=O)₂-, R⁴C(=O)NR⁴-, (R⁴)₂NC(=O)-, (R⁴)₂NC(=O)O-, (R⁴)₂NC(=O)NR⁴-, R⁴OC(=O)NR⁴-, (R⁴)₂NC(=NCN)NR⁴-, (R⁴O)₂P(=O)O-, (R⁴O)₂P(=O)NR⁴-, R⁴OS(=O)₂NR⁴-, (R⁴)₂NS(=O)₂O-; (R⁴)₂NS(=O)₂NR⁴-, R⁴S(=O)₂NR⁴-, R'S(=O)₂NHC(=O)-, R⁴S(=O)₂NHC(=O)O-, R⁴S(=O)₂NHC(=O)NR⁴-, R⁴OS(=O)₂NHC(=O)-, R⁴OS(=O)₂NHC(=O)O-, R⁴OS(=O)₂NHC(=O)NR⁴-, (R⁴)₂NS(=O)₂NHC(=O)-, (R⁴)₂NS(=O)₂NHC(=O)O-, (R⁴)₂NS(=O)₂NHC(=O)NR⁴-, R⁴C(=O)NHS(=O)₂-, R⁴C(=O)NHS(=O)₂O-, R⁴C(=O)NHS(=O)₂NR⁴-, R⁴OC(=O)NHS(=O)₂-, R⁴OC(=O)NHS(=O)₂O-, R⁴OC(=O)NHS(=O)₂NR⁴-, (R⁴)₂NC(=O)NHS(=O)₂-, (R⁴)₂NC(=O)NHS(=O)₂O-, (R⁴)₂NC(=O)NHS(=O)₂NR⁴-, heterocyclyl (which in turn may be optionally substituted with alkyl, haloalkyl or oxo), heteroaryl (which in turn may be optionally substituted with alkyl, haloalkyl, alkoxy, alkylthio, alkylsulfonyl, halogen, trifluoromethyl, dialkylamino, nitro, cyano, CO₂H, CONH₂, N-monoalkyl-substituted amido, N,N-dialkyl-substituted amido, or oxo), arylamino (which in turn may be optionally substituted with alkyl, alkoxy, alkylthio, alkylsulfonyl, halogen, trifluoromethyl, dialkylamino, nitro, cyano, CO₂H, CONH₂, N-monoalkyl-substituted amido and N,N-dialkyl-substituted amido) and heteroarylamino (which in turn may be optionally substituted with alkyl, haloalkyl, alkoxy, alkylthio, alkylsulfonyl, halogen, trifluoromethyl, dialkylamino, nitro, cyano, CO₂H, CONH₂, N-monoalkyl-substituted amido, N,N-dialkyl-substituted amido, or oxo).

Alternatively, R³ is substituted (C₂-C₆)alkyl, wherein the substituents are as described above. Alternatively, R³ is hydroxy(C₂-C₅)alkyl. Alternatively, R³ is dihydroxy(C₃-C₅)alkyl. Alternatively, R³ is ω-H₂NCO(C₁-C₃)alkyl. Alternatively, R³ is (C₁-C₂)alkoxy(C₁-C₃)alkyl. Alternatively, R³ is H₂NSO₂O(C₂-C₄)alkyl. Alternatively, R³ is H₂NSO₂NH(C₂-C₄)alkyl. Alternatively, R³ is oxo(C₂-C₄)alkyl. Alternatively, R³ is MeC(=O)NH(C₂-C₄)alkyl. Alternatively, R³ is 2-hydroxy-2-methylpropyl. Alternatively, R³ is 2-(4-morpholino)ethyl. Alternatively, R³ is MeSO₂NH(C₂-C₄)alkyl. Alternatively, R³ is MeSO₂NHCH₂CH₂CH₂-.

R⁴ is independently selected from H, (C₁-C₆)alkyl, halo(C₁-C₆)alkyl, amino(C₁-C₆)alkyl, (C₁-C₆)alkylamino(C₁-C₆)alkyl, di(C₁-C₆)alkylamino(C₁-C₆)alkyl, hydroxy(C₁-C₆)alkyl and (C₁-C₆)alkoxy(C₁-C₆)alkyl.

Q = O, NR⁵. Alternatively, Q is O. Alternatively, Q is NR⁵. Alternatively, Q is NH.

R⁵ is H, (C₁-C₆)alkyl, halo(C₁-C₆)alkyl or hydroxy(C₁-C₆)alkyl.

In a second embodiment, the 11β-HSD1 inhibitors of the invention are represented by Structural Formula Ia: 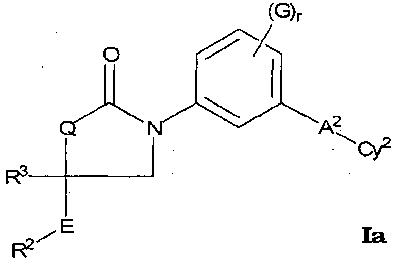 wherein:
G is independently selected from fluorine, chlorine, bromine, iodine, cyano, nitro, amino, hydroxy, carboxy, (C₁-C₆)alkyl, hydroxy(C₁-C₆)alkyl, (C₃-C₆)cycloalkyl, hydroxy(C₃-C₆)cycloalkyl, (C₄-C₇)cycloalkylalkyl, (C₂-C₆)alkynyl, (C₃-C₆)cycloalkyl(C₂-C₄)alkynyl, halo(C₁-C₆)alkyl, halo(C₃-C₆)cycloalkyl, halo(C₄-C₇)cycloalkylalkyl, (C₁-C₆)alkoxy, (C₃-C₆)cycloalkoxy, (C₄-C₇)cycloalkylalkoxy, halo(C₁-C₆)alkoxy, halo(C₃-C₆)cycloalkoxy, halo(C₄-C₇)cycloalkylalkoxy, (C₁-C₆)alkylthio, (C₃-C₆)cycloalkythio, (C₄-C₇)cycloalkylalkylthio, halo(C₁-C₆)alkylthio, halo(C₃-C₆)cycloalkythio, halo(C₄-C₇)cycloalkylalkylthio, (C₁-C₆)alkanesulfinyl, (C₃-C₆)cycloalkanesulfinyl, (C₄-C₇)cycloalkylalkanesulfinyl, halo(C₁-C₆)alkane-sulfinyl, halo(C₃-C₆)cycloalkanesulfinyl, halo(C₄-C₇)cycloalkylalkanesulfinyl, (C₁-C₆)alkanesulfonyl, (C₃-C₆)cycloalkanesulfonyl, (C₄-C₇)cycloalkylalkanesulfonyl, halo(C₁-C₆)alkanesulfonyl, halo(C₃-C₆)cycloalkanesulfonyl, halo(C₄-C₇)cyclo-alkylalkanesulfonyl, (C₁-C₆)alkylamino, di(C₁-C₆)alkylamino, (C₁-C₆)alkoxy(C₁-C₆)alkoxy, halo(C₁-C₆)alkoxy(C₁-C₆)alkoxy, (C₁-C₆)alkoxycarbonyl, H₂NCO, H₂NSO₂, (C₁-C₆)alkylaminocarbonyl, di(C₁-C₆)alkylaminocarbonyl, (C₁-C₃)alkoxy(C₁-C₃)alkylaminocarbonyl, heterocyclylcarbonyl, (C₁-C₆)alkylaminosulfonyl, di(C₁-C₆)alkylaminosulfonyl, heterocyclsulfonyl, (C₁-C₆)alkylcarbonylamino, (C₁-C₆)alkylcarbonylamino(C₁-C₆)alkyl, (C₁-C₆)alkylsulfonylamino, (C₁-C₆)alkylsulfonylamino(C₁-C₆)alkyl, (C₁-C₆)alkoxycarbonyl(C₁-C₆)alkoxy, (C₁-C₆)alkoxy(C₁-C₆)alkyl, halo(C₁-C₆)alkoxy(C₁-C₆)alkyl, hydroxy(C₁-C₆)alkoxy, heteroaryl, amino(C₁-C₆)alkyl, (C₁-C₆)alkylamino(C₁-C₆)alkyl, di(C₁-C₆)alkylamino(C₁-C₆)alkyl amino(C₂-C₆)alkoxy, (C₁-C₆)alkylamino(C₂-C₆)alkoxy, di(C₁-C₆)alkylamino(C₂-C₆)alkoxyl or (C₁-C₆)alkylcarbonyl; and
r is 0,1, 2, 3 or 4. Values and alternative values for the remainder of the variables in Structural Formula Ia are as described for Structural Formula I.

In a third embodiment, the 11β-HSD1 inhibitors of the invention are represented by Structural Formula Ib:

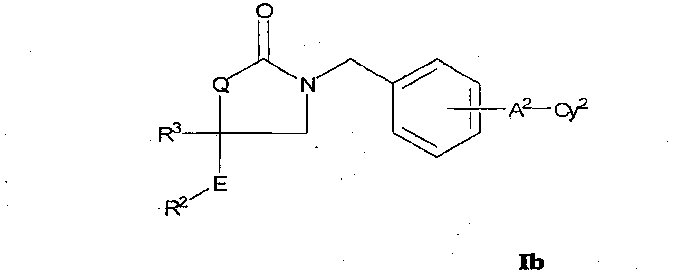

Values and alternative values for the variables in Structural Formula Ib are as described above for Structural Formula I.

In a fourth embodiment, the 11β-HSD1 inhibitors of the invention are represented by Structural Formula Ic:

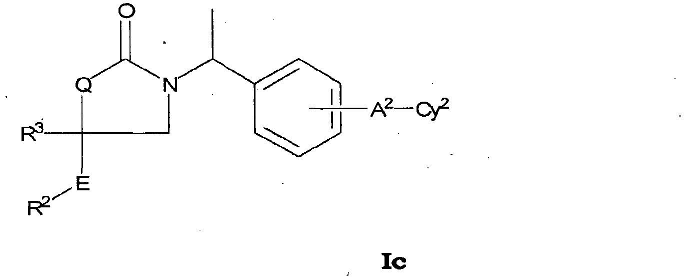

Values and alternative values for the variables in Structural Formula Ic are as described above for Structural Formula I.

In a fifth embodiment, the 11β-HSD1 inhibitors of the invention are represented by Structural Formula Id: 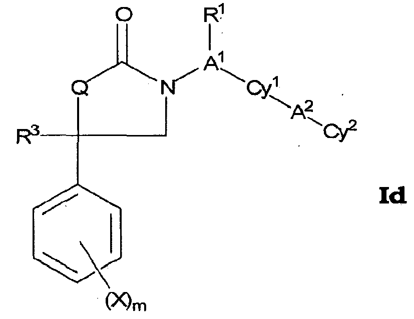 wherein:
X is fluorine, chlorine, bromine, iodine, cyano, nitro, amino, hydroxy, carboxy, (C₁-C₆)alkyl, hydroxy(C₁-C₆)alkyl, (C₃-C₆)cycloalkyl, hydroxy(C₃-C₆)cycloalkyl, (C₄-C₇)cycloalkylalkyl, (C₂-C₆)alkynyl, (C₃-C₆)cycloalkyl(C₂-C₄)alkynyl, halo(C₁-C₆)alkyl, halo(C₃-C₆)cycloalkyl, halo(C₄-C₇)cycloalkylalkyl, (C₁-C₆)alkoxy, (C₃-C₆)cycloalkoxy, (C₄-C₇)cycloalkylalkoxy, halo(C₁-C₆)alkoxy, halo(C₃-C₆)cycloalkoxy, halo(C₄-C₇)cycloalkylalkoxy, (C₁-C₆)alkylthio, (C₃-C₆)cycloalkythio, (C₄-C₇)cycloalkylalkylthio, halo(C₁-C₆)alkylthio, halo(C₃-C₆)cycloalkythio, halo(C₄-C₇)cycloalkylalkylthio, (C₁-C₆)alkanesulfinyl, (C₃-C₆)cycloalkanesulfinyl, (C₄-C₇)cycloalkylalkanesulfinyl, halo(C₁-C₆)alkane-sulfinyl, halo(C₃-C₆)cycloalkanesulfinyl, halo (C₄-C₇)cycloalkylalkanesulfinyl, (C₁-C₆)alkanesulfonyl, (C₃-C₆)cycloalkanesulfonyl, (C₄-C₇)cycloalkylalkanesulfonyl, halo(C₁-C₆)alkanesulfonyl, halo(C₃-C₆)cycloalkanesulfonyl, halo(C₄-C₇)cyclo-alkylalkanesulfonyl, (C₁-C₆)alkylamino, di(C₁-C₆)alkylamino, (C₁-C₆)alkoxy(C₁-C₆)alkoxy, halo(C₁-C₆)alkoxy(C₁-C₆)alkoxy, (C₁-C₆)alkoxycarbonyl, H₂NCO, H₂NSO₂, (C₁-C₆)alkylaminocarbonyl, di(C₁-C₆)alkylaminocarbonyl, (C₁-C₃)alkoxy(C₁-C₃)alkylaminocarbonyl, heterocyclylcarbonyl, (C₁-C₆)alkylaminosulfonyl, di(C₁-C₆)alkylaminosulfonyl, heterocyclsulfonyl, (C₁-C₆)alkylcarbonylamino, (C₁-C₆)alkylcarbonylamino(C₁-C₆)alkyl, (C₁-C₆)alkylsulfonylamino, (C₁-C₆)alkylsulfonylamino(C₁-C₆)alkyl, (C₁-C₆)alkoxycarbonyl(C₁-C₆)alkoxy, (C₁-C₆)alkoxy(C₁-C₆)alkyl, halo(C₁-C₆)alkoxy(C₁-C₆)alkyl, hydroxy(C₁-C₆)alkoxy, heteroaryl, amino(C₁-C₆)alkyl, (C₁-C₆)alkylamino(C₁-C₆)alkyl, di(C₁-C₆)alkylamino(C₁-C₆)alkyl amino(C₂-C₆)alkoxy, (C₁-C₆)alkylamino(C₂-C₆)alkoxy, di(C₁-C₆)alkylamino(C₂-C₆)alkoxyl and (C₁-C₆)alkylcarbonyl; and
m is 0, 1, 2, 3 or 4. Values and alternative values for the remainder of the variables in Structural Formula Id are as described above for Structural Formula I.

In a sixth embodiment, the 11β-HSD1 inhibitors of the invention are represented by Structural Formula Ie: 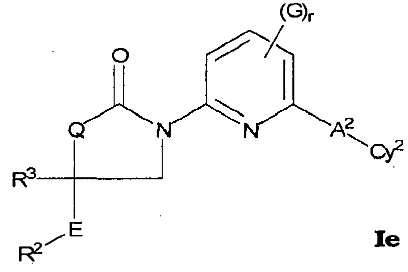 wherein:
G is independently selected from fluorine, chlorine, bromine, iodine, cyano, nitro, amino, hydroxy, carboxy, (C₁-C₆)alkyl, hydroxy(C₁-C₆)alkyl, (C₃-C₆)cycloalkyl, hydroxy(C₃-C₆)cycloalkyl, (C₄-C₇)cycloalkylalkyl, (C₂-C₆)alkyn_{Y}l, (C₃-C₆)cycloalkyl(C₂-C₄)alkynyl, halo(C₁-C₆)alkyl, halo(C₃-C₆)cycloalkyl, halo(C₄-C₇)cycloalkylalkyl, (C₁-C₆)alkoxy, (C₃-C₆)cycloalkoxy, (C₄-C₇)cycloalkylalkoxy, halo(C₁-C₆)alkoxy, halo(C₃-C₆)cycloalkoxy, halo(C₄-C₇)cycloalkylalkoxy, (C₁-C₆)alkylthio, (C₃-C₆)cycloalkythio, (C₄-C₇)cycloalkylalkylthio, halo(C₁-C₆)alkylthio, halo(C₃-C₆)cycloalkythio, halo(C₄-C₇)cycloalkylalkylthio, (C₁-C₆)alkanesulfinyl, (C₃-C₆)cycloalkanesulfinyl, (C₄-C₇)cycloalkylalkanesulfinyl, halo(C₁-C₆)alkane-sulfmyl, halo(C₃-C₆)cycloalkanesulfinyl, halo(C₄-C₇)cycloalkylalkanesulfinyl, (C₁-C₆)alkanesulfonyl, (C₃-C₆)cycloalkanesulfonyl, (C₄-C₇)cycloalkylalkanesulfonyl, halo(C₁-C₆)alkanesulfonyl, halo(C₃-C₆)cycloalkanesulfonyl, halo(C₄-C₇)cyclo-alkylalkanesulfonyl, (C₁-C₆)alkylamino, di(C₁-C₆)alkylamino, (C₁-C₆)alkoxy(C₁-C₆)alkoxy, halo(C₁-C₆)alkoxy(C₁-C₆)alkoxy, (C₁-C₆)alkoxycarbonyl, H₂NCO, H₂NSO₂, (C₁-C₆)alkylaminocarbonyl, di(C₁-C₆)alkylaminocarbonyl, (C₁-C₃)alkoxy(C₁-C₃)alkylaminocarbonyl, heterocyclylcarbonyl, (C₁-C₆)alkylaminosulfonyl, di(C₁-C₆)alkylaminosulfonyl, heterocyclsulfonyl, (C₁-C₆)alkylcarbonylamino, (C₁-C₆)alkylcarbonylamino(C₁-C₆)alkyl, (C₁-C₆)alkylsulfonylamino, (C₁-C₆)alkylsulfonylamino(C₁-C₆)alkyl, (C₁-C₆)alkoxycarbonyl(C₁-C₆)alkoxy, (C₁-C₆)alkoxy(C₁-C₆)alkyl, halo(C₁-C₆)alkoxy(C₁-C₆)alkyl,, hydroxy(C₁-C₆)alkoxy, heteroaryl, oxo, amino(C₁-C₆)alkyl, (C₁-C₆)alkylamino(C₁-C₆)alkyl, di(C₁-C₆)alkylamino(C₁-C₆)alkyl amino(C₂-C₆)alkoxy, (C₁-C₆)alkylamino(C₂-C₆)alkoxy, di(C₁-C₆)alkylamino(C₂-C₆)alkoxyl and (C₁-C₆)alkylcarbonyl; and
r is 0,1, 2, 3 or 4. Values and alternative values for the remainder of the variables in Structural Formula Ie are as described above for Structural Formula I.

In a seventh embodiment, the 11β-HSD1 inhibitors of the invention are represented by Structural Formula If: 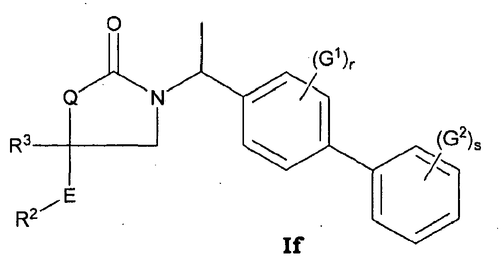 wherein:
G¹ and G² are each independently selected from fluorine, chlorine, bromine, iodine, cyano, nitro, amino, hydroxy, carboxy, (C₁-C₆)alkyl, hydroxy(C₁-C₆)alkyl, (C₃-C₆)cycloalkyl, hydroxy(C₃-C₆)cycloalkyl, (C₄-C₇)cycloalkylalkyl, (C₂-C₆)alkynyl, (C₃-C₆)cycloalkyl(C₂-C₄)alkynyl, halo(C₁-C₆)alkyl, halo(C₃-C₆)cycloalkyl, halo(C₄-C₇)cycloalkylalkyl, (C₁-C₆)alkoxy, (C₃-C₆)cycloalkoxy, (C₄-C₇)cycloalkylalkoxy, halo(C₁-C₆)alkoxy, halo(C₃-C₆)cycloalkoxy, halo(C₄-C₇)cycloalkylalkoxy, (C₁-C₆)alkylthio, (C₃-C₆)cycloalkythio, (C₄-C₇)cycloalkylalkylthio, halo(C₁-C₆)alkylthio, halo(C₃-C₆)cycloalkythio, halo(C₄-C₇)cycloalkylalkylthio, (C₁-C₆)alkanesulfinyl, (C₃-C₆)cycloalkanesulfinyl, (C₄-C₇)cycloalkylalkanesulfinyl, halo(C₁-C₆)alkane-sulfinyl, halo(C₃-C₆)cycloalkanesulfinyl, halo(C₄-C₇)cycloalkylalkanesulfinyl, (C₁-C₆)alkanesulfonyl, (C₃-C₆)cycloalkanesulfonyl, (C₄-C₇)cycloalkylalkanesulfonyl, halo(C₁-C₆)alkanesulfonyl, halo(C₃-C₆)cycloalkanesulfonyl, halo(C₄-C₇)cyclo-alkylalkanesulfonyl, (C₁-C₆)alkylamino, di(C₁-C₆)alkylamino, (C₁-C₆)alkoxy(C₁-C₆)alkoxy, halo(C₁-C₆)alkoxy(C₁-C₆)alkoxy, (C₁-C₆)alkoxycarbonyl, H₂NCO, H₂NSO₂, (C₁-C₆)alkylaminocarbonyl, di(C₁-C₆)alkylaminocarbonyl, (C₁-C₃)alkoxy(C₁-C₃)alkylaminocarbonyl, heterocyclylcarbonyl, (C₁-C₆)alkylaminosulfonyl, di(C₁-C₆)alkylaminosulfonyl, heterocyclsulfonyl, (C₁-C₆)alkylcarbonylamino, (C₁-C₆)alkylcarbonylamino(C₁-C₆)alkyl, (C₁-C₆)alkylsulfonylamino, (C₁-C₆)alkylsulfonylamino(C₁-C₆)alkyl, (C₁-C₆)alkoxycarbonyl(C₁-C₆)alkoxy, (C₁-C₆)alkoxy(C₁-C₆)alkyl, halo(C₁-C₆)alkoxy(C₁-C₆)alkyl" hydroxy(C₁-C₆)alkoxy, heteroaryl, amino(C₁-C₆)atkyl, (C₁-C₆)alkylamino(C₁-C₆)alkyl, di(C₁-C₆)alkylamino(C₁-C₆)alkyl amino(C₂-C₆)alkoxy, (C₁-C₆)alkylamino(C₂-C₆)alkoxy, di(C₁-C₆)alkylamino(C₂-C₆)alkoxyl or (C₁-C₆)alkylcarbonyl;
R⁵ is H, (C₁-C₆)alkyl, halo(C₁-C₆)alkyl, or hydroxy(C₁-C₆)alkyl; and
r and s are independently 0, 1, 2, 3 or 4. Values and alternative values for the remainder of the variables in Structural Formula If are as described above for Structural Formula I.

In an eighth embodiment, the 11β-HSD1 inhibitors of the invention are represented by Structural Formula Ig: 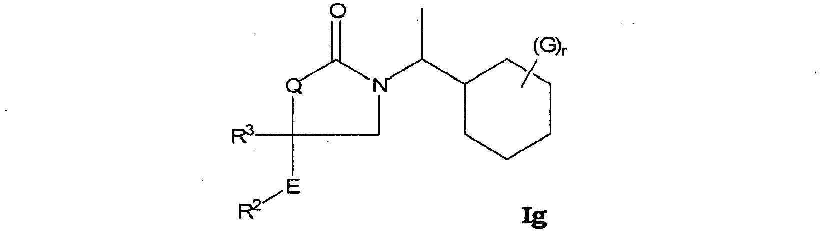 wherein:
G is independently selected from fluorine, chlorine, bromine, iodine, cyano, nitro, amino, hydroxy, carboxy, (C₁-C₆)alkyl, hydroxy(C₁-C₆)alkyl, (C₃-C₆)cycloalkyl, hydroxy(C₃-C₆)cycloalkyl, (C₄-C₇)cycloalkylalkyl, (C₂-C₆)alkenyl, halo(C₂-C₆)alkenyl, hydroxy(C₂-C₆)alkenyl, (C₂-C₆)alkynyl, (C₃-C₆)cycloalkyl(C₂-C₄)alkynyl, halo(C₁-C₆)alkyl, halo(C₃-C₆)cycloalkyl, halo(C₄-C₇)cycloalkylalkyl, (C₁-C₆)alkoxy, (C₃-C₆)cycloalkoxy, (C₄-C₇)cycloalkylalkoxy, halo(C₁-C₆)alkoxy, halo(C₃-C₆)cycloalkoxy, halo(C₄-C₇)cycloalkylalkoxy, (C₁-C₆)alkylthio, (C₃-C₆)cycloalkythio, (C₄-C₇)cycloalkylalkylthio, halo(C₁-C₆)alkylthio, halo(C₃-C₆)cycloalkythio, halo(C₄-C₇)cycloalkylalkylthio, (C₁-C₆)alkanesulfinyl, (C₃-C₆)cycloalkanesulfinyl, (C₄-C₇)cycloalkylalkanesulfinyl, halo(C₁-C₆)alkanesulfinyl, halo(C₃-C₆)cycloalkanesulfinyl, halo(C₄-C₇)cycloalkylalkanesulfinyl, (C₁-C₆)alkanesulfonyl, (C₃-C₆)cycloalkanesulfonyl, (C₄-C₇)cycloalkylalkanesulfonyl, halo(C₁-C₆)alkanesulfonyl, halo(C₃-C₆)cycloalkanesulfonyl, halo(C₄-C₇)cyclo-alkylalkanesulfonyl, (C₁-C₆)alkylamino, di(C₁-C₆)alkylamino, (C₁-C₆)alkoxy(C₁-C₆)alkoxy, halo(C₁-C₆)alkoxy(C₁-C₆)alkoxy, (C₁-C₆)alkoxycarbonyl, H₂NCO, H₂NSO₂, (C₁-C₆)alkylaminocarbonyl, di(C₁-C₆)alkylaminocarbonyl, (C₁-C₃)alkoxy(C₁-C₃)alkylaminocarbonyl; heterocyclylcarbonyl, (C₁-C₆)alkylaminosulfonyl, di(C₁-C₆)alkylaminosulfonyl, heterocyclsulfonyl, (C₁-C₆)alkylcarbonylamino, (C₁-C₆)alkylcarbonylamino(C₁-C₆)alkyl, (C₁-C₆)alkylsulfonylamino, (C₁-C₆)alkylsulfonylamino(C₁-C₆)alkyl, (C₁-C₆)alkoxycarbonyl(C₁-C₆)alkoxy, (C₁-C₆)alkoxy(C₁-C₆)alkyl, halo(C₁-C₆)alkoxy(C₁-C₆)alkyl,, hydroxy(C₁-C₆)alkoxy, heteroaryl, amino(C₁-C₆)alkyl, (C₁-C₆)alkylamino(C₁-C₆)alkyl, di(C₁-C₆)alkylamino(C₁-C₆)alkyl amino(C₂-C₆)alkoxy, (C₁-C₆)alkylamino(C₂-C₆)alkoxy, di(C₁-C₆)alkylamino(C₂-C₆)alkoxyl and (C₁-C₆)alkylcarbonyl; and
r is 0,1, 2, 3 or 4. Values and alternative values for the remainder of the variables in Structural Formula Ig are as described above for Structural Formula I.

Pharmaceutically acceptable salts of the 11β-HSD1 inhibitors disclosed herein (including those represented by any one of Structural Formulae Ia-Ig) are also included in the invention.

Specific 11β-HSD1 inhibitors of the invention and pharmaceutically acceptable salts thereof are provided in Examples 1-3 and Prophetic Examples 1a-221a and 1b-221b below.

Specific examples of compounds of Formulae I and Ia-Ig may exist in various stereoisomeric or tautomeric forms. The invention encompasses all such forms, including active compounds in the form of essentially pure enantiomers, racemic mixtures, and tautomers, including those forms not depicted structurally.

When any variable (e.g., aryl, heterocyclyl, R¹, R², etc.) occurs more than once in a compound, its definition on each occurrence is independent of any other occurrence.

The term "alkyl", used alone or as part of a larger moiety such as "alkoxy", "hydroxyalkyl", "alkoxyalkyl", "alkylamine", "dialkyamine", "alkoxycarbonyl" or "alkylaminocarbonyl", means a saturated straight or branched hydrocarbon radical having (unless otherwise specified) 1-10 carbon atoms and includes, for example, methyl, ethyl, n-propyl, isopropyl, -n-butyl, sec-butyl, isobutyl, tert-butyl, n-pentyl, n-hexyl, n-heptyl, n-octyl, n-nonyl, n-decyl and the like.

The term "cycloalkyl" means a monocyclic, bicyclic or tricyclic, saturated hydrocarbon ring having 3-10 carbon atoms and includes, for example, cyclopropyl, cyclobutyl, cyclopentyl, cyclohexyl, cycloheptyl, cyclooctyl, bicyclo[2.2.2]octyl, bicyclo[2.2.1]heptyl, spiro [4.4]nonane, adamantyl and the like.

The term "aryl" means means a 6-10 membered carbocyclic aromatic monocyclic or polycyclic ring system, such as phenyl or naphthyl. The term "aryl" may be used interchangeably with the terms "aryl ring" "aromatic ring", "aryl group" and "aromatic group".

"Heteroaromatic group", used alone or as part of a larger moiety as in "heteroaralkyl" or "heteroarylalkoxy", means a 5-10 membered monovalent monocyclic and polycylic aromatic group radical containing 1 to 4 heteroatoms independently selected from N, O, and S. Heteroaryl groups include furyl, thienyl, thiophenyl, pyrrolyl, oxazolyl, thiazolyl, imidazolyl, pyrazolyl, isoxazolyl, isothiazolyl, oxadiazolyl, triazolyl, thiadiazolyl, pyridinyl, pyridinyl-N-oxide, pyridazinyl, pyrimidinyl, pyrazinyl, indolizinyl, indolyl, isoindolyl, benzo[b]furyl, benzo[b]thienyl, indazolyl, benzimidazolyl, benzthiazolyl, purinyl, 4H-quinolizinyl, quinolinyl, isoquinolinyl, quinazolinyl, benzothienyl, benzofuranyl, 2,3-dihydrobenzofuranyl, benzodioxolyl, benzimidazolyl, indazolyl, benzisoxazolyl, benzoxazolyl, benzothiazolyl, cinnolinyl, phthalzinyl, quinazolinyl, quinoxalinyl, 1,8-naphthyridinyl, 1,2,3-triazolyl, 1,2,4-triazolyl, 1,3,4-oxadiazolyl, 1,2,5-thiadiazolyl, 1,2,5-thiadiazolyl-1-oxide, 1,2,5-thiadiazolyl-1,1-dioxide, 1,3,4-thiadiazolyl, 1,2,4-triazinyl, 1,3,5-triazinyl, tetrazolyl, and pteridinyl. The terms "heteroaryl", "heteroaromatic", "heteroaryl ring", "heteroaryl group" and "heteroaromatic group" are used interchangeably herein.

The term "heterocyclic group" means a 4-, 5-, 6- and 7-membered saturated or partially unsaturated heterocyclic ring containing 1 to 4 heteroatoms independently selected from N, O, and S, and include pyrrolidine, pyrrolidin-2-one, 1-methylpyrrolidin-2-one, piperidine, piperidin-2-one, dihydropyridine, tetrahydropyridine, piperazine, 1-(2,2,2-trifluoroethyl)piperazine, 1,2-dihydro-2-oxopyridine, 1,4-dihydro-4-oxopyridine, piperazin-2-one, 3,4,5,6-tetrahydro-4-oxopyrimidine, 3,4-dihydro-4-oxopyrimidine, tetrahydrofuran, tetrahydropyran, tetrahydrothiophene, tetrahydrothiopyran, isoxazolidine, 1,3-dioxolane, 1,3-dithiolane, 1,3-dioxane, 1,4-dioxane, 1,3-dithiane, 1,4-dithiane, oxazolidin-2-one, imidazolidin-2-one, imidazolidine-2,4-dione, tetrahydropyrimidin-2(1H)-one, morpholine, N-methylmorpholine, morpholin-3-one, 1,3-oxazinan-2-one, thiomorpholine, thiomorpholine 1,1-dioxide, tetrahydro-1,2,5-thiaoxazole 1,1-dioxide, tetrahydro-2H-1,2-thiazine 1,1-dioxide, hexahydro-1,2,6-thiadiazine 1,1-dioxide, tetrahydro-1,2,5-thiadiazole 1,1-dioxide and isothiazolidine 1,1-dioxide. The terms "heterocyclyl", "heterocycle", "heterocyclic group" and "heterocyclic ring" are used interchangeably herein.

The term "ring atom" is an atom such as C, N, O or S that is in the ring of an aryl group, heteroaryl group, cycloalkyl group or heterocyclic group. A "substitutable ring atom" in an aryl, heteroaryl cycloalkyl or heterocyclic is a carbon or nitrogen atom in the aryl, heteroaryl, cycloalkyl or heterocyclic group that is bonded to at least one hydrogen atom. The hydrogen(s) can be optionally replaced with a suitable substituent group. Thus, the term "substitutable ring atom" does not include ring carbon or nitrogen atoms when the structure depicts that they are not attached to any hydrogen atoms.

Suitable substituents for an alkyl, aryl, heteroaryl and heterocyclic group are those which do not significantly reduce the ability of the compound to inhibit the activity of 11β-HSD1. Unless otherwise specified, suitable substituents for an alkyl, aryl, heteroaryl and heterocyclyl include fluorine, chlorine, bromine, iodine, cyano, nitro, amino, hydroxy, carboxy, (C₁-C₆)alkyl, hydroxy(C₁-C₆)alkyl, (C₃-C₆)cycloalkyl, hydroxy(C₃-C₆)cycloalkyl, (C₄-C₇)cycloalkylalkyl, (C₂-C₆)alkenyl, halo(C₂-C₆)alkenyl, hydroxy(C₂-C₆)alkenyl, (C₂-C₆)alkynyl, (C₃-C₆)cycloalkyl(C₂-C₄)alkynyl, halo(C₁-C₆)alkyl, halo(C₃-C₆)cycloalkyl, halo(C₄-C₇)cycloalkylalkyl, (C₁-C₆)alkoxy, (C₃-C₆)cycloalkoxy, (C₄-C₇)cycloalkylalkoxy, halo(C₁-C₆)alkoxy, halo(C₃-C₆)cycloalkoxy, halo(C₄-C₇)cycloalkylalkoxy, (C₁-C₆)alkylthio, (C₃-C₆)cycloalkylthio, (C₄-C₇)cycloalkylalkylthio, halo(C₁-C₆)alkylthio, halo(C₃-C₆)cycloalkylthio, halo(C₄-C₇)cycloalkylalkylthio, (C₁-C₆)alkanesulfinyl, (C₃-C₆)cycloalkanesulfinyl, (C₄-C₇)cycloalkylalkanesulfinyl, halo(C₁-C₆)alkanesulfinyl, halo(C₃-C₆)cycloalkanesulfinyl, halo(C₄-C₇)cycloalkylalkanesulfinyl, (C₁-C₆)alkanesulfonyl, (C₃-C₆)cycloalkanesulfonyl, (C₄-C₇)cycloalkylalkanesulfonyl, halo(C₁-C₆)alkanesulfonyl, halo(C₃-C₆)cycloalkanesulfonyl, halo(C₄-C₇)cyclo-alkylalkanesulfonyl, (C₁-C₆)alkylamino, di(C₁-C₆)alkylamino, (C₁-C₆)alkoxy(C₁-C₆)alkoxy, halo(C₁-C₆)alkoxy(C₁-C₆)alkoxy, (C₁-C₆)alkoxycarbonyl, H₂NCO, H₂NSO₂, CONH₂, (C₁-C₆)alkylaminocarbonyl, di(C₁-C₆)alkylaminocarbonyl, (C₁-C₃)alkoxy(C₁-C₃)alkylaminocarbonyl, heterocyclylcarbonyl, (C₁-C₆)alkylaminosulfonyl, di(C₁-C₆)alkylaminosulfonyl, heterocyclosulfonyl, (C₁-C₆)alkylcarbonylamino, (C₁-C₆)alkylcarbonylamino(C₁-C₆)alkyl, (C₁-C₆)alkylsulfonylamino, (C₁-C₆)alkylsulfonylamino(C₁-C₆)alkyl, (C₁-C₆)alkoxycarbonyl(C₁-C₆)alkoxy, (C₁-C₆)alkoxy(C₁-C₆)alkyl, halo(C₁-C₆)alkoxy(C₁-C₆)alkyl, hydroxy(C₁-C₆)alkoxy, heteroaryl, oxo, amino(C₁-C₆)alkyl, (C₁-C₆)alkylamino(C₁-C₆)alkyl, di(C₁-C₆)alkylamino(C₁)-C₆)alkyl amino(C₂-C₆)alkoxy, (C₁-C₆)alkylamino(C₂-C₆)alkoxy, di(C₁-C₆)alkylamino(C₂-C₆)alkoxy and (C₁-C₆)alkylcarbonyl. Preferred substituents an alkyl, aryl, heteroaryl and heterocyclyl include, unless otherwise specified, halogen, (C₁-C₆)alkyl, hydroxy, (C₁-C₆)alkoxy, (C₁-C₆)alkylamino, di(C₁-C₆)alkylamino, NO₂, CN, CONH₂, halo(C₁-C₆)akyl or halo(C₁-C₆)alkoxy.

The compounds of the invention may be present in the form of pharmaceutically acceptable salts. For use in medicines, the salts of the compounds of the invention refer to non-toxic "pharmaceutically acceptable salts." Pharmaceutically acceptable salt forms include pharmaceutically acceptable acidic/anionic or basic/cationic salts.

Pharmaceutically acceptable acidic/anionic salts include, the acetate, benzenesulfonate, benzoate, bicarbonate, bitartrate, bromide, calcium edetate, camsylate, carbonate, chloride, citrate, dihydrochloride, edetate, edisylate, estolate, esylate, fumarate, glyceptate, gluconate, glutamate, glycollylarsanilate, hexylresorcinate, hydrobromide, hydrochloride, hydroxynaphthoate, iodide, isethionate, lactate, lactobionate, malate, maleate, mandelate, mesylate, methylsulfate, mucate, napsylate, nitrate, pamoate, pantothenate, phosphate/diphospate, polygalacturonate, salicylate, stearate, subacetate, succinate, sulfate, tannate, tartrate, teoclate, tosylate, and triethiodide salts.

The compounds of the invention include pharmaceutically acceptable anionic salt forms, wherein the anionic salts include the acetate, benzenesulfonate, benzoate, bicarbonate, bitartrate, bromide, calcium edetate, camsylate, carbonate, chloride, citrate, dihydrochloride, edetate, edisylate, estolate, esylate, fumarate, glyceptate, gluconate, glutamate, glycollylarsanilate, hexylresorcinate, hydrobromide, hydrochloride, hydroxynaphthoate, iodide, isethionate, lactate, lactobionate, malate, maleate, mandelate, mesylate, methylsulfate, mucate, napsylate, nitrate, pamoate, pantothenate, phosphate/diphospate, polygalacturonate, salicylate, stearate, subacetate, succinate, sulfate, tannate, tartrate, teoclate, tosylate, and triethiodide salts.

Salts of the disclosed 11β-HSD1 inhibitors containing an acidic functional group can be prepared by reacting with a suitable base. Such a pharmaceutically acceptable salt may be made with a base which affords a pharmaceutically acceptable cation, which includes alkali metal salts (especially sodium and potassium), alkaline earth metal salts (especially calcium and magnesium), aluminum salts and ammonium salts, as well as salts made from physiologically acceptable organic bases such as trimethylamine, triethylamine, morpholine, pyridine, piperidine, picoline, dicyclohexylamine, N,N'-dibenzylethylenediamine, 2-hydroxyethylamine, bis-(2-hydroxyethyl)amine, tri-(2-hydroxyethyl)amine, procaine, dibenzylpiperidine, dehydroabietylamine, N,N'-bisdehydroabietylamine, glucamine, N-methylglucamine, collidine, quinine, quinoline, and basic amino acids such as lysine and arginine.

The invention also includes various isomers and mixtures thereof. "Isomer" refers to compounds that have the same composition and molecular weight but differ in physical and/or chemical properties. The structural difference may be in constitution (geometric isomers) or in the ability to rotate the plane of polarized light (stereoisomers).

Certain of the disclosed 11β-HSD1 inhibitors may exist in various stereoisomeric forms. Stereoisomers are compounds that differ only in their spatial arrangement. Enantiomers are pairs of stereoisomers whose mirror images are not superimposable, most commonly because they contain an asymmetrically substituted carbon atom that acts as a chiral center. "Enantiomer" means one of a pair of molecules that are mirror images of each other and are not superimposable. Diastereomers are stereoisomers that are not related as mirror images, most commonly because they contain two or more asymmetrically substituted carbon atoms. The symbol "*" in a structural formula represents the presence of a chiral carbon center. "*R*" and "*S*" represent the configuration of substituents around one or more chiral carbon atoms. Thus, "*R**" and "*S**" denote the relative configurations of substituents around one or more chiral carbon atoms. When a chiral center is not defined as R or S, a mixture of both configurations is present.

"Racemate" or "racemic mixture" means a compound of equimolar quantities of two enantiomers, wherein such mixtures exhibit no optical activity; i.e., they do not rotate the plane of polarized light.

"Geometric isomer" means isomers that differ in the orientation of substituent atoms in relationship to a carbon-carbon double bond, to a cycloalkyl ring, or to a bridged bicyclic system. Atoms (other than H) on each side of a carbon-carbon double bond may be in an E (substituents are on opposite sides of the carbon-carbon double bond) or Z (substituents are oriented on the same side) configuration.

The compounds of the invention may be prepared as individual isomers by either isomer-specific synthesis or resolved from an isomeric mixture. Conventional resolution techniques include forming the salt of a free base of each isomer of an isomeric pair using an optically active acid (followed by fractional crystallization and regeneration of the free base), forming the salt of the acid form of each isomer of an isomeric pair using an optically active amine (followed by fractional crystallization and regeneration of the free acid), forming an ester or amide of each of the isomers of an isomeric pair using an optically pure acid, amine or alcohol (followed by chromatographic separation and removal of the chiral auxiliary), or resolving an isomeric mixture of either a starting material or a final product using various well known chromatographic methods.

When a disclosed compound is named or depicted by structure without indicating the stereochemistry, and the compound has at least one chiral center, it is to be understood that the name or structure encompasses one enantiomer of inhibitor free from the corresponding optical isomer, a racemic mixture of the inhibitor and mixtures enriched in one enantiomer relative to its corresponding optical isomer.

When the stereochemistry of a disclosed compound is named or depicted by structure, the named or depicted stereoisomer is at least 60%, 70%, 80%, 90%, 95%, 98%, 99% or 99.9% by weight pure relative to the other stereoisomers. When a single enantiomer is named or depicted by structure, the depicted or named enantiomer is at least 60%, 70%, 80%, 90%, 95%, 98%, 99% or 99.9% by weight optically pure. Percent optical purity by weight is the ratio of the weight of the enatiomer over the weight of the enantiomer plus the weight of its optical isomer. When a single geometric isomer, e.g., a geometric isomer with a double bond, is depicted by name or structure and the stereochemistry about the double is indicated, the compound is considered to be at least 60%, 70%, 80%, 90%, 95%, 98%, 99% or 99.9% steroechemically pure by weight. Percent stereochemically purity by weight is the ratio of the weight of the geometric isomer over the weight of the both geometric isomers. For example, 99% stereochemically pure means that at least 99% by weight of the compound is the indicated stereoisomer.

A pharmaceutical composition of the invention may, alternatively or in addition to a compound of Formulae I and Ia-Ig, comprise a pharmaceutically acceptable salt of a compound of Formulae I and Ia-Ig, or a prodrug or pharmaceutically active metabolite of such a compound or salt and one or more pharmaceutically acceptable carriers therefor.

"Effective amount" means that amount of active compound agent that elicits the desired biological response in a subject. Such response includes alleviation of the symptoms of the disease or disorder being treated. The effective amount of a compound of the invention in such a therapeutic method is from about 0.01 mg/kg/day to about 10 mg/kg/day, preferably from about 0.5 mg/kg/day to 5 mg/kg/day.

"Inhibiting 11β-HSD1 means to decrease the activity of the 11β-HSD1 enzyme.

"Modulating 11β-HSD1" means to impact the activity of the 11β-HSD1 enzyme by altering its natural activity. Modulation can be analogous to inhibition when a disease or disorder relating to the activity 11β-HSD1 would be effectively treated by suppressing the activity of the enzyme.

"Pharmaceutically acceptable carrier" means compounds and compositions that are of sufficient purity and quality for use in the formulation of a composition of the invention and that, when appropriately administered to an animal or human, do not produce an adverse reaction.

"Treatment" or "treating", as used herein, includes prophylactic and therapeutic treatment. "Therapeutic treatment" includes partially or totally inhibiting, delaying, or reducing the severity of the disease or disorder related to 11β-HSD1. "Prophylactic treatment" encompasses administration of a compound of the invention to a subject susceptible to a disease or disorder related to the activity or expression of 11β-HSD1 in an effort to reduce the likelihood of a subject developing the disease or disorder, or slowing or preventing progression of the disease. Prophylactic treatment includes suppression (partially or completely) of the disease or disorder, and further includes reducing the severity of the disease or disorder, if onset occurs. Prophylactic treatment is particularly advantageous for administration to mammals at risk for developing a disease or disorder related to 11β-HSD1.

The compounds of the present invention can be prepared and administered in a wide variety of oral and parenteral dosage forms. Thus, the compounds of the present invention can be administered by injection, that is, intravenously, intramuscularly, intracutaneously, subcutaneously, intraduodenally, or intraperitoneally. Additionally, the compounds of the present invention can be administered intranasally or transdermally.

It will be obvious to those skilled in the art that the following dosage forms may comprise as the active ingredient, either compounds or a corresponding pharmaceutically acceptable salt of a compound of the present invention.

For preparing pharmaceutical compositions from the compounds of the present invention, pharmaceutically acceptable carriers can either be solid or liquid. Solid form preparations include powders, tablets, pills, capsules, cachets, suppositories, and dispersable granules. A solid carrier can be one or more substances which may also act as diluents, flavoring agents, solubilizers, lubricants, suspending agents, binders, preservatives, tablet disintegrating agents, or an encapsulating material. In powders, the carrier is a finely divided solid which is in a mixture with the finely divided active ingredient.

In tablets, the active ingredient is mixed with the carrier having the necessary binding properties in suitable proportions and compacted in the shape and size desired.

The powders and tablets preferably contain from about one to about seventy percent of the active ingredient. Suitable carriers are magnesium carbonate, magnesium stearate, talc, sugar, lactose, pectin, dextrin, starch, gelatin, tragacanth, methylcellulose, sodium carboxymethylcelluose, a low melting wax, cocoa butter, and the like. Tablets, powders, cachets, lozenges, fast-melt strips, capsules and pills can be used as solid dosage forms containing the active ingredient suitable for oral administration.

For preparing suppositories, a low melting wax, such as a mixture of fatty acid glycerides or cocoa butter, is first melted and the active ingredient is dispersed homogeneously therein, as by stirring. The molten homogeneous mixture is then poured into convenient sized molds, allowed to cool, and thereby to solidify.

Liquid form preparations include solutions, suspensions, retention enemas, and emulsions, for example, water or water propylene glycol solutions. For parenteral injection, liquid preparations can be formulated in solution in aqueous polyethylene glycol solution.

Aqueous solutions suitable for oral administration can be prepared by dissolving the active ingredient in water and adding suitable colorants, flavors, stabilizing , and thickening agents as desired. Aqueous suspensions for oral administration can be prepared by dispersing the finely divided active ingredient in water with viscous material, such as natural or synthetic gums, resins, methylcellulose, sodium carboxymethyl cellulose, and other well-known suspending agents.

The pharmaceutical composition is preferably in unit dosage form. In such form, the composition is subdivided into unit doses containing appropriate quantities of the active ingredient. The unit dosage form can be a packaged preparation, the package containing discrete quantities of, for example, tablets, powders, and capsules in vials or ampules. Also, the unit dosage form can be a tablet, cachet, capsule, or lozenge itself, or it can be the appropriate amount of any of these in packaged form.

The quantity of active ingredient in a unit dose preparation may be varied or adjusted from about 0.1 mg to about 1000.0 mg, preferably from about 0.1 mg to about 100 mg. The dosages, however, may be varied depending upon the requirements of the patient, the severity of the condition being treated, and the compound being employed. Determination of the proper dosage for a particular situation is within the skill in the art. Also, the pharmaceutical composition may contain, if desired, other compatible therapeutic agents.

In therapeutic treatment or as a method-of-use as an inhibitor of 11β-HSD1 or an inhibitor in the production of cortisol in the cell, the active ingredient is preferably administered orally in a solid dosage form as disclosed above in an amount of about 0.1 mg to about 100 mg per daily dose where the dose is administered once or more than once daily.

The compounds of the invention are useful for ameliorating or treating disorders or diseases in which decreasing the level of cortisol is effective in treating a disease state. Thus, the compounds of the invention can be used in the treatment or prevention of diabetes mellitus, obesity, metabolic syndrome, insulin resistance, cardiovascular disease, dyslipidemia, atherosclerosis, lipodystrophy, osteoporosis, glaucoma, Cushing's syndrome, depression, anxiety and Alzheimer's disease, cognitive decline (including age-related cognitive decline), polycystic ovarian syndrome and infertility. In addition, compounds modulate the function of B and T cells of the immune system.

A pharmaceutical composition of the invention may, alternatively or in addition to a compound of Formulae I and Ia-Ig, comprise a pharmaceutically acceptable salt of a compound of Formulae I and Ia-Ig, or a prodrug or pharmaceutically active metabolite of such a compound or salt and one or more pharmaceutically acceptable carriers therefor.

The invention includes a compound of the invention for use in treating or ameliorating an 11β-HSD1 mediated disorder in a mammal in need thereof comprising administering to a subject in need thereof an effective amount of a compound of Formulae I and Ia-Ig, or the enantiomers, diastereomers, or salts thereof or composition thereof.

The compounds of the invention are useful for ameliorating or treating disorders or diseases in which decreasing the level of cortisol is effective in treating a disease state. Thus, the compounds of the invention can be used in the treatment or prevention of diabetes mellitus, obesity, symptoms of metabolic syndrome, glucose intolerance, hyperglycemica, hypertension, hyperlipidemia, insulin resistance, cardiovascular disease, dyslipidemia, atherosclerosis, lipodystrophy, osteoporosis, glaucoma, Cushing's syndrome, Addison's Disease, visceral fat obesity associated with glucocorticoid therapy, depression, anxiety, Alzheimer's disease, dementia, cognitive decline (including age-related cognitive decline), polycystic ovarian syndrome, infertility and hypergonadism. In addition, the compounds modulate the function of B and T cells of the immune system and can therefore be used to treat diseases such as tuberculosis, leprosy and psoriasis. They can also be used to promote wound healing, particularly in diabetic patients.

Additional diseases or disorders that are related to 11β-HSD1 activity include those selected from the group consisting of lipid disorders, hypretriglyceridemia, hypercholesterolemia, low HDL levels, high LDL levels, vascular restenosis, pancreatitis, abdominal obesity, neurodegenerative disease, retinopathy, nephropathy, neuropathy, diabetes, coronary heart disease, stroke, peripheral vascular disease, Cushing's syndrome, hyperinsulinemia, viral diseases, and Syndrome X.

The term "mammal" is preferably a human, but can also be an animal in need of veterinary treatment, e.g., companion animals (e.g., dogs, cats, and the like), farm animals (e.g., cows, sheep, pigs, horses, and the like) and laboratory animals (e.g., rats, mice, guinea pigs, and the like).

The disclosed 11β-HSD1 inhibitors can be used alone or in a combination therapy with one or more additional agents for the treatment of diabetes, dyslipidemia, cardiovascular disease, hypertension, obesity, cancer or glaucoma. Agents for the treatment of diabetes include insulins, such as Humulin® (Eli Lilly), Lantus® (Sanofi Aventis), Novolin (Novo Nordisk), and Exubera® (Pfizer); PPAR gamma agonists, such as Avandia® (rosiglitazone maleate, GSK) and Actos® (pioglitazone hydrochloride, Takeda/Eli Lilly); sulfonylureas, such as Amaryl® (glimepiride, Sanofi Aventis), Diabeta® (glyburide, Sanofi Aventis), Micronase®/Glynase® (glyburide, Pfizer), and Glucotrol®/Glucotrol XL® (glipizide, Pfizer); meglitinides, such as Prandin®/NovoNorm® (repaglinide, Novo Nordisk), Starlix® (nateglinide, Novartis), and Glufast® (mitiglinide, Takeda); biguanides, such as Glucophase®/Glucophase XR® (metformin HCl, Bristol Myers Squibb) and Glumetza (metformin HCl, Depomed); thiazolidinediones; amylin analogs; GLP-1 analogs; DPP-IV inhibitors, such as Januvia® (sitagliptin, Merck); PTB-1B inhibitors; protein kinase inhibitors (including AMP-activated protein kinase inhibitors); glucagon antagonists; glycogen synthase kinase-3 beta inhibitors; glucose-6-phosphatase inhibitors; glycogen phosphorylase inhibitors; sodium glucose co-transporter inhibitors, and α-glucosidase inhibitors, such as Precose®/Glucobay®/Prandase®/ Glucor® (acarbose, Bayer) and Glyset® (miglitol, Pfizer). Agents for the treatment of dyslipidemia and cardiovascular disease include statins, fibrates and ezetimibe. Agents for the treatment of hypertension include α-blockers, β-blockers, calcium channel blockers, diuretics, angiotensin converting enzyme (ACE) inhibitors, dual ACE and neutral endopeptidase (NEP) inhibitors, angiotensin-receptor blockers (ARBs), aldosterone synthase inhibitor, aldosterone-receptor antagonists, or endothelin receptor antagonist. Agents for the treatment of obesity include orlistat, phentermine, sibutramine and rimonabant.

An embodiment of the invention includes administering an 11β-HSD1 inhibiting compound of any one of Structural Formulae I and Ia-Ig or composition thereof in a combination therapy with one or more other 11β-HSD1 inhibitors (whether such inhibitors are also compounds of any one of Structural Formulae I or are compounds of a different class/genus), or with combination products, such as Avandamet® (metformin HCl and rosiglitazone maleate, GSK); Avandaryl® (glimepiride and rosiglitazone maleate, GSK); Metaglip® (glipizide and metformin HCl, Bristol Myers Squibb); Janumet® (sitagliptin and metformin, Merck)and Glucovance® (glyburide and metformin HCl, Bristol Myers Squibb).

The following abbreviations have the indicated meanings:

| Abbreviation | Meaning |
|---|---|
| Boc | *tert*-butoxy carbonyl or *t*-butoxy carbonyl |
| (Boc)₂O | di-*tert*-butyl dicarbonate |
| Cbz | Benzyloxycarbonyl |
| CbzCl | Benzyl chloroformate |
| DAST | diethylaminosulfur trifluoride |
| DBU | 1,8-diazabicyclo[5.4.0]undec-7-ene |
| DCC | N,N'-dicyclohexylcarbodiimide |
| DCU | N,N'-dicyclohexylurea |
| DIAD | diisopropyl azodicarboxylate |
| DIEA | N,N-diisopropylethylamine |
| DMAP | 4-(dimethylamino)pyridine |
| DMF | N,N-dimethylformamide |
| DMPU | 1,3-dimethyl-3,4,5,6-tetrahydro-2(1H)-pyrimidinone |
| 2,4-DNP | 2,4-dinitrophenylhydrazine |
| DPTBS | Diphenyl-t-butylsilyl |
| EDC.HCl, EDCI | 1-[3-(dimethylamino)propyl]-3-ethylcarbodiimide hydrochloride |
| Equiv | equivalents |
| Fmoc | 1-[[(9H-fluoren-9-ylmethoxy)carbonyl]oxy]- |
| Fmoc-OSu | 1-[[(9H-fluoren-9-ylmethoxy)carbonyl]oxy]-2,5-pyrrolidinedione |
| h, hr | hour(s) |
| HOBt | 1-hydroxybenzotriazole |
| HATU | 2-(7-Aza-1H-benzotriazole-1-yl)-1,1,3,3-tetramethyluronium hexafluorophosphate |
| HBTU | 2-(1H-Benzotriazol-1-yl)-1,1,3,3-tetramethyluronium hexafluorophosphate |
| KHMDS | potassium hexamethyldisilazane |
| LAH or LiAlH₄ | lithium aluminum hydride |
| LC-MS | liquid chromatography-mass spectroscopy |
| LHMDS | lithium hexamethyldisilazane |
| Me | methyl |
| MsCl | methanesulfonyl chloride |
| Min | minute |
| MS | mass spectrum |
| NaH | sodium hydride |
| NaHCO₃ | sodium bicarbonate |
| NaN₃ | sodium azide |
| NaOH | sodium hydroxide |
| Na₂SO₄ | sodium sulfate |
| NMM | N-methylmorpholine |
| NMP | N-methylpyrrolidinone |
| Pd₂(dba)₃ | tris(dibenzylideneacetone)dipalladium(0) |
| PE | petroleum ether |
| Quant | quantitative yield |
| Satd | saturated |
| SOCl₂ | thionyl chloride |
| SFC | supercritical fluid chromatography |
| SPA | scintillation proximity assay |
| SPE | solid phase extraction |
| TBAF | tetrabutylammonium fluoride |
| TBS | t-butyldimethylsilyl |
| TBDPS | t-butyldiphenylsilyl |
| TBSCl | t-butyldimethylsilyl chloride |
| TBDPSCl | t-butyldiphenylsilyl chloride |
| TEA | triethylamine or Et₃N |
| TEMPO | 2,2,6,6-tetramethyl-1-piperidinyloxy free radical |
| Teoc | 1-[2-(trimethylsilyl)ethoxycarbonyloxy]- |
| Teoc-OSu | 1-[2-(trimethylsilyl)ethoxycarbonyloxy]pyrrolidin-2,5-dione |
| TFA | trifluoroacetic acid |
| T1c, TLC | thin layer chromatography |
| TMS | trimethylsilyl |
| TMSCl | chlorotrimethylsilane or trimethylsilyl chloride |
| *t*_{R} | retention time |
| TsOH | p-toluenesulfonic acid |

### GENERAL DESCRIPTION OF SYNTHETIC METHODS

Compounds of Formula I can be prepared by several processes. In the discussion below, A¹, A², Cy¹, Cy², E, Q, R¹, R², R³, R⁵, Y, and n have the meanings indicated above unless otherwise noted. In cases where the synthetic intermediates and final products of Formula I described below contain potentially reactive functional groups, for example amino, hydroxyl, thiol and carboxylic acid groups, that may interfere with the desired reaction, it may be advantageous to employ protected forms of the intermediate. Methods for the selection, introduction and subsequent removal of protecting groups are well known to those skilled in the art. (T.W. Greene and P. G. M. Wuts "Protective Groups in Organic Synthesis" John Wiley & Sons, Inc., New York 1999). Such protecting group manipulations are assumed in the discussion below and not described explicitly. Generally, reagents in the reaction schemes are used in equimolar amounts; however, in certain cases it may be desirable to use an excess of one reagent to drive a reaction to completion. This is especially the case when the excess reagent can be readily removed by evaporation or extraction. Bases employed to neutralize HCl in reaction mixtures are generally used in slight to substantial excess (1.05 - 5 equivalents).

In a first process, compounds of Formula I can be prepared by reaction of intermediates of Formula II with reagents of Formula III, wherein Z¹ and Z² are leaving groups such as chloride, 1-imidazolyl or aryloxide, in an inert solvent such as THF, CH₂Cl₂, toluene or MeCN, usually in the presence of an organic or inorganic base such as triethylamine or NaHCO₃ respectively, at -10 °C to 120 °C:

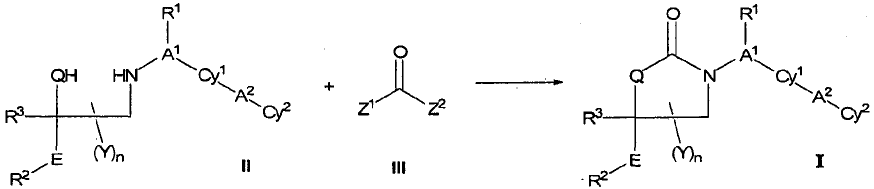

Certain instances of reagent III are especially convenient because they are commercially available. For example when Z¹ and Z² are both chloride, III is phosgene. When Z¹ and Z² are both 1-imidazolyl, III is carbonyl diimidazole. When Z¹ is chloride and Z² is p-nitrophenoxide, III is p-nitrophenyl chloroformate. When Z¹ and Z² are both OCCl₃, III is triphosgene and as little as one third of molar equivalent can be used.

Intermediates of Formula II, wherein n = 0, can be prepared by reduction of amides of Formula IV using a hydride reagent such as BH₃.THF solution, BH₃.Me₂S or LiAlH₄ in an ethereal solvent such as THF or DME at 20 °C to 100 °C for between 1 h and 48 h: 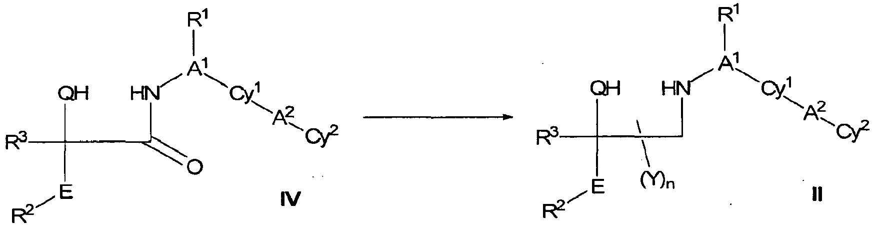 Intermediates of Formula IV can be prepared by coupling of α-hydroxyacids of Formula V (Q = OH) or suitably protected α-aminoacids of Formula V (Q = NR⁵) with amines of Formula VI using standard peptide coupling reagents such as EDC in the presence of HOBt and N,N-diisopropylethylamine in an inert solvent such as CH₂Cl₂ at 0 - 30 °C for between 1 h and 24 h:

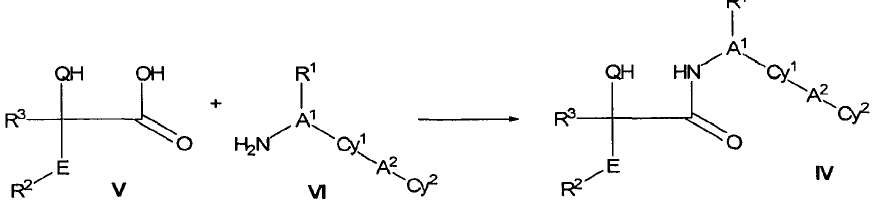

Certain α-hydroxyacids of Formula V, wherein Q = O, are commercially available. α-Hydroxyacids of Formula V can be prepared by diazotization of α-amino acids of Formula VII using NaNO₂ in H₂SO₄: 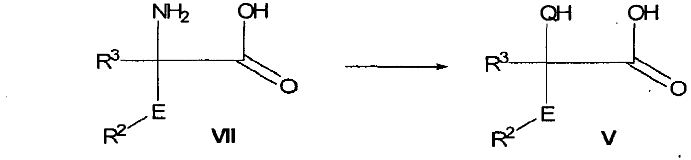 α-Hydroxyacids of Formula V, wherein Q = 0, can also be prepared from ketones Formula VIII via cyanohydrins of Formula IX:

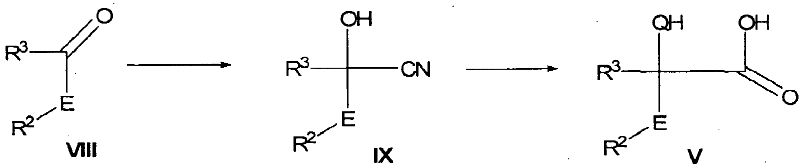

Methods for the conversion of ketones to cyanohydrins are described in Smith, M. B. and March, J. "March's Advanced Organic Chemistry" pp 1239-1240, 5th Edition, Wiley, New York, NY, 2001. Methods for the hydrolysis of cyanohydrins to α-hydroxyacids are described in Smith, M. B. and March, J. "March's Advanced Organic Chemistry" p 1179, 5th Edition, Wiley, New York, NY, 2001
α-hydroxyacids of Formula V, wherein Q = O, can also be prepared by oxidation of diols of Formula X with for example oxygen in the presence of a catalyst or using sodium chlorite and TEMPO:

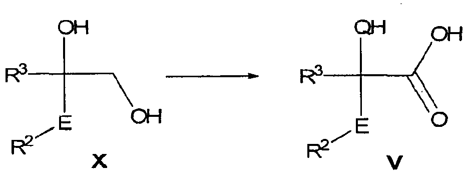

Amine intermediates of Formula VI, wherein A¹ = CH₂ and R¹ is absent, can be prepared by reduction of amides of Formula XI using a hydride reagent such as BH₃.THF solution, BH₃.Me₂S or LiAlH₄ in an ethereal solvent such as THF or DME at 20 °C to 100 °C for between 1 h and 48 h:

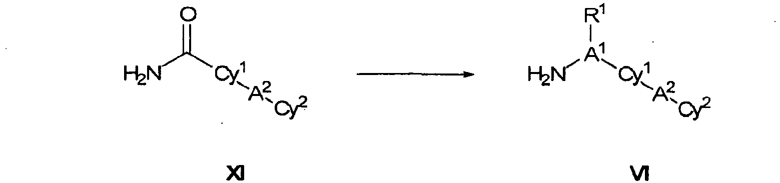

Amine intermediates of Formula VI, wherein A¹ is a bond, R¹ is absent and Cy¹ is not an aromatic or heteroaromatic ring, can be prepared from ketones of formula XII via oximes of Formula XIII or by reductive amination of ketones of Formula XII with ammonia: 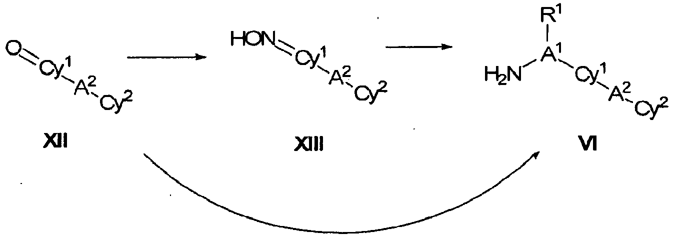 Methods for the conversion of ketones to oximes are described in Smith, M. B. and March, J. "March's Advanced Organic Chemistry" pp 1194-1195, 5th Edition, Wiley, New York, NY, 2001. Methods for the reduction of oximes to primary amines are described in Smith, M. B. and March, J. "March's Advanced Organic Chemistry" p 1555, 5th Edition, Wiley, New York, NY, 2001. Methods for the reductive amination of ketones are described in Baxter, E. W. and Reitz, A. B. "Organic Reactions" Volume 59, Ed. Overman, L. E., Wiley Interscience, 2002.

Amine intermediates of Formula VI, wherein A¹ is CH, can be prepared from ketones of Formula XIV by reductive amination with ammonia.

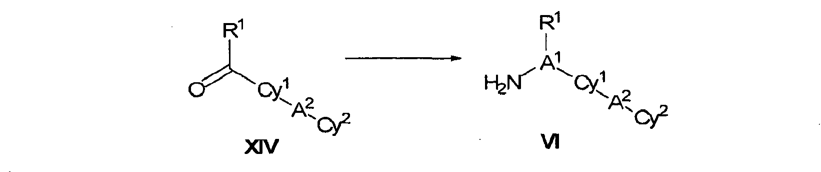

Amine intermediates of Formula VI, wherein A¹ is CH, can be prepared from alcohols of Formula XV via azides of Formula XVI. The conversion of alcohols of Formula XV to azides of Formula XVI can be accomplished with, for example, diphenylphosphoryl azide. Reduction of azides of Formula XVI to amines of Formula VII can be effected, for example, by hydrogenation in the presence of a palladium catalyst or by reaction with triphenylphosphine in wet THF.

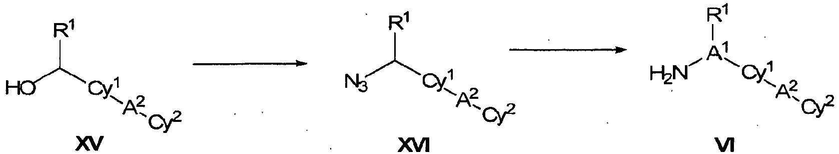

Amine intermediates of Formula VI, wherein A¹ is CH, can be prepared by reaction of sulfinyl imine intermediates of Formula XVII with organometallic reagents of Formula XVIII, wherein M is Li, MgCl, MgBr or MgI, followed by treatment with acid to remove the t-butylsulfinyl group.

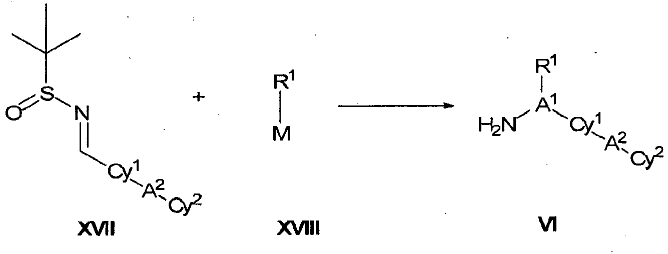

Sulfinyl imines of Formula XVII can be prepared by treatment of aldehyde intermediates of Formula XVIII with 2-methylpropane-2-sulfinamide.

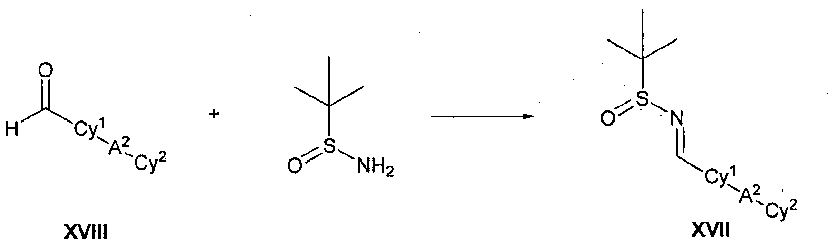

Intermediates of Formula II, wherein Q = O and n = 0, can be prepared by reaction of epoxides of Formula XIX with amines of Formula VI as described in Smith, M. B. and March, J. "March's Advanced Organic Chemistry" p 504, 5th Edition, Wiley, New York, NY, 2001: 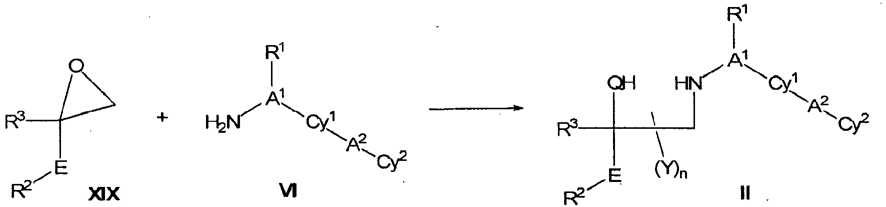 Epoxide compounds of formula XIX can, in turn, be prepared in a number of ways including those described in Aube, J. "Epoxidation and Related Processes" Chapter 3.2 in Volume 1 of "Comprehensive Organic Synthesis" Edited by B. M. Trost, I. Fleming and Stuart L. Schreiber, Pergamon Press, New York, 1992).

Intermediates of Formula II, wherein A¹ is CH₂ and R¹ is absent, can be prepared by reduction of amide intermediates of formula XX using a hydride reagent such as BH₃.THF solution, BH₃.Me₂S or LiAlH₄ in an inert solvent ethereal such as THF or DME at 20 °C to 100 °C for between 1 h and 48 h:

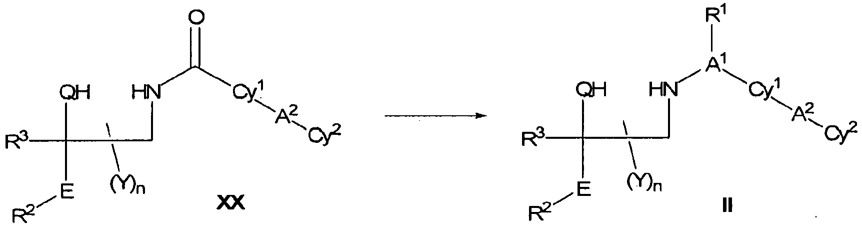

Amide intermediates of Formula XX can be prepared by reaction of an amines of Formula XXI with activated carboxylic acid of Formula XXII wherein Z³ = chloride or an activated ester, such as an N-hydroxysuccinimide ester:

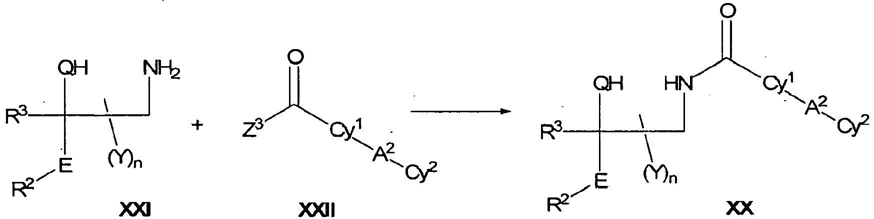

Amines of Formula XXI, wherein n = 0, can be prepared by reaction of epoxides of Formula XIX with azide ion to give azidoalcohols of Formula XXIII followed by reduction of the azide moiety with hydrogen gas or using triphenylphosphine in the presence of water:

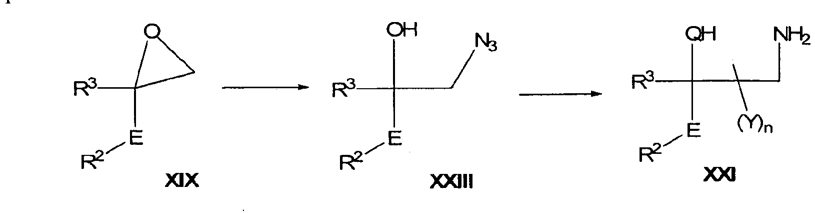

Intermediates of Formula II, wherein n = 0, can also be prepared by reductive amination of aldehyde intermediates of Formula XXIV with amines of Formula VI using for example NaCNBH₃ or NaBH(OAc)₃ as reducing agent:

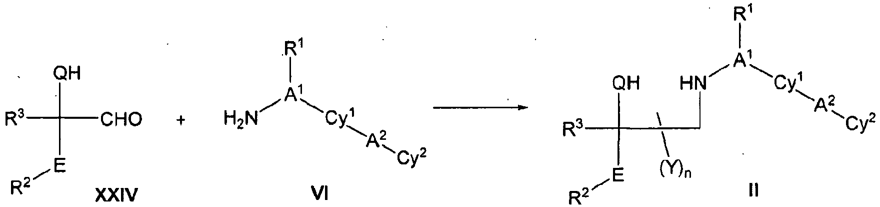

Additional methods for the synthesis of 1,2-diamines, certain of which are applicable to intermediates of Formula II wherein Q = NR³, are described in Lucet, D.; Le Gall, T.; Mioskowski, C. Angew. Chem. Int. Ed. 1998, 37, 2580-2617.

In a second process, a compound of Formula I, wherein Q = O and n =0, can be prepared by reaction of a carbamate intermediate of Formula XXV, wherein R^{a} = alkyl or benzyl, with an epoxide intermediate of Formula XIX in the presence of a strong base such as NaH in a solvent such as THF or DMF at 0 °C to 80 °C:

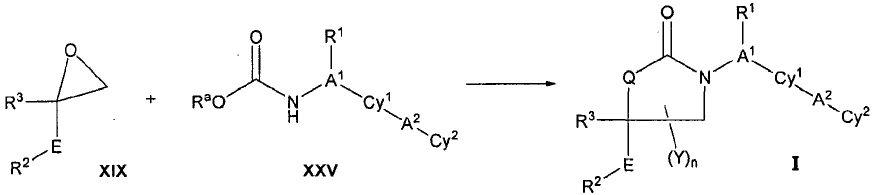

Carbamate intermediates of Formula XXV can be prepared by reaction of amines of Formula VI with chloroformates of Formula XXVI in the presence of a base such as pyridine or triethylamine in an inert solvent such as CH₂Cl₂ or THF at 0 °C to 25 °C for between 1 h and 24 h:

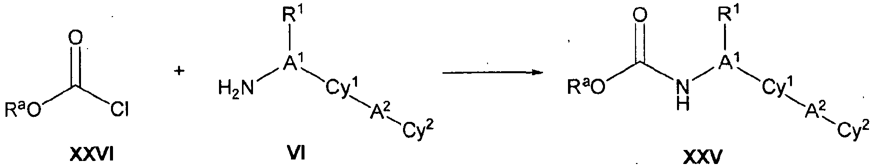

In a third process, compounds of Formula I, wherein R³ is not hydrogen, Q is O and n = 0, can be prepared by reaction of ketocarbamates of Formula XXVII with organometallic reaction of Formula XXVIII, wherein M is Li, MgCl, MgBr or MgI.

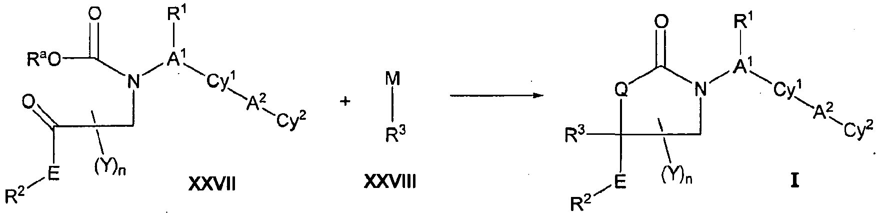

In a fourth process a compound of Formula I can be prepared from another compound of Formula I. For example:
(1) a compound of Formula I wherein Cy¹ is substituted with bromine or iodine, A² is a bond and Cy² is hydrogen can be reacted with an optionally substituted aryl or heteroarylboronic acid or ester in the presence of a palladium catalyst to give a compound of Formula I wherein A² is a bond and Cy² is optionally substituted aryl or heteroaryl.
(2) a compound of Formula I wherein R¹ or R³ is ω-hydroxy(C₂-C₆)alkyl can be oxidized to a compound of Formula I wherein R¹ or R³ is ω-carboxy(C₁-C₅)alkyl using Jones reagent.
(3) a compound of Formula I wherein R¹ or R³ is ω-carboxy(C₁-C₆)alkyl can be coupled with ammonia or a (C₁-C₆)alkylamine using a standard peptide coupling reagent such as EDC to afford a compound of Formula I wherein R¹ or R³ is ω-H₂NC(=O)(C₁-C₆)alkyl or ω-{(C₁-C₆)alkylNHC(=O)}(C₁-C₆)alkyl.
(4) a compound of Formula I wherein R¹ or R³ is ω-hydroxy(C₁-C₆)alkyl can be converted to its methanesulfonate or trifluoromethanesulfonate, treated with sodium azide and reduced to give a compound of Formula I, wherein R¹ or R³ is ω-amino(C₁-C₆)alkyl.
(5) a compound of Formula I wherein R¹ or R³ is amino(C₁-C₆)alkyl can be reacted with acetic anhydride or acetyl chloride to give a compound of Formula I wherein R¹ or R³ is {acetylamino}(C₁-C₆)alkyl.
(6) a compound of Formula I wherein R¹ or R³ is amino(C₁-C₆)alkyl can be reacted with methanesulfonyl chloride to give a compound of Formula I wherein R¹ or R³ is {methanesulfonylamino}(C₁-C₆)alkyl.
(7) a compound of Formula I, wherein R¹ or R³ is (C₂-C₆)alkenyl is hydroborated to afford a compound of Formula I wherein R¹ or R³ is hydroxy(C₂-C₆)alkyl. When the alkene is at the terminus of the (C₂-C₆)alkenyl group, the major product is generally the primary ω-hydroxy(C₂-C₆)alkenyl i and the minor product is the secondary alcohol ii. 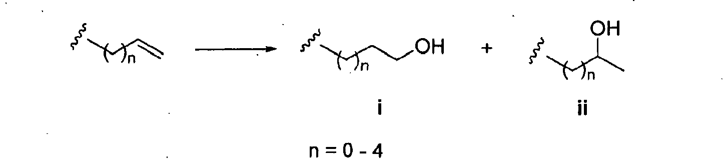
(8) a compound of Formula I, wherein R¹ is (C₂-C₆)alkenyl, can be reacted with osmium tetroxide and N-methylmorpholine-N-oxide to afford a compound of Formula I wherein R¹ is vicinal dihydroxy(C₂-C₆)alkyl,
(9) a compound of Formula I, wherein R³ is (C₂-C₆)alkenyl, can be reacted with osmium tetroxide and N-methylmorpholine-N-oxide to afford a vicinal diol compound of Formula I wherein R³ is vicinal dihydroxy(C₂-C₆)alkyl.
(10) a compound of Formula I, wherein R¹ is (C₂-C₆)alkenyl, can be reacted with ozone followed by NaBH₄ to give a compound of Formula I wherein R¹ is ω-hydroxy(C₁-C₅)alkyl.
(11) a compound of Formula I, wherein R³ is (C₂-C₆)alkenyl, can be reacted with ozone followed by NaBH₄ to give a compound of Formula I wherein R³ is ω-hydroxy(C₁-C₅)alkyl.
(12) a compound of Formula I wherein R¹ or R³ is amino(C₁-C₆)alkyl can be reacted with an (C₁-C₆)alkyl isocyanate to give a compound of Formula I wherein R¹ or R³ is (C₁-C₆)alkylaminocarbonylamino(C₁-C₆)alkyl.
(13) a compound of Formula I wherein R¹ or R³ is amino(C₁-C₆)alkyl can be reacted with an (C₁-C₆)alkyl chloroformate to give a compound of Formula I wherein R¹ or R³ is (C₁-C₆)alkoxycarbonylamino(C₁-C₆)alkyl.
(14) a compound of Formula I wherein R¹ or R³ is amino(C₁-C₆)alkyl can be reacted with chlorosulfonyl isocyanate or sulfamide to give a compound of Formula I wherein R¹ or R³ is aminosulfonylamino(C₁-C₆)alkyl.
(15) a compound of Formula I wherein R¹ or R³ is amino(C₁-C₆)alkyl can be reacted with a (C₁-C₆)alkylsulfamoyl chloride to give a compound of Formula I wherein R¹ or R³ is (C₁-C₆)alkylaminosulfonylamino(C₁-C₆)alkyl.
(16) a compound of Formula I wherein R¹ or R³ is hydroxy(C₁-C₆)alkyl can be reacted with chlorosulfonyl isocyanate to give a compound of Formula I wherein R¹ or R³ is aminosulfonyloxy(C₁-C₆)alkyl.
(17) a compound of Formula I wherein R¹ or R³ is hydroxy(C₁-C₆)alkyl can be reacted with p-nitrophenyl chloroformate, pentafluorophenyl chloroformate or carbonyl diimidazole, followed by ammonia, a (C₁-C₆)alkylamine or a di(C₁-C₆)alkylamine to give a compound of Formula I wherein R¹ or R³ is aminocarboxy(C₁-C₆)alkyl, (C₁-C₆)alkyl aminocarboxy(C₁-C₆)alkyl or di(C₁-C₆)alkyl aminocarboxy(C₁-C₆)alkyl.
(18) a compound of Formula I wherein R¹ or R³ is hydroxy(C₁-C₆)alkyl can be reacted with POCl₃ to give a compound of Formula I wherein R¹ or R³ is (HO)₂P(=O)O(C₁-C₆)alkyl.
(19) a compound of Formula I wherein Cy¹ is substituted with bromine or iodine, A² is a bond and Cy² is hydrogen can be reacted with a cyclic amine in the presence of a palladium catalyst to give a compound of Formula I wherein A² is a bond and Cy² is a cyclic amino moiety attached through its nitrogen atom.
(20) a compound of Formula I wherein R³ is MeO₂C(C₁-C₅)alkyl can be treated with MeMgBr to afford a compound of Formula I wherein R³ is Me₂(HO)C(C₁-C₅)alkyl.
(21) a compound of Formula I wherein R¹ or R³ is ω-H₂NCO(C₁-C₅)alkyl can be reacted with TFAA in the presence of pyridine to afford a compound of Formula I wherein R¹ or R³ is ω-cyano(C₁-C₅)alkyl.
(22) a compound of Formula I wherein R³ is amino(C₁-C₆)alkyl can be reacted with a 2-fluoropyridine to give a compound of Formula I wherein R³ is 2-pyridylamino(C₁-C₆)alkyl.
(23) a compound of Formula I wherein R³ is ω-hydroxy(C₁-C₆)alkyl can be converted to its methanesulfonate or trifluoromethanesulfonate, treated with a (C₁-C₆)alkylthiol followed by oxidation with m-CPBA to give a compound of Formula I wherein R³ is (C₁-C₆)alkylsulfonyl(C₁-C₆)alkyl.
(24) a compound of Formula I, wherein Q is NH, can be reacted with sodium hydride and methyl iodide in an inert solvent to give a compound of Formula I, wherein Q is NMe.

### LC-MS METHODS

### Method 1 [LC-MS (3 min)]

Column: Chromolith SpeedRod, RP-18e, 50 x 4.6 mm; Mobil phase: A: 0.01%TFA/water, B: 0.01 %TFA/CH3CN; Flow rate: 1 mL/min; Gradient:

| Time (min) | A% | B% |
|---|---|---|
| 0.0 | 90 | 10 |
| 2.0 | 10 | 90 |
| 2.4 | 10 | 90 |
| 2.5 | 90 | 10 |
| 3.0 | 90 | 10 |

### Method 2 (10-80)

| | | | |
|---|---|---|---|
| Column | YMC-PACK ODS-AQ, 50×2.0mm µm | | |
| Mobile Phase | A: water (4 L) + TFA (1.5 mL)) | | |
| | B: acetonitrile (4 L) + TFA (0.75 mL)) | | |

| | TIME(min) | A% | B% |
|---|---|---|---|
| | 0 | 90 | 10 |
| | 2.2 | 20 | 80 |
| | 2.5 | 20 | 80 |
| Flow Rate | 1mL/min | | |
| Wavelength | UV 220 nm | | |
| Oven Temp | 50 °C | | |
| MS ionization | ESI | | |

### REFERENCE

### EXAMPLE 1

### 5-allyl-3-((1S)-1-(4-bromophenyl)ethyl)-5-(4-fluorophenyl)oxazolidin-2-one

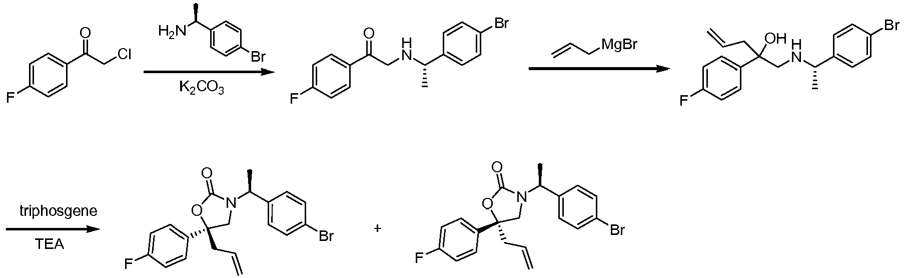

### Step 1

To a solution of (S)-1-(4-bromophenyl)ethanamine (2 g, 0.01 mol) and K₂CO₃ (4.2 g, 0.03 mol) in acetonitrile (50 mL) was added 2-chloro-1-(4-fluorophenyl)ethanone (1.72 g, 0.01 mol). The formed mixture was stirred overnight at rt. The solid was filtered, and the filtrate was concentrated to give (*S*)-2-(1-(4-bromophenyl) ethylamino) - 1- (4-fluorophenyl)ethanone (2 g, 60%), which was used for the next step without further purification.

### Step 2

To a solution of (*S*)-2-(1-(4-bromophenyl)ethylamino)-1-(4-fluorophenyl)ethanone (2 g, 0.006 mol) in THF (300 mL) was added allylmagnesium bromide (1 M, 20 mL, 0.02 mol) under nitrogen at -78 °C. The mixture was stirred at this temperature till the reaction was over. The reaction was quenched with satd aq NH₄Cl. The organic phase was separated and concentrated to give crude 1-((6)-1-(4-bromophenyl)ethylamino)-2-(4-fluorophenyl) pent-4- en-2-ol (1.8 g, 80%), which was used for the next step without further purification.

### Step 3

To a solution of 1-((*S*)-1-(4-bromophenyl)ethylamino)-2-(4-fluorophenyl)pent-4-en-2-ol (0.9 g, 0.003 mol) in CH₂Cl₂ (50 mL) and Et₃N (0.8 g, 0.009 mol) was added triphosgene (0.84 g, 0.003 mol) at 0 °C. The resulting mixture was stirred overnight. The mixture was washed with water. The organic layer was separated, and concentrated to give the crude product, which was purified by preparative TLC to afford 5-allyl-3-((1S)-1-(4-bromophenyl)ethyl)-5-(4-fluorophenyl)oxazolidin-2-one isomer 1 (403 mg, 33%) and 5-allyl -3-((1*S*)-1-(4-bromophenyl)ethyl)-5-(4-fluorophenyl)oxazolidin-2-one isomer 2 (392 mg, 32%).

### REFERENCE

### EXAMPLE 2

### 3-((1S)-1-(4-bromophenyl)ethyl)-5-(4-fluorophenyl)-5-(3-hydroxypropyl)oxazolidin-2-one

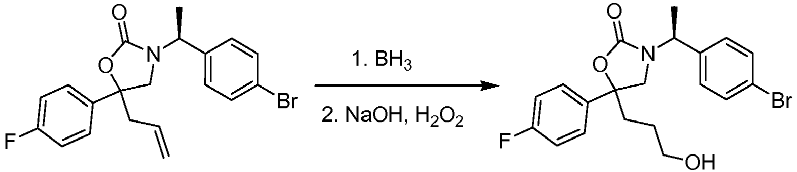

To a solution of 5-allyl-3-((1S)-1-(4-bromophenyl)ethyl)-5-(4-fluorophenyl)oxazolidin-2-one isomer 1 (50 mg, 0.13 mmol) in THF (10 mL) was added 1 M BH₃ in THF (2 mL, 2.0 mmol) at 0 °C under nitrogen atmosphere. The formed mixture was stirred for 2 h. The reaction was quenched with water. Then 3 M aq NaOH (1 mL, 3 mmol) and H₂O₂ (2 mL) were added to the above mixture. When the reaction was over, the mixture was extracted with EtOAc. The combined organic phase was concentrated to give the crude product, which was purified by preparative TLC to give 3-((1S)-1-(4-bromophenyl)ethyl)-5-(4-fluorophenyl)-5-(3-hydroxypropyl)oxazolidin-2-one isomer 1 (15 mg, 30%). ¹H NMR (CDCl₃): 1.38 (m, 2H), 1.50 (m, 3H), 1.61 (m, 2H), 2.00 (m, 2H), 3.11 (m, 1H), 3.52 (m, 3H), 5.12 (m, 1H), 6.98 (m, 4H), 7.18 (m, 2H), 7.30 (m, 2H).

Reaction of 5-allyl-3-((1S)-1-(4-bromophenyl)ethyl)-5-(4-fluorophenyl)oxazolidin-2-one isomer 2 under analogous conditions afforded 3-((1S)-1-(4-bromophenyl)ethyl)-5-(4-fluorophenyl)-5-(3-hydroxypropyl)oxazolidin-2-one isomer 2. ¹H NMR (CDCl₃): 1.18 (m, 2H), 1.39 (m, 3H), 1.41 (m, 1H), 1.86 (m, 2H), 3.18 (m, 1H), 3.48 (m, 3H), 5.12 (m, 1H), 6.98 (m, 2H), 7.17 (m, 2H), 7.23 (m, 2H), 7.48 (m, 2H).

### EXAMPLE 3

### 3-((1S)-1-(4-bromophenyl)ethyl)-5-(4-fluorophenyl)-5-(3-hydroxypropyl)oxazolidin-2-one

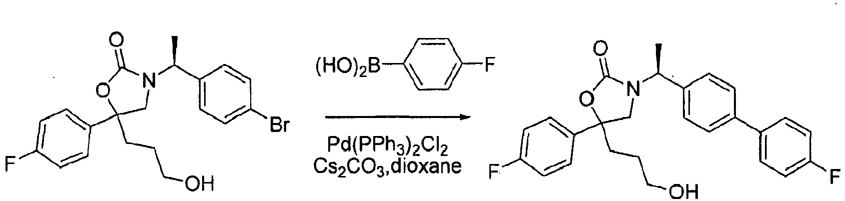

A mixture of 3-((1S)-1-(4-bromophenyl)ethyl)-5-(4-fluorophenyl)-5-(3-hydroxypropyl)oxazolidin-2-one isomer 1 (84 mg, 0.2 mmol), 4-fluorophenylboronic acid (34 mg, 0.24 mmol), Pd(Ph₃P)₂Cl₂ (30 mg), and aq. Cs₂CO₃ solution (2 mL, 2 M) in 1,4-dioxane (6 mL) was stirred and heated to reflux for 2 h. The organic phase was separated, and concentrated to give the crude product, which was purified by preparative HPLC to give 3-((1S)-1-(4'-fluorobiphenyl-4-yl)ethyl)-5-(4-fluorophenyl)-5-(3-hydroxypropyl)oxazolidin-2-one isomer 1 (23.2 mg, 27%). ¹H NMR (CDCl₃): 1.28 (m, 1H), 1.55 (m, 3H), 1.59 (m, 1H), 2.00 (m, 2H), 3.18 (m, 1H), 3.54 (m, 3H), 5.18 (m, 1H), 6.88 (m, 2H), 7.07 (m, 2H), 7.18 (m, 2H), 7.20 (m, 2H), 7.36 (m, 2H), 7.45 (m, 2H).

Reaction of 3-((1S)-1-(4-bromophenyl)ethyl)-5-(4-fluorophenyl)-5-(3-hydroxypropyl)oxazolidin-2-one isomer 2 under analogous conditions afforded 3-((1S)-1-(4'-fluorobiphenyl-4-yl)ethyl)-5-(4-fluorophenyl)-5-(3-hydroxypropyl)oxazolidin-2-one isomer 2. ¹H NMR (EDCl₃): 1.22 (m, 1H), 1.45 (m, 3H), 1.49 (m, 1H), 1.86 (m, 2H), 3.22 (m, 1H), 3.48 (m, 3H), 5.22 (m, 1H), 7.00 (m, 4H), 7.25 (m, 2H), 7.36 (m, 2H), 7.51 (m, 4H).

### REFERENCE

### EXAMPLE 4

### 3-((S)-1-(4-bromophenyl)ethyl)-5-(2-hydroxy-2-methylpropyl)-5-phenyloxazolidin-2-one

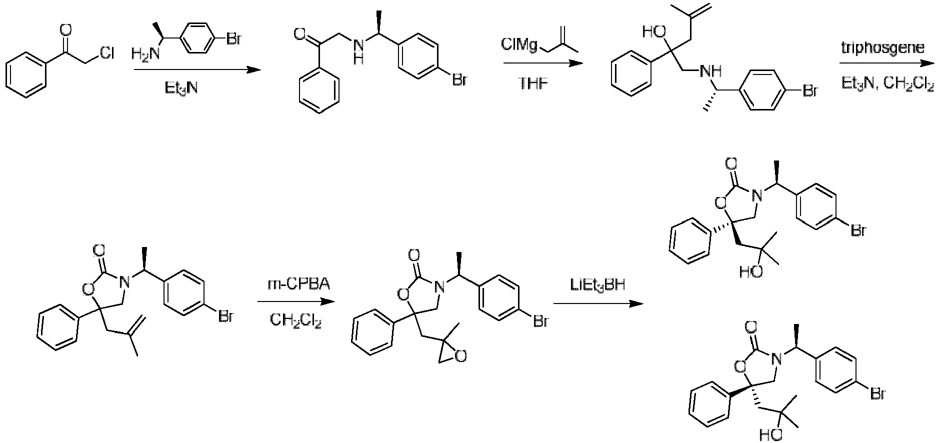

### Step 1

To a solution of 2-bromo-1-phenylethanone (1.54 g, 0.01 mol) in dry THF (50 mL) was added (*S*)-1-(4-bromophenyl)-ethyl amine (1.99 g, 0.01 mol) and Et₃N (3 g, 0.03 mol). The mixture was stirred at room temperature for 4 h, and filtered. The filtrate was concentrated to give the crude (S)-2-(1-(4-bromophenyl)ethylamino)-1-phenylethanone (2.85 g, 90%) as an oil, which was used for the next step without purification.

### Step 2

To a solution of crude (S)-2-(1-(4-bromophenyl)ethylamino)-1-phenylethanone (1 g, 3.15 mmol) in THF (20 mL) was added (2-methylallyl)magnesium chloride (10 mL, 1 mol/L) at -78 °C. The mixture was stirred at rt for 1 h, and TLC showed disappearance of the starting material. The mixture was quenched with satd aq NH₄Cl and extracted with EtOAc. The combined organic phase was dried over Na₂SO₄ and concentrated to give the crude product, which was purified by chromatography to afford 1-((*S*)-1-(4-bromophenyl)ethylamino)-4-methyl-2-phenylpent-4-en-2-ol (1.06 g, 90%).

### Step 3

To a solution of 1-((*S*)-1-(4-bromophenyl)-ethylamino)-4-methyl-2-phenylpent-4-en-2-ol (800 mg g, 2.14 mmol) in CH₂Cl₂ (30 mL) was added triphosgene (630 mg, 2.14 mmol) and Et₃N (650 mg, 6.42 mmol) at 0 °C. The mixture was stirred at rt overnight. TLC showed disappearance of the starting material. The mixture was washed with 1 N aq HCl and extracted by DCM. The combined organic phase was dried over Na₂SO₄ and concentrated to give the crude product, which was purified by chromatography to afford 3-((*S*)-1-(4-bromophenyl)-ethyl)-5-(2-methylallyl)-5-phenyloxazolidin-2-one (700 mg, 82%).

### Step 4

To a solution of 3-((*S*)-1-(4-bromophenyl)-ethyl)-5-(2-methylallyl)-5-phenyloxazolidin-2-one (700 mg, 1.75 mmol) in dry CH₂Cl₂ (100 mL) was added m-CPBA (1.5 g, 8.75 mmol) at rt. The reaction mixture was stirred until the starting material had been consumed (monitored by TLC). The mixture was diluted with (CH₃)₃COCH₃ (70 mL), washed with 30% aq Na₂S₂O₃ and aq NaHCO₃, dried over Na₂SO₄, filtered, and concentrated to give 3-((*S*)-1-(4-bromophenyl)-ethyl)-5-((2-methyloxiran-2-yl)-methyl)-5-phenyloxazolidin-2-one (650 mg, 90%), which was used directly for the next step without further purification..

### Step 5

To a solution of 3-((*S*)-1-(4-bromophenyl)-ethyl)-5-((2-methyloxiran-2-yl)methyl)-5- phenyloxazolidin-2-one (650 mg, 1.57 mmol) in THF (32 mL) was added dropwise Super-Hydride (13.6 mL, 13.6 mmol) at 0 °C under N₂ over 30 min, and the resulting solution was stirred at 10~13°C for 4 h. To the mixture was added dropwise H₂O₂ (20 mL), diluted with (CH₃)₃COCH₃ (380 mL), washed with water, followed by addition of 30% aq Na₂S₂O₃ and brine. The organic phase was dried over Na₂SO₄ and filtered. The filtrate was concentrated to give the crude product, which was purified by prep TLC to afford the the two isomers of the title compound:

Isomer 1: (*S*)-3-((*S*)-1-(4-bromophenyl)ethyl)-5-(2-hydroxy-2-methylpropyl)-5-phenyl oxazolidin-2-one (300 mg, 46%).

Isomer 2: (*R*)-3-((*S*)-1-(4-bromophenyl)-ethyl)-5-(2-hydroxy-2-methylpropyl)-5-phenyloxazolidin-2-one (300 mg, 46%).

### EXAMPLE 5 Isomer 1

### (S)-5-(2-hydroxy-2-methylpropyl)-3-((S)-1-(4-(1-methyl-2-oxo-1,2-dihydropyridin-4-yl)phenyl)ethyl)-5-phenyloxazolidin-2-one

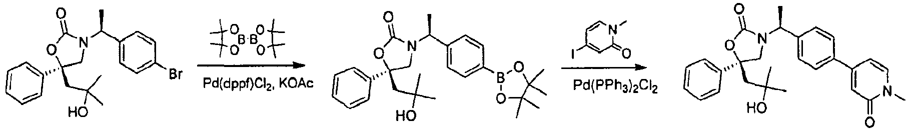

### Step 1

To a solution of (*S*)-3-((S)-1-(4-bromophenyl)-ethyl)-5-(2-hydroxy-2-methylpropyl)-5- phenyloxazolidin-2-one (300 mg, 0.72 mmol) in DMSO (10 mL) was added 4,4,5,5 -tetramethyl-1,3,2-dioxaborolan-2-yl)-1,3,2-dioxaborolane (560 mg, 2.16 mmol), CH₃COOK (995 mg, 10.13 mmol), and Pd(dppf)Cl₂ (60 mg, 0.076 mmol) under N₂. The mixture was stirred at 90 °C for 2 h. The reaction was quenched with H₂O, and extracted with EtOAc. The organic phase was dried over Na₂SO₄ and filtered. The filtrate was concentrated to give the crude product, which was purified by preparative TLC to give (*S*)-5-(2-hydroxy-2-methylpropyl)-5-phenyl-3-((*S*)-1-(4-(4,4,5,5-tetramethyl-1,3,2-dioxaborolan-2-yl)-phenyl)-ethyl)-oxazolidin-2-one (150 mg, 45%).

### Step 2

A mixture of compound (*S*)-5-(2-hydroxy-2-methylpropyl)-5-phenyl-3-((*S*)-1-(4-(4,4,5,5-tetramethyl-1,3,2-dioxaborolan-2-yl)phenyl)ethyl)oxazolidin-2-one (120 mg, 0.26 mmol), 4-iodo-1-methylpyridin-2(1H)-one (67 mg, 0.28 mmol), Pd(PPh₃)₂Cl₂ (30 mg) and aq Cs₂CO₃ (2 N, 2 mL ) solution in 1,4-dioxane (8 mL) was stirred under N₂ atmosphere at 100 °C for 2 h. LC-MS showed the reaction was complete. Water (5 mL) was added, and the mixture was extracted with EtOAc. The combined organic layers were washed with brine, dried over Na₂SO₄ and concentrated. The residue was purified by preparative TLC to give (*S*)-5-(2-hydroxy-2-methylpropyl)-3-((*S*)-1-(4-(1-methyl-2-oxo-1,2-dihydropyridin-4-yl)phenyl)ethyl)-5-phenyloxazolidin-2-one (65 mg, yield 56%). ¹H NMR (EDCl₃ 400 MHz): *δ*1.05 (s, 3H), 1.07 (s, 3H), 1.53 (d, 3H), 1.68 (s, 1H), 2.28 (s, 2H), 3.15 (d, 1H), 3.50 (s, 3H), 3.71 (m, 1H), 5.14 (m, 1H), 5.23 (s, 1H), 6.28 (d, 1H), 6.63 (s, 1H), 7.03 (d, 2H), 7.19-7.29 (m, 8H).

### EXAMPLE 5 Isomer 2

### (R)-5-(2-hydroxy-2-methylpropyl)-3-((S)-1-(4-(1-methyl-2-oxo-1,2-dihydropyridin-4-yl)phenyl)ethyl)-5-phenyloxazolidin-2-one

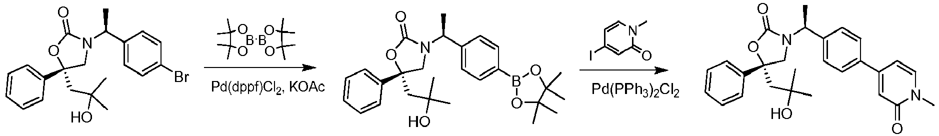

The title compound was prepared following a procedure analogous to that described in for Example 5 Isomer 1, using (R)-3-((S)-1-(4-bromophenyl)ethyl)-5-(2-hydroxy-2-methylpropyl)-5-phenyloxazolidin-2-one in Step 1. ¹H NMR (CDCl₃ 400 MHz): δ 1.02 (s, 3H), 1.06 (s, 3H), 1.45 (d, 3H), 1.71 (s, 1H), 2.20 (m, 2H), 3.40 (d, 1H), 3.48 (d, 1H), 3.60 (s, 3H), 5.28 (m, 1H), 6.45 (d, 1H), 6.80 (s, 1H), 7.28-7.45 (m, 8H), 7.58 (d, 2H).

### REFERENCE

### EXAMPLE 6

### 5-(3-hydroxy-3-methylbutyl)-3-((S)-1-(4-(1-methyl-2-oxo-1,2-dihydropyridin-4-yl)phenyl)ethyl)-5-phenyloxazolidin-2-one

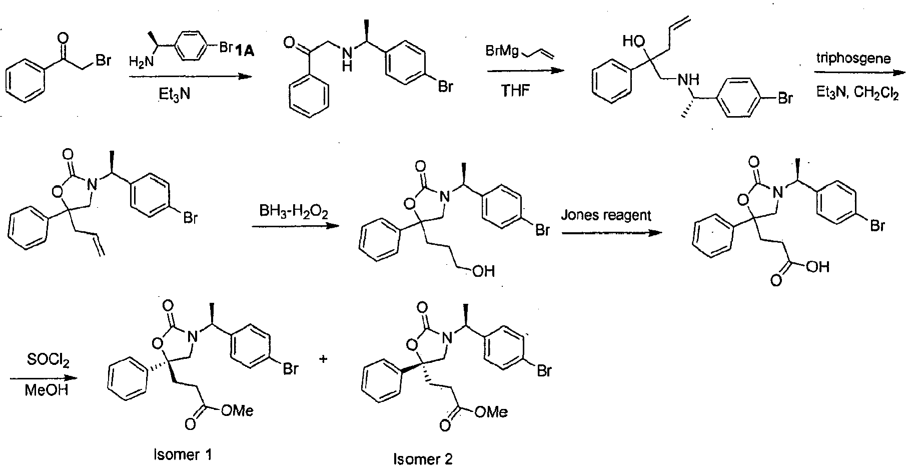

### Steps 1-3

Procedures analogous to those described in Example 4 Steps 1-3 were followed using allylmagnesium bromide in Step 2.

### Step 4

To a solution of 5-allyl-3-((*S*)-1-(4-bromophenyl)ethyl)-5-phenyloxazolidin-2-one (1 g, 2.59 mmol) in THF (10 mL) was added BH₃-THF (10 mL, 10mmol) at 0 °C under nitrogen. The mixture was stirred for 2 h, and quenched with water. The aqueous NaOH solution (2 mL, 3 M) and H₂O₂ (5 mL) was added into the mixture. When the reaction was finished, the mixture was extracted with EtOAc and concentrated to give 3-((*S*)-1-(4-bromophenyl)ethyl)-5-(3-hydroxypropyl)-5-phenyloxazolidin-2-one (0.98 g, yield 93.63%).

### Step 5

To a solution of 3-((*S*)-1-(4-bromophenyl)ethyl)-5-(3-hydroxypropyl)-5-phenyloxazolidin-2-one (0.98 g, 2.42 mmol) in acetone (20 mL) was added Jones reagent (8 mL, 2.5mol/L) at 0 °C. The solution was stirred at room temperature for 30 min. Solvent was removed in vaccum, and the residue was dissolved in a mixture of CH₂Cl₂ and water. The organic layer was separated, and the aqueous layer was extracted with CH₂Cl₂. The combined organic layer was washed with brine, dried over anhydrous Na₂SO₄ and concentrated to give 3-(3-((S)-1-(4-bromophenyl)ethyl)-2-oxo-5-phenyloxazolidin-5-yl)propanoic acid (270 mg, 26.60%).

### Step 6

To a solution of 3-(3-((S)-1-(4-bromophenyl)ethyl)-2-oxo-5-phenyloxazolidin-5-yl)propanoic acid (270 mg, 0.645 mmol) in MeOH (10 mL) was added SOCl₂ (5 mL) at 0°C under nitrogen. The reaction mixture was stirred at room temperature for 2 h, concentrated, and purified by prep TLC (3:1 PE/EA) to afford methyl 3-(3-((S)-1-(4-bromophenyl)ethyl)-2-oxo-5-phenyloxazolidin-5-yl)propanoate (74 mg, 53%) & methyl 3-((*R*)-3-((*S*)-1-(4-bromophenyl)ethyl)-2-oxo-5-phenyloxazolidin-5-yl)propanoate (73 mg, 52.32%).

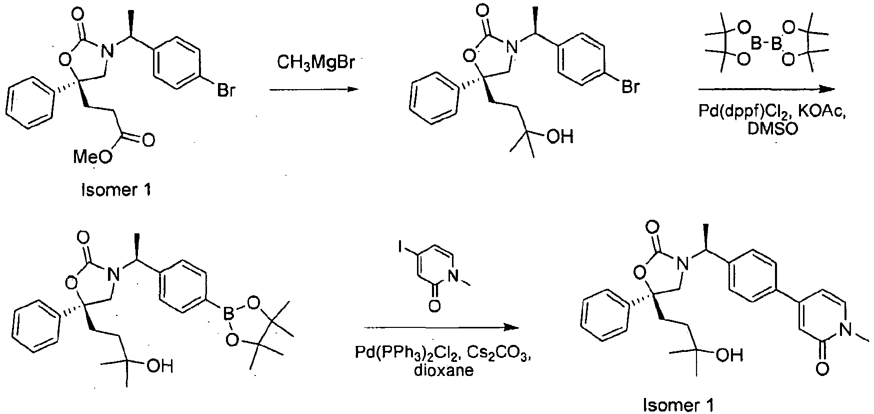

### Step 7

To a solution of methyl 3-(3-((S)-1-(4-bromophenyl)ethyl)-2-oxo-5-phenyloxazolidin-5-yl)propanoate isomer 1 (74 mg, 0.171 mmol) in THF (5 mL) was added MeMgBr (0.57 mL, 1,71 mmol) dropwise at -78. °C. under nitrogen. The reaction mixture was stirred at -78°C for 30 min., at room temperature for 30 min. The reaction mixture was quenched with water, and extracted with EtOAc. The combined organic layer was concentrated to give an oil, which was purified by prep TLC to give 3-((S)-1-(4-bromophenyl)ethyl)-5-(3-hydroxy-3-methylbutyl)-5-phenyloxazolidin-2-one isomer 1 (70 mg, yield 94.59%).

### Steps 8 and 9

Procedures analogous to those described in Example 5 Steps 1 and 2 were followed starting with 3-((S)-1-(4-bromophenyl)ethyl)-5-(3-hydroxy-3-methylbutyl)-5-phenyloxazolidin-2-one isomer 1 to afford 5-(3-hydroxy-3-methylbutyl)-3-((S)-1-(4-(1-methyl-2-oxo-1,2-dihydropyridin-4-yl)phenyl)ethyl)-5-phenyloxazolidin-2-one isomer 1. LC-MS Method 2 t_{R} = 1.00 min, m/z = 461, 443.

Procedures analogous to those described in Example 5 Steps 1 and 2, were followed starting with 3-((S)-1-(4-bromophenyl)ethyl)-5-(3-hydroxy-3-methylbutyl)-5-phenyloxazolidin-2-one isomer 2 to afford 5-(3-hydroxy-3-methylbutyl)-3-((S)-1-(4-(1-methyl-2-oxo-1,2-dihydropyridin-4-yl)phenyl)ethyl)-5-phenyloxazolidin-2-one isomer 2. LC-MS Method 2 t_{R} = 0.95 min, m/z = 461.

### REFERENCE

### EXAMPLE 7

### 5-Methyl-5-phenyl-3-m-tolyloxazolidin-2-one

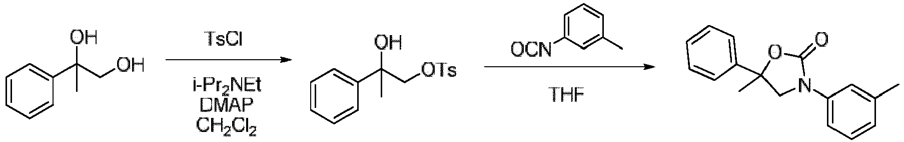

### Step 1

To a stirred solution of 2-phenylpropane-1,2-diol (1.56 g, 10.3 mmol) and i-Pr₂NEt (1.5 mL, 11.4 mmol) and DMAP (1 crystal) in CH₂Cl₂ (40 mL) was added solid p-toluenesulfonyl chloride (1.95 g, 10.3 mmol). The mixture was stirred at rt for 3 d. The mixture was diluted with ether (150 mL), washed with 5% aq HCl (2 x 30 mL) and satd aq NaHCO₃ (30 mL), and dried over MgSO₄. Removal of the salt left an oil (2.29 g) which was purified by chromatography on a 12-g silica cartridge eluted with a 0-50% EtOAc in hexanes gradient to afford 2-hydroxy-2-phenylpropyl 4-methylbenzenesulfonate (1.17 g, 37%).

### Step 2

To a stirred solution of 2-hydroxy-2-phenylpropyl 4-methylbenzenesulfonate (125 mg, 0.41 mmol) in THF (5 mL) was added 3-methylphenyl isocyanate (0.058 mL, 0.45 mmol), followed by DBU (0.075 mL, 0.49 mmol). The mixture was stirred at rt for 4 h and heated at reflux for 20 h. The mixture was diluted with ether (80 mL), washed with 5% aq HCl (20 mL) and satd aq NaHCO₃ (20 mL), and dried over MgSO₄. Removal of the solvent left an oil (70 mg) which was applied to a 2-g silica SPE cartridge which was eluted sequentially with 0, 10, 25, 50, 75 and 100% EtOAc in hexanes to give six fractions. Fractions 1 and 2 were pooled and concentrated to leave an oil (19 mg) which was purified prep HPLC to afford 5-methyl-5-phenyl-3-m-tolyloxazolidin-2-one (2.4 mg, 2%). LC-MS Method 1 t_{R} = 1.92 min, m/z = 268; ¹H NMR (CDCl₃) 1.87 (s, 3H), 2.35 (s, 3H), 4.12 (m, 2H), 6.94 (d, 1H), 7.2-7.5 (8H).

### BIOLOGICAL TEST EXAMPLE 1

The inhibition of microsomal preparation of 11β-HSD1 by compounds of the invention was measured essentially as previously described (K. Solly, S.S. Mundt, H.J. Zokian, G.J. Ding, A. Hermanowski-Vosatka, B. Strulovici, and W. Zheng, High-Throughput Screening of 11-Beta-Hydroxysteroid Dehydrogenase Type 1 in Scintillation Proximity Assay Format. Assay Drug Dev Technol 3 (2005) 377-384). All reactions were carried out at room temperature in 96 well clear flexible PET Microbeta plates (PerkinElmer). The assay begins by dispensing 49 µl of substrate solution (50mM HEPES, pH 7.4, 100mM KCl, 5mM NaCl, 2mM MgCl₂, 2 mM NADPH and 160 nM [³H]cortisone (1 Ci/mmol)) and mixing in 1 µL of the test compounds in DMSO previously diluted in half-log increments (8 points) starting at 0.1 mM. After a 10 minute pre-incubation, 50 µL of enzyme solution containing microsomes isolated from CHO cells overexpressing human 11β-HSD1 (10-20 µg/ml of total protein) was added, and the plates were incubated for 90 minutes at room temperature. The reaction was stopped by adding 50 µl of the SPA beads suspension containing10 µM 18β-glycyrrhetinic acid, 5 mg/ml protein A coated YSi SPA beads (GE Healthcare) and 3.3 µg/ml of anti-cortisol antibody (East Coast Biologies) in Superblock buffer (Bio-Rad). The plates were shaken for 120 minutes at room temperature, and the SPA signal corresponding to [³H]cortisol was measured on a Microbeta plate reader.

### BIOLOGICAL TEST EXAMPLE 2

The inhibition of 11β-HSD1 by compounds of this invention was measured in whole cells as follows. Cells for the assay were obtained from two sources: fully differentiated human omental adipocytes from Zen-Bio, Inc.; and human omental pre-adipocytes from Lonza Group Ltd. Pre-differentiated omental adipocytes from Zen-Bio Inc. were purchased in 96-well plates and were used in the assay at least two weeks after differentiation from precursor preadipocytes. Zen-Bio induced differentiation of pre-adipocytes by supplementing medium with adipogenic and lipogenic hormones (human insulin, dexamethasone, isobutylmethylxanthine and PPAR-gamma agonist). The cells were maintained in full adipocyte medium (DMEM/Ham's F-12 (1:1, v/v), HEPES pH 7.4, fetal bovine serum, penicillin, streptomycin and Amphotericin B, supplied by Zen-Bio, Inc.) at 37°C, 5% CO₂.

Pre-adipocytes were purchased from Lonza Group Ltd. and placed in culture in Preadipocyte Growth Medium-2 supplemented with fetal bovine serum, penicillin, and streptomycin (supplied by Lonza) at 37°C, 5% CO₂. Pre-adipocytes were differentiated by the addition of insulin, dexamethasone, indomethacin and isobutyl-methylxanthine (supplied by Lonza) to the Preadipocyte Growth Medium-2. Cells were exposed to the differentiating factors for 7 days, at which point the cells were differentiated and ready for the assay. One day before running the assay, the differentiated omental adipocytes were transferred into serum- and phenol-red-free medium for overnight incubation. The assay was performed in a total volume of 200 µL. The cells were pre-incubated with serum-free, phenol-red-free medium containing 0.1% (v/v) of DMSO and various concentrations of the test compounds at least 1 h before [³H] cortisone in ethanol (50Ci/mmol, ARC, Inc.) was added to achieve a final concentration of cortisone of 100 nM. The cells were incubated for 3-4 hrs at 37°C, 5% CO₂. Negative controls were incubated without radioactive substrate and received the same amount of [³H] cortisone at the end of the incubation. Formation of [³H] cortisol was monitored by analyzing 25 µL of each supernatant in a scintillation proximity assay (SPA). (Solly, K.; Mundt, S. S.;Zokian, H.J.;Ding, G. J.; Hermanowski-Vosatka, A.; Strulovici, B.; Zheng, W. Assay Drug Dev. Technol. 2005, 3, 377-384). Many compounds of the invention showed significant activity in this assay.

**TABLE OF BIOLOGICAL ASSAY RESULTS**

| | Biological Test Example 1^{a} | |
|---|---|---|
| Compound | IC₅₀ Range | Average % inhibition at 100 nM |
| Example 1 Isomer 1 | nt | nt |
| Example 1 Isomer 2 | nt | nt |
| Example 2 Isomer 1 | ++ | 78.3 |
| Example 2 Isomer 2 | # | 16.9 |
| Example 3 Isomer 1 | ++ | 91.4 |
| Example 3 Isomer 2 | # | 19.1 |
| Example 4 Isomer 1 | nt | nt |
| Example 4 Isomer 2 | nt | nt |
| Example 5 Isomer 1 | ++ | 51.9 |
| Example 5 Isomer 2 | # | 3.4 |
| Example 6 Isomer 1 | # | 18.4 |
| Example 6 Isomer 2 | # | 19.8 |
| Example 7 | - | 5.4 |

| | | |
|---|---|---|
| ^{a} ++ means IC₅₀ = <100 nM, + means IC₅₀ = 100 - 1000 nM, # means IC₅₀ > 100 nM, nt means not tested. | | |

### PROPHETIC COMPOUND TABLES

**TABLE 1**

| | | | | | | | |
|---|---|---|---|---|---|---|---|
| 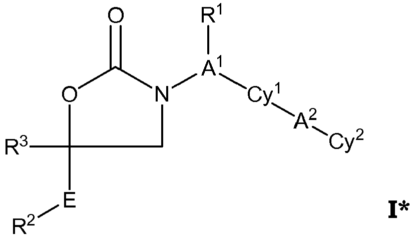 | | | | | | | |

| Prophetic Example No. | A¹-R¹ | Cy¹ | A² | Cy² | E | R² | R³ |
|---|---|---|---|---|---|---|---|
| * 1a | CHMe | 3-Me-Ph | bond | H | bond | Ph | Me |
| * 2a | CHMe | 3-Br-Ph | bond | H | bond | Ph | Me |
| 3a | bond | 1,3-C₆H₄ | bond | Ph | bond | Ph | Me |
| 4a | bond | 1,3-C₆H₄ | bond | 2-Cl-Ph | bond | Ph | Me |
| 5a | bond | 1,3-C₆H₄ | bond | 2-NC-Ph | bond | Ph | Me |
| 6a | bond | 1,3-C₆H₄ | bond | 2-MeO-Ph | bond | Ph | Me |
| 7a | bond | 1,3-C₆H₄ | bond | 2,6-diCl-Ph | bond | Ph | Me |
| 8a | bond | 1,3-C₆H₄ | bond | 2,4-diF-Ph | bond | Ph | Me |
| 9a | bond | 1,3-C₆H₄ | bond | 3-Cl-Ph | bond | Ph | Me |
| 10a | bond | 1,3-C₆H₄ | bond | 3-F-Ph | bond | Ph | Me |
| 11a | bond | 1,3-C₆H₄ | bond | 2.5-diF-Ph | bond | Ph | Me |
| 12a | bond | 1,3-C₆H₄ | bond | 3,5-diF-Ph | bond | Ph | Me |
| 13a | bond | 1,3-C₆H₄ | bond | 4-F-Ph | bond | Ph | Me |
| 14a | bond | 1,3-C₆H₄ | bond | 2-F-Ph | bond | Ph | Me |
| 15a | bond | 2,6-pyridyl | bond | 2-Cl-4-F-Ph | bond | 2-F-Ph | HOCH₂CH₂ |
| 16a | CHMe | 1,4-C₆H₄ | bond | 2,4-diF-Ph | bond | 4-F-Ph | H₂NC(=O)CH₂ |
| 17a | CHMe | 1,4-C₆H₄ | bond | 2,4-diF-Ph | bond | 4-F-Ph | HOCH₂CH(OH)CH₂ |
| 18a | bond | 1,3-C₆H₄ | bond | 2,4-diF-Ph | bond | Ph | allyl |
| 19a | bond | 1,3-C₆H₄ | bond | 2,4-diF-Ph | bond | Ph | HOCH₂CH₂ |
| 20a | bond | 1,3-(4-F)C₆H₃ | bond | 4-F-Ph | bond | 4-F-Ph | HOCH₂CH₂ |
| 21a | bond | 1,3-(4-F)C₆H₃ | bond | 4-F-Ph | bond | 2-F-Ph | HOCH₂CH₂ |
| 22a | bond | 1,3-C₆H₄ | bond | 2-Cl-4-F-Ph | bond | Ph | HOCH₂CH₂ |
| 23a | bond | 1,3-C₆H₄ | bond | 2,6-diCl-Ph | bond | Ph | HOCH₂CH₂ |
| 24a | bond | 1,3-C₆H₄ | bond | 2,4-diF-Ph | bond | Ph | H₂NC(=O)CH₂ |
| 25a | CH | 1,3-C₆H₄ | bond | 2,4-diF-Ph | bond | Ph | HOCH₂CH(OH)CH₂ |
| 26a | bond | 1,3-C₆H₄ | bond | 2,4-diF-Ph | bond | Ph | HOCH₂CH₂CH₂ |
| 27a | bond | 1,3-C₆H₄ | bond | Ph | bond | 3-Cl-Ph | Me |
| 28a | bond | 1,3-C₆H₄ | bond | 2,4-diF-Ph | bond | 2-pyridyl | Me |
| * 29a | CHMe | Ph | bond | H | bond | Ph | Me |
| * 30a | CHMe | 3-MeO-Ph | bond | H | bond | Ph | Me |
| * 31a | CHMe | 4-MeO-Ph | bond | H | bond | Ph | Me |
| * 32a | CHMe | Ph | bond | H | bond | 2-Me-Ph | Me |
| * 33a | CHMe | Ph | bond | H | bond | 4-Me-Ph | Me |
| * 34a | CHMe | Ph | bond | H | bond | 4-MeS-Ph | Me |
| * 35a | CHMe | Ph | bond | H | bond | 4-F-Ph | allyl |
| 36a | bond | 1,3-C₆H₄ | bond | 4-F-Ph | bond | Ph | HOCH₂CH₂ |
| * 37a | CHMe | Ph | bond | H | bond | 4-F-Ph | HOCH₂CH₂ |
| 38a | bond | 1,3-C₆H₄ | bond | 2,4-diF-Ph | bond | Ph | MeSO₂NHCH₂CH₂ |
| 39a | bond | 1,3-(4-F)C₆H₃ | bond | 2-Cl-4-F-Ph | bond | 4-F-Ph | HOCH₂CH₂ |
| 40a | bond | 1,3-C₆H₄ | bond | 2-Cl-4-F-Ph | bond | Ph | HOCH₂CH₂ |
| 41a | bond | 2,6-pyridyl | bond | 4-F-Ph | bond | Ph | HOCH₂CH₂ |
| 42a | bond | 2,6-pyridyl | bond | 4-F-Ph | bond | 4-F-Ph | HOCH₂CH₂ |
| 43a | bond | 2,6-pyridyl | bond | 4-F-Ph | bond | 2-F-Ph | HOCH₂CH₂ |
| 44a | bond | 1,3-(4-F)C₆H₃ | bond | 2,4-diF-Ph | bond | 4-F-Ph | HOCH₂CH₂ |
| 45a | bond | 1,3-(4-F)C₆H₃ | bond | 2,4-diF-Ph | bond | 2-F-Ph | HOCH₂CH₂ |
| 46a | bond | 2,6-pyridyl | bond | 2,4-diF-Ph | bond | Ph | HOCH₂CH₂ |
| 47a | bond | 2,6-pyridyl | bond | 2,4-diF-Ph | bond | 4-F-Ph | HOCH₂CH₂ |
| 48a | bond | 2,6-pyridyl | bond | 2,4-diF-Ph | bond | 2-F-Ph | HOCH₂CH₂ |
| * 49a | CHMe | 4-Br-Ph | bond | H | bond | 4-F-Ph | allyl |
| 50a | CHMe | 1,4-C₆H₄ | bond | 2,4-diF-Ph | bond | 4-F-Ph | allyl |
| 51a | CHMe | 1,4-C₆H₄ | bond | 2,4-diF-Ph | bond | 4-F-Ph | HOCH₂CH₂ |
| * 52a | CHMe | Ph | bond | H | bond | 4-F-Ph | vinyl |
| * 53a | CHMe | 4-Br-Ph | bond | H | bond | 4-F-Ph | HOCH₂CH₂ |
| 54a | bond | 2,6-pyridyl | bond | 2-Cl-4-F-Ph | bond | Ph | HOCH₂CH₂ |
| 55a | bond | 2,6-pyridyl | bond | 2-Cl-4-F-Ph | bond | 4-F-Ph | HOCH₂CH₂ |
| 56a | CHMe | 1,4-C₆H₄ | bond | 4-F-Ph | bond | 4-F-Ph | HOCH₂CH₂ |
| * 57a | CHMe | c-hex | bond | H | bond | 4-F-Ph | allyl |
| * 58a | CHMe | c-hex | bond | H | bond | 4-F-Ph | HOCH₂CH₂CH₂ |
| 59a | CHMe | 1,4-C₆H₄ | bond | c-Pr | bond | 4-F-Ph | allyl |
| * 60a | CHMe | 4-MeO₂C-Ph | bond | H | bond | 4-F-Ph | allyl |
| 61a | CHMe | 1,3-C₆H₄ | bond | c-Pr | bond | 4-F-Ph | HOCH₂CH₂CH₂ |
| * 62a | CHMe | 4-MeO₂C-Ph | bond | H | bond | 4-F-Ph | HOCH₂CH₂CH₂ |
| * 63a | CHEt | 4-Br-Ph | bond | H | bond | 4-F-Ph | allyl |
| 64a | bond | 2,6-(5-Cl)-pyridyl | bond | 4-F-Ph | bond | 2-F-Ph | HOCH₂CH₂ |
| 65a | CHMe | 1,4-C₆H₄ | bond | 2,4-diF-Ph | bond | 4-F-Ph | H₂NCH₂CH₂ |
| 66a | bond | 2,6-(5-Cl)-pyridyl | bond | 2,4-diF-Ph | bond | 2-F-Ph | HOCH₂CH₂ |
| 67a | CHMe | 1,4-C₆H₄ | bond | 2,4-diF-Ph | bond | 4-F-Ph | HOCH₂CH₂CH₂ |
| 68a | CHMe | 1,4-C₆H₄ | bond | 2,4-diF-Ph | bond | 4-F-Ph | MeCH(OH)CH₂ |
| 69a | CHMe | 1,4-C₆H₄ | bond | 2,4-diF-Ph | bond | 4-F-Ph | MeC(=O)CH₂ |
| 70a | CHMe | 1,4-C₆H₄ | bond | 2,4-diF-Ph | bond | 4-F-Ph | HOC(Me)₂CH₂ |
| 71a | CHMe | 1,4-C₆H₄ | bond | 2,4-diF-Ph | bond | 4-F-Ph | MeOCH₂CH₂ |
| 72a | CHMe | 1,4-C₆H₄ | bond | 2,4-diF-Ph | bond | 4-F-Ph | MeNHC(=O)NHCH₂CH₂ |
| * 73a | CHMe | 4-Br-Ph | bond | H | bond | 4-F-Ph | HOCH₂CH₂ |
| * 74a | CHMe | 4-Br-Ph | bond | H | bond | 4-F-Ph | HOCH₂CH(OH)CH₂ |
| 75a | CHMe | 1,4-C₆H₄ | bond | 2,4-diF-Ph | bond | 4-F-Ph | H₂NCOCH₂CH₂ |
| 76a | CHMe | 1,4-C₆H₄ | bond | 2,4-diF-Ph | bond | 4-F-Ph | MeNHC(=O)CH₂CH₂ |
| 77a | CHMe | 1,4-C₆H₄ | bond | 2,4-diF-Ph | bond | 4-F-Ph | MeCONHCH₂CH₂ |
| 78a | CHMe | 1,4-C₆H₄ | bond | 2,4-diF-Ph | bond | 4-F-Ph | MeNHC(=O)OCH₂CH₂ |
| 79a | CHMe | 1,4-C₆H₄ | bond | 2,4-diF-Ph | bond | 4-F-Ph | H₂NSO₂NHCH₂CH₂ |
| 80a | CHMe | 1,4-C₆H₄ | bond | 2,4-diF-Ph | bond | 4-F-Ph | H₂NSO₂OCH₂CH₂ |
| 81a | CHMe | 1,4-C₆H₄ | bond | 2,4-diF-Ph | bond | 4-F-Ph | (HO)₂P(=O)OCH₂CH₂ |
| 82a | CHMe | 1,4-C₆H₄ | bond | 2,4-diF-Ph | bond | 4-F-Ph | H₂NCH₂C(=O)NHCH₂CH₂ |
| * 83a | CHMe | 4-HOCH₂-Ph | bond | H | bond | 4-F-Ph | HOCH₂CH₂CH₂ |
| * 84a | CHMe | 4-HOC(Me)₂-Ph | bond | H | bond | 4-F-Ph | allyl |
| * 85a | CHMe | 4-Br-Ph | bond | H | bond | 2-thienyl | allyl |
| 86a | CHMe | 1,4-C₆H₄ | bond | 4-F-Ph | bond | Ph | HOCH₂CH₂ |
| 87a | CHMe | 1,4-C₆H₄ | bond | 4-F-Ph | bond | 3-thienyl | allyl |
| 88a | CHMe | 1,4-C₆H₄ | bond | 4-F-Ph | bond | Ph | HOCH₂CH₂CH₂ |
| 89a | CHMe | 1,4-C₆H₄ | bond | 2,4-diF-Ph | bond | Ph | HOCH₂CH₂ |
| 90a | CHMe | 1,4-C₆H₄ | bond | 2,4-diF-Ph | bond | 2-thienyl | allyl |
| 91a | CHMe | 1,4-C₆H₄ | bond | 4-F-Ph | bond | 2-thienyl | HOCH₂CH₂CH₂ |
| 92a | CHMe | 1,4-C₆H₄ | bond | 4-F-Ph | bond | 2-thienyl | MeCH(OH)CH₂ |
| 93a | CHMe | 1,4-C₆H₄ | bond | 4-F-Ph | bond | Ph | HOCH₂CH(OH)CH₂ |
| 94a | CHMe | 1,4-C₆H₄ | bond | 2,4-diF-Ph | bond | Ph | HOCH₂CH₂CH₂ |
| 95a | CHMe | 1,4-C₆H₄ | bond | 2,4-diF-Ph | bond | Ph | MeCH(OH)CH₂ |
| 96a | CHMe | 1,4-C₆H₄ | bond | 2,4-diF-Ph | bond | 2-thienyl | HOCH₂CH₂CH₂ |
| 97a | CHMe | Ph | bond | 2,4-diF-Ph | bond | 4-F-Ph | NCCH₂CH₂ |
| 98a | CHMe | 1,4-C₆H₄ | bond | 2,4-diF-Ph | bond | Ph | HOCH₂CH(OH)CH₂ |
| 99a | CHEt | 1,4-C₆H₄ | bond | 2,4-diF-Ph | bond | 4-F-Ph | HOCH₂CH₂ |
| 100a | CHMe | 1,4-C₆H₄ | bond | 2,4-diF-Ph | bond | 4-F-Ph | HOC(=O)CH₂CH₂ |
| 101a | CHMe | 1,4-C₆H₄ | bond | 2,4-diF-Ph | bond | 4-F-Ph | HOCH₂CH₂NHCH₂CH₂ |
| 102a | CHMe | 1,4-C₆H₄ | bond | 2,4-diF-Ph | bond | 4-F-Ph | HOCH2C(=O)NHCH₂CH₂ |
| 103a | CHMe | 1,4-C₆H₄ | bond | 2,4-diF-Ph | bond | 4-F-Ph | MeOC(=O)NHCH2CH2 |
| 104a | CHMe | 1,4-C₆H₄ | bond | 2,4-diF-Ph | bond | 4-F-Ph | 2-(4-morpholino)ethyl |
| 105a | CHMe | 1,4-C₆H₄ | bond | 2,4-diF-Ph | bond | 4-F-Ph | EtNHCONHCH₂CH₂ |
| 106a | CHMe | 1,4-C₆H₄ | bond | 2,4-diF-Ph | bond | 4-F-Ph | MeNHC(=NCN)NHCH₂CH₂ |
| 107a | CHMe | 1,4-C₆H₄ | bond | 2,4-diF-Ph | bond | 4-F-Ph | MeSO₂NHCH₂CH₂CH₂ |
| * 108a | CHMe | 4-Cl-Ph | bond | H | bond | i-Pr | HOCH₂CH₂CH₂ |
| * 109a | CHMe | 4-Me-Ph | bond | H | bond | 4-F-Ph | allyl |
| * 110a | CHMe | 4-MeO-Ph | bond | H | bond | Ph | HOCH₂CH₂ |
| * 111a | CHMe | 4-MeO-Ph | bond | H | bond | 4-F-Ph | allyl |
| * 112a | CHMe | 4-HOCH₂-Ph | bond | H | bond | Ph | HOCH₂CH₂CH₂ |
| * 113a | CHMe | 4-MeO-Ph | bond | H | bond | 4-F-Ph | HOCH₂CH₂ |
| * 114a | CHMe | 4-Cl-Ph | bond | H | bond | 4-F-Ph | allyl |
| * 115a | CHMe | c-hex | bond | H | bond | Ph | HOCH₂CH(OH)CH₂ |
| * 116a | CHMe | 4-HOCH₂CH₂-Ph | bond | H | bond | Ph | HOCH₂CH₂CH₂ |
| * 117a | CHMe | 4-MeOCH₂-Ph | bond | H | bond | Ph | HOCH₂CH₂CH₂ |
| * 118a | CHMe | 4-Br-Ph | bond | H | bond | i-Pr | HOCH₂CH₂CH₂ |
| * 119a | CHMe | 4-Cl-Ph | bond | H | bond | 4-F-Ph | HOCH₂CH₂CH₂ |
| * 120a | CHMe | 4-Cl-Ph | bond | H | bond | 4-F-Ph | MeCH(OH)CH₂ |
| * 121a | CHMe | 4-Br-Ph | bond | H | bond | Ph | allyl |
| 122a | CHMe | 1,4-C₆H₄ | bond | 3-pyridyl | bond | Ph | HOCH₂CH₂ |
| * 123a | CHMe | 4-MeO-Ph | bond | H | bond | 4-F-Ph | HOCH₂CH(OH)CH₂ |
| * 124a | CHMe | 1,4-C₆H₄ | bond | 2,4-diF-Ph | bond | i-Pr | HOCH₂CH₂ |
| * 125a | bond | 1-(*t-*BuOC(=O))pyrrolidin-3-yl | bond | H | bond | Ph | HOCH₂CH₂CH₂ |
| 126a | CHMe | 1,4-C₆H₄ | bond | 2,4-diF-Ph | bond | 4-F-Ph | MeSO₂NHCH₂CH₂ |
| 127a | CHMe | 1,4-C₆H₄ | bond | 4-pyridyl | bond | Ph | HOCH₂CH₂CH₂ |
| 128a | CHMe | 1,4-C₆H₄ | bond | 3-pyridyl | bond | Ph | HOCH₂CH₂CH₂ |
| * 129a | CHMe | 1,4-C₆H₄ | bond | 2,4-diF-Ph | bond | i-Pr | HOCH₂CH₂CH₂ |
| 130a | CHMe | 1,4-C₆H₄ | bond | 3-pyridyl | bond | 4-F-Ph | HOCH₂CH₂ |
| 131a | CHMe | 1,4-C₆H₄ | bond | 2-thienyl | bond | Ph | HOCH₂CH₂CH₂ |
| 132a | CHMe | 1,4-C₆H₄ | bond | 4-morpholinyl | bond | 4-F-Ph | allyl |
| 133a | CHMe | 1,4-C₆H₄ | bond | 4-F-Ph | bond | 2-thienyl | HOCH₂CH₂ |
| 134a | CHMe | 1,4-C₆H₄ | bond | 4-F-Ph | bond | Ph | NCCH₂CH₂ |
| * 135a | CHEt | 4-Br-Ph | bond | H | bond | Ph | HOCH₂CH₂CH₂ |
| 136a | CHMe | 1,4-C₆H₄ | bond | 2,4-diF-Ph | bond | 4-F-Ph | HOCH₂CH₂CH₂ |
| 137a | CHMe | 1,4-C₆H₄ | bond | 1-oxo-3-pyridyl | bond | Ph | HOCH₂CH₂CH₂ |
| * 138a | CHMe | 1,4-C₆H₄ | bond | 2,4-diF-Ph | bond | i-Pr | HOCH₂CH(OH)CH₂ |
| 139a | CHMe | 1,4-C₆H₄ | bond | 4-F-Ph | bond | Ph | MeCH(OH)CH₂ |
| 140a | CHMe | 1,4-C₆H₄ | bond | 3-pyridyl | bond | 4-F-Ph | HOCH₂CH₂CH₂ |
| 141a | CHMe | 1,4-C₆H₄ | bond | 2,4-diF-Ph | bond | Ph | Pr |
| * 142a | CHMe | 4-Br-Ph | bond | H | bond | 4-F-Ph | HOCH₂CH₂CH₂ |
| 143a | CHMe | 1,4-C₆H₄ | bond | 2,4-diF-Ph | bond | 4-F-Ph | MeSO₂CH₂CH₂ |
| 144a | CHMe | 1,4-C₆H₄ | bond | 5-Me-1,3,4-thiadiazol-2-yl | bond | 4-F-Ph | allyl |
| 145a | CHMe | 1,4-C₆H₄ | bond | 4-F-Ph | bond | 2-thienyl | HOCH₂CH₂CH₂ |
| 146a | CHMe | 1,4-C₆H₄ | bond | 2,4-diF-Ph | bond | 2-thienyl | HOCH₂CH2 |
| 147a | CHMe | 1,4-C₆H₄ | bond | 4-F-Ph | bond | Ph | H₂NCOCH₂CH₂ |
| 148a | CHMe | 1,4-C₆H₄ | bond | 2-MeO-5-pyridyl | bond | Ph | HOCH₂CH₂CH₂ |
| 149a | CHMe | 1,4-C₆H₄ | bond | 3-pyridyl | bond | 4-F-Ph | H₂NCOCH₂CH₂ |
| 150a | CHEt | 1,4-C₆H₄ | bond | 4-F-Ph | bond | Ph | HOCH₂CH₂CH₂ |
| 151a | CHMe | 1,4-C₆H₄ | bond | 4-F-Ph | bond | Ph | HOC(Me)₂CH₂ |
| * 152a | CHEt | 4-Br-Ph | bond | H | bond | Ph | HOCH₂CH(OH)CH₂ |
| * 153a | CHMe | 4-Br-Ph | bond | H | bond | 4-F-Ph | H₂NCOCH₂CH₂ |
| * 154a | CHEt | 4-Br-Ph | bond | H | bond | 4-F-Ph | HOCH₂CH₂CH₂ |
| 155a | CHMe | 1,4-C₆H₄ | bond | 2,4-diF-Ph | bond | 4-F-Ph | NCCH₂ |
| 156a | CHMe | 1,4-C₆H₄ | bond | 2,4-die-5-thiazolyl | bond | 4-F-Ph | allyl |
| 157a | CHMe | 1,4-C₆H₄ | bond | 4-F-Ph | bond | 4-F-Ph | HOCH₂CH₂CH₂ |
| 158a | CHMe | 1,4-C₆H₄ | bond | 4-F-Ph | bond | 2-F-Ph | HOCH₂CH₂CH₂ |
| 159a | CHMe | 1,4-C₆H₄ | bond | 4-F-Ph | bond | 3-F-Ph | HOCH₂CH₂CH₂ |
| 160a | CHMe | 1,4-C₆H₄ | bond | 4-F-Ph | bond | Ph | HOC(Me)₂CH₂CH₂ |
| 161a | CHMe | 1,4-C₆H₄ | bond | 5-MeCO-2-thienyl | bond | Ph | HOCH₂CH₂CH₂ |
| 162a | CHMe | 1,4-C₆H₄ | bond | 2,4-diF-Ph | bond | Ph | H₂NCOCH₂CH₂ |
| 163a | CHMe | 1,4-C₆H₄ | bond | 5-(H₂NCHMe)-2-thienyl | bond | Ph | HOCH₂CH₂CH₂ |
| 164a | CHEt | 1,4-C₆H₄ | bond | 4-F-Ph | bond | 4-F-Ph | HOCH₂CH₂CH₂ |
| 165a | CHEt | 1,4-C₆H₄ | bond | 2,4-diF-Ph | bond | Ph | HOCH₂CH₂CH₂ |
| 166a | CHMe | 1,4-C₆H₄ | bond | 5-(HOCHMe)-2-thienyl | bond | Ph | HOCH₂CH₂CH₂ |
| * 167a | CHEt | 4-Br-Ph | bond | H | bond | 4-F-Ph | HOCH₂CH(OH)CH₂ |
| 168a | CHMe | 1,4-C₆H₄ | bond | 2,4-diF-Ph | bond | 4-F-Ph | H₂NCH₂CH₂CH₂ |
| 169a | CHMe | 1,4-C₆H₄ | bond | 2,4-diF-Ph | bond | 4-F-Ph | MeNHCH₂CH₂ |
| 170a | CHMe | 1,4-C₆H₄ | bond | 3-(CF₃)-1-pyrazolyl | bond | 4-F-Ph | allyl |
| 171a | CHMe | 1,4-C₆H₄ | bond | 2,4-diF-Ph | bond | Ph | HOC(Me)₂CH₂CH₂ |
| 172a | CHEt | 1,4-C₆H₄ | bond | 2,4-diF-Ph | bond | 4-F-Ph | HOCH₂CH₂CH₂ |
| 173a | CHMe | 1,4-C₆H₄ | bond | 2,4-diF-Ph | bond | 4-F-Ph | MeSCH₂CH₂ |
| 174a | CHMe | Ph | bond | 2,4-diF-Ph | bond | 4-F-Ph | H₂NC(=O)NHCH₂CH₂ |
| 175a | CHMe | 1,4-C₆H₄ | bond | 2,4-diF-Ph | bond | 4-F-Ph | H₂NC(=O)OCH₂CH₂ |
| 176a | CHMe | 1,4-C₆H₄ | bond | 2,4-diF-Ph | bond | 4-F-Ph | HOCH₂CH₂OCH₂CH₂ |
| 177a | CHMe | 1,4-C₆H₄ | bond | 2,4-diF-Ph | bond | 4-F-Ph | 2-(1-imidazolyl)ethyl |
| 178a | CHMe | 1,4-C₆H₄ | bond | 2,4-diF-Ph | bond | 4-F-Ph | MeCONMeCH₂CH₂ |
| 179a | CHMe | 1,4-C₆H₄ | bond | 4-F-Ph | bond | Ph | MeSO₂NHCH₂CH₂CH₂ |
| 180a | CHMe | 1,4-C₆H₄ | bond | 2,4-diF-Ph | bond | 4-F-Ph | H₂NC(=O)NHCH₂CH₂CH₂ |
| 181a | CHMe | 1,4-C₆H₄ | bond | 2,4-diF-Ph | bond | 4-F-Ph | H₂NC(=O)OCH₂CH₂CH₂ |
| 182a | CHMe | 1,4-C₆H₄ | bond | 2,4-diF-Ph | bond | 4-F-Ph | 3-(1-aminoimidazol-1-yl)ethyl |
| 183a | CHMe | 1,4-C₆H₄ | bond | 2,4-diF-Ph | bond | 4-F-Ph | MeNHC(=O)NHCH₂CH₂CH₂ |
| 184a | CHMe | 1,4-C₆H₄ | bond | 2,4-diF-Ph | bond | 4-F-Ph | H₂NC(=O)NHCH₂CH(OH)CH₂ |
| 185a | CHMe | 1,4-C₆H₄ | bond | 4-F-Ph | bond | 4-F-Ph | MeSO₂NHCH₂CH(OH)CH₂ |
| 186a | CHMe | 1,4-C₆H₄ | bond | 4-F-Ph | bond | 4-F-Ph | MeSO₂NMeCH₂CH(OH)CH₂ |
| 187a | CHMe | 1,4-C₆H₄ | bond | 6-CF₃-3-pyridyl | bond | 4-F-Ph | HOCH₂CH₂CH₂ |
| * 188a | CHMe | 4-MeO-Ph | bond | H | bond | Ph | HOCH₂CH₂CH₂ |
| * 189a | CHMe | 3-F-Ph | bond | H | bond | 4-F-Ph | HOCH₂CH₂CH₂ |
| * 190a | CHMe | 2-F-Ph | bond | H | bond | 4-F-Ph | HOCH₂CH₂CH₂ |
| * 191a | CHMe | 4-F-Ph | bond | H | bond | 4-F-Ph | HOCH₂CH₂CH₂ |
| * 192a | CHMe | 4-MeO-Ph | bond | H | bond | Ph | HOCH₂CH(OH)CH₂ |
| * 193a | CHMe | 4-Cl-Ph | bond | H | bond | Ph | H₂NCOCH₂CH₂ |
| * 194a | CHMe | 4-MeO-Ph | bond | H | bond | 4-F-Ph | H₂NCOCH₂CH₂ |
| * 195a | CHMe | 4-F₂HCO-Ph | bond | H | bond | 4-F-Ph | allyl |
| 196a | CHMe | Ph | bond | 3-pyrazolyl | bond | Ph | HOCH₂CH₂CH₂ |
| 197a | CHMe | 1,4-C₆H₄ | bond | 5-F-3-pyridyl | bond | Ph | allyl |
| * 198a | CHMe | 3-CF₃-Ph | bond | H | bond | 4-F-Ph | HOCH₂CH₂CH₂ |
| * 199a | CHMe | 4-CF₃-Ph | bond | H | bond | 4-F-Ph | HOCH₂CH₂CH₂ |
| 200a | CHMe | 1,4-C₆H₄ | bond | 3-pyridyl | bond | Ph | H₂NCOCH₂CH₂ |
| 201a | CHMe | 1,4-C₆H₄ | bond | 4-pyridyl | bond | Ph | H₂NCOCH₂CH₂ |
| 202a | CHMe | 1,4-C₆H₄ | bond | 4-F-Ph | bond | Ph | HOCH₂CH₂CH₂ |
| 203a | CHMe | 1,4-C₆H₄ | bond | 5-F-3-pyridyl | bond | Ph | HOCH₂CH₂CH₂ |
| * 204a | CHMe | 4-MeO-Ph | bond | H | bond | 4-F-Ph | MeSO₂NHCH₂CH₂ |
| 205a | CHMe | 1,4-C₆H₄ | bond | 5-F-3-pyridyl | bond | 4-F-Ph | HOCH₂CH₂CH₂ |
| 206a | CHMe | 1,4-C₆H₄ | bond | 4-F-Ph | bond | Ph | NCC(Me)2CH2 |
| 207a | CHMe | 1,4-C₆H₄ | bond | 6-MeO-3-pyridyl | bond | Ph | H₂NCOCH₂CH₂ |
| 208a | CHMe | 1,4-C₆H₄ | bond | 5-MeO-3-pyridyl | bond | 4-F-Ph | HOCH₂CH₂CH₂ |
| 209a | CHMe | 1,4-C₆H₄ | bond | 5-Cl-3-pyridyl | bond | 4-F-Ph | HOCH₂CH₂CH₂ |
| 210a | CHMe | 1,4-C₆H₄ | bond | 3-pyridyl | bond | Ph | MeSO₂NHCH₂CH₂ |
| * 211a | CHMe | 4-F₂HCO-Ph | bond | H | bond | 4-F-Ph | HOCH₂CH₂CH₂ |
| 212a | CHMe | 1,4-C₆H₄ | bond | 4-F-Ph | bond | Ph | (HO)₂P(=O)OCH₂CH₂CH₂ |
| 213a | CHMe | 1,4-C₆H₄ | bond | 2-Me-4-pyridyl | bond | 4-F-Ph | HOCH₂CH₂CH₂ |
| * 214a | CHMe | 1,4-C₆H₄ | bond | H | bond | Ph | HOCH₂CH₂CH₂ |
| 215a | CHMe | 1,4-C₆H₄ | bond | 1-Me-6-oxo-3-(1,6-dihydropyridyl) | bond | Ph | HOCH₂CH₂CH₂ |
| * 216a | CHMe | 4-MeO-Ph | bond | H | bond | 4-F-Ph | MeSO₂NHCH₂CH₂CH₂ |
| * 217a | CHMe | 4-MeO-Ph | bond | H | bond | Ph | H₂NCOCH₂CH₂ |
| * 218a | CHMe | 4-F-Ph | bond | H | bond | 4-F-Ph | H₂NCOCH₂CH₂ |
| * 219a | CHMe | c-hex | bond | H | bond | 4-F-Ph | H₂NCOCH₂CH₂ |
| 220a | bond | 1,3-(4-F)C₆H₃ | bond | 2,4-diF-Ph | bond | 4-F-Ph | HOCH₂CH₂ |
| * 221a | CHMe | c-hex | bond | H | bond | 4-F-Ph | MeSO₂NHCH₂CH₂CH₂ |

| | | | | | | | |
|---|---|---|---|---|---|---|---|
| ^{a} Cy¹ = 1,3-C₆H₄ means 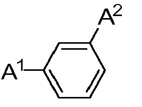 Cy¹ = 1,4-C₆H₄ means 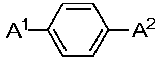 Cy¹ = 1,3-(4-F)C₆H₃ means 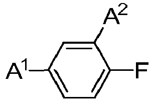 Cy¹ = 2,6-(5-Cl)-pyridyl means 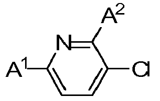 | | | | | | | |

**TABLE 2**

| | | | | | | | |
|---|---|---|---|---|---|---|---|
| 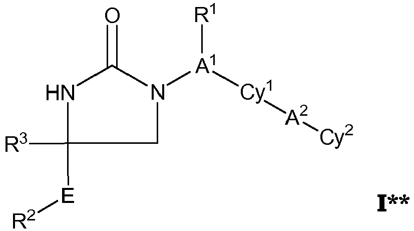 | | | | | | | |

| Prophetic Example No. | A¹-R¹ | Cy¹ | A² | Cy² | E | R² | R³ |
|---|---|---|---|---|---|---|---|
| * 1b | CHMe | 3-Me-Ph | bond | H | bond | Ph | Me |
| * 2b | CHMe | 3-Br-Ph | bond | H | bond | Ph | Me |
| 3b | bond | 1,3-C₆H₄ | bond | Ph | bond | Ph | Me |
| 4b | bond | 1,3-C₆H₄ | bond | 2-Cl-Ph | bond | Ph | Me |
| 5b | bond | 1,3-C₆H₄ | bond | 2-NC-Ph | bond | Ph | Me |
| 6b | bond | 1,3-C₆H₄ | bond | 2-MeO-Ph | bond | Ph | Me |
| 7b | bond | 1,3-C₆H₄ | bond | 2,6-diCl-Ph | bond | Ph | Me |
| 8b | bond | 1,3-C₆H₄ | bond | 2,4-diF-Ph | bond | Ph | Me |
| 9b | bond | 1,3-C₆H₄ | bond | 3-Cl-Ph | bond | Ph | Me |
| 10b | bond | 1,3-C₆H₄ | bond | 3-F-Ph | bond | Ph | Me |
| 11b | bond | 1,3-C₆H₄ | bond | 2,5-diF-Ph | bond | Ph | Me |
| 12b | bond | 1,3-C₆H₄ | bond | 3,5-diF-Ph | bond | Ph | Me |
| 13b | bond | 1,3-C₆H₄ | bond | 4-F-Ph | bond | Ph | Me |
| 14b | bond | 1,3-C₆H₄ | bond | 2-F-Ph | bond | Ph | Me |
| 15b | bond | 2,6-pyridyl | bond | 2-Cl-4-F-Ph | bond | 2-F-Ph | HOCH₂CH₂ |
| 16b | CHMe | 1,4-C₆H₄ | bond | 2,4-diF-Ph | bond | 4-F-Ph | H₂NC(=O)CH₂ |
| 17b | CHMe | 1,4-C₆H₄ | bond | 2,4-diF-Ph | bond | 4-F-Ph | HOCH₂CH(OH)CH₂ |
| 18b | bond | 1,3-C₆H₄ | bond | 2,4-diF-Ph | bond | Ph | allyl |
| 19b | bond | 1,3-C₆H₄ | bond | 2,4-diF-Ph | bond | Ph | HOCH₂CH₂ |
| 20b | bond | 1,3-(4-F)C₆H₃ | bond | 4-F-Ph | bond | 4-F-Ph | HOCH₂CH₂ |
| 21b | bond | 1,3-(4-F)C₆H₃ | bond | 4-F-Ph | bond | 2-F-Ph | HOCH₂CH₂ |
| 22b | bond | 1,3-C₆H₄ | bond | 2-Cl-4-F-Ph | bond | Ph | HOCH₂CH₂ |
| 23b | bond | 1,3-C₆H₄ | bond | 2,6-diCl-Ph | bond | Ph | HOCH₂CH₂ |
| 24b | bond | 1,3-C₆H₄ | bond | 2,4-diF-Ph | bond | Ph | H₂NC(=O)CH₂ |
| 25b | CH | 1,3-C₆H₄ | bond | 2,4-diF-Ph | bond | Ph | HOCH₂CH(OH)CH₂ |
| 26b | bond | 1,3-C₆H₄ | bond | 2,4-diF-Ph | bond | Ph 3-Cl- | HOCH₂CH₂CH₂ |
| 27b | bond | 1,3-C₆H₄ | bond | Ph | bond | Ph 2- | Me |
| 28b | bond | 1,3-C₆H₄ | bond | 2,4-diF-Ph | bond | pyridyl | Me |
| * 29b | CHMe | Ph | bond | H | bond | Ph | Me |
| * 30b | CHMe | 3-MeO-Ph | bond | H | bond | Ph | Me |
| * 31b | CHMe | 4-MeO-Ph | bond | H | bond | Ph | Me |
| * 32b | CHMe | Ph | bond | H | bond | 2-Me-Ph | Me |
| * 33b | CHMe | Ph | bond | H | bond | 4-Me-Ph | Me |
| * 34b | CHMe | Ph | bond | H | bond | 4-MeS-Ph | Me |
| * 35b | CHMe | Ph | bond | H | bond | 4-F-Ph | allyl |
| 36b | bond | 1,3-C₆H₄ | bond | 4-F-Ph | bond | Ph | HOCH₂CH₂ |
| * 37b | CHMe | Ph | bond | H | bond | 4-F-Ph | HOCH₂CH₂ |
| 38b | bond | 1,3-C₆H₄ | bond | 2,4-diF-Ph | bond | Ph | MeSO₂NHCH₂CH₂ |
| 39b | bond | 1,3-(4-F)C₆H₃ | bond | 2-Cl-4-F-Ph | bond | 4-F-Ph | HOCH₂CH₂ |
| 40b | bond | 1,3-C₆H₄ | bond | 2-Cl-4-F-Ph | bond | Ph | HOCH₂CH₂ |
| 41b | bond | 2,6-pyridyl | bond | 4-F-Ph | bond | Ph | HOCH₂CH₂ |
| 42b | bond | 2,6-pyridyl | bond | 4-F-Ph | bond | 4-F-Ph | HOCH₂CH₂ |
| 43b | bond | 2,6-pyridyl | bond | 4-F-Ph | bond | 2-F-Ph | HOCH₂CH₂ |
| 44b | bond | 1,3-(4-F)C₆H₃ | bond | 2,4-diF-Ph | bond | 4-F-Ph | HOCH₂CH₂ |
| 45b | bond | 1,3-(4-F)C₆H₃ | bond | 2,4-diF-Ph | bond | 2-F-Ph | HOCH₂CH₂ |
| 46b | bond | 2,6-pyridyl | bond | 2,4-diF-Ph | bond | Ph | HOCH₂CH₂ |
| 47b | bond | 2,6-pyridyl | bond | 2,4-diF-Ph | bond | 4-F-Ph | HOCH₂CH₂ |
| 48b | bond | 2,6-pyridyl | bond | 2,4-diF-Ph | bond | 2-F-Ph | HOCH₂CH₂ |
| * 49b | CHMe | 4-Br-Ph | bond | H | bond | 4-F-Ph | allyl |
| 50b | CHMe | 1,4-C₆H₄ | bond | 2,4-diF-Ph | bond | 4-F-Ph | allyl |
| 51b | CHMe | 1,4-C₆H₄ | bond | 2,4-diF-Ph | bond | 4-F-Ph | HOCH₂CH₂ |
| * 52b | CHMe | Ph | bond | H | bond | 4-F-Ph | vinyl |
| * 53b | CHMe | 4-Br-Ph | bond | H | bond | 4-F-Ph | HOCH₂CH₂ |
| 54b | bond | 2,6-pyridyl | bond | 2-Cl-4-F-Ph | bond | Ph | HOCH₂CH₂ |
| 55b | bond | 2,6-pyridyl | bond | 2-Cl-4-F-Ph | bond | 4-F-Ph | HOCH₂CH₂ |
| 56b | CHMe | 1,4-C₆H₄ | bond | 4-F-Ph | bond | 4-F-Ph | HOCH₂CH₂ |
| * 57b | CHMe | c-hex | bond | H | bond | 4-F-Ph | allyl |
| * 58b | CHMe | c-hex | bond | H | bond | 4-F-Ph | HOCH₂CH₂CH₂ |
| 59b | CHMe | 1,4-C₆H₄ | bond | c-Pr | bond | 4-F-Ph | allyl |
| * 60b | CHMe | 4-MeO₂C-Ph | bond | H | bond | 4-F-Ph | allyl |
| 61b | CHMe | 1,3-C₆H₄ | bond | c-Pr | bond | 4-F-Ph | HOCH₂CH₂CH₂ |
| * 62b | CHMe | 4-MeO₂C-Ph | bond | H | bond | 4-F-Ph | HOCH₂CH₂CH₂ |
| * 63b | CHEt | 4-Br-Ph | bond | H | bond | 4-F-Ph | allyl |
| 64b | bond | 2,6-(5-Cl)-pyridyl | bond | 4-F-Ph | bond | 2-F-Ph | HOCH₂CH₂ |
| 65b | CHMe | 1,4-C₆H₄ | bond | 2,4-diF-Ph | bond | 4-F-Ph | H₂NCH₂CH₂ |
| 66b | bond | 2,6-(5-Cl)-pyridyl | bond | 2,4-diF-Ph | bond | 2-F-Ph | HOCH₂CH₂ |
| 67b | CHMe | 1,4-C₆H₄ | bond | 2,4-diF-Ph | bond | 4-F-Ph | HOCH₂CH₂CH₂ |
| 68b | CHMe | 1,4-C₆H₄ | bond | 2,4-diF-Ph | bond | 4-F-Ph | MeCH(OH)CH₂ |
| 69b | CHMe | 1,4-C₆H₄ | bond | 2,4-diF-Ph | bond | 4-F-Ph | MeC(=O)CH₂ |
| 70b | CHMe | 1,4-C₆H₄ | bond | 2,4-diF-Ph | bond | 4-F-Ph | HOC(Me)₂CH₂ |
| 71b | CHMe | 1,4-C₆H₄ | bond | 2,4-diF-Ph | bond | 4-F-Ph | MeOCH₂CH₂ |
| 72b | CHMe | 1,4-C₆H₄ | bond | 2,4-diF-Ph | bond | 4-F-Ph | MeNHC(=O)NHCH₂CH₂ |
| * 73b | CHMe | 4-Br-Ph | bond | H | bond | 4-F-Ph | HOCH₂CH₂ |
| * 74b | CHMe | 4-Br-Ph | bond | H | bond | 4-F-Ph | HOCH₂CH(OH)CH₂ |
| 75b | CHMe | 1,4-C₆H₄ | bond | 2,4-diF-Ph | bond | 4-F-Ph | H₂NCOCH₂CH₂ |
| 76b | CHMe | 1,4-C₆H₄ | bond | 2,4-diF-Ph | bond | 4-F-Ph | MeNHC(=O)CH₂CH₂ |
| 77b | CHMe | 1,4-C₆H₄ | bond | 2,4-diF-Ph | bond | 4-F-Ph | MeCONHCH₂CH₂ |
| 78b | CHMe | 1,4-C₆H₄ | bond | 2,4-diF-Ph | bond | 4-F-Ph | MeNHC(=O)OCH₂CH₂ |
| 79b | CHMe | 1,4-C₆H₄ | bond | 2,4-diF-Ph | bond | 4-F-Ph | H₂NSO₂NHCH₂CH₂ |
| 80b | CHMe | 1,4-C₆H₄ | bond | 2,4-diF-Ph | bond | 4-F-Ph | H₂NSO₂OCH₂CH₂ |
| 81b | CHMe | 1,4-C₆H₄ | bond | 2,4-diF-Ph | bond | 4-F-Ph | (HO)₂P(=O)OCH₂CH₂ |
| 82b | CHMe | 1,4-C₆H₄ | bond | 2,4-diF-Ph | bond | 4-F-Ph | H₂NCH₂C(=O)NHCH₂CH₂ |
| * 83b | CHMe | 4-HOCH₂-Ph | bond | H | bond | 4-F-Ph | HOCH₂CH₂CH₂ |
| * 84b | CHMe | 4-HOC(Me)₂-Ph | bond | H | bond | 4-F-Ph | allyl |
| * 85b | CHMe | 4-Br-Ph | bond | H | bond | 2-thienyl | allyl |
| 86b | CHMe | 1,4-C₆H₄ | bond | 4-F-Ph | bond | Ph | HOCH₂CH₂ |
| 87b | CHMe | 1,4-C₆H₄ | bond | 4-F-Ph | bond | 2-thienyl | allyl |
| 88b | CHMe | 1,4-C₆H₄ | bond | 4-F-Ph | bond | Ph | HOCH₂CH₂CH₂ |
| 89b | CHMe | 1,4-C₆H₄ | bond | 2,4-diF-Ph | bond | Ph | HOCH₂CH₂ |
| 90b | CHMe | 1,4-C₆H₄ | bond | 2,4-diF-Ph | bond | 2- thienyl | allyl |
| 91b | CHMe | 1,4-C₆H₄ | bond | 4-F-Ph | bond | 2- thienyl | HOCH₂CH₂CH₂ |
| 92b | CHMe | 1,4-C₆H₄ | bond | 4-F-Ph | bond | 2- thienyl | MeCH(OH)CH₂ |
| 93b | CHMe | 1,4-C₆H₄ | bond | 4-F-Ph | bond | Ph | HOCH₂CH(OH)CH₂ |
| 94b | CHMe | 1,4-C₆H₄ | bond | 2,4-diF-Ph | bond | Ph | HOCH₂CH₂CH₂ |
| 95b | CHMe | 1,4-C₆H₄ | bond | 2,4-diF-Ph | bond | Ph | MeCH(OH)CH₂ |
| 96b | CHMe | 1,4-C₆H₄ | bond | 2,4-diF-Ph | bond | 2- thienyl | HOCH₂CH₂CH₂ |
| 97b | CHMe | Ph | bond | 2,4-diF-Ph | bond | 4-F-Ph | NCCH₂CH₂ |
| 98b | CHMe | 1,4-C₆H₄ | bond | 2,4-diF-Ph | bond | Ph | HOCH₂CH(OH)CH₂ |
| 99b | CHEt | 1,4-C₆H₄ | bond | 2,4-diF-Ph | bond | 4-F-Ph | HOCH₂CH₂ |
| 100b | CHMe | 1,4-C₆H₄ | bond | 2,4-diF-Ph | bond | 4-F-Ph | HOC(=O)CH₂CH₂ |
| 101b | CHMe | 1,4-C₆H₄ | bond | 2,4-diF-Ph | bond | 4-F-Ph | HOCH₂CH₂NHCH₂CH₂ |
| 102b | CHMe | 1,4-C₆H₄ | bond | 2,4-diF-Ph | bond | 4-F-Ph | HOCH2C(=O)NHCH₂CH₂ |
| 103b | CHMe | 1,4-C₆H₄ | bond | 2,4-diF-Ph | bond | 4-F-Ph | MeOC(=O)NHCH2CH2 |
| 104b | CHMe | 1,4-C₆H₄ | bond | 2,4-diF-Ph | bond | 4-F-Ph | 2-(4-morpholino)ethyl |
| 105b | CHMe | 1,4-C₆H₄ | bond | 2,4-diF-Ph | bond | 4-F-Ph | EtNHCONHCH₂CH₂ |
| 106b | CHMe | 1,4-C₆H₄ | bond | 2,4-diF-Ph | bond | 4-F-Ph | MeNHC(=NCN)NHCH₂CH₂ |
| 107b | CHMe | 1,4-C₆H₄ | bond | 2,4-diF-Ph | bond | 4-F-Ph | MeSO₂NHCH₂CH₂CH₂ |
| * 108b | CHMe | 4-Cl-Ph | bond | H | bond | i-Pr | HOCH₂CH₂CH₂ |
| * 109b | CHMe | 4-Me-Ph | bond | H | bond | 4-F-Ph | allyl |
| * 110b | CHMe | 4-MeO-Ph | bond | H | bond | Ph | HOCH₂CH₂ |
| * 111b | CHMe | 4-MeO-Ph | bond | H | bond | 4-F-Ph | allyl |
| * 112b | CHMe | 4-HOCH₂-Ph | bond | H | bond | Ph | HOCH₂CH₂CH₂ |
| * 113b | CHMe | 4-MeO-Ph | bond | H | bond | 4-F-Ph | HOCH₂CH₂ |
| * 114b | CHMe | 4-Cl-Ph | bond | H | bond | 4-F-Ph | allyl |
| * 115b | CHMe | c-hex | bond | H | bond | Ph | HOCH₂CH(OH)CH₂ |
| * 116b | CHMe | 4-HOCH₂CH₂-P | Ph bond | H | bond | Ph | HOCH₂CH₂CH₂ |
| * 117b | CHMe | 4-MeOCH₂-Ph | bond | H | bond | Ph | HOCH₂CH₂CH₂ |
| * 118b | CHMe | 4-Br-Ph | bond | H | bond | i-Pr | HOCH₂CH₂CH₂ |
| * 119b | CHMe | 4-Cl-Ph | bond | H | bond | 4-F-Ph | HOCH₂CH₂CH₂ |
| * 120b | CHMe | 4-Cl-Ph | bond | H | bond | 4-F-Ph | MeCH(OH)CH₂ |
| * 121b | CHMe | 4-Br-Ph | bond | H | bond | Ph | allyl |
| 122b | CHMe | 1,4-C₆H₄ | bond | 3-pyridyl | bond | Ph | HOCH₂CH₂ |
| * 123b | CHMe | 4-MeO-Ph | bond | H | bond | 4-F-Ph | HOCH₂CH(OH)CH₂ |
| * 124b | CHMe | 1,4-C₆H₄ | bond | 2,4-diF-Ph | bond | i-Pr | HOCH₂CH₂ |
| * 125b | bond | 1-(*t-*BuOC(=O))pyrrolidin-3-yl | bond | H | bond | Ph | HOCH₂CH₂CH₂ |
| 126b | CHMe | 1,4-C₆H₄ | bond | 2,4-diF-Ph | bond | 4-F-Ph | MeSO₂NHCH₂CH₂ |
| 127b | CHMe | 1,4-C₆H₄ | bond | 4-pyridyl | bond | Ph | HOCH₂CH₂CH₂ |
| 128b | CHMe | 1,4-C₆H₄ | bond | 3-pyridyl | bond | Ph | HOCH₂CH₂CH₂ |
| * 129b | CHMe | 1,4-C₆H₄ | bond | 2,4-diF-Ph | bond | i-Pr | HOCH₂CH₂CH₂ |
| 130b | CHMe | 1,4-C₆H₄ | bond | 3-pyridyl | bond | 4-F-Ph | HOCH₂CH₂ |
| 131b | CHMe | 1,4-C₆H₄ | bond | 2-thienyl | bond | Ph | HOCH₂CH₂CH₂ |
| 132b | CHMe | 1,4-C₆H₄ | bond | 4-morpholinyl | bond | 4-F-Ph | allyl |
| 133b | CHMe | 1,4-C₆H₄ | bond | 4-F-Ph | bond | 2- thienyl | HOCH₂CH₂ |
| 134b | CHMe | 1,4-C₆H₄ | bond | 4-F-Ph | bond | Ph | NCCH₂CH₂ |
| * 135b | CHEt | 4-Br-Ph | bond | H | bond | Ph | HOCH₂CH₂CH₂ |
| 136b | CHMe | 1,4-C₆H₄ | bond | 2,4-diF-Ph | bond | 4-F-Ph | HOCH₂CH₂CH₂ |
| 137b | CHMe | 1,4-C₆H₄ | bond | 1-oxo-3-pyridyl | bond | Ph | HOCH₂CH₂CH₂ |
| * 138b | CHMe | 1,4-C₆H₄ | bond | 2,4-diF-Ph | bond | i-Pr | HOCH₂CH(OH)CH₂ |
| 139b | CHMe | 1,4-C₆H₄ | bond | 4-F-Ph | bond | Ph | MeCH(OH)CH₂ |
| 140b | CHMe | 1,4-C₆H₄ | bond | 3-pyridyl | bond | 4-F-Ph | HOCH₂CH₂CH₂ |
| 141b | CHMe | 1,4-C₆H₄ | bond | 2,4-diF-Ph | bond | Ph | Pr |
| * 142b | CHMe | 4-Br-Ph | bond | H | bond | 4-F-Ph | HOCH₂CH₂CH₂ |
| 143b | CHMe | 1,4-C₆H₄ | bond | 2,4-diF-Ph | bond | 4-F-Ph | MeSO₂CH₂CH₂ |
| 144b | CHMe | 1,4-C₆H₄ | bond | 5-Me-1,3,4-thiadiazol-2-yl | bond | 4-F-Ph | allyl |
| 145b | CHMe | 1,4-C₆H₄ | bond | 4-F-Ph | bond | 2- thienyl | HOCH₂CH₂CH₂ |
| 146b | CHMe | 1,4-C₆H₄ | bond | 2,4-diF-Ph | bond | 2- thienyl | HOCH₂CH₂ |
| 147b | CHMe | 1,4-C₆H₄ | bond | 4-F-Ph | bond | Ph | H₂NCOCH₂CH₂ |
| 148b | CHMe | 1,4-C₆H₄ | bond | 2-MeO-5-pyridyl | bond | Ph | HOCH₂CH₂CH₂ |
| 149b | CHMe | 1,4-C₆H₄ | bond | 3-pyridyl | bond | 4-F-Ph | H₂NCOCH₂CH₂ |
| 150b | CHEt | 1,4-C₆H₄ | bond | 4-F-Ph | bond | Ph | HOCH₂CH₂CH₂ |
| 151b | CHMe | 1,4-C₆H₄ | bond | 4-F-Ph | bond | Ph | HOC(Me)₂CH₂ |
| * 152b | CHEt | 4-Br-Ph | bond | H | bond | Ph | HOCH₂CH(OH)CH₂ |
| * 153b | CHMe | 4-Br-Ph | bond | H | bond | 4-F-Ph | H₂NCOCH₂CH₂ |
| * 154b | CHEt | 4-Br-Ph | bond | H | bond | 4-F-Ph | HOCH₂CH₂CH₂ |
| 155b | CHMe | 1,4-C₆H₄ | bond | 2,4-diF-Ph | bond | 4-F-Ph | NCCH₂ |
| 156b | CHMe | 1,4-C₆H₄ | bond | 2,4-diMe-5-thiazolyl | bond | 4-F-Ph | allyl |
| 157b | CHMe | 1,4-C₆H₄ | bond | 4-F-Ph | bond | 4-F-Ph | HOCH₂CH₂CH₂ |
| 158b | CHMe | 1,4-C₆H₄ | bond | 4-F-Ph | bond | 2-F-Ph | HOCH₂CH₂CH₂ |
| 159b | CHMe | 1,4-C₆H₄ | bond | 4-F-Ph | bond | 3-F-Ph | HOCH₂CH₂CH₂ |
| 160b | CHMe | 1,4-C₆H₄ | bond | 4-F-Ph | bond | Ph | HOC(Me)₂CH₂CH₂ |
| 161b | CHMe | 1,4-C₆H₄ | bond | 5-MeCO-2-thienyl | bond | Ph | HOCH₂CH₂CH₂ |
| 162b | CHMe | 1,4-C₆H₄ | bond | 2,4-diF-Ph | bond | Ph | H₂NCOCH₂CH₂ |
| 163b | CHMe | 1,4-C₆H₄ | bond | 5-(H₂NCHMe)-2-thienyl | bond | Ph | HOCH₂CH₂CH₂ |
| 164b | CHEt | 1,4-C₆H₄ | bond | 4-F-Ph | bond | 4-F-Ph | HOCH₂CH₂CH₂ |
| 165b | CHEt | 1,4-C₆H₄ | bond | 2,4-diF-Ph | bond | Ph | HOCH₂CH₂CH₂ |
| 166b | CHMe | 1,4-C₆H₄ | bond | 5-(HOCHMe)-2-thienyl | bond | Ph | HOCH₂CH₂CH₂ |
| * 167b | CHEt | 4-Br-Ph | bond | H | bond | 4-F-Ph | HOCH₂CH(OH)CH₂ |
| 168b | CHMe | 1,4-C₆H₄ | bond | 2,4-diF-Ph | bond | 4-F-Ph | H₂NCH₂CH₂CH₂ |
| 169b | CHMe | 1,4-C₆H₄ | bond | 2,4-diF-Ph | bond | 4-F-Ph | MeNHCH₂CH₂ |
| 170b | CHMe | 1,4-C₆H₄ | bond | 3-(CF₃)-1-pyrazolyl | bond | 4-F-Ph | allyl |
| 171b | CHMe | 1,4-C₆H₄ | bond | 2,4-diF-Ph | bond | Ph | HOC(Me)₂CH₂CH₂ |
| 172b | CHEt | 1,4-C₆H₄ | bond | 2,4-diF-Ph | bond | 4-F-Ph | HOCH₂CH₂CH₂ |
| 173b | CHMe | 1,4-C₆H₄ | bond | 2,4-diF-Ph | bond | 4-F-Ph | MeSCH₂CH₂ |
| 174b | CHMe | Ph | bond | 2,4-diF-Ph | bond | 4-F-Ph | H₂NC(=O)NHCH₂CH₂ |
| 175b | CHMe | 1,4-C₆H₄ | bond | 2,4-diF-Ph | bond | 4-F-Ph | H₂NC(=O)OCH₂CH₂ |
| 176b | CHMe | 1,4-C₆H₄ | bond | 2,4-diF-Ph | bond | 4-F-Ph | HOCH₂CH₂OCH₂CH₂ |
| 177b | CHMe | 1,4-C₆H₄ | bond | 2,4-diF-Ph | bond | 4-F-Ph | 2-(1-imidazolyl)ethyl |
| 178b | CHMe | 1,4-C₆H₄ | bond | 2,4-diF-Ph | bond | 4-F-Ph | MeCONMeCH₂CH₂ |
| 179b | CHMe | 1,4-C₆H₄ | bond | 4-F-Ph | bond | Ph | MeSO₂NHCH₂CH₂CH₂ |
| 180b | CHMe | 1,4-C₆H₄ | bond | 2,4-diF-Ph | bond | 4-F-Ph | H₂NC(=O)NHCH₂CH₂CH₂ |
| 181b | CHMe | 1,4-C₆H₄ | bond | 2,4-diF-Ph | bond | 4-F-Ph | H₂NC(=O)OCH₂CH₂CH₂ |
| 182b | CHMe | 1,4-C₆H₄ | bond | 2,4-diF-Ph | bond | 4-F-Ph | 2-(1-aminoimidazol-1-yl)ethyl |
| 183b | CHMe | 1,4-C₆H₄ | bond | 2,4-diF-Ph | bond | 4-F-Ph | MeNHC(=O)NHCH₂CH₂CH₂ |
| 184b | CHMe | 1,4-C₆H₄ | bond | 2,4-diF-Ph | bond | 4-F-Ph | H₂NC(=O)NHCH₂CH(OH)CH₂ |
| 185b | CHMe | 1,4-C₆H₄ | bond | 4-F-Ph | bond | 4-F-Ph | MeSO₂NHCH₂CH(OH)CH₂ |
| 186b | CHMe | 1,4-C₆H₄ | bond | 4-F-Ph | bond | 4-F-Ph | MeSO₂NMeCH₂CH(OH)CH₂ |
| 187b | CHMe | 1,4-C₆H₄ | bond | 6-CF₃-3-pyridyl | bond | 4-F-Ph | HOCH₂CH₂CH₂ |
| * 188b | CHMe | 4-MeO-Ph | bond | H | bond | Ph | HOCH₂CH₂CH₂ |
| * 189b | CHMe | 3-F-Ph | bond | H | bond | 4-F-Ph | HOCH₂CH₂CH₂ |
| * 190b | CHMe | 2-F-Ph | bond | H | bond | 4-F-Ph | HOCH₂CH₂CH₂ |
| * 191b | CHMe | 4-F-Ph | bond | H | bond | 4-F-Ph | HOCH₂CH₂CH₂ |
| * 192b | CHMe | 4-MeO-Ph | bond | H | bond | Ph | HOCH₂CH(OH)CH₂ |
| * 193b | CHMe | 4-Cl-Ph | bond | H | bond | Ph | H₂NCOCH₂CH₂ |
| * 194b | CHMe | 4-MeO-Ph | bond | H | bond | 4-F-Ph | H₂NCOCH₂CH₂ |
| * 195b | CHMe | 4-F₂HCO-Ph | bond | H | bond | 4-F-Ph | allyl |
| 196b | CHMe | Ph | bond | 3-pyrazolyl | bond | Ph | HOCH₂CH₂CH₂ |
| 197b | CHMe | 1,4-C₆H₄ | bond | 5-F-3-pyridyl | bond | Ph | allyl |
| * 198b | CHMe | 3-CF₃-Ph | bond | H | bond | 4-F-Ph | HOCH₂CH₂CH₂ |
| * 199b | CHMe | 4-CF₃-Ph | bond | H | bond | 4-F-Ph | HOCH₂CH₂CH₂ |
| 200b | CHMe | 1,4-C₆H₄ | bond | 3-pyridyl | bond | Ph | H₂NCOCH₂CH₂ |
| 201b | CHMe | 1,4-C₆H₄ | bond | 4-pyridyl | bond | Ph | H₂NCOCH₂CH₂ |
| 202b | CHMe | 1,4-C₆H₄ | bond | 4-F-Ph | bond | Ph | HOCH₂CH₂CH₂ |
| 203b | CHMe | 1,4-C₆H₄ | bond | 5-F-3-pyridyl | bond | Ph | HOCH₂CH₂CH₂ |
| * 204b | CHMe | 4-MeO-Ph | bond | H | bond | 4-F-Ph | MeSO₂NHCH₂CH₂ |
| 205b | CHMe | 1,4-C₆H₄ | bond | 5-F-3-pyridyl | bond | 4-F-Ph | HOCH₂CH₂CH₂ |
| 206b | CHMe | 1,4-C₆H₄ | bond | 4-F-Ph | bond | Ph | NCC(Me)2CH2 |
| 207b | CHMe | 1,4-C₆H₄ | bond | 6-MeO-3-pyridyl | bond | Ph | H₂NCOCH₂CH₂ |
| 208b | CHMe | 1,4-C₆H₄ | bond | 5-MeO-3-pyridyl | bond | 4-F-Ph | HOCH₂CH₂CH₂ |
| 209b | CHMe | 1,4-C₆H₄ | bond | 5-Cl-3-pyridyl | bond | 4-F-Ph | HOCH₂CH₂CH₂ |
| 210b | CHMe | 1,4-C₆H₄ | bond | 3-pyridyl | bond | Ph | MeSO₂NHCH₂CH₂ |
| * 211b | CHMe | 4-F₂HCO-Ph | bond | H | bond | 4-F-Ph | HOCH₂CH₂CH₂ |
| 212b | CHMe | 1,4-C₆H₄ | bond | 4-F-Ph | bond | Ph | (HO)₂P(=O)OCH₂CH₂CH₂ |
| 213b | CHMe | 1,4-C₆H₄ | bond | 2-Me-4-pyridyl | bond | 4-F-Ph | HOCH₂CH₂CH₂ |
| * 214b | CHMe | 1,4-C₆H₄ | bond | H | bond | Ph | HOCH₂CH₂CH₂ |
| 215b | CHMe | 1,4-C₆H₄ | bond | 1-Me-6-oxo-3-(1,6-dihydropyridyl) | bond | Ph | HOCH₂CH₂CH₂ |
| * 216b | CHMe | 4-MeO-Ph | bond | H | bond | 4-F-Ph | MeSO₂NHCH₂CH₂CH₂ |
| * 217b | CHMe | 4-MeO-Ph | bond | H | bond | Ph | H₂NCOCH₂CH₂ |
| * 218b | CHMe | 4-F-Ph | bond | H | bond | 4-F-Ph | H₂NCOCH₂CH₂ |
| * 219b | CHMe | c-hex | bond | H | bond | 4-F-Ph | H₂NCOCH₂CH₂ |
| 220b | bond | 1,3-(4-F)C₆H₃ | bond | 2,4-diF-Ph | bond | 4-F-Ph | HOCH₂CH₂ |
| * 221b | CHMe | c-hex | bond | H | bond | 4-F-Ph | MeSO₂NHCH₂CH₂CH₂ |

| | | | | | | | |
|---|---|---|---|---|---|---|---|
| ^{a} Cy¹ = 1,3-C₆H₄ means 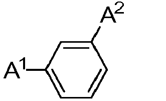 Cy¹ = 1,4-C₆H₄ means 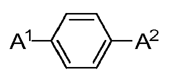 Cy¹ = 1,3-(4-F)C₆H₃ means 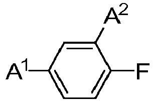 Cy¹ = 2,6-(5-Cl)-pyridyl means 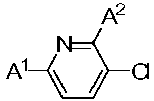 * Reference example | | | | | | | |

All publications, patents and patent applications mentioned in this specification are herein incorporated by reference to the same extent as if each individual publication or patent application were specifically and individually designated as having been incorporated by reference. It is understood that the examples and embodiments described herein are for illustrative purposes only, and it will be appreciated that the invention is susceptible to modification, variation and change without departing from the proper scope or fair meaning of the appended claims.

## Claims

1. A compound of Formula (I): 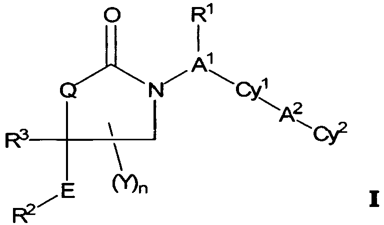 wherein:
R¹ is (a) absent or (b) selected from (C₁-C₆)alkyl, (C₂-C₆)alkenyl, (C₂-C₆)alkynyl and (C₁-C₃)alkoxy(C₁-C₃)alkyl, wherein each is optionally substituted with up to four groups independently selected from fluorine, cyano, oxo, R⁴, R⁴O-, (R⁴)₂N-, R⁴O₂C-, R⁴S, R⁴S(=O)-, R⁴S(=O)₂-, R⁴C(=O)NR⁴-, (R⁴)₂NC(=O)-, (R⁴)₂NC(=O)O-, (R⁴)₂NC(O)NR⁴-, R⁴OC(=O)NR⁴-, (R⁴)₂NC(=NCN)NR⁴-, (R⁴O)₂P(=O)O-, (R⁴O)₂P(=O)NR⁴-, R⁴OS(=O)₂NR⁴-, (R⁴)₂NS(=O)₂O-, (R⁴)₂NS(=O)₂NR⁴-, R⁴S(=O)₂NR⁴-, R⁴S(=O)₂NHC(=O)-, R⁴S(=O)₂NHC(=O)O-, R⁴S(-O)₂NHC(=O)NR⁴-, R⁴OS(=O)₂NHC(=O)-, R⁴OS(=O)₂NHC(=O)O-, R⁴OS(=O)₂NHC(=O)NR⁴-, (R⁴)₂NS(=O)₂NHC(=O)-, (R⁴)₂NS(=O)₂NHC(=O)O-, (R⁴)₂NS(=O)₂NHC(=O)NR⁴-, R⁴C(=O)NHS(=O)₂-, R⁴C(=O)NHS(=O)₂O-, R⁴C(=O)NHS(=O)₂NR⁴-, R⁴OC(=O)NHS(=O)₂-, R⁴OC(=O)NHS(=O)₂O-, R⁴OC(=O)NHS(=O)₂NR⁴-, (R⁴)₂NC(=O)NHS(=O)₂-, (R⁴)₂NC(=O)NHS(=O)₂O-, (R⁴)₂NC(=O)NHS(=O)₂NR⁴-, aryl, cycloalkyl, heterocyclyl, heteroaryl, arylamino and heteroarylamino;
A¹ is (a) a bond, or (b) (C₁-C₃)alkylene, CH₂CH₂O, wherein the oxygen is attached to Cy¹, or CH₂C(=O), wherein the carbonyl carbon is attached to Cy¹;
Cy¹ is aryl, heteroaryl, monocyclic cycloalkyl or heterocyclyl, wherein each is optionally substituted with 1 to 4 groups independently selected from fluorine, chlorine, bromine, iodine, cyano, nitro, amino, hydroxy, carboxy, (C₁-C₆)alkyl, hydroxy(C₁-C₆)alkyl, (C₃-C₆)cycloalkyl, hydroxy(C₃-C₆)cycloalkyl, (C₄-C₇)cycloalkylalkyl, (C₂-C₆)alkenyl, halo(C₂-C₆)alkenyl, hydroxy(C₂-C₆)alkenyl, (C₂-C₆)alkynyl, (C₃-C₆)cycloalkyl(C₂-C₄)alkynyl, halo(C₁-C₆)alkyl, halo(C₃-C₆)cycloalkyl, halO(C₄-C₇)cycloalkylalkyl, (C₁-C₆)alkoxy, (C₃-C₆)cycloalkoxy, (C₄-C₇)cycloalkylalkoxy, halo(C₁-C₆)alkoxy, halo(C₃-C₆)cycloalkoxy, halO(C₄-C₇)cycloalkylalkoxy, (C₁-C₆)alkylthio, (C₃-C₆)cycloalkythio, (C₄-C₇)cycloalkylalkylthio, halo(C₁-C₆)alkylthio, halo(C₃-C₆)cycloalkythio, halo(C₄-C₇)cycloalkylalkylthio, (C₁-C₆)alkanesulfinyl, (C₃-C₆)cycloalkanesulfinyl, (C₄-C₇)cycloalkylalkanesulfinyl, halo(C₁-C₆)alkanesulfinyl, halo(C₃-C₆)cycloalkanesulfinyl, halo(C₄-C₇)cycloalkylalkanesulfinyl, (C₁-C₆)alkanesulfonyl, (C₃-C₆)cycloalkanesulfonyl, (C₄-C₇)cycloalkylalkanesulfonyl, halo(C₁-C₆)alkanesulfonyl, halo(C₃-C₆)cycloalkanesulfonyl, halo(C₄-C₇)cyclo-alkylalkanesulfonyl, (C₁-C₆)alkylamino, di(C₁-C₆)alkylamino, (C₁-C₆)alkoxy(C₁-C₆)alkoxy, halo(C₁-C₆)alkoxy(C₁-C₆)alkoxy, (C₁-C₆)alkoxycarbonyl, H₂NCO, H₂NSO₂, (C₁-C₆)alkylaminocarbonyl, di(C₁-C₆)alkylaminocarbonyl, (C₁-C₃)alkoxy(C₁-C₃)alkylaminocarbonyl, heterocyclylcarbonyl, (C₁-C₆)alkylaminosulfonyl, di(C₁-C₆)alkylaminosulfonyl, heterocyclosulfonyl, (C₁-C₆)alkylcarbonylamino, (C₁-C₆)alkylcarbonylamino(C₁-C₆)alkyl, (C₁-C₆)alkylsulfonylamino, (C₁-C₆)alkylsulfonylamino(C₁-C₆)alkyl, (C₁-C₆)alkoxycarbonyl(C₁-C₆)alkoxy, (C₁-C₆)alkoxy(C₁-C₆)alkyl, halo(C₁-C₆)alkoxy(C₁-C₆)alkyl, hydroxy(C₁-C₆)alkoxy, heteroaryl oxo, amino(C₁-C₆)alkyl, (C₁-C₆)alkylamino(C₁-C₆)alkyl, di(C₁-C₆)alkylamino(C₁-C₆)alkyl amino(C₂-C₆)alkoxy, (C₁-C₆)alkylamino(C₂-C₆)alkoxy, di(C₁-C₆)alkylamino(C₂-C₆)alkoxy, (C₁-C₆)alkylcarbonyl, (C₃-C₆)cycloalkylcarbonyl, (C₃-C₆)cycloalkylaminocarbonyl, {(C₃-C₆)cycloalkyl} {(C₁-C₆)alkyl}aminocarbonyl, di(C₃-C₆)cycloalkylaminocarbonyl, (C₃-C₆)cycloalkylaminosulfonyl, {(C₃-C₆)cycloalkyl} {(C₁-C₆)alkyl}aminosulfonyl, di(C₃-C₆)cycloalkylaminosulfonyl, cyano(C₁-C₆)alkyl, aminocarbonyl(C₁-C₆)alkyl, (C₁-C₆)alkylaminocarbonyl(C₁-C₆)alkyl, di(C₁-C₆)alkylaminocarbonyl(C₁-C₆)alkyl, (C₃-C₆)cycloalkylaminocarbonyl(C₁-C₆)alkyl, {(C₃-C₆)cycloalkyl}{(C₁-C₆)alkyl}aminocarbonyl(C₁-C₆)alkyl and di(C₃-C₆)cycloalkylaminocarbonyl(C₁-C₆)alkyl;
A² is (a) a bond, O, S or NR⁴; or (b) (C₁-C₃)alkylene or (C₁-C₂)alkyleneoxy, each of which is optionally substituted with 1 to 4 groups independently selected from methyl, ethyl, trifluoromethyl or oxo;
Cy² is aryl, heteroaryl, cycloalkyl or heterocyclyl, wherein each is optionally substituted with 1 to 4 groups independently selected from fluorine, chlorine, bromine, iodine, cyano, nitro, amino, hydroxy, carboxy, (C₁-C₆)alkyl, hydroxy(C₁-C₆)alkyl, (C₃-C₆)cycloalkyl, hydroxy(C₃-C₆)cycloalkyl, (C₄-C₇)cycloalkylalkyl, (C₂-C₆)alkenyl, halo(C₂-C₆)alkenyl, hydroxy(C₂-C₆)alkenyl, (C₂-C₆)alkynyl, (C₃-C₆)cycloalkyl(C₂-C₄)alkynyl, halo(C₁-C₆)alkyl, halo(C₃-C₆)cycloalkyl, halo(C₄-C₇)cycloalkylalkyl, (C₁-C₆)alkoxy, (C₃-C₆)cycloalkoxy, (C₄-C₇)cycloalkylalkoxy, halo(C₁-C₆)alkoxy, halo(C₃-C₆)cycloalkoxy, halo(C₄-C₇)cycloalkylalkoxy, (C₁-C₆)alkylthio, (C₃-C₆)cycloalkythio, (C₄-C₇)cycloalkylalkylthio, halo(C₁-C₆)alkylthio, halo(C₃-C₆)cycloalkythio, halo(C₄-C₇)cycloalkylalkylthio, (C₁-C₆)alkanesulfinyl, (C₃-C₆)cycloalkanesulfinyl, (C₄-C₇)cycloalkylalkanesulfinyl, halo(C₁-C₆)alkanesulfinyl, halo(C₃-C₆)cycloalkanesulfinyl, halo(C₄-C₇)cycloalkylalkanesulfinyl, (C₁-C₆)alkanesulfonyl, (C₃-C₆)cycloalkanesulfonyl, (C₄-C₇)cycloalkylalkanesulfonyl, halo(C₁-C₆)alkanesulfonyl, halo(C₃-C₆)cycloalkanesulfonyl, halo(C₄-C₇)cyclo-alkylalkanesulfonyl, (C₁-C₆)alkylamino, di(C₁-C₆)alkylamino, (C₁-C₆)alkoxy(C₁-C₆)alkoxy, halo(C₁-C₆)alkoxy(C₁-C₆)alkoxy, (C₁-C₆)alkoxycarbonyl, H₂NCO, H₂NSO₂, (C₁-C₆)alkylaminocarbonyl, di(C₁-C₆)alkylaminocarbonyl, (C₁-C₃)alkoxy(C₁-C₃)alkylaminocarbonyl, heterocyclylcarbonyl, (C₁-C₆)alkylaminosulfonyl, di(C₁-C₆)alkylaminosulfonyl, heterocyclosulfonyl, (C₁-C₆)alkylcarbonylamino, (C₁-C₆)alkylcarbonylamino(C₁-C₆)alkyl, (C₁-C₆)alkylsulfonylamino, (C₁-C₆)alkylsulfonylamino(C₁-C₆)alkyl, (C₁-C₆)alkoxycarbonyl(C₁-C₆)alkoxy, (C₁-C₆)alkoxy(C₁-C₆)alkyl, halo(C₁-C₆)alkoxy(C₁-C₆)alkyl, hydroxy(C₁-C₆)alkoxy, heteroaryl, oxo, amino(C₁-C₆)alkyl, (C₁-C₆)alkylamino(C₁-C₆)alkyl, di(C₁-C₆)alkylamino(C₁-C₆)alkyl amino(C₂-C₆)alkoxy, (C₁-C₆)alkylamino(C₂-C₆)alkoxy, di(C₁-C₆)alkylamino(C₂-C₆)alkoxy, (C₁-C₆)alkylcarbonyl, (C₃-C₆)cycloalkylcarbonyl, (C₃-C₆)cycloalkylaminocarbonyl, {(C₃-C₆)cycloalkyl} {(C₁-C₆)alkyl}aminocarbonyl, di(C₃-C₆)cycloalkylaminocarbonyl, (C₃-C₆)cycloalkylaminosulfonyl, {(C₃-C₆)cycloalkyl} {(C₁-C₆)alkyl}aminosulfonyl, di(C₃-C₆)cycloalkylaminosulfonyl, cyano(C₁-C₆)alkyl, aminocarbonyl(C₁-C₆)alkyl, (C₁-C₆)alkylaminocarbonyl(C₁-C₆)alkyl, di(C₁-C₆)alkylaminocarbonyl(C₁-C₆)alkyl, (C₃-C₆)cycloalkylaminocarbonyl(C₁-C₆)alkyl, {(C₃-C₆)cycloalkyl}{(C₁-C₆)alkyl}aminocarbonyl(C₁-C₆)alkyl and di(C₃-C₆)cycloalkylaminocarbonyl(C₁-C₆)alkyl;
Y is (C₁-C₆)alkyl or halo(C₁-C₆)alkyl;
n is 0, 1 or 2;
E is (a) a bond or (b) (C₁-C₃)alkylene or (C₁-C₂)alkylenyloxy, wherein the O is attached to R², each of which is optionally substituted with 1 to 4 groups independently selected from methyl, ethyl, trifluoromethyl or oxo;
R² is aryl, heteroaryl, cycloalkyl or heterocyclyl, wherein each is optionally substituted with up to 4 groups independently selected from fluorine, chlorine, bromine, iodine, cyano, nitro, amino, hydroxy, carboxy, (C₁-C₆)alkyl, hydroxy(C₁-C₆)alkyl, (C₃-C₆)cycloalkyl, hydroxy(C₃-C₆)cycloalkyl, (C₄-C₇)cycloalkylalkyl, (C₂-C₆)alkenyl, halo(C₂-C₆)alkenyl, hydroxy(C₂-C₆)alkenyl, (C₂-C₆)alkynyl, (C₃-C₆)cycloalkyl(C₂-C₄)alkynyl, halo(C₁-C₆)alkyl, halo(C₃-C₆)cycloalkyl, halo(C₄-C₇)cycloalkylalkyl, (C₁-C₆)alkoxy, (C₃-C₆)cycloalkoxy, (C₄-C₇)cycloalkylalkoxy, halo(C₁-C₆)alkoxy, halo(C₃-C₆)cycloalkoxy, halo(C₄-C₇)cycloalkylalkoxy, (C₁-C₆)alkylthio, (C₃-C₆)cycloalkylthio, (C₄-C₇)cycloalkylalkylthio, halo(C₁-C₆)alkylthio, halo(C₃-C₆)cycloalkylthio, halo(C₄-C₇)cycloalkylalkylthio, (C₁-C₆)alkanesulfinyl, (C₃-C₆)cycloalkanesulfinyl, (C₄-C₇)cycloalkylalkanesulfinyl, halo(C₁-C₆)alkanesulfinyl, halo(C₃-C₆)cycloalkanesulfinyl, halo(C₄-C₇)cycloalkylalkanesulfinyl, (C₁-C₆)alkanesulfonyl, (C₃-C₆)cycloalkanesulfonyl, (C₄-C₇)cycloalkylalkanesulfonyl, halo(C₁-C₆)alkanesulfonyl, halo(C₃-C₆)cycloalkanesulfonyl, halo(C₄-C₇)cyclo-alkylalkanesulfonyl, (C₁-C₆)alkylamino, di(C₁-C₆)alkylamino, (C₁-C₆)alkoxy(C₁-C₆)alkoxy, halo(C₁-C₆)alkoxy(C₁-C₆)alkoxy, (C₁-C₆)alkoxycarbonyl, H₂NCO, H₂NSO₂, (C₁-C₆)alkylaminocarbonyl, di(C₁-C₆)alkylaminocarbonyl, (C₁-C₃)alkoxy(C₁-C₃)alkylaminocarbonyl, heterocyclylcarbonyl, (C₁-C₆)alkylaminosulfonyl, di(C₁-C₆)alkylaminosulfonyl, heterocyclosulfonyl, (C₁-C₆)alkylcarbonylamino, (C₁-C₆)alkylcarbonylamino(C₁-C₆)alkyl, (C₁-C₆)alkylsulfonylamino, (C₁-C₆)alkylsulfonylamino(C₁-C₆)alkyl, (C₁-C₆)alkoxycarbonyl(C₁-C₆)alkoxy, (C₁-C₆)alkoxy(C₁-C₆)alkyl, halo(C₁-C₆)alkoxy(C₁-C₆)alkyl, hydroxy(C₁-C₆)alkoxy, heteroaryl, oxo, amino(C₁-C₆)alkyl, (C₁-C₆)alkylamino(C₁-C₆)alkyl, di(C₁-C₆)alkylamino(C₁-C₆)alkyl amino(C₂-C₆)alkoxy, (C₁-C₆)alkylamino(C₂-C₆)alkoxy, di(C₁-C₆)alkylamino(C₂-C₆)alkoxy, (C₁-C₆)alkylcarbonyl, (C₃-C₆)cycloalkylcarbonyl, (C₃-C₆)cycloalkylaminocarbonyl, {(C₃-C₆)cycloalkyl} {(C₁-C₆)alkyl}aminocarbonyl, di(C₃-C₆)cycloalkylaminocarbonyl, (C₃-C₆)cycloalkylaminosulfonyl, {(C₃-C₆)cycloalkyl}{(C₁-C₆)alkyl}aminosulfonyl, di(C₃-C₆)cycloalkylaminosulfonyl, cyano(C₁-C₆)alkyl, aminocarbonyl(C₁-C₆)alkyl, (C₁-C₆)alkylaminocarbonyl(C₁-C₆)alkyl, di(C₁-C₆)alkylaminocarbonyl(C₁-C₆)alkyl, (C₃-C₆)cycloalkylaminocarbonyl(C₁-C₆)alkyl, {(C₃-C₆)cycloalkyl}{(C₁-C₆)alkyl}aminocarbonyl(C₁-C₆)alkyl and di(C₃-C₆)cycloalkylaminocarbonyl(C₁-C₆)alkyl;
R³ is selected from (C₂-C₆)alkyl, (C₂-C₆)alkenyl, (C₂-C₆)alkynyl and (C₁-C₃)alkoxy(C₁-C₃)alkyl, wherein the (C₂-C₆)alkyl is substituted with, and each of the (C₂-C₆)alkenyl, (C₂-C₆)alkynyl and (C₁-C₃)alkoxy(C₁-C₃)alkyl is optionally substituted with, up to four groups independently selected from fluorine, cyano, oxo, halo(C₁-C₆)alkyl, amino(C₁-C₆)alkyl, (C₁-C₆)alkylamino(C₁-C₆)alkyl, di(C₁-C₆)alkylamino(C₁-C₆)alkyl, hydroxy(C₁-C₆)alkyl, (C₁-C₆)alkoxy(C₁-C₆)alkyl, R⁴O-, (R⁴)₂N₋, R⁴O₂C-, R⁴S, R⁴S(=O)-, R⁴S(=O)₂-, R⁴C(O)NR⁴-, (R⁴)₂NC(=O)-, (R⁴)₂NC(=O)O-, (R⁴)₂NC(=O)NR⁴-, R⁴OC(-O)NR⁴-, (R⁴)₂NC(=NCN)NR⁴-, (R⁴O)₂P(=O)O-, (R⁴O)₂P(=O)NR⁴-, R⁴OS(=O)₂NR⁴-, (R⁴)₂NS(=O)₂O-, (R⁴)₂NS(=O)₂NR⁴-, R⁴S(=O)₂NR⁴-, R⁴S(=O)₂NHC(=O)-, R⁴S(=O)₂NHC(=O)O-, R⁴S(=O)₂NHC(=O)NR⁴-, R⁴OS(=O)₂NHC(=O)-, R⁴OS(=O)₂NHC(=O)O-, R⁴OS(=O)₂NHC(=O)NR⁴-, (R⁴)₂NS(=O)₂NHC(=O)-, (R⁴)₂NS(=O)₂NHC(=O)O-, (R⁴)₂NS(=O)₂NHC(=O)NR⁴-, R⁴C(=O)NHS(=O)₂-, R⁴C(=O)NHS(=O)₂O-, R⁴C(=O)NHS(=O)₂NR⁴-, R⁴OC(=O)NHS(=O)₂-, R⁴OC(=O)NHS(=O)₂O-, R⁴OC(=O)NHS(=O)₂NR⁴-, (R⁴)₂NC(=O)NHS(=O)₂-, (R⁴)₂NC(=O)NHS(=O)₂O-, (R⁴)₂NC(=O)NHS(=O)₂NR⁴-, heterocyclyl (which in turn may be optionally substituted with alkyl, haloalkyl or oxo), heteroaryl (which in turn may be optionally substituted with alkyl, haloalkyl, alkoxy, alkylthio, alkylsulfonyl, halogen, trifluoromethyl, dialkylamino, nitro, cyano, CO₂H, CONH₂, N-monoalkyl-substituted amido, N,N-dialkyl-substituted amido, or oxo), arylamino (which in turn may be optionally substituted with alkyl, alkoxy, alkylthio, alkylsulfonyl, halogen, trifluoromethyl, dialkylamino, nitro, cyano, CO₂H, CONH₂, N-monoalkyl-substituted amido and N,N-dialkyl-substituted amido) and heteroarylamino (which in turn may be optionally substituted with alkyl, haloalkyl, alkoxy, alkylthio, alkylsulfonyl, halogen, trifluoromethyl, dialkylamino, nitro, cyano, CO₂H, CONH₂, N-monoalkyl-substituted amido, N,N-dialkyl-substituted amido, or oxo);
R⁴ is independently selected from H, (C₁-C₆)alkyl, halo(C₁-C₆)alkyl, amino(C₁-C₆)alkyl, (C₁-C₆)alkylamino(C₁-C₆)alkyl, di(C₁-C₆)alkylamino(C₁-C₆)alkyl, hydroxy(C₁-C₆)alkyl and (C₁-C₆)alkoxy(C₁-C₆)alkyl;
Q = O, NR⁵; and
R⁵ is H, (C₁-C₆)alkyl, halo(C₁-C₆)alkyl or hydroxy(C₁-C₆)alkyl;
or a pharmaceutically acceptable salt, enantiomer or diastereomer thereof.

2. The compound of Claim 1, wherein
Cy¹ is aryl, heteroaryl, monocyclic cycloalkyl or heterocyclyl, wherein each is optionally substituted with 1 to 4 groups independently selected from fluorine, chlorine, bromine, iodine, cyano, nitro, amino, hydroxy, carboxy, (C₁-C₆)alkyl, hydroxy(C₁-C₆)alkyl, (C₃-C₆)cycloalkyl, hydroxy(C₃-C₆)cycloalkyl, (C₄-C₇)cycloalkylalkyl, (C₂-C₆)alkenyl, halo(C₂-C₆)alkenyl, hydroxy(C₂-C₆)alkenyl, (C₂-C₆)alkynyl, (C₃-C₆)cycloalkyl(C₂-C₄)alkynyl, halo(C₁-C₆)alkyl, halo(C₃-C₆)cycloalkyl, halo(C₄-C₇)cycloalkylalkyl, (C₁-C₆)alkoxy, (C₃-C₆)cycloalkoxy, (C₄-C₇)cycloalkylalkoxy, halo(C₁-C₆)alkoxy, halo(C₃-C₆)cycloalkoxy, halo(C₄-C₇)cycloalkylaikoxy, (C₁-C₆)alkylthio, (C₃-C₆)cycloalkythio, (C₄-C₇)cycloalkylalkylthio, halo(C₁-C₆)alkylthio, halo(C₃-C₆)cycloalkythio, halo(C₄-C₇)cycloalkylalkylthio, (C₁-C₆)alkanesulfinyl, (C₃-C₆)cycloalkanesulfinyl, (C₄-C₇)cycloalkylalkanesulfinyl, halo(C₁-C₆)alkanesulfinyl, halo(C₃-C₆)cycloalkanesulfinyl, halo(C₄-C₇)cycloalkylalkanesulfinyl, (C₁-C₆)alkanesulfonyl, (C₃-C₆)cycloalkanesulfonyl, (C₄-C₇)cycloalkylalkanesulfonyl, halo(C₁-C₆)alkanesulfonyl, halo(C₃-C₆)cycloalkanesulfonyl, halo(C₄-C₇)cyclo-alkylalkanesulfonyl, (C₁-C₆)alkylamino, di(C₁-C₆)alkylamino, (C₁-C₆)alkoxy(C₁-C₆)alkoxy, halo(C₁-C₆)alkoxy(C₁-C₆)alkoxy, (C₁-C₆)alkoxycarbonyl, H₂NCO, H₂NSO₂, (C₁-C₆)alkylaminocarbonyl, di(C₁-C₆)alkylaminocarbonyl, (C₁-C₃)alkoxy(C₁-C₃)alkylaminocarbonyl, heterocyclylcarbonyl, (C₁-C₆)alkylaminosulfonyl, di(C₁-C₆)alkylaminosulfonyl, heterocyclosulfonyl, (C₁-C₆)alkylcarbonylamino, (C₁-C₆)alkylcarbonylamino(C₁-C₆)alkyl, (C₁-C₆)alkylsulfonylamino, (C₁-C₆)alkylsulfonylamino(C₁-C₆)alkyl, (C₁-C₆)alkoxycarbonyl(C₁-C₆)alkoxy, (C₁-C₆)alkoxy(C₁-C₆)alkyl, halo(C₁-C₆)alkoxy(C₁-C₆)alkyl, hydroxy(C₁-C₆)alkoxy, heteroaryl oxo, amino(C₁-C₆)alkyl, (C₁-C₆)alkylamino(C₁-C₆)alkyl, di(C₁-C₆)alkylamino(C₁-C₆)alkyl amino(C₂-C₆)alkoxy, (C₁-C₆)alkylamino(C₂-C₆)alkoxy, di(C₁-C₆)alkylamino(C₂-C₆)alkoxy and (C₁-C₆)alkylcarbonyl;
Cy² is aryl, heteroaryl, cycloalkyl or heterocyclyl, wherein each is optionally substituted with 1 to 4 groups independently selected from fluorine, chlorine, bromine, iodine, cyano, nitro, amino, hydroxy, carboxy, (C₁-C₆)alkyl, hydroxy(C₁-C₆)alkyl, (C₃-C₆)cycloalkyl, hydroxy(C₃-C₆)cycloalkyl, (C₄-C₇)cycloalkylalkyl, (C₂-C₆)alkenyl, halo(C₂-C₆)alkenyl, hydroxy(C₂-C₆)alkenyl, (C₂-C₆)alkynyl, (C₃-C₆)cycloalkyl(C₂-C₄)alkynyl, halo(C₁-C₆)alkyl, halo(C₃-C₆)cycloalkyl, halo(C₄-C₇)cycloalkylalkyl, (C₁-C₆)alkoxy, (C₃-C₆)cycloalkoxy, (C₄-C₇)cycloalkylalkoxy, halo(C₁-C₆)alkoxy, halo(C₃-C₆)cycloalkoxy, halo(C₄-C₇)cycloalkylalkoxy, (C₁-C₆)alkylthio, (C₃-C₆)cycloalkythio, (C₄-C₇)cycloalkylalkylthio, halo(C₁-C₆)alkylthio, halo(C₃-C₆)cycloalkythio, halo(C₄-C₇)cycloalkylalkylthio, (C₁-C₆)alkanesulfinyl, (C₃-C₆)cycloalkanesulfinyl, (C₄-C₇)cycloalkylalkanesulfinyl, halo(C₁-C₆)alkanesulfinyl, halo(C₃-C₆)cycloalkanesulfinyl, halo(C₄-C₇)cycloalkylalkanesulfinyl, (C₁-C₆)alkanesulfonyl, (C₃-C₆)cycloalkanesulfonyl, (C₄-C₇)cycloalkylalkanesulfonyl, halo(C₁-C₆)alkanesulfonyl, halo(C₃-C₆)cycloalkanesulfonyl, halo(C₄-C₇)cyclo-alkylalkanesulfonyl, (C₁-C₆)alkylamino, di(C₁-C₆)alkylamino, (C₁-C₆)alkoxy(C₁-C₆)alkoxy, halo(C₁-C₆)alkoxy(C₁-C₆)alkoxy, (C₁-C₆)alkoxycarbonyl, H₂NCO, H₂NSO₂, (C₁-C₆)alkylaminocarbonyl, di(C₁-C₆)alkylaminocarbonyl, (C₁-C₃)alkoxy(C₁-C₃)alkylaminocarbonyl, heterocyclylcarbonyl, (C₁-C₆)alkylaminosulfonyl, di(C₁-C₆)alkylaminosulfonyl, heterocyclosulfonyl, (C₁-C₆)alkylcarbonylamino, (C₁-C₆)alkylcarbonylamino(C₁-C₆)alkyl, (C₁-C₆)alkylsulfonylamino, (C₁-C₆)alkylsulfonylamino(C₁-C₆)alkyl, (C₁-C₆)alkoxycarbonyl(C₁-C₆)alkoxy, (C₁-C₆)alkoxy(C₁-C₆)alkyl, halo(C₁-C₆)alkoxy(C₁-C₆)alkyl, hydroxy(C₁-C₆)alkoxy, heteroaryl, oxo, amino(C₁-C₆)alkyl, (C₁-C₆)alkylamino(C₁-C₆)alkyl, di(C₁-C₆)alkylamino(C₁-C₆)alkyl amino(C₂-C₆)alkoxy, (C₁-C₆)alkylamino(C₂-C₆)alkoxy, di(C₁-C₆)alkylamino(C₂-C₆)alkoxy and (C₁-C₆)alkylcarbonyl; and
R² is aryl, heteroaryl, cycloalkyl or heterocyclyl, wherein each is optionally substituted with up to 4 groups independently selected from fluorine, chlorine, bromine, iodine, cyano, nitro, amino, hydroxy, carboxy, (C₁-C₆)alkyl, hydroxy(C₁-C₆)alkyl, (C₃-C₆)cycloalkyl, hydroxy(C₃-C₆)cycloalkyl, (C₄-C₇)cycloalkylalkyl, (C₂-C₆)alkenyl, halo(C₂-C₆)alkenyl, hydroxy(C₂-C₆)alkenyl, (C₂-C₆)alkynyl, (C₃-C₆)cycloalkyl(C₂-C₄)alkynyl, halo(C₁-C₆)alkyl, halo(C₃-C₆)cycloalkyl, halo(C₄-C₇)cycloalkylalkyl, (C₁-C₆)alkoxy, (C₃-C₆)cycloalkoxy, (C₄-C₇)cycloalkylalkoxy, halo(C₁-C₆)alkoxy, halo(C₃-C₆)cycloalkoxy, halo(C₄-C₇)cycloalkylalkoxy, (C₁-C₆)alkylthio, (C₃-C₆)cycloalkylthio, (C₄-C₇)cycloalkylalkylthio, halo(C₁-C₆)alkylthio, halo(C₃-C₆)cycloalkylthio, halo(C₄-C₇)cycloalkylalkylthio, (C₁-C₆)alkanesulfinyl, (C₃-C₆)cycloalkanesulfinyl, (C₄-C₇)cycloalkylalkanesulfinyl, halo(C₁-C₆)alkanesulfinyl, halo(C₃-C₆)cycloalkanesulfinyl, halo(C₄-C₇)cycloalkylalkanesulfinyl, (C₁-C₆)alkanesulfonyl, (C₃-C₆)cycloalkanesulfonyl, (C₄-C₇)cycloalkylalkanesulfonyl, halo(C₁-C₆)alkanesulfonyl, halo(C₃-C₆)cycloalkanesulfonyl, halo(C₄-C₇)cyclo-alkylalkanesulfonyl, (C₁-C₆)alkylamino, di(C₁-C₆)alkylamino, (C₁-C₆)alkoxy(C₁-C₆)alkoxy, halo(C₁-C₆)alkoxy(C₁-C₆)alkoxy, (C₁-C₆)alkoxycarbonyl, H₂NCO, H₂NSO₂, (C₁-C₆)alkylaminocarbonyl, di(C₁-C₆)alkylaminocarbonyl, (C₁-C₃)alkoxy(C₁-C₃)alkylaminocarbonyl, heterocyclylcarbonyl, (C₁-C₆)alkylaminosulfonyl, di(C₁-C₆)alkylaminosulfonyl, heterocyclosulfonyl, (C₁-C₆)alkylcarbonylamino, (C₁-C₆)alkylcarbonylamino(C₁-C₆)alkyl, (C₁-C₆)alkylsulfonylamino, (C₁-C₆)alkylsulfonylamino(C₁-C₆)alkyl, (C₁-C₆)alkoxycarbonyl(C₁-C₆)alkoxy, (C₁-C₆)alkoxy(C₁-C₆)alkyl, halo(C₁-C₆)alkoxy(C₁-C₆)alkyl, hydroxy(C₁-C₆)alkoxy, heteroaryl, oxo, amino(C₁-C₆)alkyl, (C₁-C₆)alkylamino(C₁-C₆)alkyl, di(C₁-C₆)alkylamino(C₁-C₆)alkyl amino(C₂-C₆)alkoxy, (C₁-C₆)alkylamino(C₂-C₆)alkoxy, di(C₁-C₆)alkylamino(C₂-C₆)alkoxy and (C₁-C₆)alkylcarbonyl;
or a pharmaceutically acceptable salt, enantiomer or diastereomer thereof.

3. The compound of Claims 1 or 2, wherein the compound is of Formula (Ia): 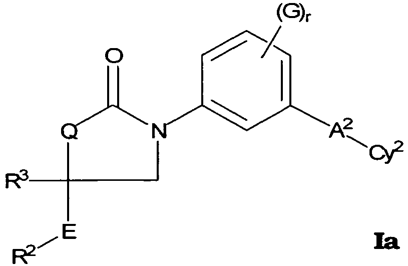 wherein:
G is independently selected from fluorine, chlorine, bromine, iodine, cyano, nitro, amino, hydroxy, carboxy, (C₁-C₆)alkyl, hydroxy(C₁-C₆)alkyl, (C₃-C₆)cycloalkyl, hydroxy(C₃-C₆)cycloalkyl, (C₄-C₇)cycloalkylalkyl, (C₂-C₆)alkynyl, (C₃-C₆)cycloalkyl(C₂-C₄)alkynyl, halo(C₁-C₆)alkyl, halo(C₃-C₆)cycloalkyl, halo(C₄-C₇)cycloalkylalkyl, (C₁-C₆)alkoxy, (C₃-C₆)cycloalkoxy, (C₄-C₇)cycloalkylalkoxy, halo(C₁-C₆)alkoxy, halo(C₃-C₆)cycloalkoxy, halo(C₄-C₇)cycloalkylalkoxy, (C₁-C₆)alkylthio, (C₃-C₆)cycloalkythio, (C₄-C₇)cycloalkylalkylthio, halo(C₁-C₆)alkylthio, halo(C₃-C₆)cycloalkythio, halo(C₄-C₇)cycloalkylalkylthio, (C₁-C₆)alkanesulfinyl, (C₃-C₆)cycloalkanesulfinyl, (C₄-C₇)cycloalkylalkanesulfinyl, halo(C₁-C₆)alkane-sulfinyl, halo(C₃-C₆)cycloalkanesulfinyl, halo(C₄-C₇)cycloalkylalkanesulfinyl, (C₁-C₆)alkanesulfonyl, (C₃-C₆)cycloalkanesulfonyl, (C₄-C₇)cycloalkylalkanesulfonyl, halo(C₁-C₆)alkanesulfonyl, halo(C₃-C₆)cycloalkanesulfonyl, halo(C₄-C₇)cyclo-alkylalkanesulfonyl, (C₁-C₆)alkylamino, di(C₁-C₆)alkylamino, (C₁-C₆)alkoxy(C₁-C₆)alkoxy, halo(C₁-C₆)alkoxy(C₁-C₆)alkoxy, (C₁-C₆)alkoxycarbonyl, H₂NCO, H₂NSO₂, (C₁-C₆)alkylaminocarbonyl, di(C₁-C₆)alkylaminocarbonyl, (C₁-C₃)alkoxy(C₁-C₃)alkylaminocarbonyl, heterocyclylcarbonyl, (C₁-C₆)alkylaminosulfonyl, di(C₁-C₆)alkylaminosulfonyl, heterocyclsulfonyl, (C₁-C₆)alkylcarbonylamino, (C₁-C₆)alkylcarbonylamino(C₁-C₆)alkyl, (C₁-C₆)alkylsulfonylamino, (C₁-C₆)alkylsulfonylamino(C₁-C₆)alkyl, (C₁-C₆)alkoxycarbonyl(C₁-C₆)alkoxy, (C₁-C₆)alkoxy(C₁-C₆)alkyl, halo(C₁-C₆)alkoxy(C₁-C₆)alkyl, hydroxy(C₁-C₆)alkoxy, heteroaryl, amino(C₁-C₆)alkyl, (C₁-C₆)alkylamino(C₁-C₆)alkyl, di(C₁-C₆)alkylamino(C₁-C₆)alkyl amino(C₂-C₆)alkoxy, (C₁-C₆)alkylamino(C₂-C₆)alkoxy, di(C₁-C₆)alkylamino(C₂-C₆)alkoxyl or (C₁-C₆)alkylcarbonyl; and r is 0,1, 2, 3 or 4;
or a pharmaceutically acceptable salt, enantiomer or diastereomer thereof.

4. The compound of Claims 1 or 2, wherein the compound is of Formula (Ib): 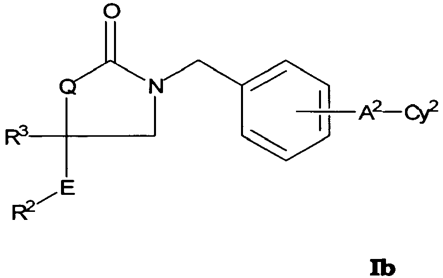 or a pharmaceutically acceptable salt, enantiomer or diastereomer thereof.

5. The compound of Claims 1 or 2, wherein the compound is of Formula (Ic): 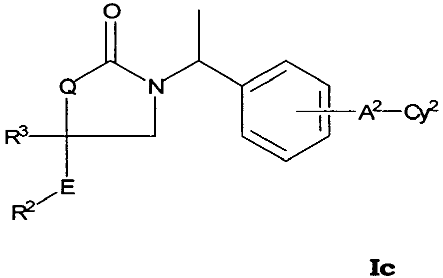 or a pharmaceutically acceptable salt, enantiomer or diastereomer thereof.

6. The compound of Claims 1 or 2, wherein the compound is of Formula (Id): 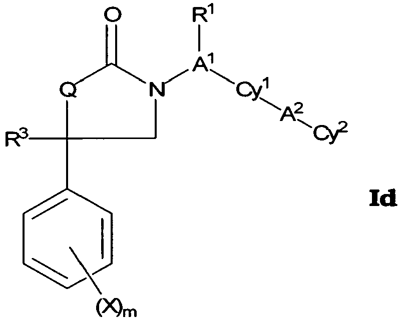 wherein:
X is fluorine, chlorine, bromine, iodine, cyano, nitro, amino, hydroxy, carboxy, (C₁-C₆)alkyl, hydroxy(C₁-C₆)alkyl, (C₃-C₆)cycloalkyl, hydroxy(C₃-C₆)cycloalkyl, (C₄-C₇)cycloalkylalkyl, (C₂-C₆)alkynyl, (C₃-C₆)cycloalkyl(C₂-C₄)alkynyl, halo(C₁-C₆)alkyl, halo(C₃-C₆)cycloalkyl, halo(C₄-C₇)cycloalkylalkyl, (C₁-C₆)alkoxy, (C₃-C₆)cycloalkoxy, (C₄-C₇)cycloalkylalkoxy, halo(C₁-C₆)alkoxy, halo(C₃-C₆)cycloalkoxy, halo(C₄-C₇)cycloalkylalkoxy, (C₁-C₆)alkylthio, (C₃-C₆)cycloalkythio, (C₄-C₇)cycloalkylalkylthio, halo(C₁-C₆)alkylthio, halo(C₃-C₆)cycloalkythio, halo(C₄-C₇)cycloalkylalkylthio, (C₁-C₆)alkanesulfinyl, (C₃-C₆)cycloalkanesulfinyl, (C₄-C₇)cycloalkylalkanesulfinyl, halo(C₁-C₆)alkane-sulfinyl, halo(C₃-C₆)cycloalkanesulfinyl, halo(C₄-C₇)cycloalkylalkanesulfinyl, (C₁-C₆)alkanesulfonyl, (C₃-C₆)cycloalkanesulfonyl, (C₄-C₇)cycloalkylalkanesulfonyl, halo(C₁-C₆)alkanesulfonyl, halo(C₃-C₆)cycloalkanesulfonyl, halo(C₄-C₇)cyclo-alkylalkanesulfonyl, (C₁-C₆)alkylamino, di(C₁-C₆)alkylamino, (C₁-C₆)alkoxy(C₁-C₆)alkoxy, halo(C₁-C₆)alkoxy(C₁-C₆)alkoxy, (C₁-C₆)alkoxycarbonyl, H₂NCO, H₂NSO₂, (C₁-C₆)alkylaminocarbonyl, di(C₁-C₆)alkylaminocarbonyl, (C₁-C₃)alkoxy(C₁-C₃)alkylaminocarbonyl, heterocyclylcarbonyl, (C₁-C₆)alkylaminosulfonyl, di(C₁-C₆)alkylaminosulfonyl, heterocyclsulfonyl, (C₁-C₆)alkylcarbonylamino, (C₁-C₆)alkylcarbonylamino(C₁-C₆)alkyl, (C₁-C₆)alkylsulfonylamino, (C₁-C₆)alkylsulfonylamino(C₁-C₆)alkyl, (C₁-C₆)alkoxycarbonyl(C₁-C₆)alkoxy, (C₁-C₆)alkoxy(C₁-C₆)alkyl, halo(C₁-C₆)alkoxy(C₁-C₆)alkyl, hydroxy(C₁-C₆)alkoxy, heteroaryl, amino(C₁-C₆)alkyl, (C₁-C₆)alkylamino(C₁-C₆)alkyl, di(C₁-C₆)alkylamino(C₁-C₆)alkyl amino(C₂-C₆)alkoxy, (C₁-C₆)alkylamino(C₂-C₆)alkoxy, di(C₁-C₆)alkylamino(C₂-C₆)alkoxyl and (C₁-C₆)alkylcarbonyl; and
m is 0, 1, 2, 3 or 4;
or a pharmaceutically acceptable salt, enantiomer or diastereomer thereof.

7. The compound of Claims 1 or 2, wherein the compound is of Formula (Ie): 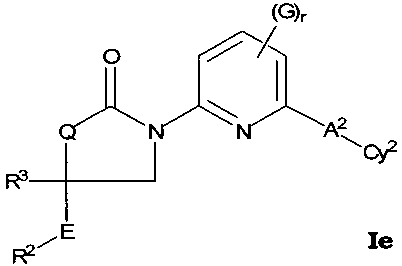 wherein:
G is independently selected from fluorine, chlorine, bromine, iodine, cyano, nitro, amino, hydroxy, carboxy, (C₁-C₆)alkyl, hydroxy(C₁-C₆)alkyl, (C₃-C₆)cycloalkyl, hydroxy(C₃-C₆)cycloalkyl, (C₄-C₇)cycloalkylalkyl, (C₂-C₆)alkynyl, (C₃-C₆)cycloalkyl(C₂-C₄)alkynyl, halo(C₁-C₆)alkyl, halo(C₃-C₆)cycloalkyl, halo(C₄-C₇)cycloalkylalkyl, (C₁-C₆)alkoxy, (C₃-C₆)cycloalkoxy, (C₄-C₇)cycloalkylalkoxy, halo(C₁-C₆)alkoxy, halo(C₃-C₆)cycloalkoxy, halo(C₄-C₇)cycloalkylalkoxy, (C₁-C₆)alkylthio, (C₃-C₆)cycloalkythio, (C₄-C₇)cycloalkylalkylthio, halo(C₁-C₆)alkylthio, halo(C₃-C₆)cycloalkythio, halo(C₄-C₇)cycloalkylalkylthio, (C₁-C₆)alkanesulfinyl, (C₃-C₆)cycloalkanesulfinyl, (C₄-C₇)cycloalkylalkanesulfinyl, halo(C₁-C₆)alkane-sulfinyl, halo(C₃-C₆)cycloalkanesulfinyl, halo(C₄-C₇)cycloalkylalkanesulfinyl, (C₁-C₆)alkanesulfonyl, (C₃-C₆)cycloalkanesulfonyl, (C₄-C₇)cycloalkylalkanesulfonyl, halo(C₁-C₆)alkanesulfonyl, halo(C₃-C₆)cycloalkanesulfonyl, halo(C₄-C₇)cyclo-alkylalkanesulfonyl, (C₁-C₆)alkylamino, di(C₁-C₆)alkylamino, (C₁-C₆)alkoxy(C₁-C₆)alkoxy, halo(C₁-C₆)alkoxy(C₁-C₆)alkoxy, (C₁-C₆)alkoxycarbonyl, H₂NCO, H₂NSO₂, (C₁-C₆)alkylaminocarbonyl, di(C₁-C₆)alkylaminocarbonyl, (C₁-C₃)alkoxy(C₁-C₃)alkylaminocarbonyl, heterocyclylcarbonyl, (C₁-C₆)alkylaminosulfonyl, di(C₁-C₆)alkylaminosulfonyl, heterocyclsulfonyl, (C₁-C₆)alkylcarbonylamino, (C₁-C₆)alkylcarbonylamino(C₁-C₆)alkyl, (C₁-C₆)alkylsulfonylamino, (C₁-C₆)alkylsulfonylamino(C₁-C₆)alkyl, (C₁-C₆)alkoxycarbonyl(C₁-C₆)alkoxy, (C₁-C₆)alkoxy(C₁-C₆)alkyl, halo(C₁-C₆)alkoxy(C₁-C₆)alkyl, hydroxy(C₁-C₆)alkoxy, heteroaryl, oxo, amino(C₁-C₆)alkyl, (C₁-C₆)alkylamino(C₁-C₆)alkyl, di(C₁-C₆)alkylamino(C₁-C₆)alkyl amino(C₂-C₆)alkoxy, (C₁-C₆)alkylamino(C₂-C₆)alkoxy, di(C₁-C₆)alkylamino(C₂-C₆)alkoxyl and (C₁-C₆)alkylcarbonyl; and
r is 0,1, 2, 3 or 4;
or a pharmaceutically acceptable salt, enantiomer or diastereomer thereof.

8. The compound of Claims 1 or 2, wherein the compound is of Formula (If): 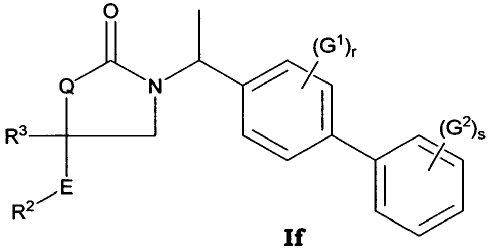 wherein:
G¹ and G² are each independently selected from fluorine, chlorine, bromine, iodine, cyano, nitro, amino, hydroxy, carboxy, (C₁-C₆)alkyl, hydroxy(C₁-C₆)alkyl, (C₃-C₆)cycloalkyl, hydroxy(C₃-C₆)cycloalkyl, (C₄-C₇)cycloalkylalkyl, (C₂-C₆)alkynyl, (C₃-C₆)cycloalkyl(C₂-C₄)alkynyl, halo(C₁-C₆)alkyl, halo(C₃-C₆)cycloalkyl, halo(C₄-C₇)cycloalkylalkyl, (C₁-C₆)alkoxy, (C₃-C₆)cycloalkoxy, (C₄-C₇)cycloalkylalkoxy, halo(C₁-C₆)alkoxy, halo(C₃-C₆)cycloalkoxy, halo(C₄-C₇)cycloalkylalkoxy, (C₁-C₆)alkylthio, (C₃-C₆)cycloalkythio, (C₄-C₇)cycloalkylalkylthio, halo(C₁-C₆)alkylthio, halo(C₃-C₆)cycloalkythio, halo(C₄-C₇)cycloalkylalkylthio, (C₁-C₆)alkanesulfinyl, (C₃-C₆)cycloalkanesulfinyl, (C₄-C₇)cycloalkylalkanesulfinyl, halo(C₁-C₆)alkane-sulfinyl, halo(C₃-C₆)cycloalkanesulfinyl, halo(C₄-C₇)cycloalkylalkanesulfinyl, (C₁-C₆)alkanesulfonyl, (C₃-C₆)cycloalkanesulfonyl, (C₄-C₇)cycloalkylalkanesulfonyl, halo(C₁-C₆)alkanesulfonyl, halo(C₃-C₆)cycloalkanesulfonyl, halo(C₄-C₇)cyclo-alkylalkanesulfonyl, (C₁-C₆)alkylamino, di(C₁-C₆)alkylamino, (C₁-C₆)alkoxy(C₁-C₆)alkoxy, halo(C₁-C₆)alkoxy(C₁-C₆)alkoxy, (C₁-C₆)alkoxycarbonyl, H₂NCO, H₂NSO₂, (C₁-C₆)alkylaminocarbonyl, di(C₁-C₆)alkylaminocarbonyl, (C₁-C₃)alkoxy(C₁-C₃)alkylaminocarbonyl, heterocyclylcarbonyl, (C₁-C₆)alkylaminosulfonyl, di(C₁-C₆)alkylaminosulfonyl, heterocyclsulfonyl, (C₁-C₆)alkylcarbonylamino, (C₁-C₆)alkylcarbonylamino(C₁-C₆)alkyl, (C₁-C₆)alkylsulfonylamino, (C₁-C₆)alkylsulfonylamino(C₁-C₆)alkyl, (C₁-C₆)alkoxycarbonyl(C₁-C₆)alkoxy, (C₁-C₆)alkoxy(C₁-C₆)alkyl, halo(C₁-C₆)alkoxy(C₁-C₆)alkyl" hydroxy(C₁-C₆)alkoxy, heteroaryl, amino(C₁-C₆)alkyl, (C₁-C₆)alkylamino(C₁-C₆)alkyl, di(C₁-C₆)alkylamino(C₁-C₆)alkyl amino(C₂-C₆)alkoxy, (C₁-C₆)alkylamino(C₂-C₆)alkoxy, di(C₁-C₆)alkylamino(C₂-C₆)alkoxyl or (C₁-C₆)alkylcarbonyl;
R⁵ is H, (C₁-C₆)alkyl, halo(C₁-C₆)alkyl, or hydroxy(C₁-C₆)alkyl; and
r and s are independently 0, 1, 2, 3 or 4;
or a pharmaceutically acceptable salt, enantiomer or diastereomer thereof.

9. The compound of any one of Claims 1 to 8, wherein Cy² is optionally substituted aryl or heteroaryl.

10. The compound of any one of Claims 1 to 8, wherein Cy² is optionally substituted cycloalkyl or heterocyclyl.

11. The compound of Claim 10, wherein Cy² is 1,2-dihydro-2-oxopyridyl or 1,2-dihydro-1-methyl-2-oxopyridyl.

12. The compound of Claim 10, wherein Cy² is optionally substituted 1,2-dihydro-2-oxopyridyl or 1,2-dihydro-1-methyl-2-oxopyridyl.

13. A pharmaceutical composition comprising: i) a pharmaceutically acceptable carrier or diluent; and ii) the compound in any one of claims 1-12; or a pharmaceutically acceptable salt, enantiomer or diastereomer thereof.

14. A compound according to any one of Claims 1 to 12 for use in treating a disease or disorder associated with activity or expression of 11β-HSD1 in a mammal.

## Patentansprüche

1. Verbindung mit Formel (I) 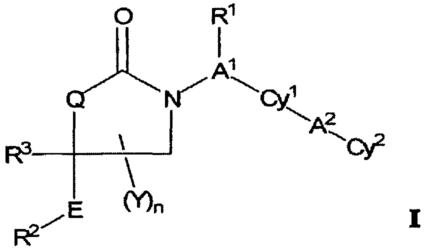 wobei:
R¹ (a) fehlt oder (b) ausgewählt ist aus (C₁-C₆)Alkyl, (C₂-C₆)Alkeny), (C₂-C₆)Alkinyl und (C₁-C₃)Alkoxy(C₁-C₃)alkyl, wobei jede wahlweise mit bis zu vier Gruppen substituiert ist, unabhängig ausgewählt aus Fluor, Cyano, Oxo, R⁴, R⁴O-, (R⁴)₂N-, (R⁴)O₂C-, R⁴S, R⁴S(=O)-, R⁴S(=O)₂-, R⁴C(=O)NR⁴-, (R⁴)₂NC(=O)-, (R⁴)₂NC(=O)O-, (R⁴)₂NC(=O)NR⁴-, R⁴OC(=O)NR⁴-, (R⁴)₂NC(=NCN)NR⁴-, (R⁴O)₂P(=O)O-, (R⁴O)₂P(=O)NR⁴-, (R⁴OS)(=O)₂NR⁴-, (R⁴)₂NS(=O)₂O-, (R⁴)₂NS(=O)₂NR⁴-, R⁴S(=O)₂NR⁴- , R⁴S(=O)₂NHC(=O)-, R⁴S(=O)₂NHC(=O)O-, R⁴S(=O)₂NHC(=O)NR⁴-, R⁴OS(=O)₂NHC(=O)-, R⁴OS(=O)₂NHC(=O)O-, R⁴OS(=O)₂NHC(=O)NR⁴-, (R⁴)₂NS(=O)₂NHC(=O)-, (R⁴)₂NS(=O)₂NHC(=O)O-, (R⁴)₂NS(=O)₂NHC(=O)NR⁴-, R⁴C(=O)NHS(=O)₂-, R⁴C(=O)NHS(=O)₂O-, R⁴C(=O)NHS(=O)₂NR⁴-, R⁴OC(=O)NHS(=O)₂-, R⁴OC(=O)NHS(=O)₂O-, R⁴OC(=O)NHS(=O)₂NR⁴-, (R⁴)₂NC(=O)NHS(=O)₂-, (R⁴)₂NC(=O)NHS(=O)₂O-, (R⁴)₂NC(=O)NHS(=O)₂NR⁴-, Aryl, Cycloalkyl, Heterocyclyl, Heteroaryl, Arylamino und Heteroarylamino;
A¹ (a) eine Bindung ist oder (b) (C₁-C₃)Alkylen, CH₂CH₂O ist, wobei der Sauerstoff an Cy¹ gebunden ist, oder CH₂C(=O) ist, wobei der Carbonyl-Kohlenstoff an Cy¹ gebunden ist. Cy¹ Aryl, Heteroaryl, monocyclisches Cycloalkyl oder Heterocyclyl ist, wobei jedes wahlweise mit 1 bis 4 Gruppen substituiert ist, unabhängig ausgewählt aus Fluor, Chlor, Brom, Iod, Cyano, Nitro, Amino, Hydroxy, Carboxy, (C₁-C₆)Alkyl, Hydroxy(C₁-C₆)alkyl, (C₃-C₆)Cycloalkyl, Hydroxy(C₃-C₆)cycloalkyl, (C₄-C₇)Cycloalkylalkyl, (C₂-C₆)Alkenyl, Halogen(C₂-C₆)alkenyl, Hydroxy(C₂-C₆)alkenyl, (C₂-C₆)Alkinyl, (C₃-C₆)Cycloalkyl(C₂-C₄)alkinyl, Halogen(C₁-C₆)alkyl, Halogen(C₃-C₆)cycloalkyl, Halogen(C₄-C₇)cycloalkylalkyl, (C₁-C₆)Alkoxy, (C₃-C₆)Cycloalkoxy, (C₄-C₇)Cycloalkylalkoxy, Halogen(C₁-C₆)alkoxy, Halogen(C₃-C₆)cycloalkoxy, Halogen(C₄-C₇)cycloalkylalkoxy, (C₁-C₆)Alkylthio, (C₃-C₆)Cycloalkylthio, (C₄-C₇)Cycloalkylalkylthio, Halogen(C₁-C₆)alkylthio, Halogen(C₃-C₆)cycloalkylthio, Halogen(C₄-C₇)cycloalkylalkylthio, (C₁-C₆)Alkansulfinyl, (C₃-C₆)Cycloalkansulfinyl, (C₄-C₇)Cycloalkylalkansulfinyl, Halogen(C₁-C₆)alkansulfinyl, Halogen(C₃-C₆)cycloalkansulfinyl, Halogen(C₄-C₇)cycloalkylalkansulfinyl, (C₁-C₆)Alkansulfonyl, (C₃-C₆)cycloalkansulfonyl, (C₄-C₇)cycloalkylalkansulfonyl, Halogen(C₁-C₆)alkansulfonyl, Halogen(C₃-C₆)cycloalkansulfonyl, Halogen(C₄-C₇)cycloalkylalkansulfonyl, (C₁-C₆)Alkylamino, Di(C₁-C₆)alkylamino, (C₁-C₆)Alkoxy(C₁-C₆)alkoxy, Halogen(C₁-C₆)alkoxy(C₁-C₆)alkoxy, (C₁-C₆)Alkoxycarbonyl, H₂NCO, H₂NSO₂, (C₁-C₆)Alkylaminocarbonyl, Di(C₁-C₆)alkylaminocarbonyl, (C₁-C₃)Alkoxy(C₁-C₃)alkylaminocarbonyl, Heterocyclylcarbonyl, (C₁-C₆)Alkylaminosulfonyl, Di(C₁-C₆)alkylaminosulfonyl, Heterocyclosulfonyl, (C₁-C₆)Alkylcarbonylamino, (C₁-C₆)Alkylcarbonylamino(C₁-C₆)alkyl, (C₁-C₆)Alkylsulfonylamino, (C₁-C₆)Alkylsulfonylamino(C₁-C₆)alkyl, (C₁-C₆)Alkoxycarbonyl(C₁-C₆)alkoxy, (C₁-C₆)Alkoxy(C₁-C₆)alkyl, Halogen(C₁-C₆)alkoxy(C₁-C₆)alkyl, Hydroxy(C₁-C₆)alkoxy, Heteroaryloxo, Amino(C₁-C₆)alkyl, (C₁-C₆)Alkylamino(C₁-C₆)alkyl, Di(C₁-C₆)alkylamino(C₁-C₆)alkylamino(C₂-C₆)alkoxy, (C₁-C₆)Alkylamino(C₂-C₆)alkoxy, Di(C₁-C₆)Alkylamino(C₂-C₆)alkoxy, (C₁-C₆)Alkylcarbonyl, (C₃-C₆)Cycloalkylcarbonyl, (C₃-C₆)Cycloalkylaminocarbonyl, {(C₃-C₆)Cycloalkyl} {(C₁-C₆)alkyl}aminocarbonyl, Di(C₃-C₆)cycloalkylaminocarbonyl, (C₃-C₆)Cycloalkylaminosulfonyl, {(C₃-C₆)Cycloalkyl} {(C₁-C₆)Alkyl}aminosulfonyl, Di(C₃-C₆)cycloalkylaminosulfonyl, Cyano(C₁-C₆)alkyl, Aminocarbonyl(C₁-C₆)alkyl, (C₁-C₆)Alkylaminocarbonyl(C₁-C₆)alkyl, Di(C₁-C₆)alkylaminocarbonyl(C₁-C₆)alkyl, (C₃-C₆)Cycloalkylaminocarbonyl(C₁-C₆)alkyl, {(C₃-C₆)Cycloalkyl} {(C₁-C₆)alkyl}aminocarbonyl(C₁-C₆)alkyl und Di(C₃-C₆)cycloalkylaminocarbonyl(C₁-C₆)alkyl ist;
A² (a) eine Bindung, O, S oder NR⁴ ist; oder (b) (C₁-C₃)Alkylen oder (C₁-C₂)Alkylenoxy ist, wobei jede davon wahlweise mit 1 bis 4 Gruppen substituiert ist, unabhängig ausgewählt aus Methyl, Ethyl, Trifluormethyl oder Oxo;
Cy² Aryl, Heteroaryl, Cycloalkyl oder Heterocyclyl ist, wobei jede wahlweise mit 1 bis 4 Gruppen substituiert ist, unabhängig ausgewählt aus Fluor, Chlor, Brom, Iod, Cyano, Nitro, Amino, Hydroxy, Carboxy, (C₁-C₆)Alkyl, Hydroxy(C₁-C₆)alkyl, (C₃-C₆)Cycloalkyl, Hydroxy(C₃-C₆)cycloalkyl, (C₄-C₇)Cycloalkylalkyl, (C₂-C₆)Alkenyl, Halogen(C₂-C₆)alkenyl, Hydroxy(C₂-C₆)alkenyl, (C₂-C₆)Alkinyl, (C₃-C₆)Cycloalkyl(C₂-C₄)alkinyl, Halogen(C₁-C₆)alkyl, Halogen(C₃-C₆)cycloalkyl, Halogen(C₄-C₇)cycloalkylalkyl, (C₁-C₆)Alkoxy, (C₃-C₆)Cycloalkoxy, (C₄-C₇)Cycloalkylalkoxy, Halogen(C₁-C₆)alkoxy, Halogen(C₃-C₆)cycloalkoxy, Halogen(C₄-C₇)cycloalkylalkoxy, (C₁-C₆)Alkylthio, (C₃-C₆)Cycloalkylthio, (C₄-C₇)Cycloalkylalkylthio, Halogen(C,-Cs)alkylthio, Halogen(C₃-C₆)cycloalkylthio, Halogen(C₄-C₇)cycloalkylalkylthio, (C₁-C₆)Alkansulfinyl, (C₃-C₆)Cycloalkansulfinyl, (C₄-C₇)Cycloalkylalkansulfinyl, Halogen(C₁-C₆)alkansulfinyl, Halogen(C₃-C₆)cycloalkansulfinyl, Halogen(C₄-C₇)cycloalkylalkansulfinyl, (C₁-C₆)Alkansulfonyl, (C₃-C₆)Cycloalkansulfonyl, (C₄-C₇)Cycloalkylalkansulfonyl, Halogen(C₁-C₆)alkansulfonyl, Halogen(C₃-C₆)cycloalkansulfonyl, Halogen(C₄-C₇)cycloalkylalkansulfonyl, (C₁-C₆)Alkylamino, Di(C₁-C₆)alkylamino, (C₁-C₆)alkoxy(C₁-C₆)alkoxy, Halogen(C₁-C₆)alkoxy(C₁-C₆)alkoxy, (C₁-C₆)Alkoxycarbonyl, H₂NCO, H₂NSO₂, (C₁-C₆)Alkylaminocarbonyl, Di(C₁-C₆)alkylaminocarbonyl, (C₁-C₃)Alkoxy(C₁-C₃)alkylaminocarbonyl, Heterocyclylcarbonyl, (C₁-C₆)Alkylaminosulfonyl, Di(C₁-C₆)alkylaminosulfonyl, Heterocyclosulfonyl, (C₁-C₆)Alkylcarbonylamino, (C₁-C₆)Alkylcarbonylamino(C₁-C₆)alkyl, (C₁-C₆)Alkylsulfonylamino, (C₁-C₆)Alkylsulfonylamino(C₁-C₆)alkyl, (C₁-C₆)Alkoxycarbonyl(C₁-C₆)alkoxy, (C₁-C₆)Alkoxy(C₁-C₆)alkyl, Halogen(C₁-C₆)alkoxy(C₁-C₆)alkyl, Hydroxy(C₁-C₆)alkoxy, Heteroaryl, Oxo, Amino(C₁-C₆)alkyl, (C₁-C₆)Alkylamino(C₁-C₆)alkyl, Di(C₁-C₆)alkylamino(C₁-C₆)alkylamino(C₂-C₆)alkoxy, (C₁-C₆)Alkylamino(C₂-C₆)alkoxy, Di(C₁-C₆)Alkylamino(C₂-C₆)alkoxy, (C₁-C₆)Alkylcarbonyl, (C₃-C₆)Cycloalkylcarbonyl, (C₃-C₆)Cycloalkylaminocarbonyl, {(C₃-C₆)Cycloalkyl} {(C₁-C₆)alkyllaminocarbonyl, Di(C₃-C₆)cycloalkylaminocarbonyl, (C₃-C₆)Cycloalkylaminosulfonyl, {(C₃-C₆)Cycloalkyl} {(C₁-C₆)alkyl}aminosulfonyl, Di(C₃-C₆)cycloalkylaminosulfonyl, Cyano(C₁-C₆)alkyl, Aminocarbonyl(C₁-C₆)alkyl, (C₁-C₆)Alkylaminocarbonyl(C₁-C₆)alkyl, Di(C₁-C₆)alkylaminocarbonyl(C₁-C₆)alkyl, (C₃-C₆)Cycloalkylaminocarbonyl(C₁-C₆)alkyl, {(C₃-C₆)Cycloalkyl} {(C₁-C₆)alkyl}aminocarbonyl(C₁-C₆)alkyl und Di(C₃-C₆)cycloalkylaminocarbonyl(C₁-C₆)alkyl;
Y (C₁-C₆)Alkyl oder Halogen(C₁-C₆)alkyl ist;
n 0, 1 oder 2 ist;
E (a) eine Bindung ist oder (b) (C₁-C₃)Alkylen oder (C₁-C₂)Alkylenyloxy ist, wobei der O an R² gebunden ist, wobei jede davon wahlweise durch 1 bis 4 Gruppen substituiert ist, ausgewählt aus Methyl, Ethyl, Trifluormethyl oder Oxo;
R² Aryl, Heteroaryl, Cycloalkyl oder Heterocyclyl ist, wobei jede wahlweise durch bis zu 4 Gruppen substituiert ist, unabhängig ausgewählt aus Fluor, Chlor, Brom, Iod, Cyano, Nitro, Amino, Hydroxy, Carboxy, (C₁-C₆)Alkyl, Hydroxy(C₁-C₆)alkyl, (C₃-C₆)Cycloalkyl, Hydroxy(C₃-C₆)cycloalkyl, (C₄-C₇)Cycloalkylalkyl, (C₂-C₆)Alkenyl, Halogen(C₂-C₆)alkenyl, Hydroxy(C₂-C₆)alkenyl, (C₂-C₆)Alkinyl, (C₃-C₆)Cycloalkyl(C₂-C₄)alkinyl, Halogen(C₁-C₆)alkyl, Halogen(C₃-C₆)cycloalkyl, Halogen(C₄-C₇)cycloalkylalkyl, (C₁-C₆)Alkoxy, (C₃-C₆)Cycloalkoxy, (C₄-C₇)Cycloalkylalkoxy, Halogen(C₁-C₆)alkoxy, Halogen(C₃-C₆)cycloalkoxy, Halogen(C₄-C₇)cycloalkylalkoxy, (C₁-C₆)Alkylthio, (C₃-C₆)Cycloalkylthio, (C₄-C₇)Cycloalkylalkylthio, Halogen(C₁-C₆)Alkylthio, Halogen(C₃-C₆)cycloalkylthio, Halogen(C₄-C₇)cycloalkylalkylthio, (C₁-C₆)Alkansulfinyl, (C₃-C₆)Cycloalkansulfinyl, (C₄-C₇)Cycloalkylalkansulfinyl, Halogen(C₁-C₆)alkansulfinyl, Halogen(C₃-C₆)cycloalkansulfinyl, Halogen(C₄-C₇)cycloalkylalkansulfinyl, (C₁-C₆)Alkansulfonyl, (C₃-C₆)Cycloalkansulfonyl, (C₄-C₇)Cycloalkylalkansulfonyl, Halogen(C₁-C₆)alkansulfonyl, Halogen(C₃-C₆)cycloalkansulfonyl, Halogen(C₄-C₇)cycloalkylalkansulfonyl, (C₁-C₆)Alkylamino, Di(C₁-C₆)alkylamino, (C₁-C₆)Alkoxy(C₁-C₆)alkoxy, Halogen(C₁-C₆)alkoxy(C₁-C₆)alkoxy, (C₁-C₆)Alkoxycarbonyl, H₂NCO, H₂NSO₂, (C₁-C₆)Alkylaminocarbonyl, Di(C₁-C₆)alkylaminocarbonyl, (C₁-C₃)Alkoxy(C₁-C₃)alkylaminocarbonyl, Heterocyclylcarbonyl, (C₁-C₆)Alkylaminosulfonyl, Di(C₁-C₆)alkylaminosulfonyl, Heterocyclosulfonyl, (C₁-C₆)Alkylcarbonylamino, (C₁-C₆)Alkylcarbonylamino(C₁-C₆)alkyl, (C₁-C₆)Alkylsulfonylamino, (C₁-C₆)Alkylsulfonylamino(C₁-C₆)alkyl, (C₁-C₆)Alkoxycarbonyl(C₁-C₆)alkoxy, (C₁-C₆)Alkoxy(C₁-C₆)alkyl, Halogen(C₁-C₆)alkoxy(C₁-C₆)alkyl, Hydroxy(C₁-C₆)alkoxy, Heteroaryl, Oxo, Amino(C₁-C₆)alkyl, (C₁-C₆)Alkylamino(C₁-C₆)alkyl, Di(C₁-C₆)alkylamino(C₁-C₆)alkylamino(C₂-C₆)alkoxy, (C₁-C₆)Alkylamino(C₂-C₆)alkoxy, Di(C₁-C₆)Alkylamino(C₂-C₆)alkoxy, (C₁-C₆)Alkylcarbonyl, (C₃-C₆)Cycloalkylcarbonyl, (C₃-C₆)Cycloalkylaminocarbonyl, {(C₃-C₆)Cycloalkyl} {(C₁-C₆)alkyllaminocarbonyl, Di(C₃-C₆)cycloalkylaminocarbonyl, (C₃-C₆)Cycloalkylaminosulfonyl, {(C₃-C₆)Cycloalkyl} {(C1-C₆)alkyl}aminosulfonyl, Di(C₃-C₆)cycloalkylaminosulfonyl, Cyano(C₁-C₆)alkyl, Aminocarbonyl(C₁-C₆)alkyl, (C₁-C₆)Alkylaminocarbonyl(C₁-C₆)alkyl, Di(C₁-C₆)alkylaminocarbonyl(C₁-C₆)alkyl, (C₃-C₆)Cycloalkylanninocarbonyl(C₁-C₆)alkyl, {(C₃-C₆)Cycloalkyl} {(C₁-C₆)alkyl}aminocarbonyl(C₁-C₆)alkyl und Di(C₃-C₆)cycloalkylaminocarbonyl(C₁-C₆)alkyl;
R³ ausgewählt ist aus (C₂-C₆)Alkyl, (C₂-C₆)Alkenyl, (C₂-C₆)Alkinyl und (C₁-C₃)Alkoxy(C₁-C₃)alkyl, wobei (C₂-C₆)Alkyl substituiert ist durch und jede aus (C₂-C₆)Alkenyl, (C₂-C₆)Alkinyl und (C₁-C₃)Alkoxy(C₁-C₃)alkyl substituiert ist durch bis zu vier Gruppen, unabhängig ausgewählt aus Fluor, Cyano, Oxo, Halogen(C₁-C₆)alkyl, Amino(C₁-C₆)alkyl, (C₁-C₆)Alkylamino(C₁-C₆)alkyl, Di(C₁-C₆)alkylamino(C₁-C₆)alkyl, Hydroxy(C₁-C₆)alkyl, (C₁-C₆)alkoxy(C₁-C₆)alkyl, R⁴O-, (R⁴)₂N-, (R⁴)O₂C-, R⁴S, R⁴S(=O)-, R⁴S(=O)₂-, R⁴C(=O)NR⁴-, (R⁴)₂NC(=O)-, (R⁴)₂NC(=O)O-, (R ⁴)₂NC(=O)NR⁴-, R⁴OC(=O)NR⁴-, (R⁴)₂NC(=NCN)NR⁴-, (R⁴O)₂P(=O)O-, (R⁴O)₂P(=O)NR⁴-, (R⁴OS)(=O)₂NR⁴-, (R¹)₂NS(=O)₂O-, (R⁴)₂NS(=O)₂NR⁴-, R⁴S(=O)₂NR⁴-, R⁴S(=O)₂NHC(=O)-, R⁴S(=O)₂NHC(=O)O-, R⁴S(=O)₂NHC(=O)NR⁴-, R⁴OS(=O)₂NHC(=O)-, R ⁴OS(=O)₂NHC(=O)O-, R⁴OS(=O)₂NHC(=O)NR⁴-, (R⁴)₂NS(=O)₂NHC(=O)-, (R⁴)₂NS(=O)₂NHC(=O)O-, (R⁴)₂NS(=O)₂NHC(=O)NR⁴-, R⁴C(=O)NHS(=O)₂-, R⁴C(=O)NHS(=O)₂O-, R⁴C(=O)NHS(=O)₂NR⁴-, R⁴OC(=O)NHS(=O)₂-, R⁴OC(=O)NHS(=O)₂O-, R⁴OC(=O)NHS(=O)₂NR⁴-, (R⁴)₂NC(=O)NHS(=O)₂-, (R⁴)₂NC(=O)NHS(=O)₂O-, (R⁴)₂NC(=O)NHS(=O)₂NR⁴-, Heterocyclyl (welches wiederum wahlweise durch Alkyl, Halogenalkyl oder Oxo substituiert sein kann), Heteroaryl (welches wiederum wahlweise durch Alkyl, Halogenalkyl, Alkoxy, Alkylthio, Alkylsulfonyl, Halogen, Trifluormethyl, Dialkylamino, Nitro, Cyano, CO₂H, CONH₂, N-monoalkyl-substituiertes Amido, N,N-Dialkyl-substituiertes Amido oder Oxo substituiert sein kann), Arylamino (welches wiederum wahlweise durch Alkyl, Alkoxy, Alkylthio, Alkylsulfonyl, Halogen, Trifluormethyl, Dialkylamino, Nitro, Cyano, CO₂H, CONH₂, N-monoalkyl-substituiertes Amido und N,N-Dialkyl-substituiertes Amido substituiert sein kann) und Heteroarylamino (welches wiederum wahlweise durch Alkyl, Halogenalkyl, Alkoxy, Alkylthio, Alkylsulfonyl, Halogen, Trifluormethyl, Dialkylamino, Nitro, Cyano, CO₂H, CONH₂, N-monoalkyl-substituiertes Amido, N,N-Dialkyl-substituiertes Amido oder Oxo substituiert sein kann);
R⁴ unabhängig ausgewählt ist aus H, (C₁-C₆)Alkyl, Halogen(C₁-C₆)alkyl, Amino(C₁-C₆)Alkyl, (C₁-C₆)Alkylamino(C₁-C₆)alkyl, Di(C₁-C₆)alkylamino(C₁-C₆)alkyl, Hydroxy(C₁-C₆)alkyl und (C₁-C₆)Alkoxy(C₁-C₆)alkyl;
Q = O, NR⁵; und R⁵ H, (C₁-C₆)Alkyl, Halogen(C₁-C₆)alkyl oder Hydroxy(C₁-C₆)alkyl ist;
oder ein pharmazeutisch unbedenkliches Salz, Enantiomer oder Diastereomer davon.

2. Verbindung nach Anspruch 1, wobei
Cy¹ Aryl, Heteroaryl, monocyclisches Cycloalkyl oder Heterocyclyl ist, wobei jedes wahlweise mit 1 bis 4 Gruppen substituiert ist, unabhängig ausgewählt aus Fluor, Chlor, Brom, Iod, Cyano, Nitro, Amino, Hydroxy, Carboxy, (C₁-C₆)Alkyl, Hydroxy(C₁-C₆)alkyl, (C₃-C₆)Cycloalkyl, Hydroxy(C₃-C₆)cycloalkyl, (C₄-C₇)Cycloalkylalkyl, (C₂-C₆)Alkenyl, Halogen(C₂-C₆)alkenyl, Hydroxy(C₂-C₆)alkenyl, (C₂-C₆)Alkinyl, (C₃-C₆)Cycloalkyl(C₂-C₄)alkinyl, Halogen(C₁-C₆)alkyl, Halogen(C₃-C₆)cycloalkyl, Halogen(C₄-C₇)cycloalkylalkyl, (C₁-C₆)Alkoxy, (C₃-C₆)Cycloalkoxy, (C₄-C₇)Cycloalkylalkoxy, Halogen(C₁-C₆)alkoxy, Halogen(C₃-C₆)cycloalkoxy, Halogen(C₄-C₇)cycloalkylalkoxy, (C₁-C₆)Alkylthio, (C₃-C₆)Cycloalkylthio, (C₄-C₇)Cycloalkylalkylthio, Halogen(C₁-C₆)alkylthio, Halogen(C₃-C₆)cycloalkylthio, Halogen(C₄-C₇)cycloalkylalkylthio, (C₁-C₆)Alkansulfinyl, (C₃-C₆)Cycloalkansulfinyl, (C₄-C₇)Cycloalkylalkansulfinyl, Halogen(C₁-C₆)alkansulfinyl, Halogen(C₃-C₆)cycloalkansulfinyl, Halogen(C₄-C₇)cycloalkylalkansulfinyl, (C₁-C₆)Alkansulfonyl, (C₃-C₆)Cycloalkansulfonyl, (C₄-C₇)Cycloalkylalkansulfonyl, Halogen(C₁-C₆)alkansulfonyl, Halogen(C₃-C₆)cycloalkansulfonyl, Halogen(C₄-C₇)cycloalkylaikansulfonyl, (C₁-C₆)Alkylamino, Di(C₁-C₆)alkylamino, (C₁-C₆)Alkoxy(C₁-C₆)alkoxy, Halogen(C₁-C₆)alkoxy(C₁-C₆)alkoxy, (C₁-C₆)Alkoxycarbonyl, H₂NCO, H₂NSO₂, (C₁-C₆)Alkylaminocarbonyl, Di(C₁-C₆)alkylaminocarbonyl, (C₁-C₃)Alkoxy(C₁-C₃)alkylaminocarbonyl, Heterocyclylcarbonyl, (C₁-C₆)Alkylaminosulfonyl, Di(C₁-C₆)alkylaminosulfonyl, Heterocyclosulfonyl, (C₁-C₆)Alkylcarbonylamino, (C₁-C₆)Alkylcarbonylamino(C₁-C₆)alkyl, (C₁-C₆)Alkylsulfonylamino, (C₁-C₆)Alkylsulfonylamino(C₁-C₆)alkyl, (C₁-C₆)Alkoxycarbonyl(C₁-C₆)alkoxy, (C₁-C₆)Alkoxy(C₁-C₆)alkyl, Halogen(C₁-C₆)alkoxy(C₁-C₆)alkyl, Hydroxy(C₁-C₆)alkoxy, Heteroaryloxo, Amino(C₁-C₆)alkyl, (C₁-C₆)Alkylamino(C₁-C₆)alkyl, Di(C₁-C₆)alkylamino(C₁-C₆)alkylamino(C₁-C₆)alkoxy, (C₁-C₆)Alkylamino(C₂-C₆)alkoxy, Di(C₁-C₆)Alkylamino(C₂-C₆)alkoxy und (C₁-C₆)Alkylcarbonyl; Cy² Aryl, Heteroaryl, Cycloalkyl oder Heterocyclyl ist, wobei jede wahlweise substituiert ist durch 1 bis 4 Gruppen, unabhängig ausgewählt aus Fluor, Chlor, Brom, Iod, Cyano, Nitro, Amino, Hydroxy, Carboxy, (C₁-C₆)Alkyl, Hydroxy(C₁-C₆)alkyl, (C₃-C₆)Cycloalkyl, Hydroxy(C₃-C₆)cycloalkyl, (C₄-C₇)Cycloalkylalkyl, (C₂-C₆)Alkenyl, Halogen(C₂-C₆)alkenyl, Hydroxy(C₂-C₆)alkenyl, (C₂-C₆)Alkinyl, (C₃-C₆)Cycloalkyl(C₂-C₄)alkinyl, Halogen(C₁-C₆)alkyl, Halogen(C₃-C₆)cycloalkyl, Halogen(C₄-C₇)cycloalkylalkyl, (C₁-C₆)Alkoxy, (C₃-C₆)Cycloalkoxy, (C₄-C₇)Cycloalkylalkoxy, Halogen(C₁-C₆)alkoxy, Halogen(C₃-C₆)cycloalkoxy, Halogen(C₄-C₇)cycloalkylalkoxy, (C₁-C₆)Alkylthio, (C₃-C₆)Cycloalkylthio, (C₄-C₇)Cycloalkylalkylthio, Halogen(C₁-C₆)alkylthio, Halogen(C₃-C₆)cycloalkylthio, Halogen(C₄-C₇)cycloalkylalkylthio, (C₁-C₆)Alkansulfinyl, (C₃-C₆)Cycloalkansulfinyl, (C₄-C₇)Cycloalkylalkansulfinyl, Halogen(C₁-C₆)alkansulfinyl, Halogen(C₃-C₆)cycloalkansulfinyl, Halogen(C₄-C₇)cycloalkylalkansulfinyl, (C₁-C₆)Alkansulfonyl, (C₃-C₆)Cycloalkansulfonyl, (C₄-C₇)Cycloalkylalkansulfonyl, Halogen(C₁-C₆)alkansulfonyl, Halogen(C₃-C₆)cycloalkansulfonyl, Halogen(C₄-C₇)cycloalkylalkansulfonyl, (C₁-C₆)Alkylamino, Di(C₁-C₆)alkylamino, (C₁-C₆)Alkoxy(C₁-C₆)alkoxy, Halogen(C₁-C₆)alkoxy(C₁-C₆)alkoxy, (C₁-C₆)Alkoxycarbonyl, H₂NCO, H₂NSO₂, (C₁-C₆)Alkylaminocarbonyl, Di(C₁-C₆)alkylaminocarbonyl, (C₁-C₃)Alkoxy(C₁-C₃)alkylaminocarbonyl, Heterocyclylcarbonyl, (C₁-C₆)Alkylaminosulfonyl, Di(C₁-C₆)alkylaminosulfonyl, Heterocyclosulfonyl, (C₁-C₆)Alkylcarbonylamino, (C₁-C₆)Alkylcarbonylamino(C₁-C₆)alkyl, (C₁-C₆)Alkylsulfonylamino, (C₁-C₆)Alkylsulfonylamino(C₁-C₆)alkyl, (C₁-C₆)Alkoxycarbonyl(C₁-C₆)alkoxy, (C₁-C₆)Alkoxy(C₁-C₆)alkyl, Halogen(C₁-C₆)alkoxy(C₁-C₆)alkyl, Hydroxy(C₁-C₆)alkoxy, Heteroaryl, Oxo, Amino(C₁-C₆)alkyl, (C₁-C₆)Alkylamino(C₁-C₆)alkyl, Di(C₁-C₆)alkylamino(C₁-C₆)alkylamino(C₂-C₆)alkoxy, (C₁-C₆)Alkylamino(C₂-C₆)alkoxy, Di(C₁-C₆)Alkylamino(C₂-C₆)alkoxy und (C₁-C₆)Alkylcarbonyl; und
R² Aryl, Heteroaryl, Cycloalkyl oder Heterocyclyl ist, wobei jede wahlweise durch bis zu vier Gruppen substituiert ist, unabhängig ausgewählt aus Fluor, Chlor, Brom, Iod, Cyano, Nitro, Amino, Hydroxy, Carboxy, (C₁-C₆)Alkyl, Hydroxy(C₁-C₆)alkyl, (C₃-C₆)Cycloalkyl, Hydroxy(C₃-C₆)cycloalkyl, (C₄-C₇)Cycloalkylalkyl, (C₂-C₆)Alkenyl, Halogen(C₂-C₆)alkenyl, Hydroxy(C₂-C₆)alkenyl, (C₂-C₆)Alkinyl, (C₃-C₆)Cycloalkyl(C₂-C₄)alkinyl, Halogen(C₁-C₆)alkyl, Halogen(C₃-C₆)cycloalkyl, Halogen(C₄-C₇)cycloalkylalkyl, (C₁-C₆)Alkoxy, (C₃-C₆)Cycloalkoxy, (C₄-C₇)Cycloalkylalkoxy, Halogen(C₁-C₆)alkoxy, Halogen(C₃-C₆)cycloalkoxy, Halogen(C₄-C₇)cycloalkylalkoxy, (C₁-C₆)Alkylthio, (C₃-C₆)Cycloalkylthio, (C₄-C₇)Cycloalkylalkylthio, Halogen(C₁-C₆)alkylthio, Halogen (C₃-C₆)cycloalkylthio, Halogen(C₄-C₇)cycloalkylalkylthio, (C₁-C₆)Alkansulfinyl, (C₃-C₆)Cycloalkansulfinyl, (C₄-C₇)Cycloalkylalkansulfinyl, Halogen(C₁-C₆)alkansulfinyl, Halogen(C₃-C₆)cycloalkansulfinyl, Halogen(C₄-C₇)cycloalkylalkansulfinyl, (C₁-C₆)Alkansulfonyl, (C₃-C₆)Cycloalkansulfonyl, (C₄-C₇)Cycloalkylalkansulfonyl, Halogen(C₁-C₆)alkansulfonyl, Halogen(C₃-C₆)cycloalkansulfonyl, Halogen(C₄-C₇)cycloalkylalkansulfonyl, (C₁-C₆)Alkylamino, Di(C₁-C₆)alkylamino, (C₁-C₆)Alkoxy(C₁-C₆)alkoxy, Halogen(C₁-C₆)alkoxy(C₁-C₆)alkoxy, (C₁-C₆)Alkoxycarbonyl, H₂NCO, H₂NSO₂, (C₁-C₆)Alkylam inocarbonyl, Di(C₁-C₆) alkylaminocarbonyl, (C₁-C₃)Alkoxy(C₁-C₃)alkylaminocarbonyl, Heterocyclylcarbonyl, (C₁-C₆)Alkylaminosulfonyl, Di(C₁-C₆)alkylaminosulfonyl, Heterocyclosulfonyl, (C₁-C₆)Alkylcarbonylamino, (C₁-C₆)Alkylcarbonylamino(C₁-C₆)alkyl, (C₁-C₆)Alkylsulfonylamino, (C₁-C₆)Alkylsulfonylamino(C₁-C₆)alkyl, (C₁-C₆)Alkoxycarbonyl(C₁-C₆)alkoxy, (C₁-C₆)Alkoxy(C₁-C₆)alkyl, Halogen(C₁-C₆)alkoxy(C₁-C₆)alkyl, Hydroxy(C₁-C₆)alkoxy, Heteroaryl, Oxo, Amino(C₁-C₆)alkyl, (C₁-C₆)Alkylamino(C₁-C₆)alkyl, Di(C₁-C₆)alkylamino(C₁-C₆)alkylamino(C₂-C₆)alkoxy, (C₁-C₆)Alkylamino(C₂-C₆)alkoxy, Di(C₁-C₆₆)alkylamino(C₂-C₆)alkoxy und (C₁-C₆)Alkylcarbonyl; oder ein pharmazeutisch unbedenkliches Salz, Enantiomer oder Diastereomer davon.

3. Verbindung nach Anspruch 1 oder 2, wobei die Verbindung, eine Verbindung mit Formel (la) ist: 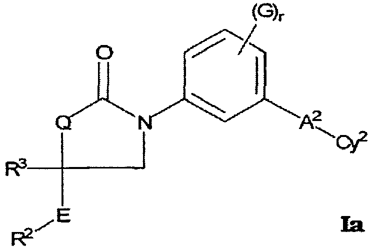 wobei:
G unabhängig ausgewählt ist aus Fluor, Chlor, Brom, lod, Cyano, Nitro, Amino, Hydroxy, Carboxy, (C₁-C₆)Alkyl, Hydroxy(C₁-C₆)alkyl, (C₃-C₆)Cycloalkyl, Hydroxy(C₃-C₆)cycloalkyl, (C₄-C₇)Cycloalkylalkyl, (C₂-C₆)Alkinyl, (C₃-C₆)Cycloalkyl(C₂-C₄)alkinyl, Halogen(C₁-C₆)alkyl, Halogen(C₃-C₆)cycloalkyl, Halogen(C₄-C₇)cycloalkylalkyl, (C₁-C₆)Alkoxy, (C₃-C₆) Cycloalkoxy, (C₄-C₇)Cycloalkylalkoxy, Halogen(C₁-C₆)alkoxy, Halogen(C₃-C₆)cycloalkoxy, Halogen(C₄-C₇)cycloalkylalkoxy, (C₁-C₆)Alkylthio, (C₃-C₆)Cycloalkylthio, (C₄-C₇)Cycloalkylalkylthio, Halogen(C₁-C₆)alkylthio, Halogen (C₃-C₆)cycloalkylthio, Halogen(C₄-C₇)cycloalkylalkylthio, (C₁-C₆)Alkansulfinyl, (C₃-C₆)Cycloalkansulfinyl, (C₄-C₇) Cycloal kylalkansulfinyl, Halogen(C₁-C₆)alkansulfinyl, Halogen(C₃-C₃)cycloalkansulfinyl, Halogen(C₄-C₇)cycloalkylalkansulfinyl, (C₁-C₆)Alkansulfonyl, (C₃-C₆)Cycloalkansulfonyl, (C₄-C₇)Cycioalkylalkansulfonyl, Halogen(C₁-C₆)alkansulfonyl, Halogen (C₃-C₆)cycloalkansulfonyl, Halogen(C₄-C₇)cycloalkylalkansulfonyl, (C₁-C₆)Alkylamino, Di(C₁-C₆)alkylamino, (C₁-C₆)Alkoxy(C₁-C₆)alkoxy, Halogen(C₁-C₆)alkoxy(C₁-C₆)alkoxy, (C₁-C₆)Alkoxycarbonyl, H₂NCO, H₂NSO₂, (C₁-C₆)Alkylaminocarbonyl, Di(C₁-C₆)alkylaminocarbonyl, (C₁-C₃)Alkoxy(C₁-C₃)alkylaminocarbonyl, Heterocyclylcarbonyl, (C₁-C₆)Alkylaminosulfonyl, Di(C₁-C₆)alkylaminosulfonyl, Heterocyclosulfonyl, (C₁-C₆)Alkyicarbonylamino, (C₁-C₆)Alkylcarbonylamino(C₁-C₆)alkyl, (C₁-C₆)Alkylsulfonylamino, (C₁-C₆)Alkylsulfonylamino(C₁-C₆)alkyl, (C₁-C₆)Alkoxycarbonyl(C₁-C₆)alkoxy, (C₁-C₆)Alkoxy(C₁-C₆)alkyl, Halogen(C₁-C₆)alkoxy(C₁-C₆)alkyl, Hydroxy(C₁-C₆)alkoxy, Heteroaryl, Amino(C₁-C₆)alkyl, (C₁-C₆)Alkylamino(C₁-C₆)alkyl, Di(C₁-C₆)alkylamino(C₁-C₆)alkylamino(C₂-C₆)alkoxy, (C₁-C₆)Alkylamino(C₂-C₆)alkoxy, Di(C₁-C₆)Alkylamino(C₂-C₆)alkoxyl oder (C₁-C₆)Alkylcarbonyl;
und r 0, 1, 2, 3 oder 4 ist;
oder ein pharmazeutisch unbedenkliches Salz, Enantiomer oder Diastereomer davon.

4. Verbindung nach Anspruch 1 oder 2, wobei die Verbindung eine Verbindung der Formel (Ib) ist: 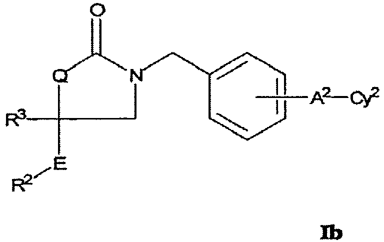 oder ein pharmazeutisch unbedenkliches Salz, Enantiomer oder Diastereomer davon.

5. Verbindung nach Anspruch 1 oder 2, wobei die Verbindung eine Verbindung mit Formel (Ic) ist: 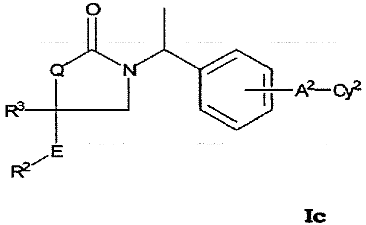 oder ein pharmazeutisch unbedenkliches Salz, Enantiomer oder Diastereomer davon.

6. Verbindung nach Anspruch 1 oder 2, wobei die Verbindung eine Verbindung mit Formel (Id) ist: 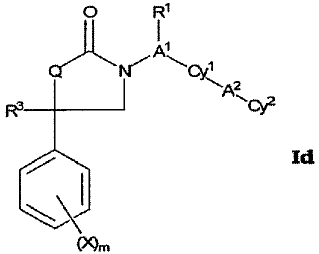 wobei:
X Fluor, Chlor, Brom, Iod, Cyano, Nitro, Amino, Hydroxy, Carboxy, (C₁-C₆)Alkyl, Hydroxy(C₁-C₆)alkyl, (C₃-C₆)Cycloalkyl, Hydroxy(C₃-C₆)cycloalkyl, (C₄-C₇)Cycloalkylalkyl, (C₂-C₆)Alkinyl, (C₃-C₆)Cycloalkyl(C₂-C₄)alkinyl, Halogen(C₁-C₆)alkyl, Halogen(C₃-C₆)cycloalkyl, Halogen(C₄-C₇)cycloalkylalkyl, (C₁-C₆)Alkoxy, (C₃-C₆)Cycloalkoxy, (C₄-C₇)Cycloalkylalkoxy, Halogen(C₁-C₆)alkoxy, Halogen(C₃-C₆)cycloalkoxy, Halogen(C₄-C₇)cycloalkylalkoxy, (C₁-C₆)Alkylthio, (C₃-C₆)Cycloalkylthio, (C₄-C₇)Cycloalkylalkylthio, Halogen(C₁-C₆)alkylthio, Halogen(C₃-C₆)cycloalkylthio, Halogen(C₄-C₇)cycloalkylalkylthio, (C₁-C₆)Alkansulfinyl, (C₃-C₆)Cycloalkansulfinyl, (C₄-C₇)Cycloalkylalkansulfinyl, Halogen(C₁-C₆)alkansulfinyl, Halogen(C₃-C₆)cycloalkansulfinyl, Halogen(C₄-C₇)cycloalkylalkansulfinyl, (C₁-C₆)Alkansulfonyl, (C₃-C₆)Cycloalkansulfonyl, (C₄-C₇)Cycloalkylalkansulfonyl, Halogen(C₁-C₆)alkansulfonyl, Halogen(C₃-C₆)cycloalkansulfonyl, Halogen(C₄-C₇)cycloalkylalkansulfonyl, (C₁-C₆)Alkylamino, Di(C₁-C₆)alkylamino, (C₁-C₆)Alkoxy(C₁-C₆)alkoxy, Halogen(C₁-C₆)alkoxy(C₁-C₆)alkoxy, (C₁-C₆)Alkoxycarbonyl, H₂NCO, H₂NSO₂, (C₁-C₆)Alkylaminocarbonyl, Di(C₁-C₆)alkylaminocarbonyl, (C₁-C₃)Alkoxy(C₁-C₃)alkylaminocarbonyl, Heterocyclylcarbonyl, (C₁-C₆)Alkylaminosulfonyl, Di(C₁-C₆)alkylaminosulfonyl, Heterocyclosulfonyl, (C₁-C₆)Alkylcarbonylamino, (C₁-C₆)Alkylcarbonylamino(C₁-C₆)alkyl, (C₁-C₆)Alkylsulfonylamino, (C₁-C₆)Alkylsulfonylamino(C₁-C₆)alkyl, (C₁-C₆)Alkoxycarbonyl(C₁-C₆)alkoxy, (C₁-C₆)Alkoxy(C₁-C₆)alkyl, Halogen(C₁-C₆)alkoxy(C₁-C₆)alkyl, Hydroxy(C₁-C₆)alkoxy, Heteroaryl, Amino(C₁-C₆)alkyl, (C₁-C₆)Alkylamino(C₁-C₆)alkyl, Di(C₁-C₆)alkylamino(C₁-C₆)alkylamino(C₂-C₆)alkoxy, (C₁-C₆)Alkylamino(C₂-C₆)alkoxy, Di(C₁-C₆)Alkylamino(C₂-C₆)alkoxyl und (C₁-C₆)Alkylcarbonyl;
und m 0, 1, 2, 3 oder 4 ist;
oder ein pharmazeutisch unbedenkliches Salz, Enantiomer oder Diastereomer davon.

7. Verbindung nach Anspruch 1 oder 2, wobei die Verbindung eine Verbindung mit Formel (le) ist: 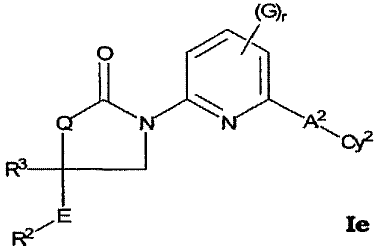 wobei:
G unabhängig ausgewählt ist aus Fluor, Chlor, Brom, lod, Cyano, Nitro, Amino, Hydroxy, Carboxy, (C₁-C₆)Alkyl, Hydroxy(C₁-C₆)alkyl, (C₃-C₆)Cycloalkyl, Hydroxy(C₃-C₆)cycloalkyl, (C₄-C₇)Cycloalkylalkyl, (C₂-C₆)Alkinyl, (C₃-C₆)Cycloalkyl(C₂-C₄)alkinyl, Halogen(C₁-C₆)alkyl, Halogen(C₃-C₆)cycloalkyl, Halogen(C₄-C₇)cycloalkylalkyl, (C₁-C₆)Alkoxy, (C₃-C₆)Cycloalkoxy, (C₄-C₇)Cycloalkylalkoxy, Halogen(C₁-C₆)alkoxy, Halogen(C₃-C₆)cycloalkoxy, Halogen(C₄-C₇)cycloalkylalkoxy, (C₁-C₆)Alkylthio, (C₃-C₆)Cycloalkylthio, (C₄-C₇)Cycloalkylalkylthio, Halogen(C₁-C₆)alkylthio, Halogen(C₃-C₆)cycloalkylthio, Halogen(C₄-C₇)cycloalkylalkylthio, (C₁-C₆)Alkansulfinyl, (C₃-C₆)Cycloalkansulfinyl, (C₄-C₇)Cycloalkylalkansulfinyl, Halogen(C₁-C₆)alkansulfinyl, Halogen(C₃-C₆)cycloalkansulfinyl, Halogen(C₄-C₇)cycloalkylalkansulfinyl, (C₁-C₆)Alkansulfonyl, (C₃-C₆)Cycloalkansulfonyl, (C₄-C₇)Cycloalkylalkansulfonyl, Halogen(C₁-C₆)alkansulfonyl, Halogen(C₃-C₆)cycloalkansulfonyl, Halogen(C₄-C₇)cycloalkylalkansulfonyl, (C₁-C₆)Alkylamino, Di(C₁-C₆)alkylamino, (C₁-C₆)Alkoxy(C₁-C₆)alkoxy, Halogen(C₁-C₆)alkoxy(C₁-C₆)alkoxy, (C₁-C₆)Alkoxycarbonyl, H₂NCO, H₂NSO₂, (C₁-C₆)Alkylaminocarbonyl, Di(C₁-C₆)alkylaminocarbonyl, (C₁-C₃)Alkoxy(C₁-C₃)alkylaminocarbonyl, Heterocyclylcarbonyl, (C₁-C₆)Alkylaminosulfonyl, Di(C₁-C₆)alkylaminosulfonyl, Heterocyclosulfonyl, (C₁-C₆)Alkylcarbonylamino, (C₁-C₆)Alkylcarbonylamino(C₁-C₆)alkyl, (C₁-C₆)Alkylsulfonylamino, (C₁-C₆)Alkylsulfonylamino(C₁-C₆)alkyl, (C₁-C₆)Alkoxycarbonyl(C₁-C₆)alkoxy, (C₁-C₆)Alkoxy(C₁-C₆)alkyl, Halogen(C₁-C₆)alkoxy(C₁-C₆)alkyl, Hydroxy(C₁-C₆)alkoxy, Heteroaryl, Oxo, Amino(C₁-C₆)alkyl, (C₁-C₆)Alkylamino(C₁-C₆)alkyl, Di(C₁-C₆)alkylamino(C₁-C₆)alkylamino(C₂-C₆)alkoxy, (C₁-C₆)Alkylamino(C₂-C₆)alkoxy, Di(C₁-C₆)Alkylamino(C₂-C₆)alkoxyl und (C₁-C₆)Alkylcarbonyl; und
r 0, 1, 2, 3 oder 4 ist;
oder ein pharmazeutisch unbedenkliches Salz, Enantiomer oder Diastereomer davon.

8. Verbindung nach Anspruch 1 oder 2, wobei die Verbindung eine Verbindung mit Formel (If) ist: 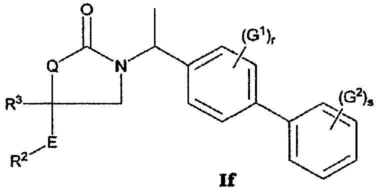 wobei:
G¹ und G² jeweils unabhängig ausgewählt sind aus Fluor, Chlor, Brom, lod, Cyano, Nitro, Amino, Hydroxy, Carboxy, (C₁-C₆)Alkyl, Hydroxy(C₁-C₆)alkyl, (C₃-C₆)Cycloalkyl, Hydroxy(C₃-C₆)cycloalkyl, (C₄-C₇)Cycloalkylalkyl, (C₂-C₆)Alkinyl, (C₃-C₆)Cycloalkyl(C₂-C₄)alkinyl, Halogen(C₁-C₆)alkyl, Halogen(C₃-C₆)cycloalkyl, Halogen(C₄-C₇)cycloalkylalkyl, (C₁-C₆)Alkoxy, (C₃-C₆)Cycloalkoxy, (C₄-C₇)Cycloalkylalkoxy, Halogen(C₁-C₆)alkoxy, Halogen(C₃-C₆)cycloalkoxy, Halogen(C₄-C₇)cycloalkylalkoxy, (C₁-C₆)Alkylthio, (C₃-C₆)Cycloalkylthio, (C₄-C₇)Cycloalkylalkylthio, Halogen(C₁-C₆)alkylthio, Halogen(C₃-C₆)cycloalkylthio, Halogen(C₄-C₇)cycloalkylalkylthio, (C₁-C₆)Alkansulfinyl, (C₃-C₆)Cycloalkansulfinyl, (C₄-C₇)Cycloalkylalkansulfinyl, Halogen(C₁-C₆)alkansulfinyl, Halogen(C₃-C₆)cycloalkansulfinyl, Halogen(C₄-C₇)cycloalkylalkansulfinyl, (C₁-C₆)Alkansulfonyl, (C₃-C₆)Cycloalkansulfonyl, (C₄-C₇)Cycloalkylalkansulfonyl, Halogen(C₁-C₆)alkansulfonyl, Halogen(C₃-C₆)cycloalkansulfonyl, Halogen(C₄-C₇)cycloalkylalkansulfonyl, (C₁-C₆)Alkylamino, Di(C₁-C₆)alkylamino, (C₁-C₆)Alkoxy(C₁-C₆)alkoxy, Halogen(C₁-C₆)alkoxy(C₁-C₆)alkoxy, (C₁-C₆)Alkoxycarbonyl, H₂NCO, H₂NSO₂, (C₁-C₆)Alkylaminocarbonyl, Di(C₁-C₆)alkylaminocarbonyl, (C₁-C₃)Alkoxy(C₁-C₃)alkylaminocarbonyl, Heterocyclylcarbonyl, (C₁-C₆)Alkylaminosulfonyl, Di(C₁-C₆)alkylaminosulfonyl, Heterocyclosulfonyl, (C₁-C₆)Alkylcarbonylamino, (C₁-C₆)Alkylcarbonylamino(C₁-C₆)alkyl, (C₁-C₆)Alkylsulfonylamino, (C₁-C₆)Alkylsulfonylamino(C₁-C₆)alkyl, (C₁-C₆)Alkoxycarbonyl(C₁-C₆)alkoxy, (C₁-C₆)Alkoxy(C₁-C₆)alkyl, Halogen(C₁-C₆)alkoxy(C₁-C₆)alkyl, Hydroxy(C₁-C₆)alkoxy, Heteroaryl, Amino(C₁-C₆)alkyl, (C₁-C₆)Alkylamino(C₁-C₆)alkyl, Di(C₁-C₆)alkylamino(C₁-C₆)alkylamino(C₂-C₆)alkoxy, (C₁-C₆)Alkylamino(C₂-C₆)alkoxy, Di(C₁-C₆)Alkylamino(C₂-C₆)alkoxyl oder (C₁-C₆)Alkylcarbonyl;
R⁵ H, (C₁-C₆)Alkyl, Halogen(C₁-C₆)alkyl oder Hydroxy(C₁-C₆)alkyl ist; und
r und s unabhängig 0, 1, 2, 3 oder 4 sind;
oder ein pharmazeutisch unbedenkliches Salz, Enantiomer oder Diastereomer davon.

9. Verbindung nach einem der Ansprüche 1 bis 8, wobei Cy² wahlweise durch Aryl oder Heteroaryl substituiert ist.

10. Verbindung nach einem der Ansprüche 1 bis 8, wobei Cy² wahlweise durch Cycloalkyl oder Heterocyclyl substituiert ist.

11. Verbindung nach Anspruch 10, wobei Cy² 1,2-Dihydro-2-oxopyridyl oder 1,2-Dihydro-1-methyl-2-oxopyridyl ist.

12. Verbindung nach Anspruch 10, wobei Cy² wahlweise durch 1,2-Dihydro-2-oxopyridyl oder 1,2-Dihydro-1-methyl-2-oxopyridyl substituiert ist.

13. Pharmazeutische Zusammensetzung umfassend: i) einen pharmazeutisch unbedenklichen Träger oder Verdünner; und ii) die Verbindung in einem der Ansprüche 1-12, oder ein pharmazeutisch unbedenkliches Salz, Enantiomer oder Diastereomer davon.

14. Verbindung nach einem der Ansprüche 1 bis 12 zur Verwendung bei der Behandlung einer Krankheit oder Störung, die mit Aktivität oder Expression von 11β-HSD1 in einem Säugetier in Verbindung steht.

## Revendications

1. Composé de Formule (I) : 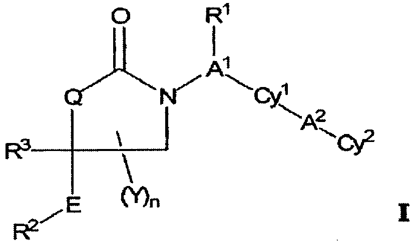 dans lequel :
R¹ est (a) absent ou (b) sélectionné parmi un alkyle en (C₁-C₆), un alkényle en (C₂-C₆), un alkynyle en (C₂-C₆) et un [alkoxy en (C₁-C₃)]alkyle en (C₁-C₃) qui, en option, sont chacun substitués par jusqu'à quatre groupements indépendamment sélectionnés parmi un fluor, cyano, oxo, R⁴, R⁴O-, (R⁴)₂N-, R⁴O₂C-, R⁴S-, R⁴S(=O)-, R⁴S(=O)₂-, R⁴C(=O)NR⁴-, (R⁴)₂NC(=O)-, (R⁴)₂NC(=O)O-, (R⁴)₂NC(=O)NR⁴-, R⁴OC(=O)NR⁴-, (R⁴)₂NC(=NCN)NR⁴-, (R⁴O)₂P(=O)O-, (R⁴O)₂P(=O)NR⁴-, R⁴OS(=O)₂NR⁴-, (R⁴)₂NS(=O)₂O-, (R⁴)₂NS(=O)₂NR⁴-, R⁴S(=O)₂NR⁴-, R⁴S(=O)₂NHC(=O)-, R⁴S(=O)₂NHC(=O)O-, R⁴S(=O)₂NHC(=O)NR⁴-, R⁴OS(=O)₂NHC(=O)-, R⁴OS(=O)₂NHC(=O)O-, R⁴OS(=O)₂NHC(=O)NR⁴-, (R⁴)₂NS(=O)₂NHC(=O)-, (R⁴)₂NS(=O)₂NHC(=O)O-, (R⁴)₂NS(=O)₂NHC(=O)NR⁴-, R⁴C(=O)NHS(=O)₂, R⁴C(=O)NHS(=O)₂O-, R⁴C(=O)NHS(=O)₂NR⁴-, R⁴OC(=O)NHS(=O)₂-, R⁴OC(=O)NHS(=O)₂O-, R⁴OC(=O)NHS(=O)₂NR⁴-, (R⁴)₂NC(=O)NHS(=O)₂-, (R⁴)₂NC(=O)NHS(=O)₂O-, (R⁴)₂NC(=O)NHS(=O)₂NR⁴-, aryle, cycloalkyle, hétérocyclyle, hétéroaryle, arylamino et hétéroarylamino ;
A¹ est (a) une liaison ; ou (b) un alkylène en (C₁-C₃), un CH₂CH₂O dans lequel l'oxygène est attaché à Cy¹ ou un CH₂C(=O) dans lequel le carbone du carbonyle est attaché à Cy¹ ;
Cy¹ est un aryle, un hétéroaryle ou un cycloalkyle ou un hétérocyclyle monocyclique qui, en option, sont chacun substitués par un 1 à 4 groupements indépendamment sélectionnés parmi un fluoré, chlore, brome, iode, cyano, nitro, amino, hydroxy, carboxy, alkyle en (C₁-C₆), hydroxyalkyle en (C₁-C₆), cycloalkyle en (C₃-C₆), hydroxycycloalkyle en (C₃-C₆), cycloalkyle en (C₄-C₇)alkyle, alkényle en (C₂-C₆), haloalkényle en (C₂-C₆), hydroxyalkényle en (C₂-C₆), alkynyle en (C₂-C₆), [cycloalkyle en (C₃-C₆)lalkynyle en (C₂-C₄), haloalkyle en (C₁-C₆), halocycloalkyle en (C₃-C₆), halocycloalkyle en (C₄-C₇)alkyle, alkoxy en (C₁-C₆), cycloalkoxy en (C₃-C₆), cycloalkyle en (C₄-C₇)alkoxy, haloalkoxy en (C₁-C₆), halocycloalkoxy en (C₃-C₆), halocycloalkyle en (C₄-C₇)alkoxy, alkyle en (C₁-C₆)thio, cycloalkyle en (C₃-C₆)thio, cycloalkyle en (C₄-C₇)alkylthio, haloalkyle en (C₁-C₆)thio, halocycloalkyle en (C₃-C₆)thio, halocycloalkyle en (C₄-C₇)alkylthio, alkane en (C₁-C₆)sulfinyle, cycloalkane en (C₃-C₆)sulfinyle, cycloalkyle en (C₄-C₇)alkanesulfinyle, haloalkane en (C₁-C₆)sulfinyle, halocycloalkane en (C₃-C₆)sulfinyle, halocycloalkyle en (C₄-C₇)alkanesulfinyle, alkane en (C₁-C₆)suifonyle, cycloalkane en (C₃-C₆)sulfonyle, cycloalkyle en (C₄-C₇)alkanesulfonyle, haloalkane en (C₁-C₆)sulfonyle, halocycloalkane en (C₃-C₆)sulfonyle, halocycloalkyle en (C₄-C₇)alkanesulfonyle, alkyle en (C₁-C₆)amino, di[alkyle en (C₁-C₆)]amino, [alkoxy en (C₁-C₆)]alkoxy en (C₁-C₆), halo[alkoxy en (C₁-C₆)]alkoxy en (C₁-C₆), alkoxy en (C₁-C₆)carbonyle, H₂NCO, H₂NSO₂, alkyle en (C₁-C₆)aminocarbonyle, di[alkyle en (C₁-C₆)]aminocarbonyle, [alkoxy en (C₁-C₃)][alkyle en (C₁-C₃)]aminocarbonyle, hétérocyclylcarbonyle, alkyle en (C₁-C₆)aminosulfonyle, di[alkyle en (C₁-C₆)]aminosulfonyle, hétérocyclosulfonyle, alkyle en (C₁-C₆)carbonylamino, [alkyle en (C₁-C₆)]carbonylaminoalkyle en (C₁-C₆), alkyle en (C₁-C₆)sulfonylamino, [alkyle en (C₁-C₆)sulfonylaminoalkyle en (C₁-C₆), alkoxy en (C₁-C₆)carbonyle, alkoxy en (C₁-C₆), [alkoxy en (C₁-C₆)]alkyle en (C₁-C₆), halo[alkoxy en (C₁-C₆)]alkyle en (C₁-C₆), hydroxyalkoxy en (C₁-C₆), hétéroaryle, oxo, aminoalkyle en (C₁-C₆), [alkyle en (C₁-C₆)]aminoalkyle en (C₁-C₆), di[alkyle en (C₁-C₆)]aminoalkyle en (C₁-C₆), aminoalkoxy en (C₂-C₆), [alkyle en (C₁-C₆)]aMinoalkoxy en (C₂-C₆), di[alkyle en (C₁-C₆)]aminoalkoxy en (C₂-C₆), alkyle en (C₁-C₆)carbonyle, cycloalkyle en (C₃-C₆)carbonyle, cycloalkyle en (C₃-C₆)aminocarbonyle, [cycloalkyle en (C₃-C₆)][alkyle en (C₁-C₆)]aminocarbonyle, di[cycloalkyle en (C₃-C₆)]aminocarbonyle, cycloalkyle en (C₃-C₆)aminosulfonyle, [cycloalkyle en (C₃-C₆)][alkyle en C₁-C₆)]aminosuifonyle, di[cycloalkyle en (C₃-C₆)]aminosulfonyle, cyanoalkyle en (C₁-C₆), aminocarbonylalkyle en (C₁-C₆), [alkyle en (C₁-C₆)]aminocarbonylalkyle en (C₁-C₆), di[alkyle en (C₁-C₆)]aminocarbonylalkyle en (C₁-C₆), [cycloalkyle en (C₃-C₆)]aminocarbonylalkyle en (C₁-C₆), [cycloalkyle en (C₃-C₆)][alkyle en (C₁-C₆)]aminocarbonylalkyle en (C₁-C₆) et di[cycloalkyle en (C₃-C₆)]aminocarbonylalkyle en (C₁-C₆) ;
A² est (a) une liaison, un O, S ou NR⁴ ; ou (b) un alkylène en (C₁-C₃) ou un alkylène en (C₁-C₂)oxy qui, en option, sont chacun substitués par 1 à 4 groupements indépendamment sélectionnés parmi un méthyle, éthyle, trifluorométhyle ou oxo ;
Cy² est un aryle, hétéroaryle, cycloalkyle ou hétérocyclyle qui, en option, sont chacun substitués par 1 à 4 groupements indépendamment sélectionnés parmi un fluore, chlore, brome, iode, cyano, nitro, amino, hydroxy, carboxy, alkyle en (C₁-C₆), hydroxyalkyle en (C₁-C₆), cycloalkyle en (C₃-C₆), hydroxycycloalkyle en (C₃-C₆), cycloalkyle en (C₄-C₇)alkyle, alkényle en (C₂-C₆), haloalkényle en (C₂-C₆), hydroxyalkényle en (C₂-C₆), alkynyle en (C₂-C₆), [cycloalkyle en (C₃-C₆)]alkynyle en (C₂-C₄), haloalkyle en (C₁-C₆), halocycloalkyle en (C₃-C₆), halocycloalkyle en (C₄-C₇) alkyle, alkoxy en (C₁-C₆), cycloalkoxy en (C₃-C₆), cycloalkyle en (C₄-C₇)alkoxy, haloalkoxy en (C₁-C₆), halocycloalkoxy en (C₃-C₆), halocycloalkyle en (C₄-C₇)alkoxy, alkyle en (C₁-C₆)thio, cycloalkyle en (C₃-C₆)thio, cycloalkyle en (C₄-C₇)alkylthio, haloalkyle en (C₁-C₆)thio, halocycloalkyle en (C₃-C₆)thio, halocycloalkyle en (C₄-C₇)alkylthio, alkane en (C₁-C₆)sulfinyle, cycloalkane en (C₃-C₆)sulfinyle, cycloalkyle en (C₄-C₇)alkanesulfinyle, haloalkane en (C₁-C₆)sulfinyle, halocycloalkane en (C₃-C₆)sulfinyle, halocycloalkyle en (C₄-C₇)alkanesulfinyle, alkane en (C₁-C₆)sulfonyle, cycloalkane en (C₃-C₆)sulfonyle, cycloalkyle en (C₄-C₇)alkanesulfonyle, haloalkane en (C₁-C₆)sulfonyle, halocycloalkane en (C₃-C₆)sulfonyle, halocycloalkyle en (C₄-C₇)alkanesulfonyle, alkyle en (C₁-C₆)amino, di[alkyle en (C₁-C₆)]amino, [alkoxy en (C₁-C₆)]alkoxy en (C₁-C₆), halo[alkoxy en (C₁-C₆)]alkoxy en (C₁-C₆), alkoxy en (C₁-C₆)carbonyle, H₂NCO, H₂NSO₂, alkyle en (C₁-C₆)aminocarbonyle, di[alkyle en (C₁-C₆)]aminocarbonyle, [alkoxy en (C₁-C₃)][alkyle en (C₁-C₃)]aminocarbonyle, hétérocyclylcarbonyle, alkyle en (C₁-C₆)aminosuifonyle, di[alkyle en (C₁-C₆)]aMinosulfonyle, hétérocyclosulfonyle, alkyle en (C₁-C₆)carbonylamino, [alkyle en (C₁-C₆)]carbonylaminoalkyle en (C₁-C₆), alkyle en (C₁-C₆)sulfonylamino, [alkyle en (C₁-C₆)]sulfonylaminoalkyle en (C₁-C₆), [alkoxy en (C₁-C₆)]carbonylalkoxy en (C₁-C₆), [alkoxy en (C₁-C₆)]alkyle en (C₁-C₆), halo[alkoxy en (C₁-C₆)]alkyle en (C₁-C₆), hydroxyalkoxy en (C₁-C₆), hétéroaryle, oxo, aminoalkyle en (C₁-C₆), [alkyle en (C₁-C₆)]aminoalkyle en (C₁-C₆), di[alkyle en (C₁-C₆)]aminoalkyle en (C₁-C₆), aminoalkoxy en (C₂-C₆), [alkyle en (C₁-C₆)]aminoalkoxy en (C₂-C₆), di[alkyle en (C₁-C₆)]aminoalkoxy en (C₂-C₆), alkyle en (C₁-C₆)carbonyle, cycloalkyle en (C₃-C₆)carbonyle, cycloalkyle en (C₃-C₆)aminocarbonyle, [cycloalkyle en (C₃-C₆)][alkyle en (C₁-C₆)]aminocarbonyle, di[cycloalkyle en (C₃-C₆)]aminocarbonyle, cycloalkyle en (C₃-C₆)aminosulfonyle, [cycloalkyle en (C₃-C₆)][alkyle en (C₁-C₆)]aminosulfonyle, di[cycloalkyle en (C₃-C₆)]aminosulfonyle, cyanoalkyle en (C₁-C₆), aminocarbonylalkyle en (C₁-C₆), [alkyle en (C₁-C₆)]aminocarbonylalkyle en (C₁-C₆), di[alkyle en (C₁-C₆)]aminocarbonylalkyle en (C₁-C₆), [cycloalkyle en (C₃-C₆)]aminocarbonylalkyle en (C₁-C₆), [cycloalkyle en (C₃-C₆)][alkyle en (C₁-C₆)]aminocarbonylalkyle en (C₁-C₆) et di[cycloalkyle en (C₃-C₆)]aminocarbonylalkyle en (C₁-C₆) ;
Y est un alkyle en (C₁-C₆) ou un haloalkyle en (C₁-C₆) ;
n prend la valeur 0, 1 ou 2 ;
E est (a) une liaison ou (b) un alkylène en (C₁-C₃) or alkylényle en (C₁-C₂)oxy dans lequel l'oxygène est attaché à R² qui, en option, sont chacun substitués par 1 à 4 groupements indépendamment sélectionnés parmi un méthyle, éthyle, trifluorométhyle ou oxo ;
R² est un aryle, hétéroaryle, cycloalkyle ou hétérocyclyle qui, en option, sont chacun substitués parjusqu'à 4 groupements indépendamment sélectionnés parmi un fluore, chlore, brome, iode, cyano, nitro, amino, hydroxy, carboxy, alkyle en (C₁-C₆), hydroxyalkyle en (C₁-C₆), cycloalkyle en (C₃-C₆), hydroxycycloalkyle en (C₃-C₆), cycloalkyle en (C₄-C₇)alkyle, alkényle en (C₂-C₆), haloalkényle en (C₂-C₆), hydroxyalkényle en (C₂-C₆), alkynyle en (C₂-C₆), [cycloalkyle en (C₃-C₆)]alkynyle en (C₂-C₄), haloalkyle en (C₁-C₆), halocycloalkyle en (C₃-C₆), halocycloalkyle en (C₄-C₇)alkyle, alkoxy en (C₁-C₆), cycloalkoxy en (C₃-C₆), cycloalkyle en (C₄-C₇)alkoxy, haloalkoxy en (C₁-C₆), halocycloalkoxy en (C₃-C₆), halocycloalkyle en (C₄-C₇)alkoxy, alkyle en (C₁-C₆)thio, cycloalkyle en (C₃-C₆)thio, cycloalkyle en (C₄-C₇)alkylthio, haloalkyle en (C₁-C₆)thio, halocycloalkyle en (C₃-C₆)thio, halocycloalkyle en (C₄-C₇)alkylthio, alkane en (C₁-C₆)sulfinyle, cycloalkane en (C₃-C₆)sulfinyle, cycloalkyle en (C₄-C₇)alkanesulfinyle, haloalkane en (C₁-C₆)sulfinyle, halocycloalkane en (C₃-C₆)sulfinyle, halocycloalkyle en (C₄-C₇)alkanesulfinyle, alkane en (C₁-C₆)sulfonyle, cycloalkane en (C₃-C₆)sulfonyle, cycloalkyle en (C₄-C₇)alkanesulfonyle, haloalkane en (C₁-C₆)sulfonyle, halocycloalkane en (C₃-C₆)sulfonyle, halocycloalkyle en (C₄-C₇)alkanesulfonyle, alkyle en (C₁-C₆)amino, di[alkyle en (C₁-C₆)]amino, [alkoxy en (C₁-C₆)lalkoxy en (C₁-C₆), halo[alkoxy en (C₁-C₆)]alkoxy en (C₁-C₆), alkoxy en (C₁-C₆)carbonyle, H₂NCO, H₂NSO₂, alkyle en (C₁-C₆)aminocarbonyle, di[alkyle en (C₁-C₆)]aminocarbonyle, [alkoxy en (C₁-C₃)1[alkyle en (C₁-C₃)]aminocarbonyle, hétérocyclylcarbonyle, alkyle en (C₁-C₆)aminosulfonyle, di[alkyle en (C₁-C₆)]aminosulfonyle, hétérocyclosulfonyle, alkyle en (C₁-C₆)carbonylamino, [alkyle en (C₁-C₆)lcarbonylaminoalkyle en (C₁-C₆), alkyle en (C₁-C₆)sulfonylamino, [alkyle en (C₁-C₆)]sulfonylaminoalkyle en (C₁-C₆), [alkoxy en (C₁-C₆)]carbonylalkoxy en (C₁-C₆), [alkoxy en (C₁-C₆)]alkyle en (C₁-C₆), halo[alkoxy en (C₁-C₆)]alkyle en (C₁-C₆), hydroxyalkoxy en (C₁-C₆), hétéroaryle, oxo, aminoalkyle en (C₁-C₆), [alkyle en (C₁-C₆)]aminoalkyle en (C₁-C₆), di[alkyle en (C₁-C₆)]aminoalkyle en (C₁-C₆), aminoalkoxy en (C₂-C₆), [alkyle en (C₁-C₆)]aminoalkoxy en (C₂-C₆), di[alkyle en (C₁-C₆)laminoalkoxy en (C₂-C₆), alkyle en (C₁-C₆)carbonyle, cycloalkyle en (C₃-C₆)carbonyle, cycloalkyle en (C₃-C₆)aminocarbonyle, [cycloalkyle en (C₃-C₆)][alkyle en (C₁-C₆)]aminocarbonyle, di[cycloalkyle en (C₃-C₆)][alkyle en (C₁-C₆)]aminocarbonyle, cycloalkyle en (C₃-C₆)aminosulfonyle, [cycloalkyle en (C₃-C₆)][alkyle en (C₁-C₆)laminosulfonyle, di[cycloalkyle en (C₃-C₆)][alkyle en (C₁-C₆)]aminosulfonyle, cyanoalkyle en (C₁-C₆), aminocarbonylalkyle en (C₁-C₆), [alkyle en (C₁-C₆)]aMinocarbonylalkyle en (C₁-C₆), di[alkyle en (C₁-C₆)]aminocarbonylalkyle en (C₁-C₆), [cycloalkyle en (C₃-C₆)]aminocarbonylalkyle en (C₁-C₆), [cycloalkyle en (C₃-C₆)][alkyle en (C₁-C₆)]aMinocarbonylalkyle en (C₁-C₆) et di[cycloalkyle en (C₃-C₆)][alkyle en (C₁-C₆)]aminocarbonylalkyle en (C₁-C₆) ;
R³ est sélectionné parmi un alkyle en (C₂-C₆), alkényle en (C₂-C₆), alkynyle en (C₂-C₆) et [alkoxy en (C₁-C₃)]alkyle en (C₁-C₃), l'alkyle en (C₂-C₆) et, en option, l'alkényle en (C₂-C₆), l'alkynyle en (C₂-C₆) et l'[alkoxy en (C₁-C₃)]alkyle en (C₁-C₃) étant chacun substitués par jusqu'à quatre groupements indépendamment sélectionnés parmi un fluore, cyano, oxo, haloalkyle en (C₁-C₆), aminoalkyle en (C₁-C₆), [alkyle en (C₁-C₆)]aminoalkyle en (C₁-C₆), di[alkyle en (C₁-C₆)]aminoalkyle en (C₁-C₆), hydroxyalkyle en (C₁-C₆), [alkoxy en (C₁-C₆)]alkyle en (C₁-C₆), R⁴O-, (R⁴)₂N-, R⁴O₂C-, R⁴S-, R⁴S(=O)-, R⁴S(=O)₂-, R⁴C(=O)NR⁴-, (R⁴)₂NC(=O)-, (R⁴)₂NC(=O)O-, (R⁴)₂NC(=O)NR⁴-, R⁴OC(=O)NR⁴-, (R⁴)₂NC(=NCN)NR⁴-, (R⁴O)₂P(=O)O-, (R⁴O)₂P(=O)NR⁴-, R⁴OS(=O)₂NR⁴-, (R⁴)₂NS(=O)₂O-, (R⁴)₂NS(=O)₂NR⁴-, R⁴S(=O)₂NR⁴-, R^{4S}(=O)₂NHC(=O)-, R⁴S(=O)₂NHC(=O)O-, R⁴S(=O)₂NHC(=O)NR⁴-, R⁴OS(=O)₂NHC(=O)-, R⁴OS(=O)₂NHC(=O)O-, R⁴OS(=O)₂NHC(=O)NR⁴-, (R⁴)₂NS(=O)₂NHC(=O)-, (R⁴)₂NS(=O)₂NHC(=O)O-, (R⁴)₂NS(=O)₂NHC(=O)NR⁴-, R⁴C(=O)NHS(=O)₂-, R⁴C(=O)NHS(=O)₂O-, R⁴C(=O)NHS(=O)₂NR⁴-, R⁴OC(=O)NHS(=O)₂-, R⁴OC(=O)NHS(=O)₂O-, R⁴OC(=O)NHS(=O)₂NR⁴-, (R⁴)₂NC(=O)NHS(=O)₂-, (R⁴)₂NC(=O)NHS(=O)₂O-, (R⁴)₂NC(=O)NHS(=O)₂NR⁴-, hétérocyclyle (qui, en option, peut lui-même être substitué par un alkyle, un haloalkyle ou un oxo), hétéroaryle (qui, en option, peut lui-même être substitué par un alkyle, haloalkyle, alkoxy, alkylthio, alkylsulfonyle, halogène, trifluorométhyle, dialkylamino, nitro, cyano, CO₂H, CONH₂, *N*-amido substitué par un monoalkyle, *N*,*N*-amido substitué par un dialkyle ou oxo), arylamino (qui, en option, peut lui-même être substitué par un alkyle, alkoxy, alkylthio, alkylsulfonyle, halogène, trifluorométhyle, dialkylamino, nitro, cyano, CO₂H, CONH₂, *N*-amido substitué par un monoalkyle et *N*,*N*-amido substitué par un dialkyle) et hétéroarylamino (qui, en option, peut lui-même être substitué par un alkyle, haloalkyle, alkoxy, alkylthio, alkylsulfonyle, halogène, trifluorométhyle, dialkylamino, nitro, cyano, CO₂H, CONH₂, *N*-amido substitué par un monoalkyle, *N*,*N-*amido substitué par un dialkyle ou oxo) ;
R⁴ est indépendamment sélectionné parmi un H, alkyle en (C₁-C₆), haloalkyle en (C₁-C₆), aminoalkyle en (C₁-C₆), [alkyle en (C₁-C₆)]aminoalkyle en (C₁-C₆), di[alkyle en (C₁-C₆)]aminoalkyle en (C₁-C₆), hydroxyalkyle en (C₁-C₆) et [alkoxy en (C₁-C₆)]alkyle en (C₁-C₆) ;
Q = O ou NR⁵ ; et
R⁵ est un H, alkyle en (C₁-C₆), haloalkyle en (C₁-C₆) ou hydroxyalkyle en (C₁-C₆) ;
ou sel, énantiomère ou diastéréoisomère pharmaceutiquement acceptable de celui-ci.

2. Composé selon la revendication 1 dans lequel :
Cy¹ est un aryle, un hétéroaryle ou un cycloalkyle ou un hétérocyclyle monocyclique qui, en option, sont chacun substitués par 1 à 4 groupements indépendamment sélectionnés parmi un fluore, chlore, brome, iode, cyano, nitro, amino, hydroxy, carboxy, alkyle en (C₁-C₆), hydroxyalkyle en (C₁-C₆), cycloalkyle en (C₃-C₆), hydroxycycloalkyle en (C₃-C₆), cycloalkyle en (C₄-C₇)alkyle, alkényle en (C₂-C₆), haloalkényle en (C₂-C₆), hydroxyalkényle en (C₂-C₆), alkynyle en (C₂-C₆), [cycloalkyle en (C₃-C₆)]alkynyle en (C₂-C₄), haloalkyle en (C₁-C₆), halocycloalkyle en (C₃-C₆), halocycloalkyle en (C₄-C₇)alkyle, alkoxy en (C₁-C₆), cycloalkoxy en (C₃-C₆), cycloalkyle en (C₄-C₇)alkoxy, haloalkoxy en (C₁-C₆), halocycloalkoxy en (C₃-C₆), halocycloalkyle en (C₄-C₇)alkoxy, alkyle en (C₁-C₆)thio, cycloalkyle en (C₃-C₆)thio, cycloalkyle en (C₄-C₇)alkylthio, haloalkyle en (C₁-C₆)thio, halocycloalkyle en (C₃-C₆)thio, halocycloalkyle en (C₄-C₇)alkylthio, alkane en (C₁-C₆)sulfinyle, cycloalkane en (C₃-C₆)sulfinyle, cycloalkyle en (C₄-C₇)alkanesulfinyle, haloalkane en (C₁-C₆)sulfinyle, halocycloalkane en (C₃-C₆)sulfinyle, halocycloalkyle en (C₄-C₇)alkanesulfinyle, alkane en (C₁-C₆)sulfonyle, cycloalkane en (C₃-C₆)sulfonyle, cycloalkyle en (C₄-C₇)alkanesulfonyle, haloalkane en (C₁-C₆)sulfonyle, halocycloalkane en (C₃-C₆)sulfonyle, halocycloalkyle en (C₄-C₇)alkanesulfonyle, alkyle en (C₁-C₆)amino, di[alkyle en (C₁-C₆)]amino, [alkoxy en (C₁-C₆)]alkoxy en (C₁-C₆), halo[alkoxy en (C₁-C₆)]alkoxy en (C₁-C₆), alkoxy en (C₁-C₆)carbonyle, H₂NCO, H₂NSO₂, [alkyle en (C₁-C₆)]aminocarbonyle, di[alkyle en (C₁-C₆)]aminocarbonyle, [alkoxy en (C₁-C₃)]alkyle en (C₁-C₃)aminocarbonyle, hétérocyclylcarbonyle, [alkyle en (C₁-C₆)]aminosulfonyle, di[alkyle en (C₁-C₆)]aminosulfonyle, hétérocyclosulfonyle, [alkyle en (C₁-C₆)]carbonylamino, [alkyle en (C₁-C₆)]carbonylaminoalkyle en (C₁-C₆), [alkyle en (C₁-C₆)]sulfonylamino, [alkyle en (C₁-C₆)]sulfonylaminoalkyle en (C₁-C₆), [alkoxy en (C₁-C₆)]carbonylalkoxy en (C₁-C₆), [alkoxy en (C₁-C₆)]alkyle en (C₁-C₆), halo[alkoxy en (C₁-C₆)]alkyle en (C₁-C₆), hydroxyalkoxy en (C₁-C₆), hétéroaryle, oxo, aminoalkyle en (C₁-C₆), [alkyle en (C₁-C₆)]aminoalkyle en (C₁-C₆), di[alkyle en (C₁-C₆)]aminoalkyle en (C₁-C₆), aminoalkoxy en (C₂-C₆), [alkyle en (C₁-C₆)]aminoalkoxy en (C₂-C₆), di[alkyle en (C₁-C₆)]aminoalkoxy en (C₂-C₆) et [alkyle en (C₁-C₆)]carbonyle ;
Cy² est un aryle, hétéroaryle, cycloalkyle ou hétérocyclyle qui, en option, sont chacun substitués par 1 à 4 groupements indépendamment sélectionnés parmi un fluore, chlore, brome, iode, cyano, nitro, amino, hydroxy, carboxy, alkyle en (C₁-C₆), hydroxyalkyle en (C₁-C₆), cycloalkyle en (C₃-C₆), hydroxycycloalkyle en (C₃-C₆), cycloalkyle en (C₄-C₇)alkyle, alkényle en (C₂-C₆), haloalkényle en (C₂-C₆), hydroxyalkényle en (C₂-C₆), alkynyle en (C₂-C₆), [cycloalkyle en (C₃-C₆)]alkynyle en (C₂-C₄), haloalkyle en (C₁-C₆), halocycloalkyle en (C₃-C₆), halocycloalkyle en (C₄-C₇)alkyle, alkoxy en (C₁-C₆), cycloalkoxy en (C₃-C₆), cycloalkyle en (C₄-C₇)alkoxy, haloalkoxy en (C₁-C₆), halocycloalkoxy en (C₃-C₆), halocycloalkyle en (C₄-C₇)alkoxy, alkyle en (C₁-C₆)thio, cycloalkyle en (C₃-C₆)thio, cycloalkyle en (C₄-C₇)alkylthio, haloalkyle en (C₁-C₆)thio, halocycloalkyle en (C₃-C₆)thio, halocycloalkyle en (C₄-C₇)alkylthio, alkane en (C₁-C₆) sulfinyle, cycloalkane en (C₃-C₆)sulfinyle, cycloalkyle en (C₄-C₇)alkanesulfinyle, haloalkane en (C₁-C₆)sulfinyle, halocycloalkane en (C₃-C₆)sulfinyle, halocycloalkyle en (C₄-C₇)alkanesulfinyle, alkane en (C₁-C₆)sulfonyle, cycloalkane en (C₃-C₆)sulfonyle, cycloalkyle en (C₄-C₇)alkanesulfonyle, haloalkane en (C₁-C₆)sulfonyle, halocycloalkane en (C₃-C₆)sulfonyle, halocycloalkyle en (C₄-C₇)alkanesulfonyle, [alkyle en (C₁-C₆)]amino, di[alkyle en (C₁-C₆)]amino, [alkoxy en (C₁-C₆)]alkoxy en (C₁-C₆), halo[alkoxy en (C₁-C₆)]alkoxy en (C₁-C₆), alkoxy en (C₁-C₆)carbonyle, H₂NCO, H₂NSO₂, [alkyle en (C₁-C₆)]aminocarbonyle, di[alkyle en (C₁-C₆)]aminocarbonyle, [alkoxy en (C₁-C₃)][alkyle en (C₁-C₃)]aminocarbonyle, hétérocyclylcarbonyle, [alkyle en (C₁-C₆)]aminosuifonyle, di[alkyle en (C₁-C₆)]aminosulfonyle, hétérocyclosulfonyle, [alkyle en (C₁-C₆)]carbonylamino, [alkyle en (C₁-C₆)]carbonylaminoalkyle en (C₁-C₆), [alkyle en (C₁-C₆)]sulfonylamino, [alkyle en (C₁-C₆)]sulfonylaminoalkyle en (C₁-C₆), [alkoxy en (C₁-C₆)]carbonylalkoxy en (C₁-C₆), [alkoxy en (C₁-C₆)]alkyle en (C₁-C₆), halo[alkoxy en (C₁-C₆)]alkyle en (C₁-C₆), hydroxyalkoxy en (C₁-C₆), hétéroaryle, oxo, aminoalkyle en (C₁-C₆), [alkyle en (C₁-C₆)]aminoalkyle en (C₁-C₆), di[alkyle en (C₁-C₆)]aminoalkyle en (C₁-C₆), aminoalkoxy en (C₂-C₆), [alkyle en (C₁-C₆)]aminoalkoxy en (C₂-C₆), di[alkyle en (C₁-C₆)]aminoalkoxy en (C₂-C₆) et alkyle en (C₁-C₆)carbonyle ; et
R² est un aryle, hétéroaryle, cycloalkyle ou hétérocyclyle qui, en option, sont chacun substitués parjusqu'à 4 groupements indépendamment sélectionnés parmi un fluore, chlore, brome, iode, cyano, nitro, amino, hydroxy, carboxy, alkyle en (C₁-C₆), hydroxyalkyle en (C₁-C₆), cycloalkyle en (C₃-C₆), hydroxycycloalkyle en (C₃-C₆), cycloalkyle en (C₄-C₇)alkyle, alkényle en (C₂-C₆), haloalkényle en (C₂-C₆), hydroxyalkényle en (C₂-C₆), alkynyle en (C₂-C₆), [cycloalkyle en (C₃-C₆)]alkynyle en (C₂-C₄), haloalkyle en (C₁-C₆), halocycloalkyle en (C₃-C₆), halocycloalkyle en (C₄-C₇)alkyle, alkoxy en (C₁-C₆), cycloalkoxy en (C₃-C₆), cycloalkyle en (C₄-C₇)alkoxy, haloalkoxy en (C₁-C₆), halocycloalkoxy en (C₃-C₆), halocycloalkyle en (C₄-C₇)alkoxy, alkyle en (C₁-C₆) thio, cycloalkyle en (C₃-C₆)thio, cycloalkyle en (C₄-C₇)alkylthio, haloalkyle en (C₁-C₆), halocycloalkyle en (C₃-C₆)thio, halocycloalkyle en (C₄-C₇)alkylthio, alkane en (C₁-C₆)sulfinyle, cycloalkane en (C₃-C₆)sulfinyle, cycloalkyle en (C₄-C₇)alkanesulfinyle, haloalkane en (C₁-C₆)sulfinyle, halocycloalkane en (C₃-C₆)sulfinyle, halocycloalkyle en (C₄-C₇)alkanesulfinyle, alkane en (C₁-C₆)sulfonyle, cycloalkane en (C₃-C₆)sulfonyle, cycloalkyle en (C₄-C₇)alkanesulfonyle, haloalkane en (C₁-C₆)sulfonyle, halocycloalkane en (C₃-C₆)sulfonyle, halocycloalkyle en (C₄-C₇)alkanesulfonyle, alkyle en (C₁-C₆)amino, di[alkyle en (C₁-C₆)]amino, [alkoxy en (C₁-C₆)]alkoxy en (C₁-C₆), halo[alkoxy en (C₁-C₆)]alkoxy en (C₁-C₆), alkoxy en (C₁-C₆)carbonyle, H₂NCO, H₂NSO₂, alkyle en (C₁-C₆)aminocarbonyle, di[alkyle en (C₁-C₆)]aminocarbonyle, [alkoxy en (C₁-C₃)][alkyle en (C₁-C₃)]aminocarbonyle, hétérocyclylcarbonyle, alkyle en (C₁-C₆)aminosulfonyle, di[alkyle en (C₁-C₆)]aminosulfonyle, hétérocyclosulfonyle, alkyle en (C₁-C₆)carbonylamino, [alkyle en (C₁-C₆)]carbonylaminoalkyle en (C₁-C₆), alkyle en (C₁-C₆)sulfonylamino, [alkyle en (C₁-C₆)]sulfonylaminoalkyle en (C₁-C₆), [alkoxy en (C₁-C₆)]carbonylalkoxy en (C₁-C₆), [alkoxy en (C₁-C₆)lalkyle en (C₁-C₆), halo[alkoxy en (C₁-C₆)]alkyle en (C₁-C₆), hydroxyalkoxy en (C₁-C₆), hétéroaryle, oxo, aminoalkyle en (C₁-C₆), [alkyle en (C₁-C₆)]aminoalkyle en (C₁-C₆), di[alkyle en (C₁-C₆)]aminoalkyle en (C₁-C₆), aminoalkoxy en (C₂-C₆), [alkyle en (C₁-C₆)]aminoalkoxy en (C₂-C₆), di[alkyle en (C₁-C₆)]aminoalkoxy en (C₂-C₆) et alkyle en (C₁-C₆)carbonyle ;
ou un sel, énantiomère ou diastéréoisomère pharmaceutiquement acceptable de celui-ci.

3. Composé selon la revendication 1 ou 2, qui est un Composé de Formule (la) : 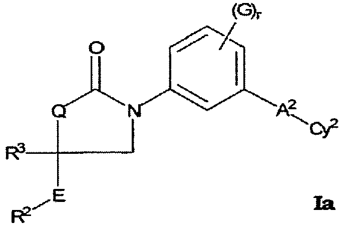 dans lequel :
G est indépendamment sélectionné parmi un fluore, chlore, brome, iode, cyano, nitro, amino, hydroxy, carboxy, alkyle en (C₁-C₆), hydroxyalkyle en (C₁-C₆), cycloalkyle en (C₃-C₆), hydroxycycloalkyle en (C₃-C₆), cycloalkyle en (C₄-C₇)alkyle, alkynyle en (C₂-C₆), [cycloalkyle en (C₃-C₆)]alkynyle en (C₂-C₄), haloalkyle en (C₁-C₆), halocycloalkyle en (C₃-C₆), halocycloalkyle en (C₄-C₇)alkyle, alkoxy en (C₁-C₆), cycloalkoxy en (C₃-C₆), cycloalkyle en (C₄-C₇)alkoxy, haloalkoxy en (C₁-C₆), halocycloalkoxy en (C₃-C₆), halocycloalkyle en (C₄-C₇)alkoxy, alkyle en (C₁-C₆)thio, cycloalkyle en (C₃-C₆)thio, cycloalkyle en (C₄-C₇)alkylthio, haloalkyle en (C₁-C₆)thio, halocycloalkyle en (C₃-C₆)thio, halocycloalkyle en (C₄-C₇)alkylthio, alkane en (C₁-C₆)sulfinyle, cycloalkane en (C₃-C₆)sulfinyle, cycloalkyle en (C₄-C₇)alkanesulfinyle, haloalkane en (C₁-C₆)sulfinyle, halocycloalkane en (C₃-C₆)sulfinyle, halocycloalkyle en (C₄-C₇)alkanesulfinyle, alkane en (C₁-C₆)sulfonyle, cycloalkane en (C₃-C₆)sulfonyle, cycloalkyle en (C₄-C₇)alkanesulfonyle, haloalkane en (C₁-C₆)sulfonyle, halocycloalkane en (C₃-C₆)sulfonyle, halocycloalkyle en (C₄-C₇)alkanesulfonyle, alkyle en (C₁-C₆)amino, di[alkyle en (C₁-C₆)]amino, [alkoxy en (C₁-C₆)]alkoxy en (C₁-C₆), halo[alkoxy en (C₁-C₆)]alkoxy en (C₁-C₆), alkoxy en (C₁-C₆)carbonyle, H₂NCO, H₂NSO₂, alkyle en (C₁-C₆)aminocarbonyle, di[alkyle en (C₁-C₆)]aminocarbonyle, [alkoxy en (C₁-C₃)][alkyle en (C₁-C₃)]aminocarbonyle, hétérocyclylcarbonyle, alkyle en (C₁-C₆)aminosulfonyle, di[alkyle en (C₁-C₆)]aminosulfonyle, hétérocyclosulfonyle, alkyle en (C₁-C₆)carbonylamino, [alkyle en (C₁-C₆)]carbonylaminoalkyle en (C₁-C₆), alkyle en (C₁-C₆)sulfonylamino, [alkyle en (C₁-C₆)]sulfonylaminoalkyle en (C₁-C₆), [alkoxy en (C₁-C₆)]carbonylalkoxy en (C₁-C₆), [alkoxy en (C₁-C₆)]alkyle en (C₁-C₆), halo[alkoxy en (C₁-C₆)]alkyle en (C₁-C₆), hydroxyalkoxy en (C₁-C₆), hétéroaryle, aminoalkyle en (C₁-C₆), [alkyle en (C₁-C₆)]aminoalkyle en (C₁-C₆), di[alkyle en (C₁-C₆)]aminoalkyle en (C₁-C₆), aminoalkoxy en (C₂-C₆), [alkyle en (C₁-C₆)]aminoalkoxy en (C₂-C₆), di[alkyle en (C₁-C₆)]aminoalkoxy en (C₂-C₆) ou alkyle en (C₁-C₆)carbonyle ; et
r prend la valeur 0, 1, 2, 3 or 4;
ou un sel, énantiomère ou diastéréoisomère pharmaceutiquement acceptable de celui-ci.

4. Composé selon la revendication 1 ou 2, qui est un Composé de Formule (Ib) : 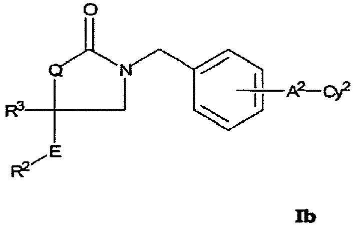 ou un sel, énantiomère ou diastéréoisomère pharmaceutiquement acceptable de celui-ci.

5. Composé selon la revendication 1 ou 2, qui est un Composé de Formule (Ic) : 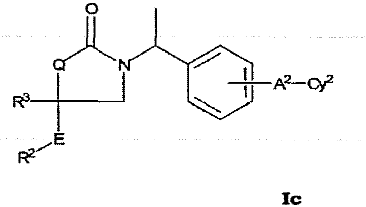 ou un sel, énantiomère ou diastéréoisomère pharmaceutiquement acceptable de celui-ci.

6. Composé selon la revendication 1 ou 2, qui est un Composé de Formule (Id) : 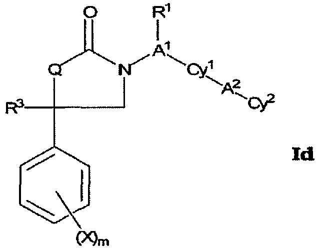 dans lequel :
X est un fluore, chlore, brome, iode, cyano, nitro, amino, hydroxy, carboxy, alkyle en (C₁-C₆), hydroxyalkyle en (C₁-C₆), cycloalkyle en (C₃-C₆), hydroxycycloalkyle en (C₃-C₆), cycloalkyle en (C₄-C₇)alkyle, alkynyle en (C₂-C₆), [cycloalkyle en (C₃-C₆)]alkynyle en (C₂-C₄), haloalkyle en (C₁-C₆), halocycloalkyle en (C₃-C₆), halocycloalkyle en (C₄-C₇)alkyle, alkoxy en (C₁-C₆), cycloalkoxy en (C₃-C₆), cycloalkyle en (C₄-C₇)alkoxy, haloalkoxy en (C₁-C₆), halocycloalkoxy en (C₃-C₆), halocycloalkyle en (C₄-C₇)alkoxy, alkyle en (C₁-C₆)thio, cycloalkyle en (C₃-C₆)thio, cycloalkyle en (C₄-C₇)alkylthio, haloalkyle en (C₁-C₆)thio, halocycloalkyle en (C₃-C₆)thio, halocycloalkyle en (C₄-C₇)alkylthio, alkane en (C₁-C₆) sulfinyle, cycloalkane en (C₃-C₆)sulfinyle, cycloalkyle en (C₄-C₇)alkanesulfinyle, haloalkane en (C₁-C₆)sulfinyle, halocycloalkane en (C₃-C₆)sulfinyle, halocycloalkyle en (C₄-C₇)alkanesulfinyle, alkane en (C₁-C₆)sulfonyle, cycloalkane en (C₃-C₆)sulfonyle, cycloalkyle en (C₄-C₇)alkanesulfonyle, haloalkane en (C₁-C₆)sulfonyle, halocycloalkane en (C₃-C₆)sulfonyle, halocycloalkyle en (C₄-C₇)alkanesulfonyle, alkyle en (C₁-C₆)amino, di[alkyle en (C₁-C₆)]amino, [alkoxy en (C₁-C₆)]alkoxy en (C₁-C₆), halo[alkoxy en (C₁-C₆)]alkoxy en (C₁-C₆), alkoxy en (C₁-C₆) carbonyle, H₂NCO, H₂NSO₂, alkyle en (C₁-C₆)aminocarbonyle, di[alkyle en (C₁-C₆)]aminocarbonyle, [alkoxy en (C₁-C₃)][alkyle en (C₁-C₃)]aminocarbonyle, hétérocyclylcarbonyle, alkyle en (C₁-C₆)aminosulfonyle, di[alkyle en (C₁-C₆)]aminosulfonyle, hétérocyclosulfonyle, alkyle en (C₁-C₆)carbonylamino, [alkyle en (C₁-C₆)]carbonylaminoalkyle en (C₁-C₆), alkyle en (C₁-C₆)sulfonylamino, [alkyle en (C₁-C₆)]sulfonylaminoalkyle en (C₁-C₆), [alkoxy en (C₁-C₆)]carbonylalkoxy en (C₁-C₆), [alkoxy en (C₁-C₆)]alkyle en (C₁-C₆), halo[alkoxy en (C₁-C₆)]alkyle en (C₁-C₆), hydroxyalkoxy en (C₁-C₆), hétéroaryle, aminoalkyle en (C₁-C₆), [alkyle en (C₁-C₆)]aminoalkyle en (C₁-C₆), di[alkyle en (C₁-C₆)]aminoalkyle en (C₁-C₆), aminoalkoxy en (C₂-C₆), [alkyle en (C₁-C₆)]aminoalkoxy en (C₂-C₆), di[alkyle en (C₁-C₆)]aminoalkoxy en (C₂-C₆) et alkyle en (C₁-C₆)carbonyle ; et
m prend la valeur 0, 1, 2, 3 ou 4;
ou un sel, énantiomère ou diastéréoisomère pharmaceutiquement acceptable de celui-ci.

7. Composé selon la revendication 1 ou 2, qui est un Composé de Formule (le) : 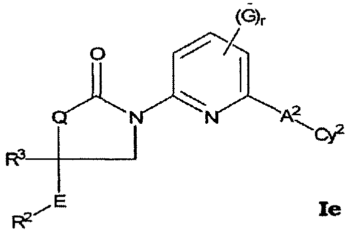 dans lequel :
G est indépendamment sélectionné parmi un fluore, chlore, brome, iode, cyano, nitro, amino, hydroxy, carboxy, alkyle en (C₁-C₆), hydroxyalkyle en (C₁-C₆), cycloalkyle en (C₃-C₆), hydroxycycloalkyle en (C₃-C₆), cycloalkyle en (C₄-C₇)alkyle, alkynyle en (C₂-C₆), [cycloalkyle en (C₃-C₆)]alkynyle en (C₂-C₄), haloalkyle en (C₁-C₆), halocycloalkyle en (C₃-C₆), halocycloalkyle en (C₄-C₇)alkyle, alkoxy en (C₁-C₆), cycloalkoxy en (C₃-C₆), cycloalkyle en (C₄-C₇)alkoxy, haloalkoxy en (C₁-C₆), halocycloalkoxy en (C₃-C₆), halocycloalkyle en (C₄-C₇)alkoxy, alkyle en (C₁-C₆)thio, cycloalkyle en (C₃-C₆)thio, cycloalkyle en (C₄-C₇)alkylthio, haloalkyle en (C₁-C₆)thio, halocycloalkyle en (C₃-C₆)thio, halocycloalkyle en (C₄-C₇)alkylthio, alkane en (C₁-C₆)sulfinyle, cycloalkane en (C₃-C₆)sulfinyle, cycloalkyle en (C₄-C₇)alkanesulfinyle, haloalkane en (C₁-C₆)sulfinyle, halocycloalkane en (C₃-C₆)sulfinyle, halocycloalkyle en (C₄-C₇)alkanesulfinyle, alkane en (C₁-C₆)sulfonyle, cycloalkane en (C₃-C₆)sulfonyle, cycloalkyle en (C₄-C₇)alkanesulfonyle, haloalkane en (C₁-C₆)sulfonyle, halocycloalkane en (C₃-C₆)sulfonyle, halocycloalkyle en (C₄-C₇)alkanesulfonyle, alkyle en (C₁-C₆)amino, di[alkyle en (C₁-C₆)]amino, [alkoxy en (C₁-C₆)]alkoxy en (C₁-C₆), halo[alkoxy en (C₁-C₆)]alkoxy en (C₁-C₆), alkoxy en (C₁-C₆) carbonyle, H₂NCO, H₂NSO₂, alkyle en (C₁-C₆)aminocarbonyle, di[alkyle en (C₁-C₆)]aminocarbonyle, [alkoxy en (C₁-C₃)][alkyle en (C₁-C₃)]aminocarbonyle, hétérocyclylcarbonyle, alkyle en (C₁-C₆)aminosulfonyle, di[alkyle en (C₁-C₆)]aminosulfonyle, hétérocyclosulfonyle, alkyle en (C₁-C₆)carbonylamino, [alkyle en (C₁-C₆)]carbonylaminoalkyle en (C₁-C₆), alkyle en (C₁-C₆)sulfonylamino, [alkyle en (C₁-C₆)]sulfonylaminoalkyle en (C₁-C₆), [alkoxy en (C₁-C₆)]carbonylalkoxy en (C₁-C₆), [alkoxy en (C₁-C₆)]alkyle en (C₁-C₆), halo[alkoxy en (C₁-C₆)]alkyle en (C₁-C₆), hydroxyalkoxy en (C₁-C₆), hétéroaryle, oxo, aminoalkyle en (C₁-C₆), [alkyle en (C₁-C₆)]aminoalkyle en (C₁-C₆), di[alkyle en (C₁-C₆)]aminoalkyle en (C₁-C₆), aminoalkoxy en (C₂-C₆), [alkyle en (C₁-C₆)]aminoalkoxy en (C₂-C₆), di[alkyle en (C₁-C₆)]aminoalkoxy en (C₂-C₆) et alkyle en (C₁-C₆)carbonyle , et
r prend la valeur 0,1, 2, 3 ou 4 ;
ou un sel, énantiomère ou diastéréoisomère pharmaceutiquement acceptable de celui-ci.

8. Composé selon la revendication 1 ou 2, qui est un Composé de Formule (If) : 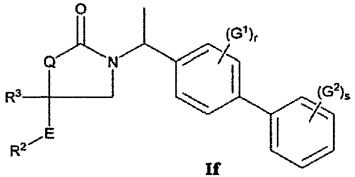 dans lequel :
G¹ et G² sont chacun indépendamment sélectionnés parmi un fluore, chlore, brome, iode, cyano, nitro, amino, hydroxy, carboxy, alkyle en (C₁-C₆), hydroxyalkyle en (C₁-C₆), cycloalkyle en (C₃-C₆), hydroxycycloalkyle en (C₃-C₆), cycloalkyle en (C₄-C₇)alkyle, alkynyle en (C₂-C₆), [cycloalkyle en (C₃-C₆)]alkynyle en (C₂-C₄), haloalkyle en (C₁-C₆), halocycloalkyle en (C₃-C₆), halocycloalkyle en (C₄-C₇)alkyle, alkoxy en (C₁-C₆), cycloalkoxy en (C₃-C₆), cycloalkyle en (C₄-C₇)alkoxy, haloalkoxy en (C₁-C₆), halocycloalkoxy en (C₃-C₆), halocycloalkyle en (C₄-C₇)alkoxy, alkyle en (C₁-C₆)thio, cycloalkyle en (C₃-C₆)thio, cycloalkyle en (C₄-C₇)alkylthio, haloalkyle en (C₁-C₆)thio, halocycloalkyle en (C₃-C₆)thio, halocycloalkyle en (C₄-C₇)alkylthio, alkane en (C₁-C₆)sulfinyle, cycloalkane en (C₃-C₆)sulfinyle, cycloalkyle en (C₄-C₇)alkanesulfinyle, haloalkane en (C₁-C₆)sulfinyle, halocycloalkane en (C₃-C₆)sulfinyle, halocycloalkyle en (C₄-C₇)alkanesulfinyle, alkane en (C₁-C₆)sulfonyle, cycloalkane en (C₃-C₆)sulfonyle, cycloalkyle en (C₄-C₇)alkanesulfonyle, haloalkane en (C₁-C₆)sulfonyle, halocycloalkane en (C₃-C₆)sulfonyle, halocycloalkyle en (C₄-C₇)alkanesulfonyle, alkyle en (C₁-C₆)amino, di[alkyle en (C₁-C₆)]amino, [alkoxy en (C₁-C₆)]alkoxy en (C₁-C₆), halo[alkoxy en (C₁-C₆)]alkoxy en (C₁-C₆), alkoxy en (C₁-C₆)carbonyle, H₂NCO, H₂NSO₂, alkyle en (C₁-C₆)aminocarbonyle, di[alkyle en (C₁-C₆)]aminocarbonyle, [alkoxy en (C₁-C₃)][alkyle en (C₁-C₃)]aminocarbonyle, hétérocyclylcarbonyle, alkyle en (C₁-C₆)aminosulfonyle, di[alkyle en (C₁-C₆)]aminosulfonyle, hétérocyclosulfonyle, alkyle en (C₁-C₆)carbonylamino, [alkyle en (C₁-C₆)]carbonylaminoalkyle en (C₁-C₆), alkyle en (C₁-C₆)sulfonylamino, [alkyle en (C₁-C₆)]sulfonylaminoalkyle en (C₁-C₆), [alkoxy en (C₁-C₆)]carbonylalkoxy en (C₁-C₆), [alkoxy en (C₁-C₆)]alkyle en (C₁-C₆), halo[alkoxy en (C₁-C₆)]alkyle en (C₁-C₆), hydroxyalkoxy en (C₁-C₆), hétéroaryle, aminoalkyle en (C₁-C₆), [alkyle en (C₁-C₆)]aminoalkyle en (C₁-C₆), di[alkyle en (C₁-C₆)]aminoalkyle en (C1-C₆), aminoalkoxy en (C₂-C₆), [alkyle en (C₁-C₆)]aminoalkoxy en (C₂-C₆), di[alkyle en (C₁-C₆)]aminoalkoxy en (C₂-C₆) ou alkyle en (C₁-C₆)carbonyle ;
R⁵ est un H, alkyle en (C₁-C₆), haloalkyle en (C₁-C₆) ou hydroxyalkyle en (C₁-C₆) ; et
r et s prennent indépendamment la valeur 0, 1, 2, 3 ou 4 ;
ou un sel, énantiomère ou diastéréoisomère pharmaceutiquement acceptable de celui-ci.

9. Composé selon l'une quelconque des revendications 1 à 8, dans lequel Cy² est en option substitué par un aryle ou un hétéroaryle.

10. Composé selon l'une quelconque des revendications 1 à 8, dans lequel Cy² est en option substitué par un cycloalkyle ou un hétérocyclyle.

11. Composé selon la revendication 10, dans lequel Cy² est un 1,2-dihydro-2-oxopyridyle ou un 1,2-dihydro-1-méthyl-2-oxopyridyle.

12. Composé selon la revendication 10, dans lequel Cy² est un 1,2-dihydro-2-oxopyridyle ou un 1,2-dihydro-1-méthyl-2-oxopyridyle en option substitué.

13. Composition pharmaceutique comprenant : i) un véhicule ou un diluant pharmaceutiquement acceptable ; et ii) le composé de l'une quelconque des revendications 1-12 ou un sel, énantiomère ou diastéréoisomère pharmaceutiquement acceptable de celui-ci.

14. Composé selon l'une quelconque des revendications 1 à 12 pour une utilisation dans le traitement d'une maladie ou d'une affection associée à l'activité ou à l'expression de 11β-HSD1 chez un mammifère.
